# EUROPEAN PATENT SPECIFICATION

(11) **EP 2 550 367 B1**
(45) Date of publication and mention of the grant of the patent: **22.03.2017**
(21) Application number: 11711830.7
(22) Date of filing: 24.03.2011
(51) Int. Cl.: C12Q 1/68

(54) **GENES AND GENES COMBINATIONS PREDICTIVE OF EARLY RESPONSE OR NON RESPONSE OF SUBJECTS SUFFERING FROM INFLAMMATORY DISEASE TO CYTOKINE TARGETING DRUGS (CYTD)**
GENE UND GENKOMBINATIONEN DIE AUFSCHLUSS GEBEN ÜBER RESPONDER UND NON-RESPONDER / PATIENTEN, DIE AN ENTZÜNDLICHEN KRANKHEITEN LEIDEN UND MIT CYTOKIN-ANTAGONISTEN BEHANDELT WERDEN
GÈNES ET COMBINAISONS DE GÈNES PRÉDICTIFS D'UNE RÉPONSE PRÉCOCE OU D'UNE NON-RÉPONSE DE SUJETS SOUFFRANT D'UNE MALADIE INFLAMMATOIRE À DES MÉDICAMENTS CIBLANT LES CYTOKINES (CYTD)

(30) Priority: 19.01.2011 US 457158 P; 24.03.2010 US 282732 P
(43) Date of publication of application: 30.01.2013
(60) Divisional application: 17154875.3
(73) Proprietor: TC LAND Expression, 44200 Nantes (FR)
(72) Inventor: CERVINO, Alessandra, F-44100 Nantes (FR); POPA-NITA, Oana Ruxandra, F-44200 Nantes (FR); BRAUD, Christophe, F-44980 Sainte-Luce-sur-Loire (FR)
(74) Representative: Regimbeau
(86) International application number: PCT/EP2011/054569
(87) International publication number: WO 2011/117366

(56) References cited:
- EP-A1- 1 857 559
- WO-A1-2006/063856
- WO-A1-2010/034864
- WO-A2-2007/038501
- WO-A2-2008/063414
- WO-A2-2008/087647
- WO-A2-2008/132176
- WO-A2-2008/150491
- LEQUERRE T ET AL: "GENE EXPRESSION PROFILING IN PBMCS AS A TOOL FOR PREDICTION OF INFLIXIMAB RESPONSIVENESS IN RHEUMATOID ARTHRITIS", ARTHRITIS & RHEUMATISM, JOHN WILEY & SONS, INC, US, 1 January 2004 (2004-01-01), XP008067698, ISSN: 0004-3591
- LINDBERG JOHAN ET AL: "Effect of infliximab on mRNA expression profiles in synovial tissue of rheumatoid arthritis patients", ARTHRITIS RESEARCH AND THERAPY, BIOMED CENTRAL, LONDON, GB, vol. 8, no. 6, 29 November 2006 (2006-11-29), page R179, XP021026935, ISSN: 1478-6354, DOI: DOI:10.1186/AR2090
- LEQUERRÉ THIERRY ET AL: "Gene profiling in white blood cells predicts infliximab responsiveness in rheumatoid arthritis", ARTHRITIS RESEARCH AND THERAPY, BIOMED CENTRAL, LONDON, GB, vol. 8, no. 4, 3 July 2006 (2006-07-03), page R105, XP021020585, ISSN: 1478-6354, DOI: DOI:10.1186/AR1924
- HAEUPL T ET AL: "GENE EXPRESSION PROFILING OF RHEUMATOID ARTHRITIS SYNOVIAL CELLS TREATED WITH ANTIRHEUMATIC DRUGS", JOURNAL OF BIOMOLECULAR SCREENING, LARCHMONT, NY, US, vol. 12, no. 3, 1 January 2007 (2007-01-01), pages 328-340, XP009083067, ISSN: 1087-0571, DOI: DOI:10.1177/1087057107299261
- KRUITHOF ELLI ET AL: "Identification of synovial biomarkers of response to experimental treatment in early-phase clinical trials in spondylarthritis", ARTHRITIS & RHEUMATISM, vol. 54, no. 6, June 2006 (2006-06), pages 1795-1804, XP002662897, ISSN: 0004-3591

### SEQUENCE LISTING

<110> TC LAND EXPRESSION
   Cervino, Alessandra
   Popa-Nita, Oana Ruxandra
   Braud, Christophe
<120> Genes and genes combinations predictive of early response or non
   reponse of subjects suffering from inflammatory disease to
   cytokine targeting drugs (CyTD)
<130> D28552
<150> US61/282,732
   <151> 2010-03-24
<150> US61/457,158
   <151> 2010-03-24
<160> 106
<170> PatentIn version 3.5
<210> 1
   <211> 4045
   <212> DNA
   <213> Homo sapiens
<400> 1
   gcagccagag ctcagcaggg ccctggagag atggccacgg tcccagcacc ggggaggact      60
   ggagagcgcg cgctgccacc gccccatgtc tcagccaggg cttccttcct cggctccacc     120
   ctgtggatgt aatggcggcc cctgctctgt cctggcgtct gcccctcctc atcctcctcc     180
   tgcccctggc tacctcttgg gcatctgcag cggtgaatgg cacttcccag ttcacatgct     240
   tctacaactc gagagccaac atctcctgtg tctggagcca agatggggct ctgcaggaca     300
   cttcctgcca agtccatgcc tggccggaca gacggcggtg gaaccaaacc tgtgagctgc     360
   tccccgtgag tcaagcatcc tgggcctgca acctgatcct cggagcccca gattctcaga     420
   aactgaccac agttgacatc gtcaccctga gggtgctgtg ccgtgagggg gtgcgatgga     480
   gggtgatggc catccaggac ttcaagccct ttgagaacct tcgcctgatg gcccccatct     540
   ccctccaagt tgtccacgtg gagacccaca gatgcaacat aagctgggaa atctcccaag     600
   cctcccacta ctttgaaaga cacctggagt tcgaggcccg gacgctgtcc ccaggccaca     660
   cctgggagga ggcccccctg ctgactctca agcagaagca ggaatggatc tgcctggaga     720
   cgctcacccc agacacccag tatgagtttc aggtgcgggt caagcctctg caaggcgagt     780
   tcacgacctg gagcccctgg agccagcccc tggccttcag gacaaagcct gcagcccttg     840
   ggaaggacac cattccgtgg ctcggccacc tcctcgtggg cctcagcggg gcttttggct     900
   tcatcatctt agtgtacttg ctgatcaact gcaggaacac cgggccatgg ctgaagaagg     960
   tcctgaagtg taacacccca gacccctcga agttcttttc ccagctgagc tcagagcatg    1020
   gaggagacgt ccagaagtgg ctctcttcgc ccttcccctc atcgtccttc agccctggcg    1080
   gcctggcacc tgagatctcg ccactagaag tgctggagag ggacaaggtg acgcagctgc 1140
   tcctgcagca ggacaaggtg cctgagcccg catccttaag cagcaaccac tcgctgacca 1200
   gctgcttcac caaccagggt tacttcttct tccacctccc ggatgccttg gagatagagg 1260
   cctgccaggt gtactttact tacgacccct actcagagga agaccctgat gagggtgtgg 1320
   ccggggcacc cacagggtct tccccccaac ccctgcagcc tctgtcaggg gaggacgacg 1380
   cctactgcac cttcccctcc agggatgacc tgctgctctt ctcccccagt ctcctcggtg 1440
   gccccagccc cccaagcact gcccctgggg gcagtggggc cggtgaagag aggatgcccc 1500
   cttctttgca agaaagagtc cccagagact gggaccccca gcccctgggg cctcccaccc 1560
   caggagtccc agacctggtg gattttcagc caccccctga gctggtgctg cgagaggctg 1620
   gggaggaggt ccctgacgct ggccccaggg agggagtcag tttcccctgg tccaggcctc 1680
   ctgggcaggg ggagttcagg gcccttaatg ctcgcctgcc cctgaacact gatgcctact 1740
   tgtccctcca agaactccag ggtcaggacc caactcactt ggtgtagaca gatggccagg 1800
   gtgggaggca ggcagctgcc tgctctgcgc cgagcctcag aaggaccctg ttgagggtcc 1860
   tcagtccact gctgaggaca ctcagtgtcc agttgcagct ggacttctcc acccggatgg 1920
   cccccaccca gtcctgcaca cttggtccat ccatttccaa acctccactg ctgctcccgg 1980
   gtcctgctgc ccgagccagg aactgtgtgt gttgcagggg ggcagtaact ccccaactcc 2040
   ctcgttaatc acaggatccc acgaatttag gctcagaagc atcgctcctc tccagccctg 2100
   cagctattca ccaatatcag tcctcgcggc tctccagggc tccctgccct gacctcttcc 2160
   ctgggttttc tgccccagcc tcctccttcc ctcccctccc cgtccacagg gcagcctgag 2220
   cgtgctttcc aaaacccaaa tatggccacg ctccccctcg gttcaaaacc ttgcacaggt 2280
   cccactgccc tcagccccac ttctcagcct ggtacttgta cctccggtgt cgtgtgggga 2340
   catccccttc tgcaatcctc cctaccgtcc tcctgagcca ctcagagctc cctcacaccc 2400
   cctctgttgc acatgctatt ccctggggct gctgtgcgct ccccctcatc taggtgacaa 2460
   acttccctga ctcttcaagt gccggttttg cttctcctgg agggaagcac tgcctccctt 2520
   aatctgccag aaacttctag cgtcagtgct ggagggagaa gctgtcaggg acccagggcg 2580
   cctggagaaa gaggccctgt tactattcct ttgggatctc tgaggcctca gagtgcttgg 2640
   ctgctgtatc tttaatgctg gggcccaagt aagggcacag atccccccac aaagtggatg 2700
   cctgctgcat cttcccacag tggcttcaca gacccacaag agaagctgat ggggagtaaa 2760
   ccctggagtc cgaggcccag gcagcagccc cgcctagtgg tgggccctga tgctgccagg 2820
   cctgggacct cccactgccc cctccactgg aggggtctcc tctgcagctc agggactggc 2880
   acactggcct ccagaagggc agctccacag ggcagggcct cattattttt cactgcccca 2940
   gacacagtgc ccaacacccc gtcgtatacc ctggatgaac gaattaatta cctggcacca 3000
   cctcgtctgg gctccctgcg cctgacattc acacagagag gcagagtccc gtgcccatta 3060
   ggtctggcat gccccctcct gcaaggggct caacccccta ccccgacccc tccacgtatc 3120
   tttcctaggc agatcacgtt gcaatggctc aaacaacatt ccaccccagc aggacagtga 3180
   ccccagtccc agctaactct gacctgggag ccctcaggca cctgcactta caggccttgc 3240
   tcacagctga ttgggcacct gaccacacgc ccccacaggc tctgaccagc agcctatgag 3300
   ggggtttggc accaagctct gtccaatcag gtaggctggg cctgaactag ccaatcagat 3360
   caactctgtc ttgggcgttt gaactcaggg agggaggccc ttgggagcag gtgcttgtgg 3420
   acaaggctcc acaagcgttg agccttggaa aggtagacaa gcgttgagcc actaagcaga 3480
   ggaccttggg ttcccaatac aaaaatacct actgctgaga gggctgctga ccatttggtc 3540
   aggattcctg ttgcctttat atccaaaata aactcccctt tcttgaggtt gtctgagtct 3600
   tgggtctatg ccttgaaaaa agctgaatta ttggacagtc tcacctcctg ccatagggtc 3660
   ctgaatgttt cagaccacaa ggggctccac acctttgctg tgtgttctgg ggcaacctac 3720
   taatcctctc tgcaagtcgg tctccttatc cccccaaatg gaaattgtat ttgccttctc 3780
   cactttggga ggctcccact tcttgggagg gttacatttt ttaagtctta atcatttgtg 3840
   acatatgtat ctatacatcc gtatctttta atgatccgtg tgtaccatct ttgtgattat 3900
   ttccttaata ttttttcttt aagtcagttc attttcgttg aaatacattt atttaaagaa 3960
   aaatctttgt tactctgtaa atgaaaaaac ccattttcgc tataaataaa aggtaactgt 4020
   acaaaataag tacaatgcaa caaaa 4045
<210> 2
   <211> 586
   <212> DNA
   <213> Homo sapiens
<400> 2
   aaacactctg tgtggctcct cggctttgac agagtgcaag acgatgactt gcaaaatgtc 60
   gcagctggaa cgcaacatag agaccatcat caacaccttc caccaatact ctgtgaagct 120
   ggggcaccca gacaccctga accaggggga attcaaagag ctggtgcgaa aagatctgca 180
   aaattttctc aagaaggaga ataagaatga aaaggtcata gaacacatca tggaggacct 240
   ggacacaaat gcagacaagc agctgagctt cgaggagttc atcatgctga tggcgaggct 300
   aacctgggcc tcccacgaga agatgcacga gggtgacgag ggccctggcc accaccataa 360
   gccaggcctc ggggagggca ccccctaaga ccacagtggc caagatcaca gtggccacgg 420
   ccacggccac agtcatggtg gccacggcca cagccactaa tcaggaggcc aggccaccct 480
   gcctctaccc aaccagggcc ccggggcctg ttatgtcaaa ctgtcttggc tgtggggcta 540
   ggggctgggg ccaaataaag tctcttcctc caagtcaaaa aaaaaa 586
<210> 3
   <211> 1593
   <212> DNA
   <213> Homo sapiens
<400> 3
   actttcaatt ctagatcagg aactgaggac atatctaaat tttctagttt tatagaaggc        60
   ttttatccac aagaatcaag atcttccctc tctgagcagg aatcctttgt gcattgaaga        120
   ctttagattc ctctctgcgg tagacgtgca cttataagta tttgatgggg tggattcgtg        180
   gtcggaggtc tcgacacagc tgggagatga gtgaatttca taattataac ttggatctga        240
   agaagagtga tttttcaaca cgatggcaaa agcaaagatg tccagtagtc aaaagcaaat        300
   gtagagaaaa tgcatctcca ttttttttct gctgcttcat cgctgtagcc atgggaatcc        360
   gtttcattat tatggtaaca atatggagtg ctgtattcct aaactcatta ttcaaccaag        420
   aagttcaaat tcccttgacc gaaagttact gtggcccatg tcctaaaaac tggatatgtt        480
   acaaaaataa ctgctaccaa ttttttgatg agagtaaaaa ctggtatgag agccaggctt       540
   cttgtatgtc tcaaaatgcc agccttctga aagtatacag caaagaggac caggatttac        600
   ttaaactggt gaagtcatat cattggatgg gactagtaca cattccaaca aatggatctt        660
   ggcagtggga agatggctcc attctctcac ccaacctact aacaataatt gaaatgcaga        720
   agggagactg tgcactctat gcctcgagct ttaaaggcta tatagaaaac tgttcaactc        780
   caaatacgta catctgcatg caaaggactg tgtaaagatg atcaaccatc tcaataaaag        840
   ccaggaacag agaagagatt acaccagcgg taacactgcc aactgagact aaaggaaaca        900
   aacaaaaaca ggacaaaatg accaaagact gtcagatttc ttagactcca caggaccaaa        960
   ccatagaaca atttcactgc aaacatgcat gattctccaa gacaaaagaa gagagatcct       1020
   aaaggcaatt cagatatccc caaggctgcc tctcccacca caagcccaga gtggatgggc       1080
   tgggggaggg gtgctgtttt aatttctaaa ggtaggacca acacccaggg gatcagtgaa       1140
   ggaagagaag gccagcagat cactgagagt gcaaccccac cctccacagg aaattgcctc       1200
   atgggcaggg ccacagcaga gagacacagc atgggcagtg ccttccctgc ctgtgggggt       1260
   catgctgcca cttttaatgg gtcctccacc caacggggtc agggaggtgg tgctgcccca       1320
   gtgggccatg attatcttaa aggcattatt ctccagcctt aagtaagatc ttaggacgtt       1380
   tcctttgcta tgatttgtac ttgcttgagt cccatgactg tttctcttcc tctctttctt       1440
   ccttttggaa tagtaatatc catcctatgt ttgtcccact attgtatttt ggaagcacat       1500
   aacttgtttg gtttcacagg ttcacagtta agaaggaatt ttgcctctga ataaatagaa       1560
   tcttgagtct catgcaaaaa aaaaaaaaaa aaa                                    1593
<210> 4
   <211> 2168
   <212> DNA
   <213> Homo sapiens
<400> 4
   ggagttagcc tcgctcaggg cgcggctaag gcgcccagat ggcctgcggg cgccaccacg 60
   tccctggtcc cagctcggga gcacatcaga ggcttagagg cgagtgggaa gggactcaga 120
   cagtgcagga cgagaaacgc ccgcggcacc aaagcccctc agagcgtcgc ccccgcctct 180
   agttctagaa agtcagtttc ccggcactgg caccccggaa cctcaggggc tgccgagctg 240
   ggggggcgct caagctgcga ggatccgggc tgcccgcgag acgaggagcg ggcgcccagg 300
   atggggtgca tgaagtccaa gttcctccag gtcggaggca atacattctc aaaaactgaa 360
   accagcgcca gcccacactg tcctgtgtac gtgccggatc ccacatccac catcaagccg 420
   gggcctaata gccacaacag caacacacca ggaatcaggg aggcaggctc tgaggacatc 480
   atcgtggttg ccctgtatga ttacgaggcc attcaccacg aagacctcag cttccagaag 540
   ggggaccaga tggtggtcct agaggaatcc ggggagtggt ggaaggctcg atccctggcc 600
   acccggaagg agggctacat cccaagcaac tatgtcgccc gcgttgactc tctggagaca 660
   gaggagtggt ttttcaaggg catcagccgg aaggacgcag agcgccaact gctggctccc 720
   ggcaacatgc tgggctcctt catgatccgg gatagcgaga ccactaaagg aagctactct 780
   ttgtccgtgc gagactacga ccctcggcag ggagataccg tgaaacatta caagatccgg 840
   accctggaca acgggggctt ctacatatcc ccccgaagca ccttcagcac tctgcaggag 900
   ctggtggacc actacaagaa ggggaacgac gggctctgcc agaaactgtc ggtgccctgc 960
   atgtcttcca agccccagaa gccttgggag aaagatgcct gggagatccc tcgggaatcc 1020
   ctcaagctgg agaagaaact tggagctggg cagtttgggg aagtctggat ggccacctac 1080
   aacaagcaca ccaaggtggc agtgaagacg atgaagccag ggagcatgtc ggtggaggcc 1140
   ttcctggcag aggccaacgt gatgaaaact ctgcagcatg acaagctggt caaacttcat 1200
   gcggtggtca ccaaggagcc catctacatc atcacggagt tcatggccaa aggaagcttg 1260
   ctggactttc tgaaaagtga tgagggcagc aagcagccat tgccaaaact cattgacttc 1320
   tcagcccaga ttgcagaagg catggccttc atcgagcaga ggaactacat ccaccgagac 1380
   ctccgagctg ccaacatctt ggtctctgca tccctggtgt gtaagattgc tgactttggc 1440
   ctggcccggg tcattgagga caacgagtac acggctcggg aaggggccaa gttccccatc 1500
   aagtggacag ctcctgaagc catcaacttt ggctccttca ccatcaagtc agacgtctgg 1560
   tcctttggta tcctgctgat ggagatcgtc acctacggcc ggatccctta cccagggatg 1620
   tcaaaccctg aagtgatccg agctctggag cgtggatacc ggatgcctcg cccagagaac 1680
   tgcccagagg agctctacaa catcatgatg cgctgctgga aaaaccgtcc ggaggagcgg 1740
   ccgaccttcg aatacatcca gagtgtgctg gatgacttct acacggccac agagagccag 1800
   taccaacagc agccatgata gggaggacca gggcagggcc agggggtgcc caggtggtgg 1860
   ctgcaaggtg gctccagcac catccgccag ggcccacacc cccttcctac tcccagacac 1920
   ccaccctcgc ttcagccaca gtttcctcat ctgtccagtg ggtaggttgg actggaaaat 1980
   ctctttttga ctcttgcaat ccacaatctg acattctcag gaagccccca agttgatatt 2040
   tctatttcct ggaatggttg gattttagtt acagctgtga tttggaaggg aaactttcaa 2100
   aatagtgaaa tgaatattta aataaaagat ataaatgcca aagtctttac caaaaaaaaa 2160
   aaaaaaaa 2168
<210> 5
   <211> 751
   <212> DNA
   <213> Homo sapiens
<400> 5
   gtatctgtgg taaacccagt gacacggggg agatgacata caaaaagggc aggacctgag 60
   aaagattaag ctgcaggctc cctgcccata aaacagggtg tgaaaggcat ctcagcggct 120
   gccccaccat ggctacctgg gccctcctgc tccttgcagc catgctcctg ggcaacccag 180
   gtctggtctt ctctcgtctg agccctgagt actacgacct ggcaagagcc cacctgcgtg 240
   atgaggagaa atcctgcccg tgcctggccc aggagggccc ccagggtgac ctgttgacca 300
   aaacacagga gctgggccgt gactacagga cctgtctgac gatagtccaa aaactgaaga 360
   agatggtgga taagcccacc cagagaagtg tttccaatgc tgcgacccgg gtgtgtagga 420
   cggggaggtc acgatggcgc gacgtctgca gaaatttcat gaggaggtat cagtctagag 480
   ttacccaggg cctcgtggcc ggagaaactg cccagcagat ctgtgaggac ctcaggttgt 540
   gtataccttc tacaggtccc ctctgagccc tctcaccttg tcctgtggaa gaagcacagg 600
   ctcctgtcct cagatcccgg gaacctcagc aacctctgcc ggctcctcgc ttcctcgatc 660
   cagaatccac tctccagtct ccctcccctg actccctctg ctgtcctccc ctctcacgag 720
   aataaagtgt caagcaagat tttaaaaaaa a 751
<210> 6
   <211> 1517
   <212> DNA
   <213> Homo sapiens
<400> 6
   aaagtgcggg gtcggccggg tgctgggccg aggccgaggc cggggcggga tccagagcgg 60
   gagccggcgc gggatctggg actcggagcg ggatccggag cgggacccag gagccggcgc 120
   ggggccatgg cgaggcgcgg gccagggtgg cggccgcttc tgctgctcgt gctgctggcg 180
   ggcgcggcgc agggcggcct ctacttccgc cggggacaga cctgctaccg gcctctgcgg 240
   ggggacgggc tggctccgct ggggcgcagc acataccccc ggcctcatga gtacctgtcc 300
   ccagcggatc tgcccaagag ctgggactgg cgcaatgtgg atggtgtcaa ctatgccagc 360
   atcacccgga accagcacat cccccaatac tgcggctcct gctgggccca cgccagcacc 420
   agcgctatgg cggatcggat caacatcaag aggaagggag cgtggccctc caccctcctg 480
   tccgtgcaga acgtcatcga ctgcggtaac gctggctcct gtgaaggggg taatgacctg 540
   tccgtgtggg actacgccca ccagcacggc atccctgacg agacctgcaa caactaccag 600
   gccaaggacc aggagtgtga caagtttaac caatgtggga catgcaatga attcaaagag 660
   tgccacgcca tccggaacta caccctctgg agggtgggag actacggctc cctctctggg 720
   agggagaaga tgatggcaga aatctacgca aatggtccca tcagctgtgg aataatggca 780
   acagaaagac tggctaacta caccggaggc atctatgccg aataccagga caccacatat 840
   ataaaccatg tcgtttctgt ggctgggtgg ggcatcagtg atgggactga gtactggatt 900
   gtccggaatt catggggtga accatggggc gagagaggct ggctgaggat cgtgaccagc 960
   acctataagg atgggaaggg cgccagatac aaccttgcca tcgaggagca ctgtacattt 1020
   ggggacccca tcgtttaagg ccatgtcact agaagcgcag tttaagaaaa ggcatggtga 1080
   cccatgacca gaggggatcc tatggttatg tgtgccaggc tggctggcag gaactggggt 1140
   ggctatcaat attggatggc gaggacagcg tggcactggc tgcgagtgtt cctgagagtt 1200
   gaaagtggga tgacttatga cacttgcaca gcatggctct gcctcacaat gatgcagtca 1260
   gccacctggt gaagaagtga cctgcgacac aggaaacgat gggacctcag tcttcttcag 1320
   cagaggactt gatattttgt atttggcaac tgtgggcaat aatatggcat ttaagaggtg 1380
   aaagagttca gacttatcac cattcttatg tcactttaga atcaagggtg ggggagggag 1440
   ggagggagtt ggcagtttca aatcgcccaa gtgatgaata aagtatctgg ctctgcacga 1500
   aaaaaaaaaa aaaaaaa 1517
<210> 7
   <211> 3227
   <212> DNA
   <213> Homo sapiens
<400> 7
   attagagcgt gtctgggagc aggttaaact ttctcctagg tggtgatgtg cctccaggtg 60
   agcgcacagg ctttgcagaa accccagcat gacaagcaag taggaaggtg acattgttgt 120
   ttgagaaaag ccagtgaccc ggttgggacg gagacacgat ggggagccgg tctccaacag 180
   catgcccttc ctgaaggcca acgaggtgac ggacagcgcg tacatgggct ccgagagcac 240
   ctacagtgag tgtgagacct tcacggacga ggacaccagc accctggtgc accctgagct 300
   gcaacctgaa ggggacgcag acagtgccgg cggctcggcc gtgccctctg agtgcctgga       360
   cgccatggag gagcccgacc atggtgccct gctgctgctc ccaggcaggc ctcaccccca       420
   tggccagtct gtcatcacgg tgatcggggg cgaggagcac tttgaggact acggtgaagg 480
   cagtgaggcg gagctgtccc cagagaccct atgcaacggg cagctgggct gcagtgaccc 540
   cgctttcctc acgcccagtc cgacaaagcg gctctccagc aagaaggtgg caaggtacct 600
   gcaccagtca ggggccctga ccatggaggc cctggaggac ccttcccccg agctcatgga 660
   gggcccagag gaggacattg ctgacaaggt tgtcttcctg gaaaggcgtg tgctggagct 720
   ggaaaaggac acggcagcca ccggtgagca acacagccgc ctgaggcagg agaacctgca 780
   gctggtgcac agagcaaacg ccctggagga gcagctgaag gagcaggagc tgagagcctg 840
   cgagatggtc ctggaagaga cccggcgtca gaaggagctc ctgtgcaaga tggagaggga 900
   gaagagcatt gagatcgaga acctgcagac caggctacag caactggacg aggagaacag 960
   tgaactccgg tcctgcacgc cctgtctgaa ggccaacatt gagcgtctgg aggaggagaa 1020
   gcagaagctg ttggatgaga tagagtcgct gacgctgcgg ctcagtgaag agcaggagaa 1080
   caagaggaga atgggggaca ggctgagtca cgagaggcac cagttccaga gggacaagga 1140
   ggccacccag gagctgatcg aggacctccg aaagcagctg gagcacctgc agctcctcaa 1200
   gctggaggcc gagcagcggc ggggccgcag cagcagcatg ggcctgcagg agtaccacag 1260
   ccgcgcccgg gagagcgagc tggagcagga ggtccgcagg ctgaagcagg acaaccgcaa 1320
   cctgaaggag cagaacgagg agctgaacgg gcagatcatt accctcagca tccagggcgc 1380
   caagagcctc ttctccacag ccttctctga gtccctggct gcagagatca gctccgtctc 1440
   ccgagatgag ctcatggagg cgattcagaa gcaggaggag atcaacttcc gcctgcagga 1500
   ctacatcgac aggatcatcg tggccatcat ggagaccaac ccgtccatcc tggaggtcaa 1560
   gtagaggcag gaaggtccag cctgagctgg attcgggact ccaacaccct ggagtggttc 1620
   cgtcagacca tgaggagcca agaccagcag gtcccacagc cgacagtgcc cagagcatgc 1680
   agggaaccct cgtgcagctg agctggggcc gccaaagacc ggggctgcca aaggggcaga 1740
   gggtggtgga gaggagaggg agaaagggaa gtcccagggc ccggggtcca cagaggatga 1800
   gggttgtggc agggccgtcc atcagcgctg accttccggg ggcccagagc ttcccagccc 1860
   tgagtcaagc tggccatgaa cgcgtacact tcagttcagc aggatgggct ggagagcctc 1920
   tctgtgcagc ggtgtggggt gagccctgct gtggcctcct tgtggtggtc cctcttccca 1980
   cgtgcagccc tgttgggaag aaaggaagaa aacaggtccc tccaggggtg ctgctgccta 2040
   agccacccac ataagtacgc tggtgccgtg tcacccatgt tgagccgctc ctgatggctg 2100
   acgggctccc agaccctcac ctcggacatg gtggtggggg aaggacgggt gggcaaggct 2160
   ggtgcgttcc ccagctctcc ctacgctgct cgggccattg cccagccaga tgtggtcacc 2220
   tcagtccagc tctggggcct ccaggccatg tggctgttcc cacggcccag tcctcgctgc 2280
   agtaacccct gggggctctg accacctatg ggggccgggc aggagcctct ggggcctcca 2340
   ctccgacatc aggacctgag atgaccgctg tgtggcgctc tctccctggg cagggtggat 2400
   gccacaggcc cctctggctc ccaggtgctg cttctccaca ggtgcggcct ggcccggcct 2460
   cctaaaggcc acaccctccc cacgcacttc ccaggccaga atccaaacat cgggaaccct 2520
   gttttcttct gggtgtgtct cacttagaaa tcgtggttct tccccgaggg tgcatgttgc 2580
   aggagggaga gggcagggaa gactcacagc agagcaggag ggggcctgtg cttctcgggg 2640
   tctgcacccc aggcacagcg gtgtcacccc gcaggaccgc gggcctgccc caacccccag     2700
   cattcccggg tgggcccaga ccccatcacc aagactggcc acccgctgcg tgtgtgtgcg     2760
   cgcgcgtgta cgtgtggccc cacatccgcc gccttccacg ctaggatgta agaggtcgcc     2820
   tcctattgta catttgggga aagccttggg tgtaaatcag tgtaaacttg gaggagagat     2880
   ttttctatca tgtagagtag gtatttttta tagattgaag gttgatcaat tttttaatac     2940
   tttcaagaga aaactgtgta tacacatgaa atatatatat atatatatat atatatatgt     3000
   ataatatata aagactggca ccctgcctct ctgtgcccag gcccagccct ggtgacatgg     3060
   caccactcag cagtgctgtc actgtaagca tggactccca ggagacagtg tgggaaacgc     3120
   tcctgcttta attccccgag aaacggctct tcctgcctgg atgcaggagg gcaggggcca     3180
   ccacagatta aagctgttac tgcacaaaaa aaaaaaaaaa aaaaaaa                   3227
<210> 8
   <211> 5537
   <212> DNA
   <213> Homo sapiens
<400> 8
   gcctttgcta aattgctgaa taagaagatg gttgagtcac ctccttcgct taaacttgtc 60
   ctcattggag gttgtcgtaa caaagatgat gaacttaggg taaaccaact gagaaggctg 120
   tctgaggatt taggagttca agaatatgtg gaatttaaaa taaacattcc atttgatgaa 180
   ttaaagaatt atttgtctga agcaacaatt ggtctgcata ccatgtggaa cgagcatttt 240
   gggattggag ttgtggagtg tatggcagct ggcacaatta tccttgcaca caattcgggg 300
   ggcccaaagc ttgacattgt ggttcctcac gaaggagata taactggctt tctggctgag 360
   agtgaagaag actatgctga aactatcgct cacattcttt ccatgtctgc agaaaagaga 420
   ctccaaatca gaaaaagtgc tcgtgcatct gtaagcagat tctctgatca ggaatttgaa 480
   gtgacattcc tatcatctgt ggaaaagtta tttaagtaat gccatatctg taaaattaaa 540
   gatattttat ataaactggt taaacacctt catatgtaaa tatttttcta aattcaatct 600
   catttgtcaa atcattttac tttagaaaac agacaaaatt tccttttaga ataaaaggaa 660
   gtgttgaaaa gaaaatggat gactagcctt cggcttccat tcttggtata catgagagag 720
   gctggctgct gagatgaatg tgaaccaggt tgcagagaat ctggctttga gccaccagga 780
   agaactagtg gatttgccaa aaaactaccc cttgagtgaa aatgaagatg agggggacag 840
   tgatggagag agaaagcatc aaaagcttct ggaagcaatc atttcccttg atggaaagaa 900
   taggcggaaa ttggctgaga ggtctgaggc tagtctgaaa gtgtcagagt tcagtgtcag 960
   ttctgaagga tcaggagaaa agctgggcct tgcagatctg cttgagcccg ttaaaacttc 1020
   atcttctttg gccactgtaa aaaagcaact gaatagagtc aaatcaaaga aggtggtgga 1080
   gttacctctt aacaaagaaa aaattgaaca gatccacaga gaagtagcat tcagtaaaac 1140
   ctcacaggtc ctctccaaat gggaccctat catcctgaag aaccagcagg cagagcagct 1200
   ggtttttccc ctggggaagg agcagccagc cattgctccc attgaacatg cgctcagtgg 1260
   ctggaaggca agaactcccc tggagcagga aatttttaac ctcctccata agaacaagca 1320
   gccagtgaca gatcctttac tgactcccat ggaaaaggcc tctctccaag ccatgagcct 1380
   ggaagaggca aagatgcacc gagcagagct tcagagggct cgggctctgc agtcctacta 1440
   tgaggccaag gctcgaaaag agaagaaaat caaaagtaaa aagtatcaca aagtcgtgaa 1500
   gaaaggaaag gccaagaaag ccttaaaaga gtttgagcag ctacagaagg ttaatccaac 1560
   tgtggcactg gaagaaatgg aaaaaattga aaatgccaga atgatggaaa gaatgagcct 1620
   taagcaccaa aacagtggga aatgggccaa gtcaaaggca attatggcca aatatgacct 1680
   ggaggctcgc caagctatgc aggaacagtt ggccaagaac aaagaactga cacagaaact 1740
   ccaggtagcc tctgagagtg aggaagagga gggaggcaca gaagtggaag aactccttgt 1800
   ccctcatgta gcgaatgaag tgcagatgaa tgtggacgga ccgaatccct ggatgttcag 1860
   gagctgcacc agtgacacca aagaggctgc aacacaggag gaccctgagc aagtgccaga 1920
   gcttgcagct catgaggttt ctgcaagtga ggcagaagaa agaccagtgg cagaggaaga 1980
   aattttgttg agagaatttg aggaaaggca atcccttaga aaaagatctg agctcaacca 2040
   ggatgctgag ccagcaagca gtcaagaaac aaaagattct agcagccagg aggtgctgtc 2100
   cgaattgagg gcactatctc agaaattgaa ggaaaaacat cagtccagga agcaaaaagc 2160
   aagttcagag gggactgttc cccaggtcca gagagaggaa cctgccccag aagaagcgga 2220
   acccctattg ctacagaggt cagagagagt acaaactctg gaagagctag aagagctggg 2280
   aaaagaagat tgttttcaaa ataaggagct tcccagacct gtgttagaag gacagcagtc 2340
   agagaggacc ccaaataatc ggcctgatgc ccctaaggag aagaaagaga aggagcaact 2400
   gatcaaccta cagaacttcc tgaccacaca gtctccttcc gtgaggtctt tggcagttcc 2460
   cacaataata gaggagctgg aagatgaaga ggagagagac caaaggcaga tgataaagga 2520
   agcttttgct ggggatgatg tcatcagaga tttcttgaaa gagaagaggg aagctgtgga 2580
   ggcgagtaag ccaaaggacg tggacctgac actacctggc tggggcgagt ggggtggtgt 2640
   gggcctaaag cccagtgcca agaaaagacg ccagtttctc attaaagccc ctgagggtcc 2700
   tccaagaaaa gataagaatt tgccaaatgt gattatcagt gagaagcgca acatccacgc 2760
   agcagctcat caggtacaag tgcttccata tccatttacc caccatcggc aatttgaaag 2820
   gaccatccag acccctatag gatccacatg gaacacccag agggctttcc aaaagctgac 2880
   tactcccaag gtcgtcacca agccaggcca tatcattaag cccataaaag cagaggatgt 2940
   gggctaccag tcttcctcaa ggtcagacct gcctgtcata cagaggaatc caaaacgaat 3000
   caccacacgt cacaataaag aagaaaaact gtaggttgtg tagctggaga agtgacagtc 3060
   aggggccctg attccacttc ctttggtcca gttttactct gctacagggt ggattccaaa 3120
   actggctcag cacattgcat gtagttgagc cacatttttt aaaaaaagaa aatggatgac 3180
   cattaattga ctagcatttt agaattgatc agacattaga acacagaaaa attctagtac 3240
   atttaaattc taaacaatac agtggatgac ccttttgaat atacctaatg atttccttaa 3300
   aaaagaaatt ttaaacagac ttgtttaatc gtgttctcaa agcatacagt caagaggtgg 3360
   gactgactga tgctttatag gtgtgtgtag ggtggtagag gccaaggtgc tgccagcaat 3420
   cctttccata ctaggtactg gtgaaaattg tttttgttta tgctgtcagc acatttgtgt 3480
   gggtctctca ttgtccctta acagtgccgc atctcagcct ggaagtcagc tttaagtcat 3540
   tcaagagaac ctcaggctgt ttttctgaca gtgatgatat gatatacaga tacatccaca 3600
   gggtatctat taccagcata atgcattgta agatggcaag gtggcatttt gaaagagcgc 3660
   tgggcaagca gttagcacat ctgggcctac cttcagcttc ttcatttaca aacttctgac 3720
   cttttgacac taaagctgct cctttatctt tctgagtctc agattcttca tctgtaatct 3780
   ggagttgtta attccagtcc ttactacctt ttagagttgg aatgagatgg aaagtagatg 3840
   aaactacttt gaaaattacg acagttaagg gctgggtgcg gtgtctcaca cctgtaatcc 3900
   cagcactttg ggaggccgag atgggtggat catgaggtca gcagttgaga ccagcctgaa 3960
   caacatggtg aaaccctgtc tgtactaaaa atacaaaaaa attagctggg cctggtggca 4020
   ggcacctgta atcccagcta cttgggagac tgaggcagga gaattgcttg aaactggaag 4080
   gcagaggttg cagtgagcca agattgtgcc actgcactct agcctgggca ataagcaaaa 4140
   ctccatctca aaaaagaaaa aaaggaaaaa gaaaattatg agagttactt aaaggtaaca 4200
   tcacatacta aatgtcttct ataatcctat atttattaat gcattacaac tctgtagatt 4260
   gttagttact aggccagtag ctaggaattg gtataaattt aatgcacctt ctatcctgaa 4320
   taactagcat ggaaaagtga atatatgtgt gagcagatat ggctataaag acctatagct 4380
   tttgcacttt atgcatatat aatcaatcct ttctagttca gtgaattgac cccatccaca 4440
   ggctgattca tctttgtgtt aaggggcaaa tgaaacggta tattatttct ttgcagtctc 4500
   ctctcagtca ttcatcaatg tggccagctt atctactccc aattatgttg ttgatacatc 4560
   tccaagccat ctgtcatcag atcaaaaagc agcaaacaga gggtcagtca caggatgttc 4620
   tgacacacca ttgtaacttt ttgttagaga tgatcccatt tagaaaaaga ctggtagaaa 4680
   ttggagtgaa aggaacccta cagattagcc cagttctctc ttattttcag ctttacagac 4740
   aagaacaatt taaatctaaa gaatttagta gattccttca gtgtcacaaa gctgtttcat 4800
   gaaagaatca agattataac ctggatattc tgactcctgg cccagtgctt tttcttactt 4860
   tgtagctaca ctttgaagta agattcaaac tgttatccac tcaattgcct tattcctgag 4920
   gatgtagtga aggaagaaaa agttttctgg aattccgtaa attatatttt aagcttattt 4980
   cttcaaaatt attttcatat atcacagata tatcattgga agatataatt tgcatatatg 5040
   ttcattatca gtgttcctaa tttggtatta catgtattct atttttttct gaatgatagc 5100
   atgaaaagtg tcaaagtggt ttgtccgcta gcgtctgtct gcagaacttt caggatgact 5160
   attaattcct ctcagatgtc atttttgagt ggtccaagcc tgctgttttg aacccacagc 5220
   agtggagatt tgtattctta tttacagttg tgtactataa agtgtgtgtt acataggttt 5280
   tgtgtaataa ttatttgtaa atattattta gatttgtatt tagacatgat ttatatctaa 5340
   tatagataca aagtctgtgt ctaaatatta tttaaagaag tgatttttca ttctcttgga 5400
   ttctttccag tgtggtgcct tttatatgcc tcacatagtc tccttgttct cctactaata 5460
   ttcccaagct ccatatgcca attaaagaag aaacaaaaat aaaagtttgt cttgcttgtg 5520
   aaacattaaa aaaaaaa 5537
<210> 9
   <211> 1022
   <212> DNA
   <213> Homo sapiens
<400> 9
   gggcgggagc ggcggtccag actggggagg gacgcgcacc ggccaggagg cttcaagagg 60
   agggcactag ggccctgcga gcggcgtctt aaccggcggc gctaggactc cgcgggaaac 120
   ggcgggggcg gagcgggcgg caccaggacc caggggaacc gcgacgggcg ggcggcgagc 180
   aggcccggga gccgggaggc tgcgggcggc ggcgctggac ccgacgcggc gagagaggcc 240
   ccgagatgcc gagcaagaag aagaagtaca acgcgcggtt cccgccggcg cggatcaaga 300
   agatcatgca gacggacgaa gagattggga aggtggcggc ggcggtgcct gtcatcatct 360
   cccgggcgct cgagctcttc ctagagtcgc tgttgaagaa ggcctgccag gtgacccagt 420
   cgcggaacgc gaagaccatg accacatccc acctgaagca gtgcatcgag ctggagcagc 480
   agtttgactt cttgaaggac ctggtggcat ctgttcccga catgcagggg gacggggaag 540
   acaaccacat ggatggggac aagggcgccc gcaggggccg gaagccaggc agcggcggcc 600
   ggaagaacgg tgggatggga acgaaaagca aggacaagaa gctgtccggg acagactcgg 660
   agcaggagga tgaatctgag gacacagata ctgatgggga agaggagaca tcacaacccc 720
   caccccaggc cagccacccc tctgcccact ttcagagccc cccgacaccc ttcctgccct 780
   tcgcctctac tctgcctttg cccccagcgc ccccgggccc ctcagcacct gatgaagagg 840
   acgaagaaga ttacgactcc tagcgccttc tgccccccag accatagccc cttttagttg 900
   gttttagttg ctctgggggg aggagagaag gtagagctgt tcttaaattt attaaaaaaa 960
   aaaataaaag ggaatctcag tgtctgttcc aggctctgcg caggaaaaaa aaaaaaaaaa 1020
   aa 1022
<210> 10
   <211> 2379
   <212> DNA
   <213> Homo sapiens
<400> 10
   gggtgcacac ccggaagtgg gtgcgggcca gccggctcgc ccgggggcca tggcagcagc      60
   ggctactgca gccgaggggg tccccagtcg gggcctcccc ggggaagtca ttcatctgaa      120
   tgtgggaggc aagagattca gtacctctcg ccagactctc acctggatcc cagactcctt      180
   cttctccagt cttctgagcg gacgcatctc gacgctgaaa gatgagaccg gagcaatctt      240
   catcgacagg gaccctacag tcttcgcccc catcctcaac ttcctgcgca ccaaagagtt      300
   ggatcccagg ggtgtccacg gttccagcct cctccatgaa gcccagttct atgggctcac      360
   tcctctggtt cgtcgcctgc agcttcgaga ggagttggat cgatcttctt gtggaaacgt      420
   cctcttcaat ggttacctgc cgccaccagt gttcccagtg aagcggcgga accggcacag      480
   cctagtgggg cctcagcagc taggaggacg gccagcccct gtccgacgga gcaacacgat      540
   gccccccaac cttggcaatg cagggctgct gggccgaatg ctggatgaga aaacccctcc      600
   ctcaccctca ggacaacctg aggagccggg gatggtgcgc ctggtgtgtg gacaccataa      660
   ttggatcgct gtggcctata cccagtttct agtctgctac aggttgaagg aagcctctgg      720
   ctggcagctg gtgttttcca gcccccgcct ggactggccc atcgaacgac tggcgctcac      780
   agcccgggtg catggtgggg ctttgggtga acatgacaag atggtggcag cagccaccgg      840
   cagcgagatc ctgctatggg ctctgcaggc ggaaggcggt ggctccgaga taggggtctt      900
   tcatctgggg gtgcctgtgg aggccttgtt cttcgtcggg aaccagctca ttgctacaag      960
   ccacacaggg cgcatcgggg tgtggaatgc cgtcaccaag cactggcagg tccaggaggt     1020
   gcagcccatc accagttatg acgcggcagg ctccttcctc ctcctgggct gcaacaacgg     1080
   ctccatttac tacgtggatg tgcagaagtt ccccttgcgc atgaaagaca acgacctcct     1140
   tgtcagcgag ctctatcggg acccagcgga ggatggggtc accgccctca gtgtctacct     1200
   cacccccaag accagtgaca gtgggaactg gatcgagatc gcctatggca ccagctcagg     1260
   gggcgtgcgg gtcatcgtgc agcacccgga gactgtgggc tcggggcctc agctcttcca     1320
   gaccttcact gtgcaccgca gccctgtcac caagatcatg ctgtcggaga agcacctcat     1380
   ctcagtctgt gccgacaaca accacgtgcg gacatggtct gtgactcgct tccgcggcat     1440
   gatttccacc cagcccggct ccaccccact cgcttccttt aagatcctgg ctctggagtc     1500
   ggcagatggg catggcggct gcagtgctgg caatgacatt ggcccctacg gtgagcggga     1560
   cgaccagcaa gtgttcatcc agaaggtggt gcccagtgcc agccagctct tcgtgcgtct     1620
   ctcatctact gggcagcggg tgtgctccgt gcgctccgtg gacggctcac ccacgacagc     1680
   cttcacagtg ctggagtgcg agggctcccg gcggctcggc tctcggcccc ggcgctacct     1740
   gctcactggc caggccaacg gcagcttggc catgtgggac ctaaccaccg ccatggacgg     1800
   cctcggccag gcccctgcag gtggcctgac ggagcaagag ctgatggaac agctggaaca     1860
   ctgtgagctg gccccgccgg ctccttcagc tccctcatgg ggctgtctcc ccagcccctc 1920
   accccgcatc tccctcacca gcctccactc agcctccagc aacacctcct tgtctggcca 1980
   ccgtgggagc ccaagccccc cgcaggctga ggcccggcgc cgtggtgggg gcagctttgt 2040
   ggaacgctgc caggaactgg tgcggagtgg gccagacctc cgacggccac ccacaccagc 2100
   cccgtggccc tccagcggtc tcggcactcc cctcacacct cccaagatga agctcaatga 2160
   aacttccttt tgaacaacgc agctgccatg atgccttggg atgccctggt cctgggggac 2220
   tcaggtgcct ccctgattcc tgtgggaacc ccgggttcag ggccagggcc tccttggaat 2280
   aaatggttat tgttactagg tccccacctt ccctcttttc tggaagccaa agtcaccctc 2340
   cccaataaag tcctcactgc caacaaaaaa aaaaaaaaa 2379
<210> 11
   <211> 4406
   <212> DNA
   <213> Homo sapiens
<400> 11
   cggtgcggag cctgtctcct tcactggatc ccgcattttc agcgcgttgc atcacctccg        60
   tgcgcccggt tgcagcgtgg acgccggatg agttgctttt aggcttgctg gcccgcgggg       120
   ctgtccaggc acgcgaggcc cctcagcaac aaaatgcttc aacaagttcc agaaaacata       180
   aattttcctg ctgaagaaga gaaaatcttg gagttttgga ctgaatttaa ttgttttcag       240
   gaatgcttaa agcaatcaaa acataaacca aaatttacct tctatgatgg tcctcctttt       300
   gcaactggac tgcctcacta tggacatata cttgcgggta caattaaaga tatagttaca       360
   agatatgctc accagagtgg gtttcatgtt gacagaagat ttggatggga ttgccatggc       420
   ttacctgtgg aatatgaaat tgataagaca ctgggaatca gaggaccaga ggatgtggcc       480
   aaaatgggga ttacagagta taacaatcag tgccgagcaa ttgtgatgag atattctgct       540
   gagtggaagt ctactgttag cagacttggc cgatggattg actttgacaa tgactataaa       600
   actctgtatc cacaattcat ggaatcagtc tggtgggtct tcaaacaact ctatgataaa       660
   ggccttgttt atagaggtgt gaaagtcatg cccttctcta cggcatgtaa cactccactt       720
   tccaacttcg agtcacacca gaattataag gatgttcaag atccttcagt atttgtaact       780
   ttccctttgg aagaagatga aactgtatct ttagttgctt ggacaaccac tccctggact       840
   ctacctagta accttgctgt gtgtgttaat ccagaaatgc aatatgtgaa aattaaagat       900
   gttgccagag gacgattact cattttaatg gaagccagat tgtcagccct ctataaattg       960
   gagagtgact atgagatcct tgaaagattt cctggtgcct atcttaaagg caagaagtac      1020
   aggcccctgt ttgactattt cctgaagtgt aaagagaatg gcgctttcac tgtgcttgtt      1080
   gacaactatg tgaaggaaga agaaggcaca ggggttgtcc accaagctcc ttacttcggt      1140
   gctgaggact atcgggtctg tatggacttt aacattattc ggaaagactc actccctgtt      1200
   tgccctgtgg atgcttcagg ctgcttcaca acggaggtga cagatttcgc aggacagtat 1260
   gtgaaggatg ctgacaaaag tatcatcagg actttgaagg aacaaggccg acttctggtt 1320
   gccaccacct tcactcacag ctaccctttt tgctggagat cagacactcc tctaatttac 1380
   aaagcagtgc ccagctggtt tgtgcgagtg gagaacatgg tggaccagct cctaaggaac 1440
   aatgacctgt gctactgggt cccagagttg gtacgagaaa aacgatttgg aaattggctg 1500
   aaagatgcac gtgactggac aatttccaga aacagatact ggggcacccc catcccactg 1560
   tgggtcagcg atgactttga ggaggtggta tgcattgggt cagtggcgga acttgaagaa 1620
   ctgtcaggag caaagatctc agatctccac agagagagtg ttgaccacct gaccattcct 1680
   tcacgctgtg ggaagggatc cttgcaccgc atctctgaag tgtttgactg ttggtttgag 1740
   agtggcagca tgccctatgc tcaggttcat tacccgtttg aaaacaagag ggagtttgag 1800
   gatgcttttc ctgcagattt cattgccgag ggcatcgacc aaaccagagg atggttttat 1860
   accctgctgg tgctggccac ggccctcttt ggacaaccgc ctttcaagaa cgtaattgtg 1920
   aatgggcttg tcctggcaag tgatggccaa aaaatgagca aacggaaaaa gaattatcca 1980
   gatccagttt ccatcatcca gaagtatggt gctgatgccc tcagattata tctgattaac 2040
   tcccctgtgg tgagagcaga aaacctccgc tttaaagaag agggtgtgcg ggacgtcctt 2100
   aaggatgtac tgctcccatg gtacaatgcc tatcgcttct taatccagaa cgttctgagg 2160
   ctccagaagg aggaagaaat agaatttctc tacaatgaga acacggttag agaaagcccc 2220
   aacattacag accggtggat cctgtccttc atgcagtctc tcattggctt ctttgagact 2280
   gaaatggcag cttataggct ttatactgtg gtgcctcgcc tggtcaagtt tgtagatatt 2340
   ctgaccaatt ggtatgttag aatgaaccgc agaagattaa agggtgaaaa tgggatggag 2400
   gattgtgtca tggccctaga aaccttgttt agtgttctgc tttctctttg cagacttatg 2460
   gctccctaca caccttttct cactgaattg atgtaccaga atctaaaggt gctgattgac 2520
   cctgtttctg ttcaggacaa ggacacactc agcattcact acctcatgct gccccgtgtt 2580
   cgagaagaat tgattgacaa gaaaacagag agtgcagtat ctcagatgca gtctgtgatt 2640
   gaacttggaa gagtgatcag agaccgaaaa actattccca taaagtatcc tttgaaagaa 2700
   attgtggtta tccatcaaga tccagaagct cttaaagata tcaagtcttt ggagaagtat 2760
   atcattgagg aactcaatgt tcgaaaagtt acactgtcta cagataaaaa caagtatggc 2820
   attcggctaa gggcagaacc agatcacatg gtcctgggga agcgtctgaa gggagccttt 2880
   aaggcagtga tgacgtccat caagcagttg agcagtgagg agctggagca gttccagaag 2940
   actgggacca ttgttgtgga aggccatgaa ttgcacgatg aagacatccg cctcatgtac 3000
   acctttgatc aggccacagg tgggactgcg caatttgaag cacactcaga tgctcaggct 3060
   ttggtcctct tagatgtcac tcctgaccag tcaatggtag atgaaggaat ggctcgggaa 3120
   gtcatcaatc gcatacagaa acttcgcaaa aagtgcaatc tggttccaac tgatgaaatc     3180
   acagtgtact ataaagcaaa gtctgaagga acatatctga atagtgttat tgaaagccac     3240
   acagagttca tatttaccac cataaaggct cccttgaaac catatccagt ttctccatcg     3300
   gataaagtcc ttattcaaga aaaaacacag ttgaagggat ctgaactgga aattacactc     3360
   accagaggat cttcccttcc tggtcctgct tgtgcatatg tcaatcttaa catttgtgca     3420
   aatggcagtg aacaaggtgg agtattgctc ctggaaaatc caaaaggtga caataggttg     3480
   gaccttttaa agctgaagag tgttgtcact agcatttttg gtgtgaaaaa tacagagctg     3540
   gctgtcttcc atgatgaaac agaaatacaa aaccaaactg acttactgag tcttagtgga     3600
   aaaacacttt gtgtgactgc aggatcggct ccctctctga tcaacagttc tagtactctt     3660
   ctttgtcagt atatcaacct acagctcctg aatgcaaagc cacaagagtg tttaatgggg     3720
   acagtgggca ctctcctgct tgaaaaccca cttgggcaga atggactcac ccaccaaggt     3780
   cttctgtatg aagcagccaa ggtgtttggc cttcggagca ggaagctaaa gctgtttctg     3840
   aatgagaccc aaacgcagga aattacagaa gacatccccg tgaagacttt gaatatgaag     3900
   actgtgtatg tttctgtgtt accaacaaca gcagacttct agcatgtact tatcaatgtt     3960
   gttcggtcag cccttcccta attacaccta tcccctacac atacatgcac atagacacac     4020
   acatgaacac actgaagata tttccttcag gtgtgtgtaa aatatgctgc ttggattgaa     4080
   attcaaatgg gattgattag tcaagtaact tgagacctca cagtaatctt cacacttaac     4140
   cttagacacc tatgcagtca tgttgggagc aggttacaat gttacttcag cccacagttt     4200
   atttctatac ttgagttctt aagtacagaa gatagaagtg atttaaatgg catagtatat     4260
   atatcatttt ctggcctttt aaaatttatt tgagacctct tgatgaaatg gacatattat     4320
   atatttctgc cacctggatt ttcctggata atttgatgga atattttaag tttcagtaaa     4380
   tcagaacaat aaacaaactc agatat                                          4406
<210> 12
   <211> 1316
   <212> DNA
   <213> Homo sapiens
<400> 12
   ctcttcccat tggctgagaa gggtcatgag gatgaactgt gcagtcacct ggcaggaacc 60
   ggaacccgcg gttataaagt aaaggaaccc gagatctgcg caggggttcc ctttgccgat 120
   ttctctcacc tcacctttca aacctaaact cgagcctact gttcaccggc ctagcattgc 180
   tctcgccatg gctctcagcg atgctgacgt gcaaaagcag ataaagcata tgatggcttt 240
   cattgaacaa gaagccaatg agaaagcaga agaaatagat gcaaaggcag aagaagagtt 300
   caacatagag aaaggtcggc ttgtgcaaac ccaaagacta aagattatgg aatattatga 360
   gaagaaagag aaacagattg agcagcagaa gaaaattcag atgtccaatt tgatgaatca 420
   agcgagactc aaagtcctca gagcaagaga tgaccttatc acaggtttgt accagttgct 480
   ggagccccga atgattgttc gttgcaggaa acaagatttc cctctggtaa aggctgcagt 540
   gcagaaggca attcctatgt acaaaattgc caccaaaaac gatgttgatg tccaaattga 600
   ccaggagtcc tacctgcctg aagacatagc tggtggagtt gagatctata atggagatcg 660
   taaaataaag gtttccaaca ccctggaaag ccggctggat ctcatagccc agcagatgat 720
   gccagaagtc cggggagcct tgtttggtgc aaatgccaac aggaagtttt tggactaagc 780
   cttcaggagg tggagctcgt cgtcagctct cctgctgtga tgtggaagct tctgatattt 840
   gaagaaacac gaatgtctct gtagcttcct cttcactgcc ccagtattgc tctgtattta 900
   tcagcgatgc ccctctgtca ctcatgcctt gcctaattgt tcacaatggt ggaaagcttc 960
   atgtaatatg atcaggaccc acctccagtt cttctgaaag tgtgacagtg tccagccggt 1020
   tctgcagcac taggggaggg ggcagatggt ggttgcatgg gcttcctggg tctccactct 1080
   ccgtctggcc taaaggtgat gtatttggtg tttggccctg cagtccccac tcttgaggct 1140
   taaggcgcat gtggcacacc actccttcca gcagtagtcg ctttactgtt acctgtttag 1200
   gcctagaagt tttccctcat ctgtaaatgt gatttaaaat ctaagccatg aatatgcttt 1260
   atttattaaa agagttatgc ggatttaatg tgatttctag tgtaaggcac tacaaa 1316
<210> 13
   <211> 541
   <212> DNA
   <213> Homo sapiens
<400> 13
   agcgcggcgg tgatggcggg gccggtgaag gaccgcgagg ccttccagag gctcaacttc      60
   ctgtaccagg ccgcccattg tgtccttgcc caggaccccg agaaccaggc gctggcgagg     120
   ttttactgct acactgagag gaccattgcg aagcggctcg tcttgcggcg ggatccctcg     180
   gtgaagagga ctctctgccg aggctgctct tccctcctcg tcccgggcct cacctgcacc     240
   cagcgccaga gacgctgcag gggacagcgc tggaccgtac agacctgcct aacatgccag     300
   cgcagccaac gcttcctcaa tgatcccggg catttactct ggggagacag gcctgaggcc     360
   cagctcggga gccaagcaga ttccaaacca ctacaaccct tgccaaacac agcccactcc     420
   atttcagacc gccttcctga ggagaaaatg cagactcagg gttccagtaa ccagtgatgg     480
   attcacccca tctcccaaat aaagtttact tgttttacat tcaaaaaaaa aaaaaaaaaa     540
   a                                                                     541
<210> 14
   <211> 1651
   <212> DNA
   <213> Homo sapiens
<400> 14
   attttgtgga gcgccagagc tgctaagtgc gtcagttgtg gagtggcgta gacgagttaa      60
   gtcctggtct gcgtggaggt cgacgactcc gtcgcagact acggacctgt ctgggtctca 120
   gccgccaaag accccgtccg gtaggtgagt ggctcacttt gagggcaagc cttctcggat 180
   cgaggcttct tcatggccgc tcagatcgtg agcggccggg gctgctctct ttgcggagga 240
   tggcgtctaa tgagcgcagt tgattcgagg aagtactagc cggacatcat gagtggctgt 300
   cgggtattca tcgggagact aaatccagcg gccagggaga aggacgtgga aagattcttc 360
   aagggatatg gacggataag agatattgat ctgaaaagag gctttggttt tgtggaattt 420
   gaggatccaa gggatgcaga tgatgctgtg tatgagcttg atggaaaaga actctgtagt 480
   gaaagggtta ctattgaaca tgctagggct cggtcacgag gtggaagagg tagaggacga 540
   tactctgacc gttttagtag tcgcagacct cgaaatgata gacgaaatgc tccacctgta 600
   agaacagaaa atcgtcttat agttgagaat ttatcctcaa gagtcagctg gcaggatctc 660
   aaagatttca tgagacaagc tggggaagta acgtttgcgg atgcacaccg acctaaatta 720
   aatgaagggg tggttgagtt tgcctcttat ggtgacttaa agaatgctat tgaaaaactt 780
   tctggaaagg aaataaatgg gagaaaaata aaattaattg aaggcagcaa aaggcacagt 840
   aggtcaagaa gcaggtctcg atcccggacc agaagttcct ctaggtctcg tagccgatcc 900
   cgttcccgta gtcgcaaatc ttacagccgg tcaagaagca ggagcaggag ccggagccgg 960
   agcaagtccc gttctgttag taggtctccc gtgcctgaga agagccagaa acgtggttct 1020
   tcaagtagat ctaagtctcc agcatctgtg gatcgccaga ggtcccggtc ccgatcaagg 1080
   tccagatcag ttgacagtgg caattaaact gtaaataact tgccctgggg gccttttttt 1140
   aaaaaacaaa aaccacaaaa attcccaaac catacttgct aaaaattctg gtaagtatgt 1200
   gcttttctgt gggggtggga tttggaaggg gggttgggtt gggctggata tctttgtaga 1260
   tgtggaccac caaggggttg ttgaaaacta attgtattaa atgtcttttg ataagccttc 1320
   tgctcacatt tttgtgaatg tctgaagtat atagtttgtg tatattgaca gagctctttt 1380
   ataactaaag caaatttaat ttttttgtac tagaaaaaaa tttgaacatt ttagttcttg 1440
   gttataaaaa tgttaattca gaattagttt aatgccttaa ttaaactaat taatagcttt 1500
   ggacacttaa aagagctcta aatttgcttg tacataaagg cttaatttgt tttccttgtt 1560
   agggtcaagg gtgtcctcca ctctttaaca gctgctggac agacacatta gagcagctgt 1620
   ttgttattga taataaaata ttataaaact a 1651
<210> 15
   <211> 5250
   <212> DNA
   <213> Homo sapiens
<400> 15
   atggcggccg cagtctctag tgtggtgaga cgagtggaag agctcgggga tctggctcag        60
   gcccacatac agcaacttag cgaagctgcc ggtgaagatg atcacttttt aattcgggcc       120
   tctgcagcat tagaaaaatt gaaactcctg tgtggagaag agaaagaatg ttcaaatcca 180
   tcaaatcttc tagaacttta cacacaggct attttggaca tgacatattt tgaggagaac 240
   aagctagtag atgaagattt tcctgaagac tcttcttcac agaaagtaaa agagctgatt 300
   agttttcttt cagaaccaga aattttagta aaggaaaata atatgcatcc aaaacactgc 360
   aatttgcttg gggatgagct actggaatgt ctctcttgga gacgaggagc cctgctgtat 420
   atgtattgtc attctcttac caaaagaaga gagtggctct taagaaaatc cagtttgctt 480
   aaaaagtacc ttcttgatgg aatcagttac ttgctacaga tgctaaatta tcgatgtcct 540
   atccagttaa atgaaggagt ttctttccaa gatctagaca cagctaaatt actgagtgca 600
   ggaatattta gtgacattca tctgctggct atgatgtaca gtggagaaat gtgttactgg 660
   ggatcgaagt attgtgctga tcagcaacca gaaaatcatg aagtggatac tagtgtgtct 720
   ggagcgggct gcactacata caaggaaccc ttggatttcc gagaagtagg agaaaaaatc 780
   ttgaaaaagt atgtatctgt gtgtgaagga cccctgaaag aacaagaatg gaatacaacg 840
   aatgcaaaac aaattttaaa cttctttcat catcgctgta actagtaagt tcatctagtc 900
   cttttcaaat agaaaaacaa caaaacccat gaaatggaaa agagagttcc aaaaaagaag 960
   acctgttgat actgaatatg agaacattac actgcataaa gatatccagc ttgggtaccc 1020
   tactaaaaca acatcaccat ctttttcatt atatattagt aaacctatga agggatgatt 1080
   ctttaatttc taaagtgttg ttcaaactca ccttaaaata cagatgttat gtcactaata 1140
   atgagtagta acccaattta gctttacaaa gattttttct attacttttt ttttttattg 1200
   tggtatatgt aacacaagtt taccattttt atcatcttta gattattttt aaatctcact 1260
   accttgaagt ttgccatgct tcagaaatta aagtcaagtt acttcactgc tttgaataca 1320
   tactatggat ataaatgtag gcaaaatgaa agattatgac taatattcag attccatttt 1380
   caataaggta aataatgaaa gatctcacaa aggaatcatt gtgtttaatt attttgccat 1440
   gtattactaa ttgtatttaa aatttctact tcccttaggc attttataaa aatttaaaaa 1500
   ttatttttct attttgttta aaagacaatg catggctttt caagtgagta atgaagataa 1560
   aaaccaagct aggttagtct accattagtt catatgtttt tgtagcacat cttttcataa 1620
   catactgaaa ttttaatgtt ttttgccttt aataataggg gtgagtgtta aaggccagag 1680
   agcctaactt gatatcttgc tcatggtctt tctgtctacc tcaactcctg ttgtcatctt 1740
   tggatgatct ttctgatacc ttgctgatac cttggacttc tgcccagtga ctttattaca 1800
   ttccagcagc ttactcccgt ggccgcactt tgaatgtcac caccttgagt ttctctttct 1860
   ggtaaaattt cagtaactga taactgccct cttttcagtt ttttcacttc cttattttat 1920
   tgacttttct ctcctttatg ctcattccac attcttcagt tccttcttac ccccttttct 1980
   tgactgctcc ccttcctcct aaactgtcag cccctttcag gctttcttcc ctatccaccc 2040
   tcagctttat gggatactat atgaactaat ctcaccagtt attcttcttt ccctggtctt    2100
   ttagtctagt cctttggcaa gccctcaacc tcagattaat cctatttatt atctaccttt    2160
   tcggggatgc tgagttcttt gtgatgggta gtccttcaaa gtagaatcac tacaagtgtc    2220
   tcacttcagc cttagaaaag ccctcaatgt aaaccttatg tttgttacta gggtggcctc    2280
   tctcccattt cccgctgtgg ctgagccaaa cttcattact ttccttaggc cctctgcccc    2340
   tcccctagca tatggctctt tctcctattg aacagaaaat tgagactatg aagaggtcaa    2400
   cttgtattta ccaactttac tcattttccc attttagagg aaaagaggtt gcctgcttcc    2460
   tatcaaaggc gactctgtgc tacatgttgg tttcatcttc gctgtctctc tcatttcccc    2520
   acaaagtgtt gtctcacttt ctgcctacct tttaggtatt gatcttcact atattttttt    2580
   ctctctctct cttttttttt tttactttac atattttata caatgtcatt cataactatg    2640
   gcttctttta ccaccagtaa taacagactg agtctcaaac ctgtaacttg tatcctgact    2700
   tatctcttgc tttctaaacc ttgtttctaa ctgtctatca aatatcttca ttagacatcc    2760
   cacagtaatt ttaagctcag tgtttccaaa cgtcaagtcc tcatctgtta tataaaatcc    2820
   tttctgcttt gtaagttttc tctttttggg attttccgca gatcagggca acacagaatg    2880
   actaccttcc cagccatgtc tctgcagtca tcctgtgtaa tcatgttcta gtccagtgtt    2940
   gctcaaacag ttaaaatcca ggctgctttt ggtaaacata caaattctga tatagagcct    3000
   ctgtggcaaa gggaatttct cagttcctgc cccagccccc ttgtttatca ttacacagct    3060
   aagattttta tttaacttag cattttagca agttttctta tgaaaatacc tttgtgcttt    3120
   ctaagtataa agatttaaat tatttttaat acggttttta gaaccacaga ctatccctct    3180
   ttttagagca tcctgatcaa ttgcaagtac ttccacttct tccctaaatg cgctttgtgt    3240
   gttttccacc tcagtagcct tgcacacatc attacttctg catccttctt tgtgaccaag    3300
   tgaataccta cctacctctt aggactcagc ttcagtgatt ctgttttcat aggcttttta    3360
   aagccctttt cagtcaaatt agatgctcct ttgttctggt cctttactct tcccctccca    3420
   tgttgtcgca ctgatcacac tgctttgtaa ttattttctt tattgttggt aaactccata    3480
   ttttaaaaat ttacctgtat acatagtgct taatgcatgg taggccctta gtaatgtttt    3540
   tgaattaatg aggcatgatt ctaaatgcca aactttgcac aaaaagttgt tgctattgga    3600
   ttccaaactg ctatgccctc tataccagtg gctgacaagg catataggga tatatgccat    3660
   cctgagagga gtaaagtgtt tagattctga acctgagaaa agagaacttt ctcttaagaa    3720
   gttggaaact tcattttacc ttcgtgaccc tggtagacca cttggtctca ctggttggta    3780
   caattatgag ggtagtactg ggtgcttttt atacaacttt ccatccccca cacagttgga    3840
   gaataatttg tagaccatgg cagttaaaat gctttttact ctggtgtatt atagaataat    3900
   gaacacattt ttatatatgt taaagcctgc taaatcattc acttagaatg aagtaacctc    3960
   aaggtaccaa ataccccaca taaggaaagt actacactaa attcactcaa ctattgtatg    4020
   cctaccatat gccagatttt atcctagacc ctgaggatat agctttttaa aaccccctcc 4080
   tgtttcagag cttatattct agtggaggaa gttagacaag cattttaagt aataaatgta 4140
   tagagagtgt caagtgatga gtaccatgaa gaagaataaa tgagggtaag gggctagtgt 4200
   gatagggaga ggggtgggat gccatcatat ttaggggtgg ttgggaactg tctactgctt 4260
   tagcatttgt gtcttcaaat ttctctcctt tggttatata ccttgcgtat tccgcacatt 4320
   gataaagttt ctttcttaca gaagttctga tattgaatta aggaatgggt cactacttaa 4380
   gactttatca tttcagctac acataaaagg tttctctccc ctatggattt tgctaatggt 4440
   tgagtgatac ctaaaggcct tgttgcattt tttacacttg gggtttctct tcggtttgaa 4500
   ttctctcatg gttaccaaaa ctctgttaaa ggacttttca cagtcgttaa atgtgtatgg 4560
   ctctttccca atatgaattt ttttgatgtt aactaggctt ttagacttag ttaaagacct 4620
   gtgtgctctg ctgcatttat agggttttgg gctgctgaga ataattgatg ctgattaagg 4680
   tgcaaatcat gactaaagac ttttctttct tcttttgcat atagtagagt gaggtgaggg 4740
   tcgtggctaa aaccccacaa taactttaca ttcctagctt tacagtcttg ctgtgaattc 4800
   tctggtgttc attaaagtgt gagctgtgtt aaaaggcatt cttagattca ttttaaccac 4860
   atttttaaaa actcacagtt ggttaacatg atggtttcag attgcttgcc tcttttctct 4920
   ttctacctta cagggctcct tttctagctt aaataagggt actccatctg ccacagaatc 4980
   caggattcta tagtttttta gtttcacttc cctatgttct tttttttttt ttaattttta 5040
   acaatgagat gataccatgt tacagcggta ttacattaag atacatttaa tatagccaca 5100
   attaaagtat gtattatgta tttatgctgg tgttctttca tgttatttct tctagtgaaa 5160
   atttccaagt aaactgttat tgagcatata catttttaaa aaaataaaac catacacccc 5220
   taaaaaaaaa aaaaaaaaaa aaaaaaaaaa 5250
<210> 16
   <211> 3113
   <212> DNA
   <213> Homo sapiens
<400> 16
   cgctcagccc gggcgggcga tgcgggcggc gcgggcggcc ccctcccccg gcccgcgtct        60
   ccgggacggc tgcgggcggc ccccccggcg gccggagggc tccctggccc cgatctgacg       120
   gcggcggcgg cggcggccac agcggcggga gcggcgcggg gaaggagcag cggctcgcag       180
   ccctcggccc gcgcccccac ccagcgccag cccgaggggg gaggcgcagc gccggagggt       240
   ggcggtcctc ggccctccca ggtctccgcg ccgggaagcc gctccgagcc ggggctggag       300
   ggttgttttg ccgttgtgtt gagcacgtca cccattaaga gccctttaaa gacctggatt       360
   gattggaagg acaaaaatta aaagcaatct gatccagcct catgcaggat ccctgcggat       420
   tttctcctta tcccatttcc atccactgtc acaatttgag aatctgcctg atttgatcag       480
   attcacctcc aggggaggtg tgataccagg gttaggagga cgtgaagtta tgggcaactt      540
   tctgatctgt ccatcagcag tctgagaaac gctggctctg aattttccgt gtcggccttt      600
   tggaaacaac aagttcctcg ctgtttgcaa agcttcagtg ctcgggtccc tgggacaccc      660
   cggccaccct cgcctggtag atgtggcatt tccatgctga ggccgcgagt cccgcctgac     720
   cccgtcgctg cctctccagg gcttctctgg gccgcgcctc tgcagactgc gcagccatgc      780
   tgcatctgct ggcgctcttc ctgcactgcc tccctctggc ctctggggac tatgacatct      840
   gcaaatcctg ggtgaccaca gatgagggcc ccacctggga gttctacgcc tgccagccca      900
   aggtgatgcg cctgaaggac tacgtcaagg tgaaggtgga gccctcaggc atcacatgtg      960
   gagacccccc tgagaggttc tgctcccatg agaatcccta cctatgcagc aacgagtgtg     1020
   acgcctccaa cccggacctg gcccacccgc ccaggctcat gttcgacaag gaggaggagg     1080
   gcctggccac ctactggcag agcatcacct ggagccgcta ccccagcccg ctggaagcca     1140
   acatcaccct ttcgtggaac aagaccgtgg agctgaccga cgacgtggtg atgaccttcg     1200
   agtacggccg gcccacggtc atggtcctgg agaagtccct ggacaacggg cgcacctggc     1260
   agccctacca gttctacgcc gaggactgca tggaggcctt cggtatgtcc gcccgccggg     1320
   cccgcgacat gtcatcctcc agcgcgcacc gcgtgctctg caccgaggag tactcgcgct     1380
   gggcaggctc caagaaggag aagcacgtgc gcttcgaggt gcgggaccgc ttcgccatct     1440
   ttgccggccc cgacctgcgc aacatggaca acctctacac gcggctggag agcgccaagg     1500
   gcctcaagga gttcttcacc ctcaccgacc tgcgcatgcg gctgctgcgc ccggcgctgg     1560
   gcggcaccta tgtgcagcgg gagaacctct acaagtactt ctacgccatc tccaacatcg     1620
   aggtcatcgg caggtgcaag tgcaacctgc acgccaacct gtgctccatg cgcgagggca     1680
   gcctgcagtg cgagtgcgag cacaacacca ccggccccga ctgcggcaag tgcaagaaga     1740
   atttccgcac ccggtcctgg cgggccggct cctacctgcc gctgccccat ggctctccca     1800
   acgcctgtgc cactgcaggt tcctttggca actgcgaatg ctacggtcac tccaaccgct     1860
   gcagctacat tgacttcctg aatgtggtga cctgcgtcag ctgcaagcac aacacgcgag     1920
   gtcagcactg ccagcactgc cggctgggct actaccgcaa cggctcggca gagctggatg     1980
   atgagaacgt ctgcattgag tgtaactgca accagatagg ctccgtgcac gaccggtgca     2040
   acgagaccgg cttctgcgag tgccgcgagg gcgcggcggg ccccaagtgc gacgactgcc     2100
   tccccacgca ctactggcgc cagggctgct accccaacgt gtgcgacgac gaccagctgc     2160
   tgtgccagaa cggaggcacc tgcctgcaga accagcgctg cgcctgcccg cgcggctaca     2220
   ccggcgtgcg ctgcgagcag ccccgctgcg accccgccga cgatgacggc ggtctggact     2280
   gcgaccgcgc gcccggggcc gccccgcgcc ccgccaccct gctcggctgc ctgctgctgc     2340
   tggggctggc cgcccgcctg ggccgctgag ccccgcccgg aggacgctcc ccgcacccgg     2400
   aggccggggg tcccggggtc ccggggcggg gccggcgtcc gaggccgggc ggtgagaagg      2460
   gtgcggcccg aggtgctccc aggtgctact cagcagggcc ccccgcccgg cccgcgctcc      2520
   cgcccgcact gccctccccc cgcagcaggg gcgccttggg actccggtcc ccgcgcctgc      2580
   gatttggttt cgtttttctt ttgtattatc cgccgcccag ttcctttttt gtctttctct      2640
   ctctctcttt tttttttttt ttttctggcg gtgagccaga gggtcgggag aaacgctgct      2700
   cgccccacac cccgtcctgc ctcccaccac acttacacac acgggactgt ggccgacacc      2760
   ccctggcctg tgccaggctc acgggcggcg gcggaccccg acctccagtt gcctacaatt      2820
   ccagtcgctg acttggtcct gttttctatt ctttattttt cctgcaaccc accagacccc      2880
   aggcctcacc ggaggcccgg tgaccacgga actcaccgtc tgggggagga ggagagaagg      2940
   aaggggtggg gggcctggaa acttcgttct gtagagaact atttttgttt gtattcactg      3000
   tcccctgcaa gggggacggg gcgggagcac tggtcaccgc gggggccgat ggtggagaat      3060
   ccgaggagta aagagtttgc tcactgctgc caaaaaaaaa aaaaaaaaaa aaa             3113
<210> 17
   <211> 2525
   <212> DNA
   <213> Homo sapiens
<400> 17
   aaagtttccg agtccattcc gggagcggga gcccatcttg ctggctgccg aggccctcgc 60
   tggaggagga gggtcagaac tcgggtgcag ccaatcgagg gcaacgctgc tacttatcag 120
   agcagaatgg gctgtagttt agtgaaatag gaaagctgca aaacactgtg gagtgctccc 180
   gtgtaaataa aaagaggaaa aaagtttctc aagtcgccgc tgcacgacgt ctggccggcg 240
   ctggagcggg ggtctgcgct ctcccgagcg gccgcgcgct ggactttatt gtgccgcaac 300
   cagccccagt tcccattgtt tgtgtttttt tcaaaatatg gcaaaggttc aggtgaacaa 360
   tgtagtggtg ctggataacc cttctccttt ctacaacccg ttccagttcg agatcacctt 420
   cgagtgcatc gaggacctgt ctgaagactt ggaatggaaa attatctatg tgggctctgc 480
   agaaagtgaa gaatacgatc aagttttaga ctctgtttta gtgggtcctg ttcccgcagg 540
   aaggcatatg tttgtatttc aggctgatgc acctaatcca ggactcattc cagatgcaga 600
   tgcagtaggc gtaactgttg tgctaattac ttgtacctat cgaggacaag aatttattag 660
   agttggctat tatgtaaata atgaatatac tgagacagaa ttaagggaaa atccaccagt 720
   aaaaccagac ttttctaagc ttcaaaggaa tattttggca tctaatccca gggtcacaag 780
   attccacatt aattgggaag ataacacaga aaaactggaa gatgcagaga gcagtaatcc 840
   aaatctacag tcacttcttt caacagatgc attaccttca gcatcaaagg gatggtccac 900
   atcagaaaac tcactaaatg tcatgttaga atcccacatg gactgcatgt gaccacctac 960
   catcccttta gtacaaatta agctattaaa aatacacaga actatttccc tgaaattccg 1020
   taagtacata gtcaaaacac aatgtgaaga atttgtttaa aaacatcctg tagaaagttt 1080
   ataagaaaac cagtatttga acaaattgtg gaatataaat acaactattt ttaagtaatt 1140
   tttttctcta atgtgttatt ttatttgttc tgaaactaat ctgattaaag catatatatt 1200
   attttcttct cctttatatg taatgaaagc acttataaag aaacaggaat cattagacca 1260
   ggttgtaaag atgtcttggc ctcccagaca gtctttggac cactatttta ctggcctttg 1320
   aaagaacaaa gtttacttca actaaaatgt ttcttcctgt gaagacatat ggtatcattt 1380
   taatttaagg ggcagatttc cattcttttt tggcatgtcc ttaaaaagag gatttgaaat 1440
   caactatatg ctaccaagaa ctttaggatc caattttcca agccaccgtg aagcctctta 1500
   tggctactat tatagttcat gagatgttgg acagcatttg gtttgtttgg taagagaaga 1560
   acaaatatgg ctaattgtta ataaggtccc ctggctatgg tttttgttct tataacagag 1620
   tttagaatat cagagcattt cttgaatcat acatcattat tgtccagtga attcaagacc 1680
   aaatacaata tcgggagaaa atacaaactc cctgtgttta agaatagggg attacaatgg 1740
   ctcaaattgg ttcatttgat ttctaaaaat accatgacct ttaaaaattc ttttaataga 1800
   tcatgttatt ggcagtattt atataaaaac catggattta gaaaggtata tttagcttta 1860
   ttataataga tacgttacta ttttggaaat atatatattt tcacacctgt agtactcttc 1920
   cccatttcct ctgacactca tgcagaatga gatcaggcat atttgtggtt gacatactct 1980
   agatagcttt tccaacaaat ctttgaaaag caatcttgga aaggaattca ttaactaaat 2040
   ggggtaaaag gtttcttttt ctttgagagt taagtccttc ctcaaaaaat tttcttatgc 2100
   tccataaaat tgaggtagaa tattttcatt attcttgcag ctaagcaaga cagccatatg 2160
   atgcaggtta tgcagtgcct tcatatctaa aacttttata ctgcactttt taaagctttt 2220
   atgttgagga aaggaaaagg gcatttgtct aaacatggat tctgagttgt atatatttcc 2280
   tatcattcta aaaaagtgaa tttgtgaagc aaagttttgc cagatgttaa actttgaatt 2340
   taatatacca gattattaaa atattgtcca ttaactagtt catagatttt aaaagtaaaa 2400
   tacttctgac tgttaaaact atataaagaa aatctcattt gtctaattgc aattaaaaga 2460
   aaaatgttaa aatattaaaa tgaaaataaa agcattactt tccaacaaaa aaaaaaaaaa 2520
   aaaaa 2525
<210> 18
   <211> 6682
   <212> DNA
   <213> Homo sapiens
<400> 18
<210> 19
   <211> 2486
   <212> DNA
   <213> Homo sapiens
<400> 19
   gacggcaaat ggcggacttc gacacctacg acgatcgggc ctacagcagc ttcggcggcg 60
   gcagagggtc ccgcggcagt gctggtggcc atggttcccg tagccagaag gagttgccca 120
   cagagccccc ctacacagca tacgtaggaa atctaccttt caatacggtt cagggcgaca 180
   tagatgctat ctttaaggat ctcagcataa ggagtgtacg gctagtcaga gacaaagaca 240
   cagataaatt taaaggattc tgctatgtag aattcgatga agtggattcc cttaaggaag 300
   ccttgacata cgatggtgca ctgttgggcg atcggtcact tcgtgtggac attgcagaag 360
   gcagaaaaca agataaaggt ggctttggat tcagaaaagg tggaccagat gacagaggct 420
   tcagggatga cttcttaggg ggcaggggag gtagtcgccc aggcgaccgg cgaacaggcc 480
   cccccatggg cagccgcttc agagatggcc ctcccctccg tggatccaac atggatttca 540
   gagaacccac agaagaggaa agagcacaga gaccacgact ccagcttaaa cctcgaacag 600
   tcgcgacgcc cctcaatcaa gtagccaatc ccaactctgc tatcttcggg ggtgccaggc 660
   ctagagagga agtcgttcaa aaggagcaag aatgagcctg cggttgggag ggaatggggc 720
   gtggggggtt agagcaggac cacagcctgg tgagtccccg ggcagccgtc ctgcagccgc 780
   cactcctgcg cctgccattg gcctcctcac agcggaaaca cagcttgtga gtgcatgtca 840
   gctgttaaca agtggttttt agtacattct gggctttgct gtatctatct agtgcctgtt 900
   tgtgcgtttt tttctttctt ccgctgcttc cccattttcc ttctgtcctt tttctcctgc 960
   tccttgtttt cccagcagca catggggttc ctcggaggag cagaggtggc cgccgtgggg 1020
   gggcgtttgg gctgcggtgc tgcgtcattt ttcctttgct ttctctttac tttagacact 1080
   ggcccaactc caggcgtttc ctttcattcc ctcagtgctt ctcttctgac ctgcatgttg 1140
   agttctgtat tgctggggct tccaacaaaa accagagtca ctgacagagg gaacagcaga 1200
   gaccttgttg gtattcagct gtgatggata tagagaatca gaggcacctt gttttcacaa 1260
   ctaggataaa aatatctgca gggtcctttc cattcctatt tagagggagt cctggctcca 1320
   tgaccccctc ccgagtggac tgtccaagca gataggctca cacgagaaac agtgaggctg 1380
   aaaggggggg ctatggaaga gcggtaggga gtccacggag aagatgcagt gaatgcttgc 1440
   atgcattcac acgtgtgtgt gtcccagcta gttcactcct ttcgccgtgc gtggtggagg 1500
   ctggcctctc tggctgggtg cagtgaatgg ccagcgggtt tcttttctgc tgggccaagg 1560
   cgctttgggg gtggaggggg tggtgctggt gctgcactgg gctgactgcg gcgctgacgc 1620
   agcgtttccc cccatccctg ttgcctgtgt gttgtgtgga tctgttccta gtataggcaa 1680
   cataatgaga tactgtgctt cccacctccc cttcagttca gagccaaaat gggtctagaa 1740
   tctggcactt tactcatttc ctttgataaa ttgtactatg cagagctgtc aggaaccttc 1800
   agatagcagt agaggactgc agctgtctag gtctgcggcc acatcttggg gacacactgg 1860
   actgttccca tgtgcagggt tcagcagtta tgtgggagtg ctaggggtta ggcttttgag 1920
   cttgaacgcc tgcgtgtgaa cagatgaaaa atccttcagt acccaagtcc cagtctgtcc 1980
   tatggggagc agtttggggg cggccggcag caggagcctg ggaaagaggc cctcgccagg 2040
   tgatggcagg gccagggtgg cctggggcac ccagcggaat gtgcttagta tttggtcacc 2100
   agccgtcatc ctgggctttt cctactgtgt cttgttacaa ggcctcagca atccacagaa 2160
   ctctctctcc ttccttccac ctgtcagctt ctctgcttct gagataagaa ccatttgtgt 2220
   aacaccaaca cttaacttca gaaagacatg cattatgtgg tgtaatcaaa cccgatgctt 2280
   tcagatgacc tacttacatc ttcaatgtgg ataagataaa gaacaaaaca catgcatcta 2340
   aactgctggg caatccagtt gacttttaaa tgtaagaatg gaattccaaa cacttaacac 2400
   attcagctat atgacagaaa gtaaatctat ggatatggta ttttgtgaat gatcttttaa 2460
   ataaaagaaa accttacgta atattt 2486
<210> 20
   <211> 2234
   <212> DNA
   <213> Homo sapiens
<400> 20
   tggcgcctgc gctggacgac tcggccggta gtggagatgt ccggccggtc taagcgggag      60
   tctcgcggtt ccactcgcgg gaagcgagag tctgagtcgc ggggcagctc cggtcgcgtc     120
   aagcgggagc gagatcggga gcgggagcct gaggcggcga gctcccgggg cagccctgtg     180
   cgcgtgaagc gggagttcga gccggcgagc gcgcgcgagg ccccggcttc tgttgtcccg     240
   tttgtgcggg tgaagcggga gcgcgaggtc gatgaggact cggagcctga gcgggaggtg     300
   cgagcaaaga atggccgagt ggattctgag gaccggagga gccgccactg cccgtacctg 360
   gacaccatta acaggagtgt gctggacttt gactttgaga aactgtgttc tatctccctc 420
   tcacacatca atgcttatgc ctgtctggtg tgtggcaagt actttcaagg ccggggtttg 480
   aagtctcacg cctacattca cagtgtccag tttagccacc atgttttcct caacctccac 540
   accctcaagt tttactgcct tccagacaac tatgagatca tcgattcctc attggaggat 600
   atcacgtatg tgttgaagcc cactttcaca aagcagcaaa ttgcaaactt ggacaagcaa 660
   gccaaattgt cccgggcata tgatggtacc acttacctgc cgggtattgt gggactgaat 720
   aacataaagg ccaatgatta tgccaacgct gtccttcagg ctctatctaa tgttcctcct 780
   ctccggaact actttctgga agaagacaat tataagaaca tcaaacgtcc tccaggggat 840
   atcatgttct tgttggtcca gcgttttgga gagctgatga gaaagctctg gaaccctcga 900
   aatttcaagg cacatgtgtc tccccatgag atgcttcagg cagttgtact ttgcagtaag 960
   aagacttttc agatcaccaa acaaggagat ggcgttgact ttctgtcttg gtttctgaat 1020
   gctctgcact cagctctggg gggcacaaag aagaaaaaga agactattgt gactgatgtt 1080
   ttccaggggt ccatgaggat cttcactaaa aagcttcccc atcctgatct gccagcagaa 1140
   gaaaaagagc agttgctcca taatgacgag taccaggaga caatggtgga gtccactttt 1200
   atgtacctga cgctggacct tcctactgcc cccctctaca aggacgagaa ggagcagctc 1260
   atcattcccc aagtgccact cttcaacatc ctggctaagt tcaatggcat cactgagaag 1320
   gaatataaga cttacaagga gaactttctg aagcgcttcc agcttaccaa gttgcctcca 1380
   tatctaatct tttgtatcaa gagattcact aagaacaact tctttgttga gaagaatcca 1440
   actattgtca atttccctat tacaaatgtg gatctgagag aatacttgtc tgaagaagta 1500
   caagcagtac acaagaatac cacctatgac ctcattgcca acatcgtgca tgacggcaag 1560
   ccctccgagg gctcctaccg gatccacgtg cttcatcatg ggacaggcaa atggtatgaa 1620
   ttacaagacc tccaggtgac tgacatcctt ccccagatga tcacactgtc agaggcttac 1680
   attcagattt ggaagaggcg agataatgat gaaaccaacc agcagggggc ttgaaggagg 1740
   cgtctagggc tttgctccca agggctgtgg ctgatgatgg taaataagaa cacagaagct 1800
   gtagctgaac acaggctggc tggtgggctt cctaggccag cccagcttgt atgggttctg 1860
   gctacaccag agcaccaaga gcccacttgc ctgggatggc cccacactgt cactcagctg 1920
   ttctttgatc atttttttct agattgatgc tcctttctcc catgcattga gctcccatct 1980
   agcttcagca gggcagaacc cttctccaga tgtgtgtaac ttatgtcttg agtatctggg 2040
   agtagttgaa gaacagataa ttccttccaa acatcaagcc ttgggattct tggagcaagc 2100
   agaaagccag taacttcgct ctgttagagg tggaggattt tcctatggtt ccccccattt 2160
   cctgatttgt atttttagat ggattaaata gtctcctgtt tttaaaccaa aaaaaaaaaa 2220
   aaaaaaaaaa aaaa 2234
<210> 21
   <211> 2701
   <212> DNA
   <213> Homo sapiens
<400> 21
   gtgcctggag cgcgcgacag cggcggggcg gggcggcctg gaggctgtgg cgcgcggccg      60
   gcagagggag gggagaggcc actggggccg tgttagtctg ccggtgggga ctcttgcagg     120
   gccgtcccca tgttgcgttt tccgacctgt ttcccatcct tccgggtggt gggagagaag     180
   cagctcccgc aggagattat tttcctggtc tggtcgccca agcgggatct cattgctttg     240
   gccaacacag ctggcgaggt tttacttcat cgactggcaa gttttcatcg agtttggagt     300
   tttccaccaa atgaaaatac aggaaaggag gtgacgtgtc tggcatggag accagatggc     360
   aaacttttgg cctttgctct tgctgatacc aagaaaattg ttttgtgtga tgtagaaaaa     420
   cctgagagct tacactcttt ttctgtggag gctccagttt cctgtatgca ttggatggaa     480
   gtgacagtag aaagcagtgt tctcacatca ttttataatg ctgaggatga atcaaatctt     540
   ctcttaccta aactacctac actgccaaaa aactatagca acacctcaaa aatatttagt     600
   gaagaaaatt ctgatgaaat tattaagctc ttgggagacg tcaggcttaa tattctcgtc     660
   cttggaggaa gctctggatt tattgagctt tatgcttatg gaatgtttaa aattgctcga     720
   gtcacaggga ttgctggtac ttgtcttgca ttatgtttat caagtgattt gaaatcatta     780
   tcagtggtca cagaagtctc taccaatggt gcttcagaag tttcatactt tcagcttgaa     840
   actaatctgt tgtactcttt cttacctgaa gtaactcgga tggccagaaa gtttactcat     900
   atttcagctc tgttacagta tataaatttg tcactaacat gtatgtgtga agcatgggaa     960
   gaaatactaa tgcagatgga ttctcgtctc accaagtttg tgcaggaaaa gaacacaacc    1020
   acatcagtgc aagatgagtt catgcacttg ctattatggg ggaaagcaag tgctgaactt    1080
   cagactctct tgatgaatca gttaacagta aagggcttga aaaagcttgg ccagtctata    1140
   gagtcatcat actccagtat acaaaaattg gtcataagtc atttacagag tggctcggag    1200
   tctttattat accatttgag tgaattgaaa ggaatggctt catggaagca aaaatatgaa    1260
   cctcttggac tagatgctgc aggaatcgaa gaagctataa ctgctgtggg ttcttttata    1320
   ctcaaggcaa atgaacttct tcaagttata gatagtagta tgaaaaactt caaagcattt    1380
   tttcggtggc tttatgtggc aatgttgaga atgacagaag accatgtgct tcccgagctg    1440
   aataagatga ctcagaaaga tatcacattt gttgctgaat ttcttactga acatttcaat    1500
   gaggctccag acctttataa tcgaaaagga aaatacttta acgttgaaag agttggtcag    1560
   tacttgaaag atgaagatga tgatcttgtg tcacccccta acacagaagg aaaccagtgg    1620
   tatgactttc ttcaaaatag cagccacctt aaagaaagtc ctttgctgtt tccttattat    1680
   cctcgaaaat cattgcattt tgtgaaaagg cggatggaga atattattga tcagtgtttg     1740
   caaaagccag cagatgtaat tggaaaatcg atgaatcaag caatctgtat tccattgtat     1800
   agagatacca gaagtgagga ttctacacgt agattgttca aatttccttt tctgtggaat     1860
   aataaaactt caaatctaca ttatcttctt tttactattc tagaagattc actttataaa     1920
   atgtgcatct taaggagaca tactgatatt tctcaatctg tgagtaatgg actaattgct     1980
   attaaatttg ggagctttac atatgccaca acagaaaaag tcagaagaag catctacagt     2040
   tgtttagatg cacagtttta tgatgatgaa actgtaacag tagttcttaa agacactgta     2100
   ggacgtgaag gaagagatag actcttggtc cagctgcctt tgtctttagt atataacagt     2160
   gaagattctg cagaatatca gttcactggg acttattcta caaggctaga tgaacagtgt     2220
   agtgctattc ccacccgtac catgcatttt gagaagcact ggagattact ggaaagtatg     2280
   aaagcacagt atgttgctgg gaatggtttt cgaaaagtgt cctgtgtgtt aagctcaaat     2340
   cttcgtcatg tgagagtatt tgaaatggac atagatgatg aatgggagct cgatgagtct     2400
   tcagatgaag aggaggaggc cagtaataag cctgtaaaaa taaaggaaga agtgttgtcg     2460
   gagtcagagg cagagaacca acaagctggt gctgccgctt tagctccaga gatagtcatt     2520
   aaagtggaaa aacttgaccc tgagctagac tcctaatcta gcttgccatt attgtgtgtg     2580
   taattatggc caaaaggaca taggagatgg actaagatgt cttggaccac ctttgtgtaa     2640
   caaagaaata aacagtaaat tttatttttt caaaaaaaaa aaaaaaaaaa aaaaaaaaaa     2700
   a                                                                     2701
<210> 22
   <211> 2022
   <212> DNA
   <213> Homo sapiens
<400> 22
   attccccaac ctgatagccc tccgcgacgc attacgcacc gcggacagct ggagaggccg        60
   aggcgctctc gctttgattt cggcgcctcc gccctcgcgg ggagagattg gctgcggccg       120
   cgggacgggg tagtgagcgc gtcacttcct gccgctgcca ggcgcgtcct cccgcgcgct       180
   atgacggcca gcgcacagcc gcgcgggcgg cggccaggag tcggagtcgg agtcgtggtg       240
   accagctgca agcatccgcg ttgcgtcctc ctggggaaga ggaaaggctc ggttggagct       300
   ggcagtttcc aactccctgg aggtcatctg gagttcggtg aaacctggga agaatgtgct       360
   caaagggaaa cctgggaaga agcagctctt cacctgaaaa atgttcactt tgcctcagtt       420
   gtgaattctt tcattgagaa ggagaattac cattatgtta ctatattaat gaaaggagaa       480
   gtggatgtga ctcatgattc agaaccaaag aatgtagagc ctgaaaaaaa tgaaagttgg       540
   gagtgggttc cttgggaaga actacctccc ctggaccagc ttttctgggg actgcgttgt       600
   ttaaaagaac aaggctatga tccatttaaa gaagatctga accatctggt gggatacaaa       660
   ggaaatcatc tctaggtggc cgagaagatt tgattttctt taaaaagaca agaataaggt      720
   ctggttaggg aatgaaaaat gtatacattt cggaacaact ccattttatc taaaaaagtt      780
   cttgtgattg ccagtttatt tgcagtctct taatgtatcc cccactcttt cagccagtac      840
   ttgagaaaat ttttctgaaa tatgtcattg aattgtattc cagacacaga atacatgata      900
   aatactgata ttatgggtaa tctgctttcc atatttacct atgatattta ctgtgcagtt      960
   tgtcattact agcttgcatg gagtaggatg cagtcaaatt tgccttagtg tttctgttca     1020
   aatagagacc tgaattcaaa tattgtagtt taggttcaaa cagagtatgg ctgttgcaaa     1080
   atttaccaaa tttgttgatt acctctttta ttaaagaaat gttggggaga gggtaatata     1140
   ttgtgacaat ttttgcaact tacagggaat aggacaggtc attagggtgt atttcccagg     1200
   tttccaattg agaatctaaa ccaagagaag tgagacaaac catctaatat ctgtatcatg     1260
   ctattaatag gctgtgatgt tgagtttctc acttactctt tctgaggtcc agggtcctta     1320
   tatgaagaga ttcatatata tgaagagatt cgtctagagc agtggttgtc aaagtgtggt     1380
   ctgggggggc ctcaagagcc tttcaagggt attctaggtc aaagctgttt ttataacact     1440
   aagacattat ttcactattt atagcttttc ctttttttta tttgagaagg agtcttgctc     1500
   tgttgcccac attggagtgc agtggcacga tctcagctca ttgcagcctc cgccacctgg     1560
   gttcaagcaa ttctcctacc tcagcttccc gagtagctgg gattataggc acccaccacc     1620
   acacctggct aatttttgta tttttagtgg agacggggtt tcaccatgtt ggccaggctg     1680
   gtctggaact cctgagctca agtgatctgc ccatctcagc ctcccaaagt gctgggatta     1740
   caggtatgag ccacaacgcc cagccatcat ttatcttttt cactcatttt ttcatgagta     1800
   tgcagaggag ttttcaagag gctatctggt gtgatattgt aataggctga atgcaaaagc     1860
   agatacaaga ttccagcttt cgtctgtcat cagacattga agagatttgg agaaatgtaa     1920
   agcagcagta ctcttctcac taatttttat tgtggttagt tagaaaaatg gttattttta     1980
   gtgggtttta atatgtgaaa aaaataaaat tagctatttg cc                        2022
<210> 23
   <211> 1278
   <212> DNA
   <213> Homo sapiens
<400> 23
   gctgaggggc agcggcttag gctccggcgt ctgcaggggt cgccgagcta acccgtggct      60
   aggcgagtgg ggcggggcgg ccggcaccat gtcgaggcag gcgaaccgtg gcaccgagag     120
   caagaaaatg agctctgagc tcttcaccct gacctatggt gccctggtca cccagctatg     180
   taaggactat gaaaatgatg aagatgtgaa taaacagctg gacaaaatgg gctttaacat     240
   tggagtccgg ctgattgaag atttcttggc tcggtcaaat gttgggaggt gccatgactt     300
   tcgggaaact gcggatgtca ttgccaaggt ggcgttcaag atgtacttgg gcatcactcc     360
   aagcattact aattggagcc cagctggtga tgaattctcc ctcattttgg aaaataaccc     420
   cttggtggac tttgtggaac ttcctgataa ccactcatcc cttatttatt ccaatctctt     480
   gtgtggggtg ttgcggggag ctttggagat ggtccagatg gctgtggagg ccaagtttgt     540
   ccaggacacc ctgaaaggag acggtgtgac agaaatccgg atgagattca tcaggcggat     600
   tgaggacaat cttccagctg gagaggaata accatcccta caactcgagg atagccatca     660
   ggagcactgt tggaatcagc aggcctctgt gctccctctg ccctccagaa ctcagtgact     720
   cttgaacatg gatgttatat attcttataa cctgtttcca ttctccattc aaataaagag     780
   cagactgcga tatagtccat ttaccccatg tgtgcacatt caggagcgac agtctctgcc     840
   cccattccct tgagaggggc tggatgtaat cacctttggt tggactagaa agagctcaaa     900
   ccattttaca ttcctgtttg aatttttcca aagcaaaact cactttgacc ccattaagag     960
   gcaagcctgg cacatctatc cctgggcctt tagaaagcca tttgcctcaa atggctatag    1020
   ggttgtgggg tggagggagg aagggctggg agggagtggg gaggaattgc tagctgtagt    1080
   gtgacacatt gtagtgtttg ccaggaaagg agccagtcat gccggaaaca ctgacttctg    1140
   ggaagccacc caggtctcat tcctccctgc tgttggaggc aacatctcct ctttttacag    1200
   agggtacatc cttttttctt acaaattctt caataaagac acattcttga gtgaaatccc    1260
   aaaaaaaaaa aaaaaaaa                                                   1278
<210> 24
   <211> 2880
   <212> DNA
   <213> Homo sapiens
<400> 24
   gtaggccatg gagccgagca gatccgggtc ttcgggtggc cgcaatgtcc caagtggacg    60
   acacccacct agtcccgagg acaaccgaca aagtccttaa tgggcccaga aggaggacgg   120
   agtcccagag aactacaact cccatcaagc acttggggga tcgccgcggc tgagggagag   180
   actatggctt aggaaccacc attcgcgtga ggctctgctc cccgcgcctg cgcagagtgc   240
   agggccacgt cgcttttgct gtaccgggga ccacgcgtct catccatggc ttccgcggac   300
   tcgcgccggg tggcagatgg cggcggtgcc gggggcacct tccagcccta cctagacacc   360
   ttgcggcagg agctgcagca gacggaccca acgctgttgt cagtagtggt ggcggttctt   420
   gcggtgctgc tgacgctagt cttctggaag ttaatccgga gcagaaggag cagtcagaga   480
   gctgttcttc ttgttggcct ttgtgattcc gggaaaacgt tgctctttgt caggttgtta   540
   acaggccttt atagagacac tcagacgtcc attactgaca gctgtgctgt atacagagtc   600
   aacaataaca ggggcaatag tctgaccttg attgaccttc ccggccatga gagtttgagg   660
   cttcagttct tagagcggtt taagtcttca gccagggcta ttgtgtttgt tgtggatagt   720
   gcagcattcc agcgagaggt gaaagatgtg gctgagtttc tgtatcaagt cctcattgac   780
   agtatgggtc tgaagaatac accatcattc ttaatagcct gcaataagca agatattgca 840
   atggcaaaat cagcaaagtt aattcaacag cagctggaga aagaactcaa caccttacga 900
   gttacccgtt ctgctgcccc cagcacactg gacagttcca gcactgcccc tgctcagctg 960
   gggaagaaag gcaaagagtt tgaattctca cagttgcccc tcaaagtgga gttcctggag 1020
   tgcagtgcca agggtggaag aggggacgtg ggctctgctg acatccagga cttggagaaa 1080
   tggctggcta aaattgcctg agaggcagct ctaaagcaca agacctggat gtgtgacaca 1140
   cagttttgga aaaaggtctg tggtagtctg gagttgatga ggaaggggta caagatgtgg 1200
   ttagaaacat ttctttgttc tggaaacaaa gtactgttga aaccagcttg gaattttttt 1260
   tttttttttt tttaagttca gttctccctt atggctgcct ttcaaacaag taccttttat 1320
   ctgatgcctg tatcttccct ttgttaaggt gtaacttgat gtagggtcaa ggtttttgtg 1380
   acaacaggca gactccacac agagaggata tgatgagaat atggccatca cctgaaaagt 1440
   tttcttatct tctgtgcttt tggtccctgg aaacaaatcc gcctatgtat gaagctagtt 1500
   gatttccagt tgcactattt ccagttgcct ctgaagttca caggcaatac attgtctagt 1560
   cctttgcgaa tttctctgat ttgtgggcac agttatgaag tttccccaca tgtgaagaca 1620
   ggtacaaaat agcagagcca agcagacagt gggtctattc ttcattagct cagtgacttg 1680
   tccacactcg tcttagcact tacgtttcaa aagcttgtca caaacccttg gagtcattcc 1740
   cagataatag aactggaaat gataaatccc ctaatgccaa gggtctagtg tgttcttagt 1800
   ggttatactg ggaagtgtgt ggagatttag gtgctgctct gctgctctgg atggctgaag 1860
   gctcctgggc catcttcatg tgctgcttga agagctccta ttttgtactc ctggctagaa 1920
   tgctgtggaa caaatacaaa gtgaaaaaag ttctctgtag atttctgaag tgcatattca 1980
   ttgatgccaa gaaaaaaaaa aagttgcctt tttgaagtga tgttttttgc tgtcttctta 2040
   aacacaaggc ttttttgaat gattagtata tttcatggta aagaaaacag cctgtctggc 2100
   tcaaagcaat taaatagaat gtaatggtga gtacaaatga gtgcacatgt caggactcag 2160
   gtctaactcc ttgtctcctg agcctaaaga ttgcaacata cacaagaaca cactcctatt 2220
   cctaccccac acactcaggg acaagcccaa ctaaagctta caaggagacc agggtggctc 2280
   tgtccagggg agaagccagt tatggaacag tgcattgaga gccatggtag gagaggccca 2340
   cagttctctg gagcatgcag caggggcacc ccacctggcc ttgaggatca gggggagtca 2400
   aaggataaag catggggctg atgacgtctg agggagtgtg atcctccatg tatggcctct 2460
   gcctgctgtc tcacatgtcc cttctggtgg tcacttgggc tctaggagta tacgtcacct 2520
   cagaccatct ggcagaaata ctccaggctc ctaccccaaa gcacatgtca gccttgctgc 2580
   tggagcacga agacaatgta aatgaaacat gaaatggagg agttgtgaga ccctgaccct 2640
   gagtccttac ttgaaagctg ctgctggtgt tctgagtgtc ttttggactc ttatttcttg 2700
   cccttttcct tattaggcaa gcagtaactt aggaagtagg taagagcaat aaatgtgaca 2760
   tgttatgtca tcatagtagg agctcatggg aataaaagtc agtggcttga tgcttctgtt 2820
   agaggcaaaa aaaaaaaaaa aaaaaaaaaa aaaaaaaaaa aaaaaaaaaa aaaaaaaaaa 2880
<210> 25
   <211> 3101
   <212> DNA
   <213> Homo sapiens
<400> 25
   acactctggc ccggttctcg gtggtgcggg agcgggcggg agcagcggcc gctctggtcg 60
   gcggacgtgc tgccgagtag tcccggaagc gaagcagcga tggcggagag tccgactgag 120
   gaggcggcaa cggcgggcgc cggggcggcg ggccccgggg cgagcagcgt tgctggtgtt 180
   gttggcgtta gcggcagcgg cggcgggttc gggccgcctt tcctgccgga tgtgtgggcg 240
   gcggcggcgg cagcgggcgg ggccgggggc ccggggagcg gcctggctcc gctgcccggg 300
   ctcccgccct cagccgctgc ccacggggcc gcgctgctta gccactggga ccccacgctc 360
   agctccgact gggacggcga gcgcaccgcg ccgcagtgtc tactccggat caagcgggat 420
   atcatgtcca tttataagga gcctcctcca ggaatgttcg ttgtacctga tactgttgac 480
   atgactaaga ttcatgcatt gatcacaggc ccatttgaca ctccttatga agggggtttc 540
   ttcctgttcg tgtttcggtg tccgcccgac tatcccatcc acccacctcg ggtcaaactg 600
   atgacaacgg gcaataacac agtgaggttt aaccccaact tctaccgcaa tgggaaagtc 660
   tgcttgagta ttctaggtac atggactgga cctgcctgga gcccagccca gagcatctcc 720
   tcagtgctca tctctatcca gtccctgatg actgagaacc cctatcacaa tgagcccggc 780
   tttgaacagg agagacatcc aggagacagc aaaaactata atgaatgtat ccggcacgag 840
   accatcagag ttgcagtctg tgacatgatg gaaggaaagt gtccctgtcc tgaaccccta 900
   cgaggggtga tggagaagtc ctttctggag tattacgact tctacgaggt ggcctgcaaa 960
   gatcgcctgc accttcaagg ccaaactatg caggaccctt ttggagagaa gcggggccac 1020
   tttgactacc agtccctctt gatgcgcctg ggactgatac gtcagaaagt gctggagagg 1080
   ctccataatg agaatgcaga aatggactct gatagcagtt catctgggac agagacagac 1140
   cttcatggga gcctgagggt ttagaccctg ctcccatctc cccttccccc actcaagagt 1200
   cccagcagaa tcccttcccc ccaccccagg gatggagagg cactgtgtat ctccctccag 1260
   actcgaagtc atcctgcaag atggcaagaa ccaagcaagc tccgatccca gggtgtggga 1320
   gtgggggcct gttcccggtc tgacctcctt ggcactggag catctggggc ttcgttcatc 1380
   cattcatccc gtatcagggg ccaaggtacc tttacaggag cacctagagc gagggccttt 1440
   ggcaaaaaca aaacaaccaa cacacctctc cacagggcca gctccttagg gataagtgga 1500
   agatggaaat tgcaattcca agagggagtg tgcccaaatg atttatgggg atacctggaa 1560
   gggagcttgg ggtgggggct gtctgtgaca cttaagcagt ctgggtggtt gtctatttgt 1620
   ctgtcttcag tcttgaagca gggcttccca atgccctttt cctccctgcc ttccttcccc      1680
   cattatttcc cacaggccag cataattttg tttttcctaa tttatagtca ctgttctaga      1740
   cagaccaaag agaaggaaca gtggtggagt ctaggctgct gatcagtaag ctttacctag      1800
   cacctgagca cctttctccc ctcccctctt tcctcaccct tttctagatg taagacagaa      1860
   agtaaatgtg actgggactt aaccaaggtc ttggtaaagc ctgcatggca ccgtaagaag      1920
   ctgaaaatac tgtttgttcc cgcaatcact gatttgaaaa gttcccaaca caggcagctg      1980
   ctgtgtatat gggattagag ccactacata gaatagtctc ttacagattt tcataaatac      2040
   tagtcacaat aagggtattt ttcttggggg tggagtaagg gggagactga tgctagtcct      2100
   tgttgtattt tgttgggctg tccttgtgta ttttcacccc agcctgtagt cctcctcact      2160
   tcaaccccag ggatttttgg ggagcaaggg tagccaatgg cagagggggt tggggctggg      2220
   actctggagg ctcctcccct tctttctctt ccttccgcct cccccgtgcc cccagctgct      2280
   cttgtcactg tctctgatgg gtatttgcct ggctttgttg cttctctatc tgtatttagc      2340
   tgcagtgatc ctttagctgg ttggctcaga aaaaaaaaaa tgtgctttag gtgccctgta      2400
   atcctgggca tcaagggaat ccatccttcc cctttttgat atgttctccc cgtacttcca      2460
   gatttattgt tatggctccc agtgggtatt ggcgattctt gtgatgcagg gcctcagtca      2520
   gtgtccagcc atgcataagg gagaggatag tgtgtacctg ccctgccctc tgctatgaag      2580
   gtctctgcct tgtggatcat gggactcccc ttggaggatc tgtgcaaagg ggggctgggc      2640
   acaaaggaga atgtcctatt tgggagggca ggaagcaaag gaactggaca gggattggtg      2700
   ggcttgggga acggaagttt atcttggata cccttgaaga ggctgggtct cttcacatga      2760
   agatcgaaaa gggaccctgc ttccaatttc cctcttccat tcctcgagct actccagggc      2820
   ttagaagaat gctcttggtc tgtgggtcca gtgttgtctg tcatccattt aagtgttccc      2880
   actttcaagt gacaatcctc tccttggccc tgccataggg cagagcatgt ctggcatagc      2940
   agcctgactt ttatgcccta atcttgagtt gaggaaatat atgcacagga gtcaaagaga      3000
   tgtctttata tctgactgta tataaatgaa gtttttttgt tttttttgtt ttcctttttg      3060
   gtgcaataaa gtttgttttg gcagaaggag aaaaaaaaaa a                          3101
<210> 26
   <211> 2213
   <212> DNA
   <213> Homo sapiens
<400> 26
   ataggcctcc tcctggcgcg cctccgcacc gcattggtgg accggccccc agcccgtcca      60
   tgggcgttgt ggccgccacc gcaggttccg cctcccgttc cctccgccct caacgtacgt     120
   cgcaccgcct ctctgtagcc gcccgcggag catcgcagcc ggcccgggcc cccgccagcc     180
   tccctcctcg cgtccctcgg tgtcctccgc gggccggcgc gatgcggctg ggcccgagga     240
   ccgcggcgtt ggggctgctg ctgctgtgcg ccgccgcggc cggcgccggc aaggccgagg 300
   agctgcacta cccgctgggc gagcgccgca gcgactacga ccgcgaggcg ctgctgggcg 360
   tccaggaaga tgtggatgaa tatgttaaac tcggccacga agagcagcaa aaaagactgc 420
   aggcgatcat aaagaaaatc gacttggact cagatggctt tctcactgaa agtgaactca 480
   gttcatggat tcagatgtct tttaagcatt atgctatgca agaagcaaaa caacagtttg 540
   ttgaatatga taaaaacagt gatgatactg tgacttggga tgaatataac attcagatgt 600
   atgatcgtgt gattgacttt gatgagaaca ctgctctgga tgatgcagaa gaggagtcct 660
   ttaggaagct tcacttaaag gacaagaagc gatttgaaaa agctaaccag gattcaggtc 720
   ccggtttgag tcttgaagaa tttattgctt ttgagcatcc tgaagaagtt gattatatga 780
   cggaatttgt cattcaagaa gctttagaag aacatgacaa aaatggtgat ggatttgtta 840
   gtttggaaga atttcttggt gattacaggt gggatccaac tgcaaatgaa gatccagaat 900
   ggatacttgt tgagaaagac agattcgtga atgattatga caaagataac gatggcaggc 960
   ttgatcccca agagctgtta ccttgggtag tacctaataa tcagggcatt gcacaagagg 1020
   aggcgcttca tctaattgat gaaatggatt tgaatggtga caaaaagctc tctgaagaag 1080
   agattctgga aaacccggac ttgtttctca ccagtgaagc cacagattat ggcagacagc 1140
   tccatgatga ctatttctat catgatgagc tttaatctct gagcctgtct cagtagagta 1200
   ctggctcctt ttataatttg ttaccagctt tacttttgtg ataaaatatt gatgttgtat 1260
   tttacactct taagtcttaa ccacagtcag aattatctta atgtagatta taattttggt 1320
   cttttaggaa aaaaaaacaa aaatctgata tttatttcaa aatgtattga agcaacaaaa 1380
   tattaatatt gtgccatatg acaacaaagt ctttcctaaa tactccatct gtttagtact 1440
   gtattgtgga atatttgagt tctatttcca tacttgaaaa catggaggat tttagagatg 1500
   cctgaacaat attatttaag tagtatgtga ccgagctata aatttttgtt tttgttctaa 1560
   gtagatttaa tttgggaact gacaggacaa tgtttttagg tttagcattt tgtttaaaaa 1620
   cctttaaaga aacctttaga aggacttaga cctcacatat taatgttgag aagttctgct 1680
   taattttaaa atggtttcta taaagggttt tattgtatga aatagaactt tatatttttg 1740
   catatgtata gatagtaatt atatttaatg tataactata gcattatggt gagtggaatt 1800
   tgacattgtc caaacctttt tcatttttga gtgattaaaa atgaaatgtc ctttgtatat 1860
   gtttgtttgc ttttctttaa ctttcagata aatattgaat ttagcatagg ttttgtggta 1920
   ttgtacatta tcttgcctca tccattcctt tatgtggata gttcccaaaa ctgtcaaatt 1980
   attcttgctg cattttctta tttttttttt aaataagaga atcatgttat aatataattg 2040
   aatgtgcacc tgacacattt taataattgg tgttgtagca aatgtggttt tgctctttgg 2100
   ggctataaat aatcttgagt aacgcaggtt ggaattacca atagagctgg tcagttatca 2160
   acagtagagc tcagtatcaa ttttactgaa attaaaatca tacttgataa gca 2213
<210> 27
   <211> 1097
   <212> DNA
   <213> Homo sapiens
<400> 27
   ctctgtcctc attgcgccca gacgggccgg cccagagctc ccgggtcgtc tttcgtgtgg 60
   ccgcgagaca ctcttgcact cctgtaatga gcctggcact gtgatgaaac acttttcccg 120
   tgtcgtttga gtgcatcttc tcaacaaccc taggagggtt cttgaagctt ttgagattaa 180
   caatggcagg aaaatcatca ctttttaaag taattctcct tggagatggt ggagttggga 240
   agagttcact tatgaacaga tatgtaacta ataagtttga tacccagctc ttccatacaa 300
   taggtgtgga atttttaaat aaagatttgg aagtggatgg acattttgtt accatgcaga 360
   tttgggacac ggcaggtcag gagcgattcc gaagcctgag gacaccattt tacagaggtt 420
   ctgactgctg cctgcttact tttagtgtcg atgattcaca aagcttccag aacttaagta 480
   actggaagaa agaattcata tattatgcag atgtgaaaga gcctgagagc tttccttttg 540
   tgattctggg taacaagatt gacataagcg aacggcaggt gtctacagaa gaagcccaag 600
   cttggtgcag ggacaacggc gactatcctt attttgaaac aagtgcaaaa gatgccacaa 660
   atgtggcagc agcctttgag gaagcggttc gaagagttct tgctaccgag gataggtcag 720
   atcatttgat tcagacagac acagtcaatc ttcaccgaaa gcccaagcct agctcatctt 780
   gctgttgatt gttagattgt tgatgcattc taaccaactc acacatatac acaaaatcaa 840
   catggggatg gagaagagaa ttagcgtttg cagcagtgta tcatctacta ataaaattaa 900
   actaatgttg ctgcttcatt agttggtggg agaagggaca catccactct tggaggaata 960
   tatttactca ataatggcac cttacattta taaattgtaa cagttgtcta ataacgtttc 1020
   tttaatttaa atatgtaagt tgcagagcta ataaatgaaa tgaccaagac tttaattata 1080
   aaaaaaaaaa aaaaaaa 1097
<210> 28
   <211> 1495
   <212> DNA
   <213> Homo sapiens
<400> 28
   gcttcttccc tggtaggttc cggaagagcc gcgcactcct tgggcgttaa gggttcgcgc 60
   gccgcagggt cgtttcagcc gagcacttgg cgtcccctcg agctcgagat ctgtgaacag 120
   ccaccatgtc gatcttcacc cccaccaacc agatccgact gaccaatgtg gccgtggtgc 180
   ggatgaagcg gggagggaag cgcttcgaaa tcgcctgcta taaaaacaag gtcgtcggct 240
   ggcggagtgg cgtggaaaaa gaccttgatg aagttctgca gacccattca gtgtttgtaa 300
   atgtttccaa aggtcaggtt gccaagaagg aagacctcat cagtgcattt gggacagacg 360
   accagactga aatctgcaag cagattttga ctaaaggaga agttcaagtg tcagataaag 420
   aacggcacac acagctggag cagatgttta gggatatcgc caccattgtg gcagacaagt 480
   gtgtgaaccc agaaacaaag agaccttaca ccgttatcct catcgagaga gccatgaagg 540
   acatccacta ctccgtgaaa cccaacaaga gcacaaagca acaggctttg gaagtgataa 600
   agcagctgaa agagaagatg aagatagagc gggcccacat gcgattgcgc ttcatcctgc 660
   cagtgaacga agggaagaag ctgaaggaga agctgaagcc actgatgaag gtggtggaga 720
   gtgaggacta cagccagcag ctggagatcg tgtgcctcat cgacccaggc tgcttcagag 780
   aaattgatga gctaataaaa aaggaaacga aaggcagggg ttctctggaa gtgctcagtc 840
   tgaaggacgt ggaggaaggc gatgagaagt ttgaatgaca ccgcccggct cctcaactgg 900
   agcacgaccg aggacgcttg ttcctcacag cagcagctcg ttctgtgacc tgccaaacgc 960
   cctgctcacg cgacgtgcca ctttccatct tgtgttaaac atttacccag gtacctgggt 1020
   atttttgttg tcaattgggg tttccagcaa aaatgaaaaa taacctaaaa tacagagtcc 1080
   agaacagctg ctcactgctg cgtctgcctt tctagttcca ggggaccaga gacagcattg 1140
   gtggataaga aggtagagtt agtccatgac agatcattgg agaggggtct gaataacaaa 1200
   gggggtacgc ctgctggaaa gaagatgggg tgtttctgaa taatgaagtg caggtatggg 1260
   gtgtgagcat ggagagaaga gttcctgggt ccctcccaat agatttataa tgactaggga 1320
   gaatttgact ttctaatttt caaccaacat gctaccaaaa ctgacttaga ttattcttgg 1380
   gaaaatatat acagtcattt aatactaatt cttaaaggtt tataatatat gttagtatag 1440
   ttaaaattct atgtaatcaa taaaacttat ttttactaaa aaaaaaaaaa aaaaa 1495
<210> 29
   <211> 10835
   <212> DNA
   <213> Homo sapiens
<400> 29
   caaaaatcag tctgatctcg ggaaacctgg agaaatttat tttctgtact ctaatgttct      60
   ttcattttgg tgaccatcaa ggtgctggga gaggaattag atggctgtaa ttcaaagtta     120
   atggaattag atgcagcagt acagaaattc ttggaacaga atggccaact gggtaagcca     180
   ctggccaaga agataggaaa actgactgaa cttcaccagc agaccattag acaagctgag     240
   aatcggctct ccaagctcaa tcaggcagca tcacatttag aagaatacaa tgaaatgctt     300
   gaattaattt tgaagtggat tgaaaaagct aaagtcttgg ctcatggaac tattgcatgg     360
   aattctgcaa gccagcttcg ggaacaatat attttgcatc agaccctgct agaagaatcc     420
   aaagaaattg acagtgagct ggaagcaatg actgagaaat tacagtacct cactagcgtg     480
   tactgtacag aaaaaatgtc tcagcaagtg gcagaactgg gacgggagac tgaggagttg     540
   cgacagatga tcaaaattcg tttgcagaac ctccaagatg cagctaagga tatgaaaaaa     600
   tttgaagcag agttgaaaaa gttacaagct gccttggagc aagcccaggc aacactgact 660
   tctccagaag ttggacgtct cagtctcaag gagcagctct ctcatcggca gcatttgttg 720
   tctgagatgg agtcactgaa gccgaaggtg caagcagtgc agctctgcca gagtgccctc 780
   cggatccccg aggatgtggt tgccagctta cctctctgtc atgctgctct gcggctgcag 840
   gaagaggcca gccggctgca gcacaccgcc atccagcagt gtaacatcat gcaggcagct 900
   gtggtacaat atgaacaata tgagcaagaa atgaaacatc tccagcaact gatagaagga 960
   gctcacagag agattgagga taaacctgtt gccaccagta acatacagga gctgcaggct 1020
   cagatttctc ggcatgagga gctggcgcag aaaattaagg gctaccagga gcagatcgct 1080
   tctttgaatt ccaagtgcaa gatgctgacg atgaaagcca agcacgccac catgctgctg 1140
   acggtgaccg aggtcgaggg gctggcggaa gggacagagg acctggatgg ggagctcctc 1200
   cccacgcctt cggcccaccc ctctgtggtc atgatgactg caggtcgctg tcacactttg 1260
   ctgtcaccgg tcactgagga gtctggggag gagggaacca acagtgagat ttcctctcca 1320
   cctgcctgtc gctccccttc acctgtggct aatacagatg cttctgttaa ccaggacatt 1380
   gcatattacc aagccttgtc tgctgagagg ttgcagacag atgctgcaaa aattcacccc 1440
   agcacatccg catcccagga gttctatgaa ccgggattgg agccatccgc tactgccaaa 1500
   ctgggtgatt tgcagcgttc ttgggaaacc ttaaagaatg tgatcagtga gaagcagcgc 1560
   acactctatg aagctttgga acgccagcag aagtaccagg actccctcca gtccatctct 1620
   acgaagatgg aggccattga gctgaaactc agtgagagcc cagagcctgg caggagtcca 1680
   gaaagccaga tggctgaaca tcaggcattg atggatgaga ttctcatgct ccaggatgaa 1740
   atcaatgagc tccagtcctc tctcgcagag gagctggtat ccgagtcttg tgaggccgac 1800
   cctgcggagc agctggcctt gcagtccacg ctcactgtct tagccgagcg aatgtccacc 1860
   atcaggatga aagcctcggg gaaacggcag cttttggagg agaagttgaa tgatcagctg 1920
   gaggaacaaa ggcaggaaca ggccctgcag aggtatcgct gtgaagccga tgagctggac 1980
   agctggctct tgagtaccaa ggccactctg gacactgcgc tgagtccacc caaggagccc 2040
   atggacatgg aggcccagct tatggactgc cagaatatgc tggtggaaat agagcagaag 2100
   gtggtggctt tatcagaact gtcagtccac aatgagaacc tgctgctgga gggcaaagct 2160
   cacaccaagg acgaggccga gcagctggct ggaaagctga gaaggctcaa ggggagcctg 2220
   ctggagctgc agagagccct gcatgataag cagctcaaca tgcagggaac agcacaggag 2280
   aaggaggaga gcgatgttga cctaacagcc acgcagagcc ccggcgtcca ggaatggctg 2340
   gcccaagctc gcaccacatg gacccagcag cggcagagca gtctccagca acaaaaagag 2400
   ttagaacagg aattagccga gcagaagagt ctccttcgct cagtagccag tcgtggagag 2460
   gagattctaa ttcaacattc ggcggcagag acctctggtg atgctggcga aaaacctgat 2520
   gtgttatccc aggagttggg gatggaaggg gagaaatcat ccgctgaaga ccagatgaga 2580
   atgaaatggg aaagcctaca tcaagaattt agtaccaagc agaaactact acagaatgtt 2640
   ctggaacagg aacaagagca agtgctttat agcaggccaa atcgactctt gtctggtgtg 2700
   ccactgtaca aaggggacgt gccaacccaa gataaatctg cagttacatc tttgctggat 2760
   ggactgaacc aagccttcga ggaggtttca tcccagagtg gaggggcaaa gaggcagagt 2820
   atacacttgg agcagaagtt gtatgatgga gtctcagcca cctctacttg gttggatgac 2880
   gttgaagaac gtttatttgt tgccacagca cttttaccag aagaaacaga gacttgtctc 2940
   ttcaaccaag agattcttgc caaagacatt aaggaaatgt ctgaagaaat ggataagaac 3000
   aaaaacttgt tttcccaagc ttttccagag aatggtgata atcgagatgt tattgaagat 3060
   actttgggtt gtcttttggg caggttatcc ttgctagact cagtagtgaa tcaacgatgt 3120
   catcagatga aagaaagact tcagcaaata ctaaatttcc agaatgatct gaaagtgctg 3180
   tttacatcac tggctgacaa caaatacatc attctgcaaa aactggcaaa tgtgtttgaa 3240
   cagcccgtag cagaacaaat agaggcaata caacaggctg aagatggact caaagaattt 3300
   gatgcaggaa tcattgaatt aaagaggcgt ggtgacaagc tacaggtcga gcagccgtcc 3360
   atgcaagaac tctccaagct ccaggacatg tatgatgagc tgatgatgat cattggctcc 3420
   cggaggagtg gtctgaatca gaaccttaca ctcaagagtc agtatgagag ggccctacaa 3480
   gatctggctg acctgctaga aactggtcag gagaagatgg caggagacca gaaaatcatc 3540
   gtgtcttcca aagaggaaat ccagcaacta cttgacaaac ataaggaata ctttcagggc 3600
   ctggaatctc atatgatctt gactgaaaca ctcttcagaa agataatcag ctttgcagtc 3660
   caaaaggaaa cccagttcca tacagagctg atggctcagg cttctgctgt actgaaacgg 3720
   gctcacaaga ggggtgtgga gctggagtac attctagaga cgtggtccca tctggatgag 3780
   gaccagcagg agctcagcag acagctggag gtggtggaaa gcagcatccc aagcgtgggt 3840
   ctggtggagg agaacgagga caggcttatt gaccgcataa cactctacca gcatttaaaa 3900
   tctagcctta atgaatacca gcccaaatta tatcaagtat tagatgatgg gaaacgactt 3960
   ctgatatcca tcagctgctc agatctagaa agccaactaa atcaacttgg agagtgctgg 4020
   ctaagtaaca ccaataaaat gtctaaggaa cttcacagac tggaaacaat attgaaacac 4080
   tggaccagat atcaaagtga atctgcagat ctaattcact ggttacaatc tgcaaaagac 4140
   cggctagaat tttggactca gcaatctgtg acagtcccac aagagctgga aatggtccgt 4200
   gatcatctaa atgctttcct ggagttttct aaagaagtgg atgcccaatc ttccctgaaa 4260
   tcatctgttc tgagtactgg aaatcagctc cttcgactaa aaaaggtgga cacagccacg 4320
   ctgcgctctg agctgtcgcg cattgatagc cagtggactg acctgctaac caatatccca 4380
   gccgtccagg agaagctcca ccagctccag atggataaac tgccttcccg ccatgccatt 4440
   tctgaagtca tgagttggat ttctctaatg gaaaatgtta ttcagaagga tgaagataat 4500
   attaaaaatt ccataggtta caaggcaatt catgaatacc ttcagaaata taagggtttt 4560
   aagatagaca ttaactgtaa acagctgaca gtggattttg tgaaccagtc cgtgctacaa 4620
   atcagcagtc aggatgtgga aagtaagcgt agtgataaga ctgattttgc tgagcaactt 4680
   ggagcaatga ataaaagttg gcaaattctg caaggtctag taactgagaa gatccagctg 4740
   ttggaaggct tattggaatc ttggtcagaa tatgaaaata atgtacaatg tctgaaaaca 4800
   tggtttgaaa cccaggaaaa gagactaaaa caacagcatc gaattggaga tcaggcttct 4860
   gttcaaaatg cactgaaaga ctgtcaggat ctggaagatt tgattaaagc aaaagaaaaa 4920
   gaagtagaga aaattgagca gaatggactt gctttgattc agaacaagaa agaagacgtc 4980
   tctagcattg tcatgagcac actgcgagag ctcggccaaa cctgggcaaa tttagatcac 5040
   atggttggac aattaaagat actgctgaaa tcagtgcttg accaatggag tagtcacaaa 5100
   gtggcctttg acaagataaa cagttacctc atggaggcca gatactctct ttcccgattc 5160
   cgtctgctga ctggctcctt agaagctgtg caagttcagg tggacaatct tcagaatctc 5220
   caagatgatc tggaaaaaca ggaaaggagc ttacagaaat ttggctctat caccaaccaa 5280
   ttattaaaag agtgtcaccc acccgtgaca gaaactctta ccaatacact gaaagaagtc 5340
   aacatgagat ggaataactt gctggaagag attgctgagc agctacagtc cagcaaggcc 5400
   ctacttcagc tttggcaaag atacaaggac tactccaaac agtgtgcttc gacagttcag 5460
   cagcaggagg atcgaaccaa tgagctgttg aaggcagcca caaacaagga cattgccgat 5520
   gatgaggttg ccacatggat tcaagattgc aacgacctcc tcaaaggact gggcacagtt 5580
   aaagattccc tcttttttct ccatgagctg ggagagcaac tgaagcaaca agtggatgct 5640
   tccgcagcat cagctattca atcggatcaa ctctctttga gtcaacactt gtgtgccctg 5700
   gagcaagctc tctgcaaaca gcagacttca ttacaggctg gagttcttga ttatgaaacc 5760
   tttgccaaga gtttagaagc tttggaggcc tggatagtgg aagctgaaga aatactacaa 5820
   gggcaggacc ctagccactc atctgacctc tccacaatcc aggaaaggat ggaagaactt 5880
   aagggacaga tgttaaaatt cagcagcatg gctccagatt tagaccgtct aaatgagctt 5940
   ggatataggt tacccttgaa tgataaggaa atcaaaagaa tgcagaatct gaaccgccat 6000
   tggtctctga tctcctctca gactacagaa agattcagca agttgcagtc atttttgcta 6060
   caacatcaga ctttcttgga aaaatgtgaa acatggatgg aattcctagt tcagacagaa 6120
   caaaagttag cagtagagat ttcaggaaat tatcagcacc ttttggaaca gcagagagca 6180
   cacgagttgt ttcaagccga gatgttcagt cgtcagcaga ttttgcactc aatcattatt 6240
   gatgggcaac gtcttctaga acaaggtcaa gttgatgaca gggatgaatt caacctgaaa 6300
   ttgacactcc tcagtaatca atggcaggga gtgattcgca gggcccagca gaggcggggg 6360
   atcattgaca gccagattcg ccagtggcag cgctataggg agatggcaga aaagcttcgt 6420
   aaatggttgg ttgaagtgtc ctacctcccc atgagtggtc tcggaagtgt tcctatacca 6480
   ctgcaacaag caaggaccct ctttgatgaa gtgcagttca aagaaaaagt gtttctgcgg 6540
   caacaaggca gctacatcct gactgtggag gctggcaagc aactccttct ctcggcggac 6600
   agtggcgctg aggccgcctt gcaggccgaa ctcgctgaaa tccaagagaa atggaaatca 6660
   gccagcatgc ggctggaaga acagaagaaa aaactagcct tcttgttgaa agactgggaa 6720
   aaatgtgaga aaggaatagc agattccctg gagaaactac gaactttcaa aaagaagctt 6780
   tcgcagtctc tcccggatca ccatgaagag ctccatgcag aacaaatgcg ttgcaaggaa 6840
   ttagaaaatg cagttgggag ctggacagat gacttgaccc agttgagcct gctgaaggac 6900
   accctctctg cctatatcag tgctgatgat atctccattc ttaatgaacg cgtagagctt 6960
   ctgcaaaggc agtgggaaga actatgccac cagctctcct taaggcggca gcaaataggt 7020
   gaaagattga atgaatgggc agtcttcagt gaaaagaaca aggaactctg tgagtggttg 7080
   actcaaatgg aaagcaaagt ttctcagaat ggagacattc tcattgaaga aatgatagag 7140
   aagctcaaga aggattatca agaggaaatt gctattgctc aagagaacaa aatacagctc 7200
   caacaaatgg gagaacgact tgctaaagcc agccatgaaa gcaaagcatc tgagattgaa 7260
   tacaagctgg gaaaggtcaa cgaccggtgg cagcatctcc tggacctcat tgcagccagg 7320
   gtgaagaagc tgaaggagac cctggtagcc gtgcagcagc ttgataagaa catgagcagc 7380
   ctgaggacct ggctcgctca catcgagtca gagctggcca agccaatagt ctacgattcc 7440
   tgtaactcgg aagaaataca gagaaagctt aatgagcagc aggagcttca gagagacata 7500
   gagaagcaca gtacaggtgt tgcatctgtc ctcaacctgt gtgaagtcct gctgcacgac 7560
   tgtgacgcct gtgccactga tgccgagtgt gactctatac agcaggctac gagaaacctg 7620
   gaccggcggt ggagaaacat ttgtgctatg tccatggaaa ggaggctgaa aatcgaagag 7680
   acgtggcgat tgtggcagaa atttctggat gactattcac gttttgaaga ttggctgaag 7740
   tcttcagaaa ggacagctgc ttttcccagc tcttctgggg tgatctatac agttgccaag 7800
   gaagaactaa agaaatttga ggctttccag cgacaggtcc acgagtgcct gacgcagctg 7860
   gaactgatca acaagcagta ccgccgcctg gccagggaga accgcactga ttcagcatgt 7920
   agcctcaaac agatggttca cgaaggcaac cagagatggg acaacctgca aaagcgtgtc 7980
   acctccatct tgcgcagact caagcatttt attggccagc gtgaggagtt tgagactgcg 8040
   cgggacagca ttctggtctg gctcacagag atggatctgc agctcactaa tattgaacat 8100
   ttttctgagt gtgatgttca agctaaaata aagcaactca aggccttcca gcaggaaatt 8160
   tcactgaacc acaataagat tgagcagata attgcccaag gagaacagct gatagaaaag 8220
   agtgagccct tggatgcagc gatcatcgag gaggaactag atgagctccg acggtactgc 8280
   caggaggtct tcgggcgtgt ggaaagatac cataagaaac tgatccgcct gcctctccca 8340
   gacgatgagc acgacctctc agacagggag ctggagctgg aagactctgc agctctgtcg 8400
   gacctgcact ggcacgaccg ctctgcagac agcctgcttt ctccacagcc ttcctccaat 8460
   ctctccctct cgctcgctca gcccctccgg agcgagcggt caggacgaga caccccggct 8520
   agtgtggact ccatccccct ggagtgggat cacgactatg acctcagtcg ggacctggag 8580
   tctgcaatgt ccagagctct gccctctgag gatgaagaag gtcaggatga caaagatttc 8640
   tacctccggg gagctgttgg cttatcaggg gaccacagtg ccctagagtc acagatccga 8700
   caactgggca aagccctgga tgatagccgt tttcagatac agcaaaccga aaatatcatt 8760
   cgcagcaaaa ctcccacggg gccggagcta gacaccagct acaaaggcta catgaaactg 8820
   ctgggcgaat gcagtagcag tatagactcc gtgaagagac tggagcacaa actgaaggag 8880
   gaagaggaga gccttcctgg ctttgttaac ctgcatagta ccgaaaccca aacggctggt 8940
   gtgattgacc gatgggagct tctccaggcc caggcattga gcaaggagtt gaggatgaag 9000
   cagaacctcc agaagtggca gcagtttaac tcagacttga acagcatctg ggcctggctg 9060
   ggggacacgg aggaggagtt ggaacagctc cagcgtctgg aactcagcac tgacatccag 9120
   accatcgagc tccagatcaa aaagctcaag gagctccaga aagctgtgga ccaccgcaaa 9180
   gccatcatcc tctccatcaa tctctgcagc cctgagttca cccaggctga cagcaaggag 9240
   agccgggacc tgcaggatcg cttgtcgcag atgaatgggc gctgggaccg agtgtgctct 9300
   ctgctggagg agtggcgggg cctgctgcag gatgccctga tgcagtgcca gggtttccat 9360
   gaaatgagcc atggtttgct tcttatgctg gagaacattg acagaaggaa aaatgaaatt 9420
   gtccctattg attctaacct tgatgcagag atacttcagg accatcacaa acagcttatg 9480
   caaataaagc atgagctgtt ggaatcccaa ctcagagtag cctctttgca agacatgtct 9540
   tgccaactac tggtgaatgc tgaaggaaca gactgtttag aagccaaaga aaaagtccat 9600
   gttattggaa atcggctcaa acttctcttg aaggaggtca gtcgtcatat caaggaactg 9660
   gagaagttat tagacgtgtc aagtagtcag caggatttgt cttcctggtc ttctgctgat 9720
   gaactggaca cctcagggtc tgtgagtccc acatcaggaa ggagcacccc aaacagacag 9780
   aaaacgccac gaggcaagtg tagtctctca cagcctggac cctctgtcag cagtccacat 9840
   agcaggtcca caaaaggtgg ctccgattcc tccctttctg agccagggcc aggtcggtcc 9900
   ggccgcggct tcctgttcag agtcctccga gcagctcttc cccttcagct tctcctgctc 9960
   ctcctcatcg ggcttgcctg ccttgtacca atgtcagagg aagactacag ctgtgccctc 10020
   tccaacaact ttgcccggtc attccacccc atgctcagat acacgaatgg ccctcctcca 10080
   ctctgaacta agcagatgcc atctgcagaa gtgctggtag cataaggagg atcgggtcat 10140
   aagcaatccc aaactaccaa caagaggacc ttgatcttgg cgaaagccct cggtgtggca 10200
   gctttagccc tcctccagat cacatgtgtg caaattatgg cttcagaggt ggaagataaa 10260
   cagtgacggg ggaacaaaca gacaacaaga aggtttggaa gaaatctggt ttgagactct 10320
   gaaccttagc actaaggaga ttgagtaagg acctccaaag ttccccggac tcatgaattc     10380
   tgggcccttg gcccattctg tgcacagcca aggacttcag tagaccatct gggcagcttt     10440
   cccatggtgc tgctccaacc atcagataaa tgaccctccc aagcaccatg tcagtgtcgt     10500
   acaatctacc aaccaaccag tgctgaagag attttagaac cttgtaacat acaattttta     10560
   agagcttata tggcagcttc ctttttacct tgttttcctt tggggcatga tgttttaacc     10620
   tttgctttag aagcacaagc tgtaaatcta aaaggcactt ttttttagag gtataaagaa     10680
   aaactagatg taataaataa gatcatggaa ggctttatgt gaaaaaagtt gaatgttata     10740
   gtaaaaaaaa aaagatattt atgtatgtac agtttgctaa agccaagttt tgtttgtatt     10800
   gatttctttg catttattat agatattata aaata                                10835
<210> 30
   <211> 27436
   <212> DNA
   <213> Homo sapiens
<400> 30
   cggccgccgg ccgcagcggg ctgagattgt tgtcctctgt caccagggcg gctgggctcc 60
   cgcagtcctg cagaccgcgc ccgatcccgg cgacagggcg ggcggacagc cgcgcatccc 120
   cggggtcccg ccgagcctgg gcgcagagag ccgggaggaa gcgttcgctc gcttcgcctt 180
   gctgctggga aactgaacga ggccgagaga gaaggttctt gagttcatgt aagaggacag 240
   tcttaaaacg gaagaagaaa aagaagcagt tcagtctttg ggagagctgc ctccttgttg 300
   agtgctgcaa aggcctggaa ttcatttatg acagaataga tctagaaaag tccaagcatg 360
   ttttctagag tggtgtagcc ctgtgctgcc tccagtgaag agtctcttgg tgttggcttc 420
   gtgcttccgg agggaccatg gcaacctcca gaggggcctc ccggtgtcct cgggatatcg 480
   ccaatgtgat gcagaggctg caagatgagc aagagatagt acaaaaacga actttcacaa 540
   aatggatcaa ctctcatctg gccaagcgga aacctccaat ggtggtggac gatctttttg 600
   aagacatgaa agatggtgtt aaactgcttg cccttctgga ggtcctgtct gggcagaaac 660
   tgccttgtga acaaggacgc cggatgaagc gaatccatgc tgtggctaac attggcacgg 720
   cactcaagtt cctcgaagga agaaagtcca tgcacagagg atcaccgatt aaattagtca 780
   acattaactc caccgatata gctgatggcc gaccctcaat agttcttgga ttgatgtgga 840
   ccattattct atatttccag attgaagagt tgaccagcaa cctgccccag ctccagtctt 900
   tgtccagcag cgcatcctcc gtggacagca tagttagctc tgagactccc agcccaccaa 960
   gtaaacggaa ggtgaccacc aagatccaag gaaatgctaa gaaggcttta ttaaagtggg 1020
   ttcagtacac agctggcaag cagactggaa tagaagtaaa agattttggg aagagttgga 1080
   gaagcggggt tgcctttcat tcagttattc atgccattcg accggaattg gtggacttgg 1140
   agacagtgaa aggcagatcc aaccgagaaa atttggagga tgctttcact atcgccgaaa 1200
   cagaactggg gatcccaaga ctgctagatc ctgaagacgt tgatgtggat aaaccagatg 1260
   agaaatctat tatgacctat gtagcccagt ttctgaaaca ttatcctgac atccacaatg 1320
   caagcactga tgggcaagag gatgatgaaa tacttccagg tttcccatct tttgcaaatt 1380
   ctgtacaaaa ttttaagaga gaagacagag taatttttaa ggaaatgaaa gtttggatag 1440
   aacaatttga gagagatttg acaagagcac agatggtgga atcaaattta caggataaat 1500
   atcagtcatt taagcacttc agagttcaat atgaaatgaa gaggaaacag attgaacatt 1560
   taatacaacc attacacaga gacggtaaat tgtcacttga ccaagcattg gtaaaacaat 1620
   cttgggatag agtgacctcc aggctctttg actggcatat acagcttgat aaatctcttc 1680
   ctgcacctct gggcaccata ggtgcctggc tgtacagagc ggaggtggcc ctgagagagg 1740
   aaataaccgt tcaacaggtc cacgaggaaa cagcaaacac gatacaacgg aaacttgagc 1800
   aacataagga tctgcttcaa aacacggatg cccacaaaag agcattccat gaaatctacc 1860
   ggaccaggtc tgttaacggg attccagtgc cacctgatca attagaggac atggccgaga 1920
   ggtttcattt tgtttcctcc acatcagagc tacacctaat gaaaatggaa tttttagaat 1980
   taaagtaccg tctgctctca ctgctggttc ttgcagagtc aaagctgaag tcttggatca 2040
   ttaagtacgg gaggagagag tcagtggagc agcttctaca aaactacgtg tcttttatag 2100
   aaaatagcaa gttctttgaa caatatgagg tgacatacca gatcttgaaa cagacagctg 2160
   agatgtatgt caaagcagat ggttcagtgg aagaagctga gaatgtgatg aaattcatga 2220
   atgaaaccac cgctcagtgg aggaatctct cagtagaagt gaggagtgtg aggagcatgc 2280
   tggaagaagt gatctctaac tgggatcgct atggcaatac agtggctagt ctgcaagcct 2340
   ggctagagga tgctgaaaaa atgctcaatc aatcagaaaa tgccaaaaag gatttttttc 2400
   gaaatttacc tcattggatt cagcagcata ctgccatgaa cgatgctggc aattttctaa 2460
   ttgaaacctg tgatgagatg gtttcccgtg acctgaagca gcaattactg ttgctaaatg 2520
   ggcggtggag ggagttgttt atggaagtca agcaatatgc tcaagctgat gagatggaca 2580
   gaatgaagaa ggaatacaca gactgtgttg ttaccctgtc tgcttttgca acggaagccc 2640
   ataagaaact ttctgaaccc ttagaagtct cttttatgaa tgtcaagcta ttaattcaag 2700
   acttggagga tattgagcag agggtgcctg tgatggatgc ccaatacaag ataattacaa 2760
   agacagcaca cctcattacc aaagaaagcc cccaagaaga aggaaaagaa atgtttgcga 2820
   ccatgtcaaa gctcaaagag cagctaacca aggtcaaaga atgttactcc ccactccttt 2880
   atgagtctca gcagctgttg attccgttgg aggaattaga aaagcagatg acgtcctttt 2940
   atgactcact tgggaaaatc aatgaaatta tcacagttct tgagcgtgag gcacaatcga 3000
   gtgccctttt taaacaaaaa catcaggaac tgttagcttg tcaagaaaac tgtaagaaaa 3060
   ccttgacact tattgagaaa ggcagtcaaa gtgttcaaaa gtttgtgacc ttgagcaacg 3120
   tgttaaagca ttttgatcag acgaggctac aaagacagat tgcagatatt catgttgctt 3180
   ttcagagtat ggtaaagaaa actggagatt ggaagaagca tgtggaaacc aacagtcgct 3240
   tgatgaagaa gtttgaggag tctcgagcag agttggagaa ggtactgcgg attgctcagg 3300
   agggcctgga ggaaaagggg gatccagagg agctcctgcg gagacacact gagtttttca 3360
   gtcagctgga tcagagggtg ctcaatgctt tcctgaaagc ttgtgatgaa ctcaccgaca 3420
   tccttccaga gcaggagcag caggggctgc aggaagctgt tcgaaagctc cacaaacaat 3480
   ggaaggatct tcaaggagaa gccccttatc atttgcttca tctgaagatt gatgtggaga 3540
   agaataggtt cttagcctct gtagaagaat gcagaactga gctggatcga gagaccaagc 3600
   tgatgcccca ggaaggcagt gaaaagataa ttaaagagca cagggttttc ttcagtgaca 3660
   aaggtcctca tcatctctgt gagaaaaggt tacagctcat cgaggaactc tgtgtgaaac 3720
   tcccagtgcg ggacccagta agggacacac ctggaacctg tcacgtgact ctcaaagagc 3780
   tcagagctgc cattgacagc acctacagga agctcatgga agacccagac aagtggaagg 3840
   actacactag cagattctct gagttctcat cttggatatc tacaaatgag acacaattaa 3900
   aggggatcaa gggtgaggcc atcgatactg ccaaccacgg agaggttaaa cgtgccgttg 3960
   aagagatcag aaatggtgtt accaaaaggg gtgagaccct cagctggctg aaatccaggc 4020
   tgaaagtttt gacagaagtt tcttctgaga atgaagccca aaagcaggga gatgagctgg 4080
   caaaattatc cagctctttc aaggctcttg tgacgctgct gtcagaggtt gaaaagatgc 4140
   taagcaattt tggggactgt gtccagtaca aagaaatagt caaaaattct ctcgaagaat 4200
   taatttctgg ctctaaagaa gtccaggaac aagctgagaa gatcttggat actgaaaatc 4260
   tgtttgaagc acagcagtta cttcttcatc accagcaaaa gacaaagcgg atctcagcaa 4320
   agaagagaga tgtgcagcag cagatcgcgc aggcgcagca gggagaaggg gggctgcctg 4380
   accgaggcca cgaggagctg cggaagctgg agagcacact ggatggcctg gagcgcagcc 4440
   gggagaggca ggaacgccgc atccaggtca cattaagaaa atgggagcga tttgaaacaa 4500
   acaaagaaac agtagtaaga tacctttttc aaacaggttc cagtcatgaa cgcttcttga 4560
   gttttagcag tttggaaagt ttatcttcag aactggaaca aacaaaggag ttttctaaac 4620
   ggacagaaag tattgcagtc caggctgaga accttgtaaa ggaagcttca gagataccgc 4680
   ttgggcccca aaataagcag ctgcttcaac agcaggccaa gtcaatcaaa gaacaagtca 4740
   aaaaattaga agacacgctt gaagaagata ttaaaaccat ggaaatggtg aaaaccaagt 4800
   gggatcattt tggcagtaat tttgagactc tgtccgtctg gataactgag aaagaaaaag 4860
   aactcaatgc cttggaaact tcgtcatctg ccatggacat gcaaatcagc caaattaagg 4920
   tcacaattca ggaaatagaa agtaagctca gcagcattgt aggattagaa gaagaagccc 4980
   agtcttttgc tcagtttgtt accactggag aatctgctcg aattaaagcc aagttgacac 5040
   aaataagaag atacggggaa gagcttcgag agcatgcaca gtgtctggaa ggaacaatcc 5100
   tgggacattt atctcagcag caaaagtttg aagagaacct tagaaagatc cagcaatctg 5160
   tgtctgaatt tgaagataaa cttgctgttc caattaaaat atgttcttca gctacagaaa 5220
   catacaaagt tcttcaagaa catatggatc tctgccaggc cctggagtca ctgagcagcg 5280
   cgatcactgc cttctcagcc agtgccagga aggttgtgaa cagagattcc tgtgttcagg 5340
   aggctgcggc tctacagcag caatacgagg acatcctaag gagggcgaag gagagacaga 5400
   cggcgctgga gaatctgctg gcccactggc agaggctaga gaaagaacta tcatcctttt 5460
   tgacctggtt agagcggggt gaagctaaag ccagttcccc agaaatggac atttctgcag 5520
   acagagtcaa agtggaaggt gaacttcagt taatacaggc actgcaaaat gaagttgtat 5580
   cccaggcctc attctatagc aaacttttgc aattgaagga atcattgttc tcagtagcct 5640
   ccaaagatga tgtgaaaatg atgaaactac atttggagca gttggatgag agatggagag 5700
   atttaccaca gatcattaac aaaaggatta attttcttca gtctgtggtt gctgaacacc 5760
   agcaatttga tgagctgctg ctttcctttt ctgtctggat taaactgttt ctcagtgaat 5820
   tacaaactac ctctgagatt agcataatgg accatcaagt agcccttact cggcataagg 5880
   accacgcagc agaagtagag agcaaaaagg gcgaattgca gagtctgcag ggtcacttag 5940
   caaagttggg ttctctgggc cgtgctgagg acctccacct cctgcaggga aaggctgagg 6000
   actgcttcca gctgtttgag gaggccagcc aggttgtgga gaggcggcag cttgccctgt 6060
   cccatttggc agaattcctc cagagccatg cctctctgtc cggcattctc cgccagctga 6120
   ggcaaacagt ggaagcaacc aacagtatga ataagaacga gtctgatttg atagaaaagg 6180
   acctcaatga tgctcttcaa aatgctaaag cattagaatc tgctgccgtc agtctggatg 6240
   gcattctttc caaagcccaa taccatctga aaatcgggag ctctgagcaa aggacttcct 6300
   gcagagccac ggctgatcag ctctgtggag aggtagagag gatccagaac cttctgggaa 6360
   ccaagcagag tgaggcagat gctctggcag tgttgaaaaa agcattccaa gaccagaaag 6420
   aggagcttct gaaaagcatt gaggacattg aagaaaggac tgacaaagag cgattgaaag 6480
   aacctacccg ccaagctctt cagcagaggt taagagtgtt taatcagcta gaagatgaat 6540
   tgaattctca cgagcatgaa ctatgttggt tgaaagacaa agccaagcaa attgcccaga 6600
   aagatgtagc ttttgcacct gaagttgaca gggagataaa ccgcttagag gtcacctggg 6660
   atgataccaa aagactaatt catgaaaatc agggtcagtg ctgtggactt attgacttaa 6720
   tgagagaata tcagaacctg aaatcagctg tatctaaagt cttagaaaat gccagcagtg 6780
   tgattgtaac cagaactacc ataaaagatc aggaggatct taaatgggct ttttccaagc 6840
   atgaaactgc caagaacaaa atgaattaca aacagaaaga cttggataac tttaccagca 6900
   aaggaaaaca cttgttatct gagctgaaga aaattcacag tagtgatttc agcttggtga 6960
   aaacagacat ggagagcacc gtggacaaat ggctggatgt atcagagaaa cttgaagaaa 7020
   acatggatag gctgagagta agcctgtcca tttgggatga tgtactgtca actagagatg 7080
   agattgaggg atggtcaaac aactgcgttc cacagatggc agaaaacatc agcaacctgg 7140
   ataaccacct cagagctgaa gaactgctta aagaatttga gtctgaagtt aaaaacaaag 7200
   cattgagatt ggaagaactg cattccaaag ttaatgatct gaaagaatta actaaaaatc 7260
   tagaaacacc gccagacctt cagtttatag aagcagactt aatgcagaaa ctggagcatg 7320
   ccaaagaaat aactgaagta gcaaaaggaa ccctgaagga tttcacggct caaagtacac 7380
   aagtggagaa gtttattaat gacataacaa catggttcac aaaagtggaa gaatcgttga 7440
   tgaactgtgc ccaaaatgag acttgtgaag cattgaaaaa agtcaaggat atacaaaaag 7500
   aacttcaaag tcaacaaagc aacatcagct ctacccaaga aaatctcaat agcttgtgcc 7560
   gcaagtacca ctcagctgag ttggagagcc tgggccgtgc aatgactggt ctgataaaga 7620
   aacatgaagc cgtgagccag ttgtgctcca aaacccaggc cagcctgcag gaatctctgg 7680
   aaaaacactt cagtgagtct atgcaggaat tccaagaatg gtttttggga gcaaaggcag 7740
   cagcaaaaga atcatcagat cgcaccggtg acagcaaagt tctagaagca aagctccatg 7800
   atcttcagaa cattttggac tcagtcagtg atgggcagag caaacttgat gcagtgactc 7860
   aagaaggaca aactttgtat gcacatttgt ctaaacaaat tgtcagtagc attcaagaac 7920
   aaatcacaaa ggccaatgaa gagtttcaag catttctgaa acaatgcctt aaagataagc 7980
   aggctcttca agactgtgct tcagaacttg gaagctttga agatcagcac agaaaactga 8040
   acttatggat ccatgaaatg gaagaaaggt tcaatacgga aaacttggga gagagtaaac 8100
   agcacattcc tgagaagaaa aatgaagttc ataaagttga aatgtttttg ggagaactgc 8160
   tggctgcaag agagtctctt gataagcttt cccagagagg gcagcttctg agtgaagaag 8220
   gccacggtgc tgggcaggag ggccgcctgt gttcccagct cctcacaagc caccagaacc 8280
   tacttagaat gaccaaagag aaactccgga gctgccaggt ggcccttcag gagcacgaag 8340
   ccctggagga agcactgcaa agcatgtggt tctgggtgaa ggccattcag gacagactgg 8400
   cctgtgcaga gagcactctt gggagcaaag acaccctgga gaaacggctg tcacaaatac 8460
   aggatattct cctgatgaaa ggtgaagggg aagttaagtt gaatatggcc attggcaagg 8520
   gggaacaggc cttgagaagt agcaacaaag aaggtcagag ggtgattcag actcagttag 8580
   agacccttaa agaagtgtgg gctgacatca tgagctcctc cgtccacgct caaagcactt 8640
   tagagtctgt gattagccaa tggaatgact atgtagagag gaaaaaccag ttggagcagt 8700
   ggatggaatc agtggatcaa aaaatagaac atcccttaca accacagcca ggtctgaaag 8760
   agaagttcgt cctgcttgac cacctccagt ccatcctgtc tgaggcagaa gatcacacga 8820
   gagcccttca ccgtctaatt gcgaagtcca gggagctcta cgaaaagaca gaggatgagt 8880
   ctttcaagga cacagctcaa gaggagctga aaacacagtt taatgatata atgactgttg 8940
   ccaaggaaaa aatgaggaaa gtggaagaga ttgtgaaaga tcatctaatg tatttagatg 9000
   cggtccacga gttcacagat tggctccatt cagcaaagga agaacttcac cggtggtcag 9060
   atatgtctgg agattcatca gccacccaga aaaagttatc aaaaattaag gagctgatag 9120
   attccagaga gattggtgca agccgtctca gcagagtgga gtcgctggct cccgaagtga 9180
   aacagaacac aactgccagt gggtgtgagc tcatgcacac ggagatgcag gccctgcgtg 9240
   ccgactggaa gcagtgggaa gacagtgtat tccaaacgca gagctgtttg gagaacctgg 9300
   tcagccagat ggccctttcg gagcaggaat tctcaggcca agtggctcaa ctggagcagg 9360
   ccctggaaca gttcagtgcc cttctgaaaa cctgggctca gcagttaacc ctcctggaag 9420
   gcaagaacac ggatgaggag atagtggaat gctggcacaa aggacaagag atactggatg 9480
   ctttgcaaaa agcagagcct agaacagagg atctcaagtc tcagctgaat gaactttgtc 9540
   gattttccag agacctgagt acctacagtg gaaaagtttc tggcttgatt aaagagtata 9600
   attgtctttg tttgcaagca tccaagggct gccagaataa agaacagatt ttacagcaaa 9660
   gatttcgaaa ggccttcagg gatttccagc agtggttggt taatgcaaaa atcactaccg 9720
   ccaagtgttt tgatatacct caaaatataa gtgaagtttc aactagtctt cagaaaatac 9780
   aggagttttt gtcagaaagt gaaaatggac agcacaagct aaacatgatg ctgtctaaag 9840
   gggaacttct gagtaccctg ctgaccaaag agaaagcgaa agggatccag gccaaagtta 9900
   cagctgcaaa agaagattgg aaaaattttc attcaaatct ccaccaaaaa gaatctgctc 9960
   tagagaatct aaagatccaa atgaaggact ttgaagtaag tgctgagcct atccaggact 10020
   ggctgagtaa aactgagaag atggtccatg aaagcagcaa tcgcctctat gatctgccag 10080
   caaagaggag ggagcagcag aagctccagt ctgtccttga ggaaatacac tgctacgagc 10140
   ctcagctcaa caggctgaaa gagaaagctc agcagctgtg ggaaggacaa gctgccagca 10200
   agagctttag gcacagagtg tcgcagctgt cttctcagta tctagcgcta agcaatttaa 10260
   caaaggagaa agtgtcaaga ctggatagaa tcgttgcaga acacaatcag ttctctcttg 10320
   ggattaaaga attacaagac tggatgacgg atgcgattca catgctggat tcatactgcc 10380
   acccgacatc cgacaaaagt gtgctggaca gcaggacgct caagctcgag gctctattat 10440
   cagtcaaaca ggaaaaagag attcagatga aaatgatagt gaccagggga gaatctgtcc 10500
   ttcagaatac ttctccagaa ggcattccca ctattcagca gcagctgcag agtgtgaagg 10560
   atatgtgggc atcccttttg tctgcaggga ttcgttgtaa aagccaactc gaaggagctc 10620
   tctccaagtg gacaagttat caggatggcg ttcgacagtt ctccggttgg atggatagta 10680
   tggaagccaa cctgaatgaa tcagaaaggc agcatgcgga gctgcgggat aaaacaacga 10740
   tgctcggaaa agccaagtta ttaaatgaag aagtgctgag ttacagcagc ttgctggaga 10800
   ccatcgaagt caaaggggct ggcatgacag aacactatgt cacccagcta gaactccagg 10860
   atctacagga acgatacaga gccatccaag agagggccaa ggaagccgta accaagtctg 10920
   aaaaacttgt ccgcctgcac caagagtatc agagagacct aaaggcattt gaagtttggt 10980
   tggggcaaga acaagaaaag ctcgaccagt attcagttct tgaaggtgat gcccacactc 11040
   atgagacaac attgcgtgat cttcaggagc tacaggtaca ctgtgcagag gggcaggccc 11100
   tgttgaactc agtgctgcac accagagagg atgtgatccc atcaggtatc ccacaggcag 11160
   aggaccgggc tttagagtct ctccggcaag actggcaggc ttaccagcac aggctgtccg 11220
   agactcgaac tcagttcaat aacgtggtga acaaattgag gctaatggag caaaagtttc 11280
   agcaagtaga tgaatggctc aaaacagcag aggagaaaaa gtggcacgaa gaagtgactg 11340
   catacagaga tgaagttgag gaagtgggag ctagagctca ggagatactg gacgagagcc 11400
   acgtgaacag cagaatgggt tgccaggcca cccagctgac ttccagatac caggccctgc 11460
   ttctccaagt gctggaacaa ataaaattcc tggaggagga gattcagagt ttggaggaat 11520
   cagaatcatc cctcagttcc tattctgatt ggtatggctc tactcataaa aacttcaaga 11580
   atgtggctac caagattgac aaagtagata cagtaatgat ggggaagaaa ttgaagacgt 11640
   tggaggtttt gctcaaagac atggagaaag gtcacagttt gctgaaatca gcccgggaga 11700
   aaggagagag ggctgttaaa tacttggagg aaggcgaggc agagaggtta agaaaggaga 11760
   ttcatgatca catggagcag ttgaaggaac tgaccagcac tgtccggaaa gaacacatga 11820
   cgctggaaaa aggtcttcat ttagcaaagg aattctcaga taaatgcaaa gcactgacac 11880
   agtggatagc agaataccag gaaattctac atgttcctga agaacccaaa atggaattat 11940
   atgagaaaaa agctcagtta tctaaataca agtcacttca acaaacggtg ctgtcccatg 12000
   aaccatcagt aaagtcagtg agagagaagg gtgaagctct tttggaactg gtgcaggacg 12060
   tcactttaaa ggacaaaata gatcaacttc aaagtgatta ccaggacctg tgcagcatag 12120
   gaaaggcaat gatggaagaa attgctggtt ttgaagaccg tttgaacaat cttcaaatga 12180
   aaggtgatac tttgattggc caatgtgcag accacctgca agcgaaactt aaacaaaacg 12240
   tgcatgctca tctgcagggc acaaaggaca gctactcagc gatctgcagc acagctcaga 12300
   ggatgtacca gagtttggaa cacgaacttc agaagcacgt cagccgacaa gacaccctgc 12360
   agcagtgcca ggcctggctt tctgcagtcc agccggattt agagccaagt cctcaaccac 12420
   ctcttagtag ggcagaagcc attaagcagg tcaaacactt cagagctttg caagagcagg 12480
   caaggaccta cctagatctc ctttgctcca tgtgtgacct gtcaaatgct tcggtgaaaa 12540
   ccacagcaaa agacattcaa caaacagagc aaacgattga acaaaagctt gtccaggccc 12600
   agaacttaac tcagggctgg gaagagatca agcacctgaa gtctgagctc tggatttacc 12660
   tgcaagatgc tgatcagcaa ctgcagaaca tgaagaggag gcactctgag ctggagctga 12720
   acattgcaca gaacatggtt tcacaagtta aggattttgt taagaaacta cagagcaaac 12780
   aggcatccgt gaacaccata atagaaaagg tgaataagtt aacaaagaag gaggaatcgc 12840
   ctgaacacaa ggaaataaat catttaaatg atcagtggct cgatttgtgc cgtcagtcta 12900
   acaacctgtg cttgcaaagg gaagaggatc ttcagagaac aagagattac catgactgta 12960
   tgaatgttgt tgaagtgttc ctagaaaaat ttactacaga atgggataac ttggccagat 13020
   ctgatgcaga gagtacagct gtccacctgg aagctttgaa aaagttagca ttggcattgc 13080
   aggagagaaa gtatgctatt gaagatctga aagatcaaaa gcagaaaatg atagagcatc 13140
   tgaatttaga tgacaaggag ttagtcaaag aacagacgag tcatttagag caacgttggt 13200
   ttcagcttga ggacctcatt aaaaggaaaa tccaagtgtc agtcaccaac ttggaggagt 13260
   taaatgtggt gcagtccaga tttcaggagc taatggagtg ggcagaagag caacaaccca 13320
   acatcgccga ggcccttaag cagagccctc ctccagatat ggctcagaac cttctcatgg 13380
   atcacctggc catctgcagt gaactggagg ccaagcagat gctcctgaaa tcgcttataa 13440
   aggacgcaga cagggtcatg gcagatcttg gtctcaatga gcgacaggtc atccagaagg 13500
   ctctctctga tgcacaaagc cacgtgaatt gtctcagtga cttagtgggc cagcgaagaa 13560
   agtacttaaa caaagccttg tccgagaaaa cccagtttct catggcagtg ttccaggcca 13620
   ccagccaaat tcagcaacat gagcgaaaga taatgttccg tgaacacatc tgtctgttac 13680
   cagatgatgt gagcaaacaa gtcaaaacat gtaagagtgc acaagccagc ctcaagactt 13740
   accaaaatga agtcactgga ctttgggccc agggtcgcga actaatgaag gaagtcacag 13800
   agcaggaaaa gagtgaagtg ctggggaagc ttcaggaatt gcagagtgtc tatgacagtg 13860
   ttttacaaaa gtgcagtcac cggttacaag aactagagaa gaatttggtt tctaggaagc 13920
   attttaagga agattttgat aaagcttgcc actggctaaa acaagcagat attgttacat 13980
   ttcctgaaat caacctaatg aatgagagtt ctgagcttca tacacaactg gctaaatacc 14040
   aaaacattct tgaacaatct ccagaatatg aaaatcttct acttacgctg cagagaactg 14100
   ggcagaccat attaccatcg ctgaatgaag tcgatcattc ctacctcagt gaaaagctaa 14160
   atgctttgcc tcgacaattt aatgtaattg ttgccttggc taaagacaag ttctataaag 14220
   tccaggaagc aattcttgct cggaaggaat atgcttcctt gattgagttg acaacccagt 14280
   ctctgagtga acttgaagcc caattcttga ggatgagcaa agttcccacc gacctggccg 14340
   ttgaggaggc tctttctctg caagatggtt gcagagccat tctggacgag gtggcgggcc 14400
   ttggggaggc ggtggatgaa ctgaaccaga aaaaagaagg ttttcgcagc acaggtcagc 14460
   cttggcagcc agacaagatg ctgcaccttg tcaccttata tcacaggctg aagcgacaaa 14520
   cagaacagag ggttagctta ttagaagaca ccaccagtgc ttaccaagaa cacgagaaga 14580
   tgtgccaaca gctggagaga caactgaagt ctgtaaaaga ggagcagtcc aaagtgaatg 14640
   aggaaacgct gcctgcagag gagaagctca aaatgtatca ctccctggca ggaagtctcc 14700
   aggactcagg gattgtactg aaacgagtaa ccatacatct tgaagatctt gccccacacc 14760
   ttgacccctt ggcttatgag aaagccaggc atcagatcca gtcctggcaa ggggagttaa 14820
   aactgttgac ttctgccatt ggtgagacgg tgacagaatg tgagagccga atggtgcaga 14880
   gtatagactt ccagactgag atgagtcgct ccctggactg gctgaggaga gtgaaggcag 14940
   agctcagtgg gccggtgtac ctagacctca acctgcagga catccaagag gaaatcagaa 15000
   aaatccaaat tcatcaggaa gaggtccagt ccagcttgag aatcatgaat gcgctgagtc 15060
   acaaggaaaa ggagaagttc acaaaggcca aggagctgat ttctgcggat ttagaacaca 15120
   gcctcgctga gctctcagag ctggatggag acatccagga agccttacgc accagacagg 15180
   ctaccttgac tgaaatatat agccagtgtc aaaggtatta tcaggtattt caagcagcca 15240
   atgactggct tgaggatgcc caagaattgt tacagctggc aggcaatggc ctagacgtgg 15300
   agagcgcaga ggaaaatctc aaaagccaca tggaattttt cagtacagag gatcagttcc 15360
   atagtaacct ggaggagctc cacagcctgg tagccaccct ggacccactc atcaagccaa 15420
   ccggcaaaga agacctagaa cagaaagtgg cttctctgga actcaggagc cagaggatga 15480
   gccgggactc tggtgcccaa gtggatctct tgcagagatg cacagctcaa tggcacgatt 15540
   accagaaagc aagggaagag gttattgaat tgatgaatga tacagaaaag aaattgtctg 15600
   agttttcttt gttgaagact tcgtctagtc atgaagcgga agaaaaattg tcagaacaca 15660
   aggctttagt gtcagtggtt aactctttcc atgagaaaat tgtggccctt gaggaaaaag 15720
   cttcacaact ggagaaaacc ggaaatgatg ccagcaaagc caccctgagc aggtcaatga 15780
   ccaccgtctg gcagcgctgg acacgccttc gagctgtggc ccaggaccag gagaagatcc 15840
   tggaagatgc agtggatgag tggacgggct ttaacaacaa ggttaaaaag gccactgaaa 15900
   tgattgatca gctgcaagat aagttacctg gaagttcagc agagaaagca tcgaaagcag 15960
   agctcttaac tcttcttgaa taccacgaca cgttcgttct ggagctggag cagcagcagt 16020
   cggccttggg catgctgcgg cagcaaaccc tgagcatgct ccaggatgga gccgccccaa 16080
   cccctgggga agagcctccg ctcatgcagg aaatcaccgc catgcaagat cggtgcctga 16140
   acatgcagga gaaagtgaag actaatggaa agttggtgaa gcaagagctg aaggaccgag 16200
   aaatggtgga gactcagatc aattctgtga aatgttgggt tcaggaaacg aaagaatatt 16260
   tagggaatcc aacaatagaa atagatgctc aacttgaaga acttcagatt ctcctaacag 16320
   aagccacaaa tcaccgacag aacattgaaa aaatggcaga agaacagaag gagaagtact 16380
   taggtcttta taccatatta ccttctgaac tctcccttca gttggctgaa gtggcgttag 16440
   atctaaagat ccgagatcag atccaagaca aaataaaaga agttgagcag agcaaggcca 16500
   cgagccagga actcagccgg caaattcaga agttagctaa agacctcaca actattctaa 16560
   ctaagctgaa agcgaagaca gataatgtag ttcaagctaa aactgaccaa aaggtgctgg 16620
   gagaggaatt agatggctgt aattcaaagt taatggaatt agatgcagca gtacagaaat 16680
   tcttggaaca gaatggccaa ctgggtaagc cactggccaa gaagatagga aaactgactg 16740
   aacttcacca gcagaccatt agacaagctg agaatcggct ctccaagctc aatcaggcag 16800
   catcacattt agaagaatac aatgaaatgc ttgaattaat tttgaagtgg attgaaaaag 16860
   ctaaagtctt ggctcatgga actattgcat ggaattctgc aagccagctt cgggaacaat 16920
   atattttgca tcagaccctg ctagaagaat ccaaagaaat tgacagtgag ctggaagcaa 16980
   tgactgagaa attacagtac ctcactagcg tgtactgtac agaaaaaatg tctcagcaag 17040
   tggcagaact gggacgggag actgaggagt tgcgacagat gatcaaaatt cgtttgcaga 17100
   acctccaaga tgcagctaag gatatgaaaa aatttgaagc agagttgaaa aagttacaag 17160
   ctgccttgga gcaagcccag gcaacactga cttctccaga agttggacgt ctcagtctca 17220
   aggagcagct ctctcatcgg cagcatttgt tgtctgagat ggagtcactg aagccgaagg 17280
   tgcaagcagt gcagctctgc cagagtgccc tccggatccc cgaggatgtg gttgccagct 17340
   tacctctctg tcatgctgct ctgcggctgc aggaagaggc cagccggctg cagcacaccg 17400
   ccatccagca gtgtaacatc atgcaggcag ctgtggtaca atatgaacaa tatgagcaag 17460
   aaatgaaaca tctccagcaa ctgatagaag gagctcacag agagattgag gataaacctg 17520
   ttgccaccag taacatacag gagctgcagg ctcagatttc tcggcatgag gagctggcgc 17580
   agaaaattaa gggctaccag gagcagatcg cttctttgaa ttccaagtgc aagatgctga 17640
   cgatgaaagc caagcacgcc accatgctgc tgacggtgac cgaggtcgag gggctggcgg 17700
   aagggacaga ggacctggat ggggagctcc tccccacgcc ttcggcccac ccctctgtgg 17760
   tcatgatgac tgcaggtcgc tgtcacactt tgctgtcacc ggtcactgag gagtctgggg 17820
   aggagggaac caacagtgag atttcctctc cacctgcctg tcgctcccct tcacctgtgg 17880
   ctaatacaga tgcttctgtt aaccaggaca ttgcatatta ccaagccttg tctgctgaga 17940
   ggttgcagac agatgctgca aaaattcacc ccagcacatc cgcatcccag gagttctatg 18000
   aaccgggatt ggagccatcc gctactgcca aactgggtga tttgcagcgt tcttgggaaa 18060
   ccttaaagaa tgtgatcagt gagaagcagc gcacactcta tgaagctttg gaacgccagc 18120
   agaagtacca ggactccctc cagtccatct ctacgaagat ggaggccatt gagctgaaac 18180
   tcagtgagag cccagagcct ggcaggagtc cagaaagcca gatggctgaa catcaggcat 18240
   tgatggatga gattctcatg ctccaggatg aaatcaatga gctccagtcc tctctcgcag 18300
   aggagctggt atccgagtct tgtgaggccg accctgcgga gcagctggcc ttgcagtcca 18360
   cgctcactgt cttagccgag cgaatgtcca ccatcaggat gaaagcctcg gggaaacggc 18420
   agcttttgga ggagaagttg aatgatcagc tggaggaaca aaggcaggaa caggccctgc 18480
   agaggtatcg ctgtgaagcc gatgagctgg acagctggct cttgagtacc aaggccactc 18540
   tggacactgc gctgagtcca cccaaggagc ccatggacat ggaggcccag cttatggact 18600
   gccagaatat gctggtggaa atagagcaga aggtggtggc tttatcagaa ctgtcagtcc 18660
   acaatgagaa cctgctgctg gagggcaaag ctcacaccaa ggacgaggcc gagcagctgg 18720
   ctggaaagct gagaaggctc aaggggagcc tgctggagct gcagagagcc ctgcatgata 18780
   agcagctcaa catgcaggga acagcacagg agaaggagga gagcgatgtt gacctaacag 18840
   ccacgcagag ccccggcgtc caggaatggc tggcccaagc tcgcaccaca tggacccagc 18900
   agcggcagag cagtctccag caacaaaaag agttagaaca ggaattagcc gagcagaaga 18960
   gtctccttcg ctcagtagcc agtcgtggag aggagattct aattcaacat tcggcggcag 19020
   agacctctgg tgatgctggc gaaaaacctg atgtgttatc ccaggagttg gggatggaag 19080
   gggagaaatc atccgctgaa gaccagatga gaatgaaatg ggaaagccta catcaagaat 19140
   ttagtaccaa gcagaaacta ctacagaatg ttctggaaca ggaacaagag caagtgcttt 19200
   atagcaggcc aaatcgactc ttgtctggtg tgccactgta caaaggggac gtgccaaccc 19260
   aagataaatc tgcagttaca tctttgctgg atggactgaa ccaagccttc gaggaggttt 19320
   catcccagag tggaggggca aagaggcaga gtatacactt ggagcagaag ttgtatgatg 19380
   gagtctcagc cacctctact tggttggatg acgttgaaga acgtttattt gttgccacag 19440
   cacttttacc agaagaaaca gagacttgtc tcttcaacca agagattctt gccaaagaca 19500
   ttaaggaaat gtctgaagaa atggataaga acaaaaactt gttttcccaa gcttttccag 19560
   agaatggtga taatcgagat gttattgaag atactttggg ttgtcttttg ggcaggttat 19620
   ccttgctaga ctcagtagtg aatcaacgat gtcatcagat gaaagaaaga cttcagcaaa 19680
   tactaaattt ccagaatgat ctgaaagtgc tgtttacatc actggctgac aacaaataca 19740
   tcattctgca aaaactggca aatgtgtttg aacagcccgt agcagaacaa atagaggcaa 19800
   tacaacaggc tgaagatgga ctcaaagaat ttgatgcagg aatcattgaa ttaaagaggc 19860
   gtggtgacaa gctacaggtc gagcagccgt ccatgcaaga actctccaag ctccaggaca 19920
   tgtatgatga gctgatgatg atcattggct cccggaggag tggtctgaat cagaacctta 19980
   cactcaagag tcagtatgag agggccctac aagatctggc tgacctgcta gaaactggtc 20040
   aggagaagat ggcaggagac cagaaaatca tcgtgtcttc caaagaggaa atccagcaac 20100
   tacttgacaa acataaggaa tactttcagg gcctggaatc tcatatgatc ttgactgaaa 20160
   cactcttcag aaagataatc agctttgcag tccaaaagga aacccagttc catacagagc 20220
   tgatggctca ggcttctgct gtactgaaac gggctcacaa gaggggtgtg gagctggagt 20280
   acattctaga gacgtggtcc catctggatg aggaccagca ggagctcagc agacagctgg 20340
   aggtggtgga aagcagcatc ccaagcgtgg gtctggtgga ggagaacgag gacaggctta 20400
   ttgaccgcat aacactctac cagcatttaa aatctagcct taatgaatac cagcccaaat 20460
   tatatcaagt attagatgat gggaaacgac ttctgatatc catcagctgc tcagatctag 20520
   aaagccaact aaatcaactt ggagagtgct ggctaagtaa caccaataaa atgtctaagg 20580
   aacttcacag actggaaaca atattgaaac actggaccag atatcaaagt gaatctgcag 20640
   atctaattca ctggttacaa tctgcaaaag accggctaga attttggact cagcaatctg 20700
   tgacagtccc acaagagctg gaaatggtcc gtgatcatct aaatgctttc ctggagtttt 20760
   ctaaagaagt ggatgcccaa tcttccctga aatcatctgt tctgagtact ggaaatcagc 20820
   tccttcgact aaaaaaggtg gacacagcca cgctgcgctc tgagctgtcg cgcattgata 20880
   gccagtggac tgacctgcta accaatatcc cagccgtcca ggagaagctc caccagctcc 20940
   agatggataa actgccttcc cgccatgcca tttctgaagt catgagttgg atttctctaa 21000
   tggaaaatgt tattcagaag gatgaagata atattaaaaa ttccataggt tacaaggcaa 21060
   ttcatgaata ccttcagaaa tataagggtt ttaagataga cattaactgt aaacagctga 21120
   cagtggattt tgtgaaccag tccgtgctac aaatcagcag tcaggatgtg gaaagtaagc 21180
   gtagtgataa gactgatttt gctgagcaac ttggagcaat gaataaaagt tggcaaattc 21240
   tgcaaggtct agtaactgag aagatccagc tgttggaagg cttattggaa tcttggtcag 21300
   aatatgaaaa taatgtacaa tgtctgaaaa catggtttga aacccaggaa aagagactaa 21360
   aacaacagca tcgaattgga gatcaggctt ctgttcaaaa tgcactgaaa gactgtcagg 21420
   atctggaaga tttgattaaa gcaaaagaaa aagaagtaga gaaaattgag cagaatggac 21480
   ttgctttgat tcagaacaag aaagaagacg tctctagcat tgtcatgagc acactgcgag 21540
   agctcggcca aacctgggca aatttagatc acatggttgg acaattaaag atactgctga 21600
   aatcagtgct tgaccaatgg agtagtcaca aagtggcctt tgacaagata aacagttacc 21660
   tcatggaggc cagatactct ctttcccgat tccgtctgct gactggctcc ttagaagctg 21720
   tgcaagttca ggtggacaat cttcagaatc tccaagatga tctggaaaaa caggaaagga 21780
   gcttacagaa atttggctct atcaccaacc aattattaaa agagtgtcac ccacccgtga 21840
   cagaaactct taccaataca ctgaaagaag tcaacatgag atggaataac ttgctggaag 21900
   agattgctga gcagctacag tccagcaagg ccctacttca gctttggcaa agatacaagg 21960
   actactccaa acagtgtgct tcgacagttc agcagcagga ggatcgaacc aatgagctgt 22020
   tgaaggcagc cacaaacaag gacattgccg atgatgaggt tgccacatgg attcaagatt 22080
   gcaacgacct cctcaaagga ctgggcacag ttaaagattc cctctttttt ctccatgagc 22140
   tgggagagca actgaagcaa caagtggatg cttccgcagc atcagctatt caatcggatc 22200
   aactctcttt gagtcaacac ttgtgtgccc tggagcaagc tctctgcaaa cagcagactt 22260
   cattacaggc tggagttctt gattatgaaa cctttgccaa gagtttagaa gctttggagg 22320
   cctggatagt ggaagctgaa gaaatactac aagggcagga ccctagccac tcatctgacc 22380
   tctccacaat ccaggaaagg atggaagaac ttaagggaca gatgttaaaa ttcagcagca 22440
   tggctccaga tttagaccgt ctaaatgagc ttggatatag gttacccttg aatgataagg 22500
   aaatcaaaag aatgcagaat ctgaaccgcc attggtctct gatctcctct cagactacag 22560
   aaagattcag caagttgcag tcatttttgc tacaacatca gactttcttg gaaaaatgtg 22620
   aaacatggat ggaattccta gttcagacag aacaaaagtt agcagtagag atttcaggaa 22680
   attatcagca ccttttggaa cagcagagag cacacgagtt gtttcaagcc gagatgttca 22740
   gtcgtcagca gattttgcac tcaatcatta ttgatgggca acgtcttcta gaacaaggtc 22800
   aagttgatga cagggatgaa ttcaacctga aattgacact cctcagtaat caatggcagg 22860
   gagtgattcg cagggcccag cagaggcggg ggatcattga cagccagatt cgccagtggc 22920
   agcgctatag ggagatggca gaaaagcttc gtaaatggtt ggttgaagtg tcctacctcc 22980
   ccatgagtgg tctcggaagt gttcctatac cactgcaaca agcaaggacc ctctttgatg 23040
   aagtgcagtt caaagaaaaa gtgtttctgc ggcaacaagg cagctacatc ctgactgtgg 23100
   aggctggcaa gcaactcctt ctctcggcgg acagtggcgc tgaggccgcc ttgcaggccg 23160
   aactcgctga aatccaagag aaatggaaat cagccagcat gcggctggaa gaacagaaga 23220
   aaaaactagc cttcttgttg aaagactggg aaaaatgtga gaaaggaata gcagattccc 23280
   tggagaaact acgaactttc aaaaagaagc tttcgcagtc tctcccggat caccatgaag 23340
   agctccatgc agaacaaatg cgttgcaagg aattagaaaa tgcagttggg agctggacag 23400
   atgacttgac ccagttgagc ctgctgaagg acaccctctc tgcctatatc agtgctgatg 23460
   atatctccat tcttaatgaa cgcgtagagc ttctgcaaag gcagtgggaa gaactatgcc 23520
   accagctctc cttaaggcgg cagcaaatag gtgaaagatt gaatgaatgg gcagtcttca 23580
   gtgaaaagaa caaggaactc tgtgagtggt tgactcaaat ggaaagcaaa gtttctcaga 23640
   atggagacat tctcattgaa gaaatgatag agaagctcaa gaaggattat caagaggaaa 23700
   ttgctattgc tcaagagaac aaaatacagc tccaacaaat gggagaacga cttgctaaag 23760
   ccagccatga aagcaaagca tctgagattg aatacaagct gggaaaggtc aacgaccggt 23820
   ggcagcatct cctggacctc attgcagcca gggtgaagaa gctgaaggag accctggtag 23880
   ccgtgcagca gcttgataag aacatgagca gcctgaggac ctggctcgct cacatcgagt 23940
   cagagctggc caagccaata gtctacgatt cctgtaactc ggaagaaata cagagaaagc 24000
   ttaatgagca gcaggagctt cagagagaca tagagaagca cagtacaggt gttgcatctg 24060
   tcctcaacct gtgtgaagtc ctgctgcacg actgtgacgc ctgtgccact gatgccgagt 24120
   gtgactctat acagcaggct acgagaaacc tggaccggcg gtggagaaac atttgtgcta 24180
   tgtccatgga aaggaggctg aaaatcgaag agacgtggcg attgtggcag aaatttctgg 24240
   atgactattc acgttttgaa gattggctga agtcttcaga aaggacagct gcttttccca 24300
   gctcttctgg ggtgatctat acagttgcca aggaagaact aaagaaattt gaggctttcc 24360
   agcgacaggt ccacgagtgc ctgacgcagc tggaactgat caacaagcag taccgccgcc 24420
   tggccaggga gaaccgcact gattcagcat gtagcctcaa acagatggtt cacgaaggca 24480
   accagagatg ggacaacctg caaaagcgtg tcacctccat cttgcgcaga ctcaagcatt 24540
   ttattggcca gcgtgaggag tttgagactg cgcgggacag cattctggtc tggctcacag 24600
   agatggatct gcagctcact aatattgaac atttttctga gtgtgatgtt caagctaaaa 24660
   taaagcaact caaggccttc cagcaggaaa tttcactgaa ccacaataag attgagcaga 24720
   taattgccca aggagaacag ctgatagaaa agagtgagcc cttggatgca gcgatcatcg 24780
   aggaggaact agatgagctc cgacggtact gccaggaggt cttcgggcgt gtggaaagat 24840
   accataagaa actgatccgc ctgcctctcc cagacgatga gcacgacctc tcagacaggg 24900
   agctggagct ggaagactct gcagctctgt cggacctgca ctggcacgac cgctctgcag 24960
   acagcctgct ttctccacag ccttcctcca atctctccct ctcgctcgct cagcccctcc 25020
   ggagcgagcg gtcaggacga gacaccccgg ctagtgtgga ctccatcccc ctggagtggg 25080
   atcacgacta tgacctcagt cgggacctgg agtctgcaat gtccagagct ctgccctctg 25140
   aggatgaaga aggtcaggat gacaaagatt tctacctccg gggagctgtt ggcttatcag 25200
   atgtaatgat ccccgaaagc cctgaggcct atgtaaaact cacagaaaat gcaatcaaaa 25260
   atacctccgg ggaccacagt gccctagagt cacagatccg acaactgggc aaagccctgg 25320
   atgatagccg ttttcagata cagcaaaccg aaaatatcat tcgcagcaaa actcccacgg 25380
   ggccggagct agacaccagc tacaaaggct acatgaaact gctgggcgaa tgcagtagca 25440
   gtatagactc cgtgaagaga ctggagcaca aactgaagga ggaagaggag agccttcctg 25500
   gctttgttaa cctgcatagt accgaaaccc aaacggctgg tgtgattgac cgatgggagc 25560
   ttctccaggc ccaggcattg agcaaggagt tgaggatgaa gcagaacctc cagaagtggc 25620
   agcagtttaa ctcagacttg aacagcatct gggcctggct gggggacacg gaggaggagt 25680
   tggaacagct ccagcgtctg gaactcagca ctgacatcca gaccatcgag ctccagatca 25740
   aaaagctcaa ggagctccag aaagctgtgg accaccgcaa agccatcatc ctctccatca 25800
   atctctgcag ccctgagttc acccaggctg acagcaagga gagccgggac ctgcaggatc 25860
   gcttgtcgca gatgaatggg cgctgggacc gagtgtgctc tctgctggag gagtggcggg 25920
   gcctgctgca ggatgccctg atgcagtgcc agggtttcca tgaaatgagc catggtttgc 25980
   ttcttatgct ggagaacatt gacagaagga aaaatgaaat tgtccctatt gattctaacc 26040
   ttgatgcaga gatacttcag gaccatcaca aacagcttat gcaaataaag catgagctgt 26100
   tggaatccca actcagagta gcctctttgc aagacatgtc ttgccaacta ctggtgaatg 26160
   ctgaaggaac agactgttta gaagccaaag aaaaagtcca tgttattgga aatcggctca 26220
   aacttctctt gaaggaggtc agtcgtcata tcaaggaact ggagaagtta ttagacgtgt 26280
   caagtagtca gcaggatttg tcttcctggt cttctgctga tgaactggac acctcagggt 26340
   ctgtgagtcc cacatcagga aggagcaccc caaacagaca gaaaacgcca cgaggcaagt 26400
   gtagtctctc acagcctgga ccctctgtca gcagtccaca tagcaggtcc acaaaaggtg 26460
   gctccgattc ctccctttct gagccagggc caggtcggtc cggccgcggc ttcctgttca 26520
   gagtcctccg agcagctctt ccccttcagc ttctcctgct cctcctcatc gggcttgcct 26580
   gccttgtacc aatgtcagag gaagactaca gctgtgccct ctccaacaac tttgcccggt     26640
   cattccaccc catgctcaga tacacgaatg gccctcctcc actctgaact aagcagatgc     26700
   catctgcaga agtgctggta gcataaggag gatcgggtca taagcaatcc caaactacca     26760
   acaagaggac cttgatcttg gcgaaagccc tcggtgtggc agctttagcc ctcctccaga     26820
   tcacatgtgt gcaaattatg gcttcagagg tggaagataa acagtgacgg gggaacaaac     26880
   agacaacaag aaggtttgga agaaatctgg tttgagactc tgaaccttag cactaaggag     26940
   attgagtaag gacctccaaa gttccccgga ctcatgaatt ctgggccctt ggcccattct     27000
   gtgcacagcc aaggacttca gtagaccatc tgggcagctt tcccatggtg ctgctccaac     27060
   catcagataa atgaccctcc caagcaccat gtcagtgtcg tacaatctac caaccaacca     27120
   gtgctgaaga gattttagaa ccttgtaaca tacaattttt aagagcttat atggcagctt     27180
   cctttttacc ttgttttcct ttggggcatg atgttttaac ctttgcttta gaagcacaag     27240
   ctgtaaatct aaaaggcact tttttttaga ggtataaaga aaaactagat gtaataaata     27300
   agatcatgga aggctttatg tgaaaaaagt tgaatgttat agtaaaaaaa aaaagatatt     27360
   tatgtatgta cagtttgcta aagccaagtt ttgtttgtat tgatttcttt gcatttatta     27420
   tagatattat aaaata                                                     27436
<210> 31
   <211> 3894
   <212> DNA
   <213> Homo sapiens
<400> 31
   aaggtaaagc cactagagag aaactgaaag aaaacattct taagataaat tgaattgaca        60
   ttttctctct aaaatatgat ttatagacca cagataggaa ttaagagttt cctgataatt       120
   ttggcttcat attattttaa aggattatca agaggaaatt gctattgctc aagagaacaa       180
   aatacagctc caacaaatgg gagaacgact tgctaaagcc agccatgaaa gcaaagcatc       240
   tgagattgaa tacaagctgg gaaaggtcaa cgaccggtgg cagcatctcc tggacctcat       300
   tgcagccagg gtgaagaagc tgaaggagac cctggtagcc gtgcagcagc ttgataagaa       360
   catgagcagc ctgaggacct ggctcgctca catcgagtca gagctggcca agccaatagt       420
   ctacgattcc tgtaactcgg aagaaataca gagaaagctt aatgagcagc aggagcttca       480
   gagagacata gagaagcaca gtacaggtgt tgcatctgtc ctcaacctgt gtgaagtcct       540
   gctgcacgac tgtgacgcct gtgccactga tgccgagtgt gactctatac agcaggctac       600
   gagaaacctg gaccggcggt ggagaaacat ttgtgctatg tccatggaaa ggaggctgaa       660
   aatcgaagag acgtggcgat tgtggcagaa atttctggat gactattcac gttttgaaga       720
   ttggctgaag tcttcagaaa ggacagctgc ttttcccagc tcttctgggg tgatctatac       780
   agttgccaag gaagaactaa agaaatttga ggctttccag cgacaggtcc acgagtgcct       840
   gacgcagctg gaactgatca acaagcagta ccgccgcctg gccagggaga accgcactga 900
   ttcagcatgt agcctcaaac agatggttca cgaaggcaac cagagatggg acaacctgca 960
   aaagcgtgtc acctccatct tgcgcagact caagcatttt attggccagc gtgaggagtt 1020
   tgagactgcg cgggacagca ttctggtctg gctcacagag atggatctgc agctcactaa 1080
   tattgaacat ttttctgagt gtgatgttca agctaaaata aagcaactca aggccttcca 1140
   gcaggaaatt tcactgaacc acaataagat tgagcagata attgcccaag gagaacagct 1200
   gatagaaaag agtgagccct tggatgcagc gatcatcgag gaggaactag atgagctccg 1260
   acggtactgc caggaggtct tcgggcgtgt ggaaagatac cataagaaac tgatccgcct 1320
   gcctctccca gacgatgagc acgacctctc agacagggag ctggagctgg aagactctgc 1380
   agctctgtcg gacctgcact ggcacgaccg ctctgcagac agcctgcttt ctccacagcc 1440
   ttcctccaat ctctccctct cgctcgctca gcccctccgg agcgagcggt caggacgaga 1500
   caccccggct agtgtggact ccatccccct ggagtgggat cacgactatg acctcagtcg 1560
   ggacctggag tctgcaatgt ccagagctct gccctctgag gatgaagaag gtcaggatga 1620
   caaagatttc tacctccggg gagctgttgg cttatcagat gtaatgatcc ccgaaagccc 1680
   tgaggcctat gtaaaactca cagaaaatgc aatcaaaaat acctccgggg accacagtgc 1740
   cctagagtca cagatccgac aactgggcaa agccctggat gatagccgtt ttcagataca 1800
   gcaaaccgaa aatatcattc gcagcaaaac tcccacgggg ccggagctag acaccagcta 1860
   caaaggctac atgaaactgc tgggcgaatg cagtagcagt atagactccg tgaagagact 1920
   ggagcacaaa ctgaaggagg aagaggagag ccttcctggc tttgttaacc tgcatagtac 1980
   cgaaacccaa acggctggtg tgattgaccg atgggagctt ctccaggccc aggcattgag 2040
   caaggagttg aggatgaagc agaacctcca gaagtggcag cagtttaact cagacttgaa 2100
   cagcatctgg gcctggctgg gggacacgga ggaggagttg gaacagctcc agcgtctgga 2160
   actcagcact gacatccaga ccatcgagct ccagatcaaa aagctcaagg agctccagaa 2220
   agctgtggac caccgcaaag ccatcatcct ctccatcaat ctctgcagcc ctgagttcac 2280
   ccaggctgac agcaaggaga gccgggacct gcaggatcgc ttgtcgcaga tgaatgggcg 2340
   ctgggaccga gtgtgctctc tgctggagga gtggcggggc ctgctgcagg atgccctgat 2400
   gcagtgccag ggtttccatg aaatgagcca tggtttgctt cttatgctgg agaacattga 2460
   cagaaggaaa aatgaaattg tccctattga ttctaacctt gatgcagaga tacttcagga 2520
   ccatcacaaa cagcttatgc aaataaagca tgagctgttg gaatcccaac tcagagtagc 2580
   ctctttgcaa gacatgtctt gccaactact ggtgaatgct gaaggaacag actgtttaga 2640
   agccaaagaa aaagtccatg ttattggaaa tcggctcaaa cttctcttga aggaggtcag 2700
   tcgtcatatc aaggaactgg agaagttatt agacgtgtca agtagtcagc aggatttgtc 2760
   ttcctggtct tctgctgatg aactggacac ctcagggtct gtgagtccca catcaggaag 2820
   gagcacccca aacagacaga aaacgccacg aggcaagtgt agtctctcac agcctggacc 2880
   ctctgtcagc agtccacata gcaggtccac aaaaggtggc tccgattcct ccctttctga 2940
   gccagggcca ggtcggtccg gccgcggctt cctgttcaga gtcctccgag cagctcttcc 3000
   ccttcagctt ctcctgctcc tcctcatcgg gcttgcctgc cttgtaccaa tgtcagagga 3060
   agactacagc tgtgccctct ccaacaactt tgcccggtca ttccacccca tgctcagata 3120
   cacgaatggc cctcctccac tctgaactaa gcagatgcca tctgcagaag tgctggtagc 3180
   ataaggagga tcgggtcata agcaatccca aactaccaac aagaggacct tgatcttggc 3240
   gaaagccctc ggtgtggcag ctttagccct cctccagatc acatgtgtgc aaattatggc 3300
   ttcagaggtg gaagataaac agtgacgggg gaacaaacag acaacaagaa ggtttggaag 3360
   aaatctggtt tgagactctg aaccttagca ctaaggagat tgagtaagga cctccaaagt 3420
   tccccggact catgaattct gggcccttgg cccattctgt gcacagccaa ggacttcagt 3480
   agaccatctg ggcagctttc ccatggtgct gctccaacca tcagataaat gaccctccca 3540
   agcaccatgt cagtgtcgta caatctacca accaaccagt gctgaagaga ttttagaacc 3600
   ttgtaacata caatttttaa gagcttatat ggcagcttcc tttttacctt gttttccttt 3660
   ggggcatgat gttttaacct ttgctttaga agcacaagct gtaaatctaa aaggcacttt 3720
   tttttagagg tataaagaaa aactagatgt aataaataag atcatggaag gctttatgtg 3780
   aaaaaagttg aatgttatag taaaaaaaaa aagatattta tgtatgtaca gtttgctaaa 3840
   gccaagtttt gtttgtattg atttctttgc atttattata gatattataa aata 3894
<210> 32
   <211> 27745
   <212> DNA
   <213> Homo sapiens
<400> 32
   cccgggcagt ggaaaccgtg ggcaaaagtt agctggcagg acagcgcagc tcctccaggc      60
   agcggaggca gcgcgtcccg gctctcaggg acatttcctt cccacctcga cccccgggag     120
   gtggtcccgg tataaaggct cgctgagcgg gtgggtcaca gcacagcttt gcagctgcgg     180
   agaaacgccc aaggccgtgc atctccagga gggcggctgg gctcccgcag tcctgcagac     240
   cgcgcccgat cccggcgaca gggcgggcgg acagccgcgc atccccgggg tcccgccgag     300
   cctgggcgca gagagccggg aggaagcgtt cgctcgcttc gccttgctgc tgggaaactg     360
   aacgaggccg agagagaagg ttcttgagtt catgtaagag gacagtctta aaacggaaga     420
   agaaaaagaa gcagttcagt ctttgggaga gctgcctcct tgttgagtgc tgcaaaggcc     480
   tggaattcat ttatgacaga atagatctag aaaagtccaa gcatgttttc tagagtggtg     540
   tagccctgtg ctgcctccag tgaagagtct cttggtgttg gcttcgtgct tccggaggga     600
   ccatggcaac ctccagaggg gcctcccggt gtcctcggga tatcgccaat gtgatgcaga      660
   ggctgcaaga tgagcaagag atagtacaaa aacgaacttt cacaaaatgg atcaactctc      720
   atctggccaa gcggaaacct ccaatggtgg tggacgatct ttttgaagac atgaaagatg      780
   gtgttaaact gcttgccctt ctggaggtcc tgtctgggca gaaactgcct tgtgaacaag      840
   gacgccggat gaagcgaatc catgctgtgg ctaacattgg cacggcactc aagttcctcg      900
   aaggaagaaa gattaaatta gtcaacatta actccaccga tatagctgat ggccgaccct      960
   caatagttct tggattgatg tggaccatta ttctatattt ccagattgaa gagttgacca     1020
   gcaacctgcc ccagctccag tctttgtcca gcagcgcatc ctccgtggac agcatagtta     1080
   gctctgagac tcccagccca ccaagtaaac ggaaggtgac caccaagatc caaggaaatg     1140
   ctaagaaggc tttattaaag tgggttcagt acacagctgg caagcagact ggaatagaag     1200
   taaaagattt tgggaagagt tggagaagcg gggttgcctt tcattcagtt attcatgcca     1260
   ttcgaccgga attggtggac ttggagacag tgaaaggcag atccaaccga gaaaatttgg     1320
   aggatgcttt cactatcgcc gaaacagaac tggggatccc aagactgcta gatcctgaag     1380
   acgttgatgt ggataaacca gatgagaaat ctattatgac ctatgtagcc cagtttctga     1440
   aacattatcc tgacatccac aatgcaagca ctgatgggca agaggatgat gaaatacttc     1500
   caggtttccc atcttttgca aattctgtac aaaattttaa gagagaagac agagtaattt     1560
   ttaaggaaat gaaagtttgg atagaacaat ttgagagaga tttgacaaga gcacagatgg     1620
   tggaatcaaa tttacaggat aaatatcagt catttaagca cttcagagtt caatatgaaa     1680
   tgaagaggaa acagattgaa catttaatac aaccattaca cagagacggt aaattgtcac     1740
   ttgaccaagc attggtaaaa caatcttggg atagagtgac ctccaggctc tttgactggc     1800
   atatacagct tgataaatct cttcctgcac ctctgggcac cataggtgcc tggctgtaca     1860
   gagcggaggt ggccctgaga gaggaaataa ccgttcaaca ggtccacgag gaaacagcaa     1920
   acacgataca acggaaactt gagcaacata aggatctgct tcaaaacacg gatgcccaca     1980
   aaagagcatt ccatgaaatc taccggacca ggtctgttaa cgggattcca gtgccacctg     2040
   atcaattaga ggacatggcc gagaggtttc attttgtttc ctccacatca gagctacacc     2100
   taatgaaaat ggaattttta gaattaaagt accgtctgct ctcactgctg gttcttgcag     2160
   agtcaaagct gaagtcttgg atcattaagt acgggaggag agagtcagtg gagcagcttc     2220
   tacaaaacta cgtgtctttt atagaaaata gcaagttctt tgaacaatat gaggtgacat     2280
   accagatctt gaaacagaca gctgagatgt atgtcaaagc agatggttca gtggaagaag     2340
   ctgagaatgt gatgaaattc atgaatgaaa ccaccgctca gtggaggaat ctctcagtag     2400
   aagtgaggag tgtgaggagc atgctggaag aagtgatctc taactgggat cgctatggca     2460
   atacagtggc tagtctgcaa gcctggctag aggatgctga aaaaatgctc aatcaatcag     2520
   aaaatgccaa aaaggatttt tttcgaaatt tacctcattg gattcagcag catactgcca     2580
   tgaacgatgc tggcaatttt ctaattgaaa cctgtgatga gatggtttcc cgtgacctga 2640
   agcagcaatt actgttgcta aatgggcggt ggagggagtt gtttatggaa gtcaagcaat 2700
   atgctcaagc tgatgagatg gacagaatga agaaggaata cacagactgt gttgttaccc 2760
   tgtctgcttt tgcaacggaa gcccataaga aactttctga acccttagaa gtctctttta 2820
   tgaatgtcaa gctattaatt caagacttgg aggatattga gcagagggtg cctgtgatgg 2880
   atgcccaata caagataatt acaaagacag cacacctcat taccaaagaa agcccccaag 2940
   aagaaggaaa agaaatgttt gcgaccatgt caaagctcaa agagcagcta accaaggtca 3000
   aagaatgtta ctccccactc ctttatgagt ctcagcagct gttgattccg ttggaggaat 3060
   tagaaaagca gatgacgtcc ttttatgact cacttgggaa aatcaatgaa attatcacag 3120
   ttcttgagcg tgaggcacaa tcgagtgccc tttttaaaca aaaacatcag gaactgttag 3180
   cttgtcaaga aaactgtaag aaaaccttga cacttattga gaaaggcagt caaagtgttc 3240
   aaaagtttgt gaccttgagc aacgtgttaa agcattttga tcagacgagg ctacaaagac 3300
   agattgcaga tattcatgtt gcttttcaga gtatggtaaa gaaaactgga gattggaaga 3360
   agcatgtgga aaccaacagt cgcttgatga agaagtttga ggagtctcga gcagagttgg 3420
   agaaggtact gcggattgct caggagggcc tggaggaaaa gggggatcca gaggagctcc 3480
   tgcggagaca cactgagttt ttcagtcagc tggatcagag ggtgctcaat gctttcctga 3540
   aagcttgtga tgaactcacc gacatccttc cagagcagga gcagcagggg ctgcaggaag 3600
   ctgttcgaaa gctccacaaa caatggaagg atcttcaagg agaagcccct tatcatttgc 3660
   ttcatctgaa gattgatgtg gagaagaata ggttcttagc ctctgtagaa gaatgcagaa 3720
   ctgagctgga tcgagagacc aagctgatgc cccaggaagg cagtgaaaag ataattaaag 3780
   agcacagggt tttcttcagt gacaaaggtc ctcatcatct ctgtgagaaa aggttacagc 3840
   tcatcgagga actctgtgtg aaactcccag tgcgggaccc agtaagggac acacctggaa 3900
   cctgtcacgt gactctcaaa gagctcagag ctgccattga cagcacctac aggaagctca 3960
   tggaagaccc agacaagtgg aaggactaca ctagcagatt ctctgagttc tcatcttgga 4020
   tatctacaaa tgagacacaa ttaaagggga tcaagggtga ggccatcgat actgccaacc 4080
   acggagaggt taaacgtgcc gttgaagaga tcagaaatgg tgttaccaaa aggggtgaga 4140
   ccctcagctg gctgaaatcc aggctgaaag ttttgacaga agtttcttct gagaatgaag 4200
   cccaaaagca gggagatgag ctggcaaaat tatccagctc tttcaaggct cttgtgacgc 4260
   tgctgtcaga ggttgaaaag atgctaagca attttgggga ctgtgtccag tacaaagaaa 4320
   tagtcaaaaa ttctctcgaa gaattaattt ctggctctaa agaagtccag gaacaagctg 4380
   agaagatctt ggatactgaa aatctgtttg aagcacagca gttacttctt catcaccagc 4440
   aaaagacaaa gcggatctca gcaaagaaga gagatgtgca gcagcagatc gcgcaggcgc 4500
   agcagggaga aggggggctg cctgaccgag gccacgagga gctgcggaag ctggagagca 4560
   cactggatgg cctggagcgc agccgggaga ggcaggaacg ccgcatccag gtcacattaa 4620
   gaaaatggga gcgatttgaa acaaacaaag aaacagtagt aagatacctt tttcaaacag 4680
   gttccagtca tgaacgcttc ttgagtttta gcagtttgga aagtttatct tcagaactgg 4740
   aacaaacaaa ggagttttct aaacggacag aaagtattgc agtccaggct gagaaccttg 4800
   taaaggaagc ttcagagata ccgcttgggc cccaaaataa gcagctgctt caacagcagg 4860
   ccaagtcaat caaagaacaa gtcaaaaaat tagaagacac gcttgaagaa gatattaaaa 4920
   ccatggaaat ggtgaaaacc aagtgggatc attttggcag taattttgag actctgtccg 4980
   tctggataac tgagaaagaa aaagaactca atgccttgga aacttcgtca tctgccatgg 5040
   acatgcaaat cagccaaatt aaggtcacaa ttcaggaaat agaaagtaag ctcagcagca 5100
   ttgtaggatt agaagaagaa gcccagtctt ttgctcagtt tgttaccact ggagaatctg 5160
   ctcgaattaa agccaagttg acacaaataa gaagatacgg ggaagagctt cgagagcatg 5220
   cacagtgtct ggaaggaaca atcctgggac atttatctca gcagcaaaag tttgaagaga 5280
   accttagaaa gatccagcaa tctgtgtctg aatttgaaga taaacttgct gttccaatta 5340
   aaatatgttc ttcagctaca gaaacataca aagttcttca agaacatatg gatctctgcc 5400
   aggccctgga gtcactgagc agcgcgatca ctgccttctc agccagtgcc aggaaggttg 5460
   tgaacagaga ttcctgtgtt caggaggctg cggctctaca gcagcaatac gaggacatcc 5520
   taaggagggc gaaggagaga cagacggcgc tggagaatct gctggcccac tggcagaggc 5580
   tagagaaaga actatcatcc tttttgacct ggttagagcg gggtgaagct aaagccagtt 5640
   ccccagaaat ggacatttct gcagacagag tcaaagtgga aggtgaactt cagttaatac 5700
   aggcactgca aaatgaagtt gtatcccagg cctcattcta tagcaaactt ttgcaattga 5760
   aggaatcatt gttctcagta gcctccaaag atgatgtgaa aatgatgaaa ctacatttgg 5820
   agcagttgga tgagagatgg agagatttac cacagatcat taacaaaagg attaattttc 5880
   ttcagtctgt ggttgctgaa caccagcaat ttgatgagct gctgctttcc ttttctgtct 5940
   ggattaaact gtttctcagt gaattacaaa ctacctctga gattagcata atggaccatc 6000
   aagtagccct tactcggcat aaggaccacg cagcagaagt agagagcaaa aagggcgaat 6060
   tgcagagtct gcagggtcac ttagcaaagt tgggttctct gggccgtgct gaggacctcc 6120
   acctcctgca gggaaaggct gaggactgct tccagctgtt tgaggaggcc agccaggttg 6180
   tggagaggcg gcagcttgcc ctgtcccatt tggcagaatt cctccagagc catgcctctc 6240
   tgtccggcat tctccgccag ctgaggcaaa cagtggaagc aaccaacagt atgaataaga 6300
   acgagtctga tttgatagaa aaggacctca atgatgctct tcaaaatgct aaagcattag 6360
   aatctgctgc cgtcagtctg gatggcattc tttccaaagc ccaataccat ctgaaaatcg 6420
   ggagctctga gcaaaggact tcctgcagag ccacggctga tcagctctgt ggagaggtag 6480
   agaggatcca gaaccttctg ggaaccaagc agagtgaggc agatgctctg gcagtgttga 6540
   aaaaagcatt ccaagaccag aaagaggagc ttctgaaaag cattgaggac attgaagaaa 6600
   ggactgacaa agagcgattg aaagaaccta cccgccaagc tcttcagcag aggttaagag 6660
   tgtttaatca gctagaagat gaattgaatt ctcacgagca tgaactatgt tggttgaaag 6720
   acaaagccaa gcaaattgcc cagaaagatg tagcttttgc acctgaagtt gacagggaga 6780
   taaaccgctt agaggtcacc tgggatgata ccaaaagact aattcatgaa aatcagggtc 6840
   agtgctgtgg acttattgac ttaatgagag aatatcagaa cctgaaatca gctgtatcta 6900
   aagtcttaga aaatgccagc agtgtgattg taaccagaac taccataaaa gatcaggagg 6960
   atcttaaatg ggctttttcc aagcatgaaa ctgccaagaa caaaatgaat tacaaacaga 7020
   aagacttgga taactttacc agcaaaggaa aacacttgtt atctgagctg aagaaaattc 7080
   acagtagtga tttcagcttg gtgaaaacag acatggagag caccgtggac aaatggctgg 7140
   atgtatcaga gaaacttgaa gaaaacatgg ataggctgag agtaagcctg tccatttggg 7200
   atgatgtact gtcaactaga gatgagattg agggatggtc aaacaactgc gttccacaga 7260
   tggcagaaaa catcagcaac ctggataacc acctcagagc tgaagaactg cttaaagaat 7320
   ttgagtctga agttaaaaac aaagcattga gattggaaga actgcattcc aaagttaatg 7380
   atctgaaaga attaactaaa aatctagaaa caccgccaga ccttcagttt atagaagcag 7440
   acttaatgca gaaactggag catgccaaag aaataactga agtagcaaaa ggaaccctga 7500
   aggatttcac ggctcaaagt acacaagtgg agaagtttat taatgacata acaacatggt 7560
   tcacaaaagt ggaagaatcg ttgatgaact gtgcccaaaa tgagacttgt gaagcattga 7620
   aaaaagtcaa ggatatacaa aaagaacttc aaagtcaaca aagcaacatc agctctaccc 7680
   aagaaaatct caatagcttg tgccgcaagt accactcagc tgagttggag agcctgggcc 7740
   gtgcaatgac tggtctgata aagaaacatg aagccgtgag ccagttgtgc tccaaaaccc 7800
   aggccagcct gcaggaatct ctggaaaaac acttcagtga gtctatgcag gaattccaag 7860
   aatggttttt gggagcaaag gcagcagcaa aagaatcatc agatcgcacc ggtgacagca 7920
   aagttctaga agcaaagctc catgatcttc agaacatttt ggactcagtc agtgatgggc 7980
   agagcaaact tgatgcagtg actcaagaag gacaaacttt gtatgcacat ttgtctaaac 8040
   aaattgtcag tagcattcaa gaacaaatca caaaggccaa tgaagagttt caagcatttc 8100
   tgaaacaatg ccttaaagat aagcaggctc ttcaagactg tgcttcagaa cttggaagct 8160
   ttgaagatca gcacagaaaa ctgaacttat ggatccatga aatggaagaa aggttcaata 8220
   cggaaaactt gggagagagt aaacagcaca ttcctgagaa gaaaaatgaa gttcataaag 8280
   ttgaaatgtt tttgggagaa ctgctggctg caagagagtc tcttgataag ctttcccaga 8340
   gagggcagct tctgagtgaa gaaggccacg gtgctgggca ggagggccgc ctgtgttccc 8400
   agctcctcac aagccaccag aacctactta gaatgaccaa agagaaactc cggagctgcc 8460
   aggtggccct tcaggagcac gaagccctgg aggaagcact gcaaagcatg tggttctggg 8520
   tgaaggccat tcaggacaga ctggcctgtg cagagagcac tcttgggagc aaagacaccc 8580
   tggagaaacg gctgtcacaa atacaggata ttctcctgat gaaaggtgaa ggggaagtta 8640
   agttgaatat ggccattggc aagggggaac aggccttgag aagtagcaac aaagaaggtc 8700
   agagggtgat tcagactcag ttagagaccc ttaaagaagt gtgggctgac atcatgagct 8760
   cctccgtcca cgctcaaagc actttagagt ctgtgattag ccaatggaat gactatgtag 8820
   agaggaaaaa ccagttggag cagtggatgg aatcagtgga tcaaaaaata gaacatccct 8880
   tacaaccaca gccaggtctg aaagagaagt tcgtcctgct tgaccacctc cagtccatcc 8940
   tgtctgaggc agaagatcac acgagagccc ttcaccgtct aattgcgaag tccagggagc 9000
   tctacgaaaa gacagaggat gagtctttca aggacacagc tcaagaggag ctgaaaacac 9060
   agtttaatga tataatgact gttgccaagg aaaaaatgag gaaagtggaa gagattgtga 9120
   aagatcatct aatgtattta gatgcggtcc acgagttcac agattggctc cattcagcaa 9180
   aggaagaact tcaccggtgg tcagatatgt ctggagattc atcagccacc cagaaaaagt 9240
   tatcaaaaat taaggagctg atagattcca gagagattgg tgcaagccgt ctcagcagag 9300
   tggagtcgct ggctcccgaa gtgaaacaga acacaactgc cagtgggtgt gagctcatgc 9360
   acacggagat gcaggccctg cgtgccgact ggaagcagtg ggaagacagt gtattccaaa 9420
   cgcagagctg tttggagaac ctggtcagcc agatggccct ttcggagcag gaattctcag 9480
   gccaagtggc tcaactggag caggccctgg aacagttcag tgcccttctg aaaacctggg 9540
   ctcagcagtt aaccctcctg gaaggcaaga acacggatga ggagatagtg gaatgctggc 9600
   acaaaggaca agagatactg gatgctttgc aaaaagcaga gcctagaaca gaggatctca 9660
   agtctcagct gaatgaactt tgtcgatttt ccagagacct gagtacctac agtggaaaag 9720
   tttctggctt gattaaagag tataattgtc tttgtttgca agcatccaag ggctgccaga 9780
   ataaagaaca gattttacag caaagatttc gaaaggcctt cagggatttc cagcagtggt 9840
   tggttaatgc aaaaatcact accgccaagt gttttgatat acctcaaaat ataagtgaag 9900
   tttcaactag tcttcagaaa atacaggagt ttttgtcaga aagtgaaaat ggacagcaca 9960
   agctaaacat gatgctgtct aaaggggaac ttctgagtac cctgctgacc aaagagaaag 10020
   cgaaagggat ccaggccaaa gttacagctg caaaagaaga ttggaaaaat tttcattcaa 10080
   atctccacca aaaagaatct gctctagaga atctaaagat ccaaatgaag gactttgaag 10140
   taagtgctga gcctatccag gactggctga gtaaaactga gaagatggtc catgaaagca 10200
   gcaatcgcct ctatgatctg ccagcaaaga ggagggagca gcagaagctc cagtctgtcc 10260
   ttgaggaaat acactgctac gagcctcagc tcaacaggct gaaagagaaa gctcagcagc 10320
   tgtgggaagg acaagctgcc agcaagagct ttaggcacag agtgtcgcag ctgtcttctc 10380
   agtatctagc gctaagcaat ttaacaaagg agaaagtgtc aagactggat agaatcgttg 10440
   cagaacacaa tcagttctct cttgggatta aagaattaca agactggatg acggatgcga 10500
   ttcacatgct ggattcatac tgccacccga catccgacaa aagtgtgctg gacagcagga 10560
   cgctcaagct cgaggctcta ttatcagtca aacaggaaaa agagattcag atgaaaatga 10620
   tagtgaccag gggagaatct gtccttcaga atacttctcc agaaggcatt cccactattc 10680
   agcagcagct gcagagtgtg aaggatatgt gggcatccct tttgtctgca gggattcgtt 10740
   gtaaaagcca actcgaagga gctctctcca agtggacaag ttatcaggat ggcgttcgac 10800
   agttctccgg ttggatggat agtatggaag ccaacctgaa tgaatcagaa aggcagcatg 10860
   cggagctgcg ggataaaaca acgatgctcg gaaaagccaa gttattaaat gaagaagtgc 10920
   tgagttacag cagcttgctg gagaccatcg aagtcaaagg ggctggcatg acagaacact 10980
   atgtcaccca gctagaactc caggatctac aggaacgata cagagccatc caagagaggg 11040
   ccaaggaagc cgtaaccaag tctgaaaaac ttgtccgcct gcaccaagag tatcagagag 11100
   acctaaaggc atttgaagtt tggttggggc aagaacaaga aaagctcgac cagtattcag 11160
   ttcttgaagg tgatgcccac actcatgaga caacattgcg tgatcttcag gagctacagg 11220
   tacactgtgc agaggggcag gccctgttga actcagtgct gcacaccaga gaggatgtga 11280
   tcccatcagg tatcccacag gcagaggacc gggctttaga gtctctccgg caagactggc 11340
   aggcttacca gcacaggctg tccgagactc gaactcagtt caataacgtg gtgaacaaat 11400
   tgaggctaat ggagcaaaag tttcagcaag tagatgaatg gctcaaaaca gcagaggaga 11460
   aagttagtcc caggaccaga cgtcagtcta acagggcaac caaggagata caattacatc 11520
   agatgaagaa gtggcacgaa gaagtgactg catacagaga tgaagttgag gaagtgggag 11580
   ctagagctca ggagatactg gacgagagcc acgtgaacag cagaatgggt tgccaggcca 11640
   cccagctgac ttccagatac caggccctgc ttctccaagt gctggaacaa ataaaattcc 11700
   tggaggagga gattcagagt ttggaggaat cagaatcatc cctcagttcc tattctgatt 11760
   ggtatggctc tactcataaa aacttcaaga atgtggctac caagattgac aaagtagata 11820
   cagtaatgat ggggaagaaa ttgaagacgt tggaggtttt gctcaaagac atggagaaag 11880
   gtcacagttt gctgaaatca gcccgggaga aaggagagag ggctgttaaa tacttggagg 11940
   aaggcgaggc agagaggtta agaaaggaga ttcatgatca catggagcag ttgaaggaac 12000
   tgaccagcac tgtccggaaa gaacacatga cgctggaaaa aggtcttcat ttagcaaagg 12060
   aattctcaga taaatgcaaa gcactgacac agtggatagc agaataccag gaaattctac 12120
   atgttcctga agaacccaaa atggaattat atgagaaaaa agctcagtta tctaaataca 12180
   agtcacttca acaaacggtg ctgtcccatg aaccatcagt aaagtcagtg agagagaagg 12240
   gtgaagctct tttggaactg gtgcaggacg tcactttaaa ggacaaaata gatcaacttc 12300
   aaagtgatta ccaggacctg tgcagcatag gaaaggagca tgtcttcagt ctggaggcga 12360
   aagtcaaaga ccatgaagac tacaacagtg agctccaaga ggtcgaaaag tggctgctgc 12420
   agatgtctgg cagactggtg gcacctgacc tcctggagac aagcagcctg gagacaatca 12480
   cccagcaatt ggcccaccac aaggcaatga tggaagaaat tgctggtttt gaagaccgtt 12540
   tgaacaatct tcaaatgaaa ggtgatactt tgattggcca atgtgcagac cacctgcaag 12600
   cgaaacttaa acaaaacgtg catgctcatc tgcagggcac aaaggacagc tactcagcga 12660
   tctgcagcac agctcagagg atgtaccaga gtttggaaca cgaacttcag aagcacgtca 12720
   gccgacaaga caccctgcag cagtgccagg cctggctttc tgcagtccag ccggatttag 12780
   agccaagtcc tcaaccacct cttagtaggg cagaagccat taagcaggtc aaacacttca 12840
   gagctttgca agagcaggca aggacctacc tagatctcct ttgctccatg tgtgacctgt 12900
   caaatgcttc ggtgaaaacc acagcaaaag acattcaaca aacagagcaa acgattgaac 12960
   aaaagcttgt ccaggcccag aacttaactc agggctggga agagatcaag cacctgaagt 13020
   ctgagctctg gatttacctg caagatgctg atcagcaact gcagaacatg aagaggaggc 13080
   actctgagct ggagctgaac attgcacaga acatggtttc acaagttaag gattttgtta 13140
   agaaactaca gagcaaacag gcatccgtga acaccataat agaaaaggtg aataagttaa 13200
   caaagaagga ggaatcgcct gaacacaagg aaataaatca tttaaatgat cagtggctcg 13260
   atttgtgccg tcagtctaac aacctgtgct tgcaaaggga agaggatctt cagagaacaa 13320
   gagattacca tgactgtatg aatgttgttg aagtgttcct agaaaaattt actacagaat 13380
   gggataactt ggccagatct gatgcagaga gtacagctgt ccacctggaa gctttgaaaa 13440
   agttagcatt ggcattgcag gagagaaagt atgctattga agatctgaaa gatcaaaagc 13500
   agaaaatgat agagcatctg aatttagatg acaaggagtt agtcaaagaa cagacgagtc 13560
   atttagagca acgttggttt cagcttgagg acctcattaa aaggaaaatc caagtgtcag 13620
   tcaccaactt ggaggagtta aatgtggtgc agtccagatt tcaggagcta atggagtggg 13680
   cagaagagca acaacccaac atcgccgagg cccttaagca gagccctcct ccagatatgg 13740
   ctcagaacct tctcatggat cacctggcca tctgcagtga actggaggcc aagcagatgc 13800
   tcctgaaatc gcttataaag gacgcagaca gggtcatggc agatcttggt ctcaatgagc 13860
   gacaggtcat ccagaaggct ctctctgatg cacaaagcca cgtgaattgt ctcagtgact 13920
   tagtgggcca gcgaagaaag tacttaaaca aagccttgtc cgagaaaacc cagtttctca 13980
   tggcagtgtt ccaggccacc agccaaattc agcaacatga gcgaaagata atgttccgtg 14040
   aacacatctg tctgttacca gatgatgtga gcaaacaagt caaaacatgt aagagtgcac 14100
   aagccagcct caagacttac caaaatgaag tcactggact ttgggcccag ggtcgcgaac 14160
   taatgaagga agtcacagag caggaaaaga gtgaagtgct ggggaagctt caggaattgc 14220
   agagtgtcta tgacagtgtt ttacaaaagt gcagtcaccg gttacaagaa ctagagaaga 14280
   atttggtttc taggaagcat tttaaggaag attttgataa agcttgccac tggctaaaac 14340
   aagcagatat tgttacattt cctgaaatca acctaatgaa tgagagttct gagcttcata 14400
   cacaactggc taaataccaa aacattcttg aacaatctcc agaatatgaa aatcttctac 14460
   ttacgctgca gagaactggg cagaccatat taccatcgct gaatgaagtc gatcattcct 14520
   acctcagtga aaagctaaat gctttgcctc gacaatttaa tgtaattgtt gccttggcta 14580
   aagacaagtt ctataaagtc caggaagcaa ttcttgctcg gaaggaatat gcttccttga 14640
   ttgagttgac aacccagtct ctgagtgaac ttgaagccca attcttgagg atgagcaaag 14700
   ttcccaccga cctggccgtt gaggaggctc tttctctgca agatggttgc agagccattc 14760
   tggacgaggt ggcgggcctt ggggaggcgg tggatgaact gaaccagaaa aaagaaggtt 14820
   ttcgcagcac aggtcagcct tggcagccag acaagatgct gcaccttgtc accttatatc 14880
   acaggctgaa gcgacaaaca gaacagaggg ttagcttatt agaagacacc accagtgctt 14940
   accaagaaca cgagaagatg tgccaacagc tggagagaca actgaagtct gtaaaagagg 15000
   agcagtccaa agtgaatgag gaaacgctgc ctgcagagga gaagctcaaa atgtatcact 15060
   ccctggcagg aagtctccag gactcaggga ttgtactgaa acgagtaacc atacatcttg 15120
   aagatcttgc cccacacctt gaccccttgg cttatgagaa agccaggcat cagatccagt 15180
   cctggcaagg ggagttaaaa ctgttgactt ctgccattgg tgagacggtg acagaatgtg 15240
   agagccgaat ggtgcagagt atagacttcc agactgagat gagtcgctcc ctggactggc 15300
   tgaggagagt gaaggcagag ctcagtgggc cggtgtacct agacctcaac ctgcaggaca 15360
   tccaagagga aatcagaaaa atccaaattc atcaggaaga ggtccagtcc agcttgagaa 15420
   tcatgaatgc gctgagtcac aaggaaaagg agaagttcac aaaggccaag gagctgattt 15480
   ctgcggattt agaacacagc ctcgctgagc tctcagagct ggatggagac atccaggaag 15540
   ccttacgcac cagacaggct accttgactg aaatatatag ccagtgtcaa aggtattatc 15600
   aggtatttca agcagccaat gactggcttg aggatgccca agaattgtta cagctggcag 15660
   gcaatggcct agacgtggag agcgcagagg aaaatctcaa aagccacatg gaatttttca 15720
   gtacagagga tcagttccat agtaacctgg aggagctcca cagcctggta gccaccctgg 15780
   acccactcat caagccaacc ggcaaagaag acctagaaca gaaagtggct tctctggaac 15840
   tcaggagcca gaggatgagc cgggactctg gtgcccaagt ggatctcttg cagagatgca 15900
   cagctcaatg gcacgattac cagaaagcaa gggaagaggt tattgaattg atgaatgata 15960
   cagaaaagaa attgtctgag ttttctttgt tgaagacttc gtctagtcat gaagcggaag 16020
   aaaaattgtc agaacacaag gctttagtgt cagtggttaa ctctttccat gagaaaattg 16080
   tggcccttga ggaaaaagct tcacaactgg agaaaaccgg aaatgatgcc agcaaagcca 16140
   ccctgagcag gtcaatgacc accgtctggc agcgctggac acgccttcga gctgtggccc 16200
   aggaccagga gaagatcctg gaagatgcag tggatgagtg gacgggcttt aacaacaagg 16260
   ttaaaaaggc cactgaaatg attgatcagc tgcaagataa gttacctgga agttcagcag 16320
   agaaagcatc gaaagcagag ctcttaactc ttcttgaata ccacgacacg ttcgttctgg 16380
   agctggagca gcagcagtcg gccttgggca tgctgcggca gcaaaccctg agcatgctcc 16440
   aggatggagc cgccccaacc cctggggaag agcctccgct catgcaggaa atcaccgcca 16500
   tgcaagatcg gtgcctgaac atgcaggaga aagtgaagac taatggaaag ttggtgaagc 16560
   aagagctgaa ggaccgagaa atggtggaga ctcagatcaa ttctgtgaaa tgttgggttc 16620
   aggaaacgaa agaatattta gggaatccaa caatagaaat agatgctcaa cttgaagaac 16680
   ttcagattct cctaacagaa gccacaaatc accgacagaa cattgaaaaa atggcagaag 16740
   aacagaagga gaagtactta ggtctttata ccatattacc ttctgaactc tcccttcagt 16800
   tggctgaagt ggcgttagat ctaaagatcc gagatcagat ccaagacaaa ataaaagaag 16860
   ttgagcagag caaggccacg agccaggaac tcagccggca aattcagaag ttagctaaag 16920
   acctcacaac tattctaact aagctgaaag cgaagacaga taatgtagtt caagctaaaa 16980
   ctgaccaaaa ggtgctggga gaggaattag atggctgtaa ttcaaagtta atggaattag 17040
   atgcagcagt acagaaattc ttggaacaga atggccaact gggtaagcca ctggccaaga 17100
   agataggaaa actgactgaa cttcaccagc agaccattag acaagctgag aatcggctct 17160
   ccaagctcaa tcaggcagca tcacatttag aagaatacaa tgaaatgctt gaattaattt 17220
   tgaagtggat tgaaaaagct aaagtcttgg ctcatggaac tattgcatgg aattctgcaa 17280
   gccagcttcg ggaacaatat attttgcatc agaccctgct agaagaatcc aaagaaattg 17340
   acagtgagct ggaagcaatg actgagaaat tacagtacct cactagcgtg tactgtacag 17400
   aaaaaatgtc tcagcaagtg gcagaactgg gacgggagac tgaggagttg cgacagatga 17460
   tcaaaattcg tttgcagaac ctccaagatg cagctaagga tatgaaaaaa tttgaagcag 17520
   agttgaaaaa gttacaagct gccttggagc aagcccaggc aacactgact tctccagaag 17580
   ttggacgtct cagtctcaag gagcagctct ctcatcggca gcatttgttg tctgagatgg 17640
   agtcactgaa gccgaaggtg caagcagtgc agctctgcca gagtgccctc cggatccccg 17700
   aggatgtggt tgccagctta cctctctgtc atgctgctct gcggctgcag gaagaggcca 17760
   gccggctgca gcacaccgcc atccagcagt gtaacatcat gcaggcagct gtggtacaat 17820
   atgaacaata tgagcaagaa atgaaacatc tccagcaact gatagaagga gctcacagag 17880
   agattgagga taaacctgtt gccaccagta acatacagga gctgcaggct cagatttctc 17940
   ggcatgagga gctggcgcag aaaattaagg gctaccagga gcagatcgct tctttgaatt 18000
   ccaagtgcaa gatgctgacg atgaaagcca agcacgccac catgctgctg acggtgaccg 18060
   aggtcgaggg gctggcggaa gggacagagg acctggatgg ggagctcctc cccacgcctt 18120
   cggcccaccc ctctgtggtc atgatgactg caggtcgctg tcacactttg ctgtcaccgg 18180
   tcactgagga gtctggggag gagggaacca acagtgagat ttcctctcca cctgcctgtc 18240
   gctccccttc acctgtggct aatacagatg cttctgttaa ccaggacatt gcatattacc 18300
   aagccttgtc tgctgagagg ttgcagacag atgctgcaaa aattcacccc agcacatccg 18360
   catcccagga gttctatgaa ccgggattgg agccatccgc tactgccaaa ctgggtgatt 18420
   tgcagcgttc ttgggaaacc ttaaagaatg tgatcagtga gaagcagcgc acactctatg 18480
   aagctttgga acgccagcag aagtaccagg actccctcca gtccatctct acgaagatgg 18540
   aggccattga gctgaaactc agtgagagcc cagagcctgg caggagtcca gaaagccaga 18600
   tggctgaaca tcaggcattg atggatgaga ttctcatgct ccaggatgaa atcaatgagc 18660
   tccagtcctc tctcgcagag gagctggtat ccgagtcttg tgaggccgac cctgcggagc 18720
   agctggcctt gcagtccacg ctcactgtct tagccgagcg aatgtccacc atcaggatga 18780
   aagcctcggg gaaacggcag cttttggagg agaagttgaa tgatcagctg gaggaacaaa 18840
   ggcaggaaca ggccctgcag aggtatcgct gtgaagccga tgagctggac agctggctct 18900
   tgagtaccaa ggccactctg gacactgcgc tgagtccacc caaggagccc atggacatgg 18960
   aggcccagct tatggactgc cagaatatgc tggtggaaat agagcagaag gtggtggctt 19020
   tatcagaact gtcagtccac aatgagaacc tgctgctgga gggcaaagct cacaccaagg 19080
   acgaggccga gcagctggct ggaaagctga gaaggctcaa ggggagcctg ctggagctgc 19140
   agagagccct gcatgataag cagctcaaca tgcagggaac agcacaggag aaggaggaga 19200
   gcgatgttga cctaacagcc acgcagagcc ccggcgtcca ggaatggctg gcccaagctc 19260
   gcaccacatg gacccagcag cggcagagca gtctccagca acaaaaagag ttagaacagg 19320
   aattagccga gcagaagagt ctccttcgct cagtagccag tcgtggagag gagattctaa 19380
   ttcaacattc ggcggcagag acctctggtg atgctggcga aaaacctgat gtgttatccc 19440
   aggagttggg gatggaaggg gagaaatcat ccgctgaaga ccagatgaga atgaaatggg 19500
   aaagcctaca tcaagaattt agtaccaagc agaaactact acagaatgtt ctggaacagg 19560
   aacaagagca agtgctttat agcaggccaa atcgactctt gtctggtgtg ccactgtaca 19620
   aaggggacgt gccaacccaa gataaatctg cagttacatc tttgctggat ggactgaacc 19680
   aagccttcga ggaggtttca tcccagagtg gaggggcaaa gaggcagagt atacacttgg 19740
   agcagaagtt gtatgatgga gtctcagcca cctctacttg gttggatgac gttgaagaac 19800
   gtttatttgt tgccacagca cttttaccag aagaaacaga gacttgtctc ttcaaccaag 19860
   agattcttgc caaagacatt aaggaaatgt ctgaagaaat ggataagaac aaaaacttgt 19920
   tttcccaagc ttttccagag aatggtgata atcgagatgt tattgaagat actttgggtt 19980
   gtcttttggg caggttatcc ttgctagact cagtagtgaa tcaacgatgt catcagatga 20040
   aagaaagact tcagcaaata ctaaatttcc agaatgatct gaaagtgctg tttacatcac 20100
   tggctgacaa caaatacatc attctgcaaa aactggcaaa tgtgtttgaa cagcccgtag 20160
   cagaacaaat agaggcaata caacaggctg aagatggact caaagaattt gatgcaggaa 20220
   tcattgaatt aaagaggcgt ggtgacaagc tacaggtcga gcagccgtcc atgcaagaac 20280
   tctccaagct ccaggacatg tatgatgagc tgatgatgat cattggctcc cggaggagtg 20340
   gtctgaatca gaaccttaca ctcaagagtc agtatgagag ggccctacaa gatctggctg 20400
   acctgctaga aactggtcag gagaagatgg caggagacca gaaaatcatc gtgtcttcca 20460
   aagaggaaat ccagcaacta cttgacaaac ataaggaata ctttcagggc ctggaatctc 20520
   atatgatctt gactgaaaca ctcttcagaa agataatcag ctttgcagtc caaaaggaaa 20580
   cccagttcca tacagagctg atggctcagg cttctgctgt actgaaacgg gctcacaaga 20640
   ggggtgtgga gctggagtac attctagaga cgtggtccca tctggatgag gaccagcagg 20700
   agctcagcag acagctggag gtggtggaaa gcagcatccc aagcgtgggt ctggtggagg 20760
   agaacgagga caggcttatt gaccgcataa cactctacca gcatttaaaa tctagcctta 20820
   atgaatacca gcccaaatta tatcaagtat tagatgatgg gaaacgactt ctgatatcca 20880
   tcagctgctc agatctagaa agccaactaa atcaacttgg agagtgctgg ctaagtaaca 20940
   ccaataaaat gtctaaggaa cttcacagac tggaaacaat attgaaacac tggaccagat 21000
   atcaaagtga atctgcagat ctaattcact ggttacaatc tgcaaaagac cggctagaat 21060
   tttggactca gcaatctgtg acagtcccac aagagctgga aatggtccgt gatcatctaa 21120
   atgctttcct ggagttttct aaagaagtgg atgcccaatc ttccctgaaa tcatctgttc 21180
   tgagtactgg aaatcagctc cttcgactaa aaaaggtgga cacagccacg ctgcgctctg 21240
   agctgtcgcg cattgatagc cagtggactg acctgctaac caatatccca gccgtccagg 21300
   agaagctcca ccagctccag atggataaac tgccttcccg ccatgccatt tctgaagtca 21360
   tgagttggat ttctctaatg gaaaatgtta ttcagaagga tgaagataat attaaaaatt 21420
   ccataggtta caaggcaatt catgaatacc ttcagaaata taagggtttt aagatagaca 21480
   ttaactgtaa acagctgaca gtggattttg tgaaccagtc cgtgctacaa atcagcagtc 21540
   aggatgtgga aagtaagcgt agtgataaga ctgattttgc tgagcaactt ggagcaatga 21600
   ataaaagttg gcaaattctg caaggtctag taactgagaa gatccagctg ttggaaggct 21660
   tattggaatc ttggtcagaa tatgaaaata atgtacaatg tctgaaaaca tggtttgaaa 21720
   cccaggaaaa gagactaaaa caacagcatc gaattggaga tcaggcttct gttcaaaatg 21780
   cactgaaaga ctgtcaggat ctggaagatt tgattaaagc aaaagaaaaa gaagtagaga 21840
   aaattgagca gaatggactt gctttgattc agaacaagaa agaagacgtc tctagcattg 21900
   tcatgagcac actgcgagag ctcggccaaa cctgggcaaa tttagatcac atggttggac 21960
   aattaaagat actgctgaaa tcagtgcttg accaatggag tagtcacaaa gtggcctttg 22020
   acaagataaa cagttacctc atggaggcca gatactctct ttcccgattc cgtctgctga 22080
   ctggctcctt agaagctgtg caagttcagg tggacaatct tcagaatctc caagatgatc 22140
   tggaaaaaca ggaaaggagc ttacagaaat ttggctctat caccaaccaa ttattaaaag 22200
   agtgtcaccc acccgtgaca gaaactctta ccaatacact gaaagaagtc aacatgagat 22260
   ggaataactt gctggaagag attgctgagc agctacagtc cagcaaggcc ctacttcagc 22320
   tttggcaaag atacaaggac tactccaaac agtgtgcttc gacagttcag cagcaggagg 22380
   atcgaaccaa tgagctgttg aaggcagcca caaacaagga cattgccgat gatgaggttg 22440
   ccacatggat tcaagattgc aacgacctcc tcaaaggact gggcacagtt aaagattccc 22500
   tctttttcct ccatgagctg ggagagcaac tgaagcaaca agtggatgct tccgcagcat 22560
   cagctattca atcggatcaa ctctctttga gtcaacactt gtgtgccctg gagcaagctc 22620
   tctgcaaaca gcagacttca ttacaggctg gagttcttga ttatgaaacc tttgccaaga 22680
   gtttagaagc tttggaggcc tggatagtgg aagctgaaga aatactacaa gggcaggacc 22740
   ctagccactc atctgacctc tccacaatcc aggaaaggat ggaagaactt aagggacaga 22800
   tgttaaaatt cagcagcatg gctccagatt tagaccgtct aaatgagctt ggatataggt 22860
   tacccttgaa tgataaggaa atcaaaagaa tgcagaatct gaaccgccat tggtctctga 22920
   tctcctctca gactacagaa agattcagca agttgcagtc atttttgcta caacatcaga 22980
   ctttcttgga aaaatgtgaa acatggatgg aattcctagt tcagacagaa caaaagttag 23040
   cagtagagat ttcaggaaat tatcagcacc ttttggaaca gcagagagca cacgagttgt 23100
   ttcaagccga gatgttcagt cgtcagcaga ttttgcactc aatcattatt gatgggcaac 23160
   gtcttctaga acaaggtcaa gttgatgaca gggatgaatt caacctgaaa ttgacactcc 23220
   tcagtaatca atggcaggga gtgattcgca gggcccagca gaggcggggg atcattgaca 23280
   gccagattcg ccagtggcag cgctataggg agatggcaga aaagcttcgt aaatggttgg 23340
   ttgaagtgtc ctacctcccc atgagtggtc tcggaagtgt tcctatacca ctgcaacaag 23400
   caaggaccct ctttgatgaa gtgcagttca aagaaaaagt gtttctgcgg caacaaggca 23460
   gctacatcct gactgtggag gctggcaagc aactccttct ctcggcggac agtggcgctg 23520
   aggccgcctt gcaggccgaa ctcgctgaaa tccaagagaa atggaaatca gccagcatgc 23580
   ggctggaaga acagaagaaa aaactagcct tcttgttgaa agactgggaa aaatgtgaga 23640
   aaggaatagc agattccctg gagaaactac gaactttcaa aaagaagctt tcgcagtctc 23700
   tcccggatca ccatgaagag ctccatgcag aacaaatgcg ttgcaaggaa ttagaaaatg 23760
   cagttgggag ctggacagat gacttgaccc agttgagcct gctgaaggac accctctctg 23820
   cctatatcag tgctgatgat atctccattc ttaatgaacg cgtagagctt ctgcaaaggc 23880
   agtgggaaga actatgccac cagctctcct taaggcggca gcaaataggt gaaagattga 23940
   atgaatgggc agtcttcagt gaaaagaaca aggaactctg tgagtggttg actcaaatgg 24000
   aaagcaaagt ttctcagaat ggagacattc tcattgaaga aatgatagag aagctcaaga 24060
   aggattatca agaggaaatt gctattgctc aagagaacaa aatacagctc caacaaatgg 24120
   gagaacgact tgctaaagcc agccatgaaa gcaaagcatc tgagattgaa tacaagctgg 24180
   gaaaggtcaa cgaccggtgg cagcatctcc tggacctcat tgcagccagg gtgaagaagc 24240
   tgaaggagac cctggtagcc gtgcagcagc ttgataagaa catgagcagc ctgaggacct 24300
   ggctcgctca catcgagtca gagctggcca agccaatagt ctacgattcc tgtaactcgg 24360
   aagaaataca gagaaagctt aatgagcagc aggagcttca gagagacata gagaagcaca 24420
   gtacaggtgt tgcatctgtc ctcaacctgt gtgaagtcct gctgcacgac tgtgacgcct 24480
   gtgccactga tgccgagtgt gactctatac agcaggctac gagaaacctg gaccggcggt 24540
   ggagaaacat ttgtgctatg tccatggaaa ggaggctgaa aatcgaagag acgtggcgat 24600
   tgtggcagaa atttctggat gactattcac gttttgaaga ttggctgaag tcttcagaaa 24660
   ggacagctgc ttttcccagc tcttctgggg tgatctatac agttgccaag gaagaactaa 24720
   agaaatttga ggctttccag cgacaggtcc acgagtgcct gacgcagctg gaactgatca 24780
   acaagcagta ccgccgcctg gccagggaga accgcactga ttcagcatgt agcctcaaac 24840
   agatggttca cgaaggcaac cagagatggg acaacctgca aaagcgtgtc acctccatct 24900
   tgcgcagact caagcatttt attggccagc gtgaggagtt tgagactgcg cgggacagca 24960
   ttctggtctg gctcacagag atggatctgc agctcactaa tattgaacat ttttctgagt 25020
   gtgatgttca agctaaaata aagcaactca aggccttcca gcaggaaatt tcactgaacc 25080
   acaataagat tgagcagata attgcccaag gagaacagct gatagaaaag agtgagccct 25140
   tggatgcagc gatcatcgag gaggaactag atgagctccg acggtactgc caggaggtct 25200
   tcgggcgtgt ggaaagatac cataagaaac tgatccgcct gcctctccca gacgatgagc 25260
   acgacctctc agacagggag ctggagctgg aagactctgc agctctgtcg gacctgcact 25320
   ggcacgaccg ctctgcagac agcctgcttt ctccacagcc ttcctccaat ctctccctct 25380
   cgctcgctca gcccctccgg agcgagcggt caggacgaga caccccggct agtgtggact 25440
   ccatccccct ggagtgggat cacgactatg acctcagtcg ggacctggag tctgcaatgt 25500
   ccagagctct gccctctgag gatgaagaag gtcaggatga caaagatttc tacctccggg 25560
   gagctgttgg cttatcaggg gaccacagtg ccctagagtc acagatccga caactgggca 25620
   aagccctgga tgatagccgt tttcagatac agcaaaccga aaatatcatt cgcagcaaaa 25680
   ctcccacggg gccggagcta gacaccagct acaaaggcta catgaaactg ctgggcgaat 25740
   gcagtagcag tatagactcc gtgaagagac tggagcacaa actgaaggag gaagaggaga 25800
   gccttcctgg ctttgttaac ctgcatagta ccgaaaccca aacggctggt gtgattgacc 25860
   gatgggagct tctccaggcc caggcattga gcaaggagtt gaggatgaag cagaacctcc 25920
   agaagtggca gcagtttaac tcagacttga acagcatctg ggcctggctg ggggacacgg 25980
   aggaggagtt ggaacagctc cagcgtctgg aactcagcac tgacatccag accatcgagc    26040
   tccagatcaa aaagctcaag gagctccaga aagctgtgga ccaccgcaaa gccatcatcc    26100
   tctccatcaa tctctgcagc cctgagttca cccaggctga cagcaaggag agccgggacc    26160
   tgcaggatcg cttgtcgcag atgaatgggc gctgggaccg agtgtgctct ctgctggagg    26220
   agtggcgggg cctgctgcag gatgccctga tgcagtgcca gggtttccat gaaatgagcc    26280
   atggtttgct tcttatgctg gagaacattg acagaaggaa aaatgaaatt gtccctattg    26340
   attctaacct tgatgcagag atacttcagg accatcacaa acagcttatg caaataaagc    26400
   atgagctgtt ggaatcccaa ctcagagtag cctctttgca agacatgtct tgccaactac    26460
   tggtgaatgc tgaaggaaca gactgtttag aagccaaaga aaaagtccat gttattggaa    26520
   atcggctcaa acttctcttg aaggaggtca gtcgtcatat caaggaactg gagaagttat    26580
   tagacgtgtc aagtagtcag caggatttgt cttcctggtc ttctgctgat gaactggaca    26640
   cctcagggtc tgtgagtccc acatcaggaa ggagcacccc aaacagacag aaaacgccac    26700
   gaggcaagtg tagtctctca cagcctggac cctctgtcag cagtccacat agcaggtcca    26760
   caaaaggtgg ctccgattcc tccctttctg agccagggcc aggtcggtcc ggccgcggct    26820
   tcctgttcag agtcctccga gcagctcttc cccttcagct tctcctgctc ctcctcatcg    26880
   ggcttgcctg ccttgtacca atgtcagagg aagactacag ctgtgccctc tccaacaact    26940
   ttgcccggtc attccacccc atgctcagat acacgaatgg ccctcctcca ctctgaacta    27000
   agcagatgcc atctgcagaa gtgctggtag cataaggagg atcgggtcat aagcaatccc    27060
   aaactaccaa caagaggacc ttgatcttgg cgaaagccct cggtgtggca gctttagccc    27120
   tcctccagat cacatgtgtg caaattatgg cttcagaggt ggaagataaa cagtgacggg    27180
   ggaacaaaca gacaacaaga aggtttggaa gaaatctggt ttgagactct gaaccttagc    27240
   actaaggaga ttgagtaagg acctccaaag ttccccggac tcatgaattc tgggcccttg    27300
   gcccattctg tgcacagcca aggacttcag tagaccatct gggcagcttt cccatggtgc    27360
   tgctccaacc atcagataaa tgaccctccc aagcaccatg tcagtgtcgt acaatctacc    27420
   aaccaaccag tgctgaagag attttagaac cttgtaacat acaattttta agagcttata    27480
   tggcagcttc ctttttacct tgttttcctt tggggcatga tgttttaacc tttgctttag    27540
   aagcacaagc tgtaaatcta aaaggcactt ttttttagag gtataaagaa aaactagatg    27600
   taataaataa gatcatggaa ggctttatgt gaaaaaagtt gaatgttata gtaaaaaaaa    27660
   aaagatattt atgtatgtac agtttgctaa agccaagttt tgtttgtatt gatttctttg    27720
   catttattat agatattata aaata                                          27745
<210> 33
   <211> 690
   <212> DNA
   <213> Homo sapiens
<400> 33
   ccgggcgctc ctagcggtct cccggcccct gccgccctgc cactatgtcc cgccgctcta      60
   tgctgcttgc ctgggctctt cccagcctcc ttcgactcgg agcggctcag gagacagaag     120
   acccggcctg ctgcagcccc atagtgcccc ggaacgagtg gaaggccctg gcatcagagt     180
   gcgcccagca cctgagcctg cccttacgct atgtggtggt atcgcacacg gcgggcagca     240
   gctgcaacac ccccgcctcg tgccagcagc aggcccggaa tgtgcagcac taccacatga     300
   agacactggg ctggtgcgac gtgggctaca acttcctgat tggagaagac gggctcgtat     360
   acgagggccg tggctggaac ttcacgggtg cccactcagg tcacttatgg aaccccatgt     420
   ccattggcat cagcttcatg ggcaactaca tggatcgggt gcccacaccc caggccatcc     480
   gggcagccca gggtctactg gcctgcggtg tggctcaggg agccctgagg tccaactatg     540
   tgctcaaagg acaccgggat gtgcagcgta cactctctcc aggcaaccag ctctaccacc     600
   tcatccagaa ttggccacac taccgctccc cctgaggccc tgctgatccg caccccattc     660
   ctcccctccc atggccaaaa accccactgt                                      690
<210> 34
   <211> 2677
   <212> DNA
   <213> Homo sapiens
<400> 34
   catttggccc ggggatggtc acacgcgcgg gggccggaac tgccgtcgcc ggcgcggtcg      60
   ttgtcgcatt gctctcggcc gcactcgcgc tgtacgggcc gccactggac gcagttttag     120
   aaagagcgtt ttcgctacgt aaagcacatt cgataaagga tatggaaaat actttgcagc     180
   tggtgagaaa tatcatacct cctctgtctt ccacaaagca caaagggcaa gatggaagaa     240
   taggcgtagt tggaggctgt caggagtaca ctggagcccc atattttgca gcaatctcag     300
   ctctcaaagt gggcgcagac ttgtcccacg tgttctgtgc cagtgcggcc gcacctgtga     360
   ttaaggccta cagcccggag ctgatcgtcc acccagttct tgacagcccc aatgctgttc     420
   atgaggtgga gaagtggctg ccccggctgc atgctcttgt cgtaggacct ggcttgggta     480
   gagatgatgc gcttctcaga aatgtccagg gcattttgga agtgtcaaag gccagggaca     540
   tccctgttgt catcgacgcg gatggcctgt ggctggtcgc tcagcagccg gccctcatcc     600
   atggctaccg gaaggctgtg ctcactccca accacgtgga gttcagcaga ctgtatgacg     660
   ctgtgctcag aggccctatg gacagcgatg acagccatgg atctgtgcta agactcagcc     720
   aagccctggg caacgtgacg gtggtccaga aaggagagcg cgacatcctc tccaacggcc     780
   agcaggtgct tgtgtgcagc caggaaggca gcagccgcag gtgtggaggg caaggggacc     840
   tcctgtcggg ctccctgggc gtcctggtac actgggcgct ccttgctgga ccacagaaaa     900
   caaatggggg catatcagac ttgaaattga caatttgggg tcctgagatt gaaacaggag     960
   tcaaaaccag agcccagggt agctgcggcc cccggaccac gacgcccact tccccacacc     1020
   tcctgctgtc cccctctccg caggtccagc cctctcctgg tggccgcgtt tggcgcctgc     1080
   tctctcacca ggcagtgcaa ccaccaagcc ttccagaagc acggtcgctc caccaccacc     1140
   tccgacatga tcgccgaggt gggggccgcc ttcagcaagc tctttgaaac ctgagcccgc     1200
   gcagaccaga agtaaacagg caccttggac gggggagagc gtgtgtgtga tgggaaaatc     1260
   cggacccacg cgtgtgctga aggcgtacgg tgcttgccag attttcaact tgagcataaa     1320
   ttggttgcca ttgagaattt aagaatctgg aatattgcag cttttggtta aacttaatgc     1380
   atggttggag atgttatggc gacactaaac aaagtattcc tgaactttcc ttagctcctt     1440
   ggtagtaact gggaagacag aaatgaagaa aatcacatga gaatgaagaa ttctttagca     1500
   gctcaacaga gtttctcggc ctgctcccag atcggcgaag tttctacttg ttactctctc     1560
   tgccggcgcc cttcgttcct cctctgcttc ccttccctag tctttcctcc ggcagggagc     1620
   tgggcagggg tccccgggtg tctccctgag tcccgactgc actgactggg tccatcagag     1680
   ggctgcttcg ttctccagct catcttcttt taaagtggtg actagcttgg tggtatctgg     1740
   ctgctggtgt ttggcttatt gacatactcc agggtaatca atgatgactt tgtttggaaa     1800
   ccctttggga ggcaccatgg gaacagaagg aaacatgagt gacgctgacc cttgagtgtg     1860
   tgggtgggga gctctgagac gcctcctgtc ccacgctctc cggtgtccgt gtctacacag     1920
   gggtccccat gatacccacc ggccccagca gggcagaccg gaccggggac gggcacggtg     1980
   aagggctgca gcctggggtc tgacgtggcc cctagtgctg tctcaggaga aggctctgga     2040
   ggacttgagg catgctgggc ctggtgcagt gatggcgcta aggagacccg gggaaagaca     2100
   gtatcgtggt cacgtatgct taggaagcag cacagccgtg tccttaggga tgttcgcgtc     2160
   cagtaaagac actggtaact gcggtttcag ccaacactct tcatggcagt gtcgacctcg     2220
   ggttagcttc tgttgtcttt gtggatggtt ttcctggagc ggcctgacgt tgacgtgttc     2280
   tctggtccca tgtcttagcg gggcatggta cggtttcgtg cctgacgcgt gcattagggt     2340
   gttctcttat actttcagta gcatctttcc acagcaaggg ccaaaccctc ctggttccct     2400
   tcagagtctt tttggcctga tgatgactct tgagtgatac cctgtgatgc agacatgccc     2460
   cagatggatt ctactttctt taaaactagg gactttcaag attaaaaaaa agattgtcac     2520
   tactaatttg acgcctaact tcagaagctt cactgtctac atgtgaactt ttccagaaaa     2580
   actgtgccat ggacattttt cctctgggga attaacatct aaattctggt aactattaaa     2640
   agacagatct ggttaattta aaaaaaaaaa aaaaaaa                              2677
<210> 35
   <211> 2063
   <212> DNA
   <213> Homo sapiens
<400> 35
   catcccacaa tcctctcgtc acgtatttcc ggtttatcta gctcagccgt aagtagtttc 60
   tctatcagtc gcgcagctgt gttcgcggac tcaggtggaa ggaatttctt ctcttcgttg 120
   acgttgctgg tgttcactgt ttggaattag tcaagtttcg ggaatcaccg tcgctgccat 180
   caacatgtcg gtcccaagcg ctctcatgaa gcaaccgccc attcagtcta cggctggggc 240
   cgtcccagtt cgcaatgaga aaggtgagat ttcaatggaa aaagtgaagg taaagcgtta 300
   tgtgtccgga aaaaggccag actatgcccc tatggagtcc tcagatgagg aggatgaaga 360
   atttcagttc attaagaaag ccaaagaaca agaagcagag cctgaggaac aggaggagga 420
   ttcatccagt gacccccggc tacggcgttt acagaaccgt attagtgaag atgtggaaga 480
   gagattggct cgacatcgaa aaatagtgga acctgaagtg gtaggagaga gtgactcaga 540
   agtagaagga gatgcttggc gcatggaacg agaagacagc agtgaagaag aggaggagga 600
   aattgatgat gaggaaatag agcggcggcg tggcatgatg cgtcagcgag cacaggagag 660
   aaaaaatgaa gagatggaag tcatggaagt ggaagatgag ggtcgttctg gagaggagtc 720
   agaatcagag tctgagtatg aagagtacac agacagtgaa gatgagatgg agcctcgcct 780
   taagccagtc ttcattcgaa agaaggaccg agtgacagtt caagaacgtg aagccgaagc 840
   attgaaacag aaggagctgg agcaggaagc aaaacgcatg gctgaggaaa ggcgcaagta 900
   cacactcaag attgtcgaag aggaaaccaa aaaagagctg gaagagaaca agcgatccct 960
   ggctgcattg gatgcactca atactgatga tgaaaatgat gaggaggaat atgaggcatg 1020
   gaaagttcga gagctaaaaa gaatcaagag ggacagagaa gatcgagaag cgcttgagaa 1080
   ggagaaagca gaaattgaac gcatgcgaaa cctgactgag gaagagagga gagctgaact 1140
   tcgggcaaac ggcaaagtca ttaccaacaa agctgttaag ggcaaataca agttcttaca 1200
   gaagtattat caccggggtg ccttcttcat ggatgaggat gaagaagtat acaagagaga 1260
   tttcagcgct cctaccctgg aggatcattt caataaaacc attcttccta aagtcatgca 1320
   ggtcaagaac tttggacgct caggtcgcac caaatacact caccttgtgg atcaagatac 1380
   cacctccttt gactcagctt ggggccaaga gagtgcccag aacacaaagt tcttcaaaca 1440
   aaaggcagct ggggtacgag atgtatttga gcggccatct gccaagaagc ggaaaactac 1500
   ctagggtcca actgcttatt cttccaactg tggaacacaa ggggagtctc agcatctggt 1560
   ccttgatttg gttttttcat tgtttccttg gcccctgtat ccagatattg gacttactgc 1620
   tatacttgtg atactgggta gcccagactt tgaaggtgct ttgtgaggtt tggactcatg 1680
   ctgagaaacc cacaggaaag cactgtccag gtaggattag aggcttccca cttaaaacta 1740
   tttctgagaa atcttaggtt ttatcactgc tatggtttcc catatttact tgggactgtt 1800
   ctgactttct ttttccagcc cttagcttgg gttagaaaag tggacatgta agtgaacaat 1860
   gcattacttc taccttaggt ttaggagtaa tatacccgga aatctaagct catggaaaca 1920
   tgttttccat ttttgcttgg agtccgtttt tctagttgta catacttttt gatccatata 1980
   tgtgtgcatg tcaagaaata aaagaatcac acaacaagga gatttttagc atgatactgg 2040
   aaataaaatg tccaagctga caa 2063
<210> 36
   <211> 3080
   <212> DNA
   <213> Homo sapiens
<400> 36
   gggtgcctca tattgccaga caagagctca gacctgagga gagtgactag cttctctgtg 60
   tcccaggtgg ccaccttcca ctgtggaagc tcatggactc cattgggtct tcagggttgc 120
   ggcaggggga agaaaccctg agttgctctg aggagggctt gcccgggccc tcagacagct 180
   cagagctggt gcaggagtgc ctgcagcagt tcaaggtgac aagggcacag ctacagcaga 240
   tccaagccag cctcttgggt tccatggagc aggcgctgag gggacaggcc agccctgccc 300
   ctgcggtccg gatgctgcct acatacgtgg ggtccacccc acatggcact gagcaaggag 360
   acttcgtggt gctggagctg ggggccacag gggcctcact gcgtgttttg tgggtgactc 420
   taactggcat tgaggggcat agggtggagc ccagaagcca ggagtttgtg atcccccaag 480
   aggtgatgct gggtgctggc cagcagctct ttgactttgc tgcccactgc ctgtctgagt 540
   tcctggatgc gcagcctgtg aacaaacagg gtctgcagct tggcttcagc ttctctttcc 600
   cttgtcacca gacgggcttg gacaggagca ccctcatttc ctggaccaaa ggttttaggt 660
   gcagtggtgt ggaaggccag gatgtggtcc agctgctgag agatgccatt cggaggcagg 720
   gggcctacaa catcgacgtg gttgctgtgg tgaacgacac agtgggcacc atgatgggct 780
   gtgagccggg ggtcaggccg tgtgaggttg ggctagttgt agacacgggc accaacgcgt 840
   gttacatgga ggaggcacgg catgtggcag tgctggacga agaccggggc cgcgtctgcg 900
   tcagcgtcga gtggggctcc ttcagcgatg atggggcgct gggaccagtg ctgaccacct 960
   tcgaccatac cctggaccat gagtccctga atcctggtgc tcagaggttt gagaagatga 1020
   tcggaggcct gtacctgggt gagctggtgc ggctggtgct ggctcacttg gcccggtgtg 1080
   gggtcctctt tggtggctgc acctcccctg ccctgctgag ccaaggcagc atcctcctgg 1140
   aacacgtggc tgagatggag gacccctcta ctggggcagc ccgtgtccat gctatcctgc 1200
   aggacttggg cctgagccct ggggcttcgg atgttgagct tgtgcagcac gtctgtgcgg 1260
   ccgtgtgcac gcgggctgcc cagctctgtg ctgccgccct ggccgctgtt ctctcctgcc 1320
   tccagcacag ccgggagcaa caaacactcc aggttgctgt ggccaccgga ggccgagtgt 1380
   gtgagcggca ccccaggttc tgcagcgtcc tgcaggggac agtgatgctc ctggccccgg 1440
   aatgcgatgt ctccttaatc ccctctgtgg atggtggtgg ccggggagtg gcgatggtga 1500
   ctgccgtggc tgcccgtctg gctgcccacc ggcgcctgct ggaggagacc ctggccccat 1560
   tccggttgaa ccatgatcaa ctggctgcgg ttcaggcaca gatgcggaag gccatggcca 1620
   aggggctccg aggggaggcc tcctcccttc gcatgctgcc cactttcgtc cgggccaccc 1680
   ctgacggcag cgagcgaggg gatttcctgg ccctggacct cgggggcacg aacttccgtg 1740
   tcctcctggt acgtgtgacc acaggcgtgc agatcaccag cgagatctac tccattcccg 1800
   agactgtggc ccagggttct gggcagcagc tctttgacca catcgtggac tgcatcgtgg 1860
   acttccagca gaagcagggc ctgagcgggc agagcctccc actgggtttt accttctcct 1920
   tcccatgtag gcagcttggc ctagaccagg gcatcctcct gaactggacc aagggtttca 1980
   aggcatcaga ctgcgagggc caagatgtcg tgagtctgtt gcgggaagcc atcactcgca 2040
   gacaggcagt ggagctgaat gtggttgcca ttgtcaatga cacggtgggg accatgatgt 2100
   cctgtggcta tgaggacccc cgttgcgaga taggcctcat tgtcggaacc ggcaccaatg 2160
   cctgctacat ggaggagctc cggaatgtgg cgggcgtgcc tggggactca ggccgcatgt 2220
   gcatcaacat ggagtggggc gcctttgggg acgatggctc tctggccatg ctcagcaccc 2280
   gctttgatgc aagtgtggac caggcgtcca tcaaccccgg caagcagagg tttgaaaaga 2340
   tgatcagcgg catgtacctg ggggagatcg tccgccacat ccttttacat ttaaccagcc 2400
   ttggcgttct cttccggggc cagcagatcc agcgccttca gaccagggac atcttcaaga 2460
   ccaagttcct ctctgagatc gaaagtgaca gcctggccct gcggcaggtc cgagccatcc 2520
   tagaggatct ggggctaccc ctgacctcag atgacgccct gatggtgcta gaggtgtgcc 2580
   aggctgtgtc ccagagggct gcccagctct gtggggcggg tgtagctgcc gtggtggaga 2640
   agatccggga gaaccggggc ctggaagagc tggcagtgtc tgtgggggtg gatggaacgc 2700
   tctacaagct gcacccgcgc ttctccagcc tggtggcggc cacagtgcgg gagctggccc 2760
   ctcgctgtgt ggtcacgttc ctgcagtcag aggatgggtc cggcaaaggt gcggccctgg 2820
   tcaccgctgt tgcctgccgc cttgcgcagt tgactcgtgt ctgaggaaac ctccaggctg 2880
   aggaggtctc cgccgcagcc ttgctggagc cgggtcgggg tctgcctgtt tcccagccag 2940
   gcccagccac ccaggactcc tgggacatcc catgtgtgac ccctctgcgg ccatttggcc 3000
   ttgctccctg gctttccctg agagaagtag cactcaggtt agcaatatat atatataatt 3060
   tatttacaaa aaaaaaaaaa 3080
<210> 37
   <211> 2180
   <212> DNA
   <213> Homo sapiens
<400> 37
   ggcgcactgc tcccctctgg actgcggagg cactgcactg cgcgggacgt gggagggtgc      60
   ctctgcagcc cctcaaggcc gccgcccctg cggggagaac tggagaatgc ggacacctag     120
   atgaggccct ggcacccaag ctggggcccc cggaatctca cgttcccttt actcgccagg   180
   catttcgggg caaaacgaag cggagccccc caaaccccca gacctaacca agttcagaga 240
   ttcgcagaag cacgcccccc accccaaatt tattgtgctc tacccaaaat ggaataggac      300
   taggtttatt tacccattgt gagggtagag aggcgagtct ggaggagcag ggattgggag      360
   aaggggtgga aaaatactct gattcttaaa aatactttgt aacctaaagt ccttaaattg      420
   tggaagaaag gaatactcct cctttccatt gtagtctaga gttaagattt caaatccata      480
   aattagagga cctaaaatta gagggcaatt aactgctcat tcattgggcc cccagtcagc      540
   acgggggtgc tggaagagat cgggaataat agcgcagacc aatgagccta gggagatgct      600
   ttcatcgtct ctccttccct caagtgttct ggaacctatc atttgaatta gccgagtcag      660
   gcaggagggg gcggggaatc cttccgccct tcttaggagg ggctgcattg cagggggaga      720
   gtgaactgac agactcagtc actgaagagg gaaaaggagt gagaagacaa agccgtcaaa      780
   gccccaacag ctttgtattt ctccagcccg gcgcagaccc cggagctccc gaggcactcc      840
   ctccatcttt ggaacacgcc agtaattgat tgataacagg aagctatgag ggaccctgtg      900
   agtagccagt acagttcctt tcttttctgg aggatgccca tcccagaact ggatctgtcg      960
   gagctggaag gcctgggtct gtcagataca gccacctaca aggtcaaaga cagcagcgtt     1020
   ggcaaaatga tcgggcaagc aactgcagca gaccaggaga aaaaccctga aggtgatggc     1080
   ctccttgagt acagcacctt caacttctgg agagctccca ttgccagcat ccactccttc     1140
   gaactggact tgctctaagg ccaagacttc tctctcccat caccttgccc tcattgtctt     1200
   ccctctcaag ccccttcctt tccactcctt tcccatttta atcttgttct ctccctactg     1260
   tgttggtggt gctgatgaat ctgccagagt tgagttctat gtatttattt atctatctgt     1320
   ctactccatt tctctcaaaa gccctcaagt cacaaagtaa atggttcaag caatggagta     1380
   ctgggtcaca gggattcctc ctttcccccc caaatattaa ctccagaaac taggcctgac     1440
   tggggacacc tgagagtagt atagtagtgc aaaatggaag actgattttt gactctatta     1500
   taatcagctt cagagattcc ttaaaccttc ctaatttcct gctccagggc agtaaacaca     1560
   aatatttctt caaggggtga tgaaaacctc ggaagtttta atttgaggtt atctgctacg     1620
   aaacagtatt tctaaaaggc taaagtgata agtctcttgc ttttttttga tcctgctctt     1680
   atattctttt ttttcctcag agaaatcagg agggtagtta gaggtataaa acaggaggaa     1740
   atattatgga aaatgaaaat agggaaaata attgaatcat tttagaagta gctaatttct     1800
   tttctcaaaa gagtgtccct tcttcacacc tactcacttt acaactttgc tcctaactgt     1860
   gggttgaaaa ctctagctaa agaaagttat caaatcttaa catgcattcc tactattatg     1920
   atagttttta aggtttcaat tcaatcttct gaacggcata agtcctattt tagccttacc     1980
   tcctgcattt gcaatacgta atactgatca gtgggcacag ttcttcagct acattgagac     2040
   cctgaaatga acaattatat tctgactcga catcttgtcc ccaatccttc caaaaatatt     2100
   gatggtgatt tgtgctacca tttactcgtt tatttaataa agacattcaa tcccaggaaa     2160
   aaaaaaaaaa aaaaaaaaaa 2180
<210> 38
   <211> 2046
   <212> DNA
   <213> Homo sapiens
<400> 38
   gcggcttgtt ttctttcctc cagtctcggg gctgcaggct gagcgcgatg cgcggagacc      60
   cccgcggggg cggcggcggc cgtgagcccc gatgaggccc gagcgtcccc ggccgcgcgg     120
   cagcgccccc ggcccgatgg agaccccgcc gtgggaccca gcccgcaacg actcgctgcc     180
   gcccacgctg accccggccg tgccccccta cgtgaagctt ggcctcaccg tcgtctacac     240
   cgtgttctac gcgctgctct tcgtgttcat ctacgtgcag ctctggctgg tgctgcgtta     300
   ccgccacaag cggctcagct accagagcgt cttcctcttt ctctgcctct tctgggcctc     360
   cctgcggacc gtcctcttct ccttctactt caaagacttc gtggcggcca attcgctcag     420
   ccccttcgtc ttctggctgc tctactgctt ccctgtgtgc ctgcagtttt tcaccctcac     480
   gctgatgaac ttgtacttca cgcaggtgat tttcaaagcc aagtcaaaat attctccaga     540
   attactcaaa taccggttgc ccctctacct ggcctccctc ttcatcagcc ttgttttcct     600
   gttggtgaat ttaacctgtg ctgtgctggt aaagacggga aattgggaga ggaaggttat     660
   cgtctctgtg cgagtggcca ttaatgacac gctcttcgtg ctgtgtgccg tctctctctc     720
   catctgtctc tacaaaatct ctaagatgtc cttagccaac atttacttgg agtccaaggg     780
   ctcctccgtg tgtcaagtga ctgccatcgg tgtcaccgtg atactgcttt acacctctcg     840
   ggcctgctac aacctgttca tcctgtcatt ttctcagaac aagagcgtcc attcctttga     900
   ttatgactgg tacaatgtat cagaccaggc agatttgaag aatcagctgg gagatgctgg     960
   atacgtatta tttggagtgg tgttatttgt ttgggaactc ttacctacca ccttagtcgt    1020
   ttatttcttc cgagttagaa atcctacaaa ggaccttacc aaccctggaa tggtccccag    1080
   ccatggattc agtcccagat cttatttctt tgacaaccct cgaagatatg acagtgatga    1140
   tgaccttgcc tggaacattg cccctcaggg acttcaggga ggttttgctc cagattacta    1200
   tgattgggga caacaaacta acagcttcct ggcacaagca ggaactttgc aagactcaac    1260
   tttggatcct gacaaaccaa gccttgggta gcatcagtta acagttttat ggacgattcc    1320
   tcagatgaaa agcttcagaa aagcatagtg acagctgaat ttttagggca cttttcctta     1380
   agaaatagaa cttgattttt atttgttaca ggtttccaat ggccccatag gaataagcaa    1440
   taatgtagac tgataaaccc ttattttagt actaaagagg gagccttgct atttcagtgg    1500
   gtataattta aactttttaa agaaaatctg tacttttata aagatgtatt ttgtataact    1560
   taaataataa tgctaaagta tactagggtt tttttttctt gagaatgtta ctgcaatcat    1620
   gttgtagttt gcacagactt ttatgcataa ttcactttaa aaatatagaa tatatggtct    1680
   aatagttttt taaagctttt ggactaaagt attccacaaa tcttacctct ttaggtcact    1740
   gatggtcact ccgattctga gtgccacatt ggtagactcc taaaatacag ttgacaactt    1800
   agccaattgc aactccagtg ttgataatta aaatgaaatg gtaaagcagc agactgtaag    1860
   gtctttagag attttttttt taaggttcag gccgtaggtt cctcaaggaa tctcttaagt    1920
   tttgcccaaa gactggtact tcctttcagt agggcgctaa tgtatacaca ttaatgataa    1980
   gttgataaca ttaaaaatgt agctgactta tcctattaaa cctcctctgc tatgttcaca    2040
   gaaaaa                                                               2046
<210> 39
   <211> 1025
   <212> DNA
   <213> Homo sapiens
<400> 39
   cagctgttac cgcgtcacat gagggaggcc ggcggccact cggcggggga ggggaccgtg      60
   gctggagccc ggggcggggc cgcgcggcag gcggggcggg agccgggggg cgcagctaga     120
   gagccccgga gccgcggcgg gagaggaacg cgcagccagc cttgggaagc ccaggcccgg     180
   cagccatggc ggtggaagga ggaatgaaat gtgtgaagtt cttgctctac gtcctcctgc     240
   tggccttttg cgcctgtgca gtgggactga ttgccgtggg tgtcggggca cagcttgtcc     300
   tgagtcagac cataatccag ggggctaccc ctggctctct gttgccagtg gtcatcatcg     360
   cagtgggtgt cttcctcttc ctggtggctt ttgtgggctg ctgcggggcc tgcaaggaga     420
   actattgtct tatgatcacg tttgccatct ttctgtctct tatcatgttg gtggaggtgg     480
   ccgcagccat tgctggctat gtgtttagag ataaggtgat gtcagagttt aataacaact     540
   tccggcagca gatggagaat tacccgaaaa acaaccacac tgcttcgatc ctggacagga     600
   tgcaggcaga ttttaagtgc tgtggggctg ctaactacac agattgggag aaaatccctt     660
   ccatgtcgaa gaaccgagtc cccgactcct gctgcattaa tgttactgtg ggctgtggga     720
   ttaatttcaa cgagaaggcg atccataagg agggctgtgt ggagaagatt gggggctggc     780
   tgaggaaaaa tgtgctggtg gtagctgcag cagcccttgg aattgctttt gttttgggaa     840
   ttgtctttgc ctgctgcctc gtgaagagta tcagaagtgg ctacgaggtg atgtaggggt     900
   ctggtctcct cagcctcctc atctggggga gtggaatagt atcctccagg tttttcaatt     960
   aaacggatta ttttttcaga ccgaaaagag atggtctgag tttgtcttag aaaaaaaaaa    1020
   aaaaa                                                                1025
<210> 40
   <211> 1031
   <212> DNA
   <213> Homo sapiens
<400> 40
   cagctgttac cgcgtcacat gagggaggcc ggcggccact cggcggggga ggggaccgtg      60
   gctggagccc ggggcggggc cgcgcggcag gcggggcggg agccgggggg cgcagctaga     120
   gagccccgga gccgcggcgg gagaggaacg cgcagccagc cttgggaagc ccaggcccgg     180
   cagccatggc ggtggaagga ggaatgaaat gtgtgaagtt cttgctctac gtcctcctgc     240
   tggccttttg cgcctgtgca gtgggactga ttgccgtggg tgtcggggca cagcttgtcc     300
   tgagtcagac cataatccag ggggctaccc ctggctctct gttgccagtg gtcatcatcg      360
   cagtgggtgt cttcctcttc ctggtggctt ttgtgggctg ctgcggggcc tgcaaggaga     420
   actattgtct tatgatcacg tttgccatct ttctgtctct tatcatgttg gtggaggtgg     480
   ccgcagccat tgctggctat gtgtttagag ataaggtgat gtcagagttt aataacaact     540
   tccggcagca gatggagaat tacccgaaaa acaaccacac tgcttcgatc ctggacagga     600
   tgcaggcaga ttttaagtgc tgtggggctg ctaactacac agattgggag aaaatccctt     660
   ccatgtcgaa gaaccgagtc cccgactcct gctgcattaa tgttactgtg ggctgtggga     720
   ttaatttcaa cgagaaggcg atccataagg agggctgtgt ggagaagatt gggggctggc     780
   tgaggaaaaa tgtgctggtg gtagctgcag cagcccttgg aattgctttt gtcgaggttt     840
   tgggaattgt ctttgcctgc tgcctcgtga agagtatcag aagtggctac gaggtgatgt     900
   aggggtctgg tctcctcagc ctcctcatct gggggagtgg aatagtatcc tccaggtttt     960
   tcaattaaac ggattatttt ttcagaccga aaagagatgg tctgagtttg tcttagaaaa    1020
   aaaaaaaaaa a                                                         1031
<210> 41
   <211> 3300
   <212> DNA
   <213> Homo sapiens
<400> 41
   ccctcgccgc agtcgcgggc accccgctcg gcgtcggtgc ctgagggagg ccgcgatggc      60
   ggccgaggcc ctggcggcgg aggccgtggc gtcgcgcctg gagcggcagg aggaggacat     120
   ccgctggctg tggtcggagg tcgagcgcct gagggacgag cagctgaacg cgccctacag     180
   ctgccaggcg gaggggccgt gcctcacgcg ggaggtggcg cagctccggg ccgagaactg     240
   cgacctgcgc caccgcctgt gcagcctgcg gctgtgcctc gccgaggagc ggagccgcca     300
   ggccacgctg gagagcgcgg agctagaggc ggcgcaggag gccggcgcac agcctcctcc     360
   tagtcaaagc caagacaagg acatgaaaaa gaagaaaatg aaggaaagcg aggctgacag     420
   cgaggtgaag catcaaccaa ttttcataaa agaaagattg aagctttttg aaatactgaa     480
   gaaagaccat cagctcttac ttgccattta tggaaaaaag ggggatacaa gcaacatcat     540
   cacagtaaga gtggctgatg ggcaaacagt gcaaggggaa gtctggaaaa caacgcctta     600
   ccaagtggct gctgaaatta gtcaggaact ggctgaaagc acggtaatag ccaaagtcaa     660
   tggtgaactg tgggacctgg accgcccatt ggaaggggac tcttctctag agctgcttac      720
   atttgataat gaggaagctc aagctgtgta ctggcactcc agtgctcaca ttcttgggga      780
   ggccatggag ctttactatg gaggccacct gtgctacggt ccgcccattg aaaatggatt      840
   ttattatgac atgttcattg aagacagagc agtgtccagc acagaattgt cagccctgga      900
   gaatatatgt aaagccatca taaaagaaaa gcaacctttt gaaagactag aagtcagcaa      960
   ggaaatcctc ctggaaatgt ttaagtacaa taaatttaaa tgccgcattc tgaatgagaa     1020
   agttaacact gcaactacca ccgtgtacag gtgcggtcca ttaattgacc tttgcaaagg     1080
   tccacatgta agacacactg gaaaaattaa aaccatcaaa atttttaaga attcctcaac     1140
   atattgggag ggcaatccgg aaatggaaac attgcagagg atctatggaa tatcctttcc     1200
   tgataacaag atgatgagag actgggaaaa gttccaagag gaagcaaaga accgagatca     1260
   caggaagatc gggaaggaac aagaactttt ctttttccac gatttgagtc ctggaagctg     1320
   ttttttcctt cccagaggag ccttcattta taatacgctt acagatttca tacgagagga     1380
   atatcacaaa cgggacttca cggaggtgct ctctcccaat atgtacaaca gtaaactctg     1440
   ggaagcctca ggccactggc agcattacag cgagaacatg tttacctttg agattgaaaa     1500
   ggacactttt gccctcaaac ccatgaattg tccagggcac tgtctaatgt ttgcccatcg     1560
   tccacgatct tggagggaaa tgcctattag atttgctgat tttggagttc tgcatagaaa     1620
   tgaactgtcg gggactctca gcggcttgac cagagtgagg cgcttccagc aggacgatgc     1680
   tcacattttt tgcacagtgg agcagattga agaagaaata aaggggtgtt tgcagttttt     1740
   gcaatctgtt tactcaacat ttggcttctc ctttcaatta aacctgtcaa caaggccgga     1800
   aaacttccta ggagagattg agatgtggaa tgaggctgag aagcaactgc agaacagctt     1860
   gatggacttt ggagaaccgt ggaaaatgaa cccaggagat ggagcatttt atggccctaa     1920
   aattgacata aaaatcaagg atgctattgg cagataccat caatgtgcta caattcagct     1980
   ggacttccaa ctgcctatta gatttaatct cacatatgtt agtaaggatg gggatgataa     2040
   gaagagacct gtgatcattc atcgagccat tttgggatca gtggaaagaa tgatagccat     2100
   tctttcagaa aactatggcg gaaaatggcc tttctggcta tctcctcgtc aggtgatggt     2160
   catccctgtg gggccaactt gtgaaaaata tgcacttcag gtatccagtg aattttttga     2220
   agaaggattt atggctgacg ttgacttgga tcacagttgt acactaaata agaaaatacg     2280
   aaatgcacag ctggctcagt ataattttat tttggtggtt ggagaaaagg aaaagataga     2340
   taatgctgta aacgtgcgaa caagagacaa caaaattcat ggagagattt tagtaacttc     2400
   tgccattgat aaactgaaga atctcaggaa gacacggaca ctcaatgctg aggaggcctt     2460
   ttgaagtcct tccctgatat ttgcttctgt gtaactttgt tttgaccctt aaaaatgtat     2520
   ttttcttaac atgttagtac ttctacgact ttggagccac tgatgggtcc actcatggcc     2580
   tcagctgaga aaggagacga tgaacgtgta gctgacatgc acgaagttta atttactcat     2640
   gtccacgggg gacgtttaga gggcacgtgg gaaattttcc agcaatcaat gccttgagaa 2700
   acttaaatgg ggaaatatta ttcatcgaga aagtgaaaca aaacactagg aaatgattat 2760
   gaaatgttag tgattttcaa aagatgggct tcaaataaaa gtctgcagag ttttttttaa 2820
   ataggaggga aaatcttatt ttctaatatg tctcaggtat ttttatgact tctactaaaa 2880
   ttcacactga aactttatct tctaaactgg aatcattact taattttaac taaccaacaa 2940
   ccacaaaagc agcagctact gctaaatatt ggattactga caaaggaatt cagttttgtg 3000
   gaatctggtg tttgcactat aggttaagag ttgccattta aatgtttctt attcataatt 3060
   aggttttgtt ccgctttaga aaaaaataaa ttcccaaatg aattgcatca gtgtttccag 3120
   cgtttcagct gtgggcttct accaccacta agccgctcac ctagagttgt tacttttttg 3180
   atgggatgac attccatgga tttatgtact gaaatggatt tcagatttta aagagaggaa 3240
   tgctctacgt gctcagaact gaattaaaat ggcattatgt caatagagaa ttcaaaaaaa 3300
<210> 42
   <211> 3468
   <212> DNA
   <213> Homo sapiens
<400> 42
   ttcagcttgt gggtagcact cgggccgagc catgcaggcg gcgcgcgtgg actacatcgc 60
   tccctggtgg gtcgtgtggc tgcacagcgt cccgcacgtc ggcctgcgcc tgcagcccgt 120
   gaacagcacc ttcagccccg gcgacgagag ttaccaggag tcgctgctgt tcctggggct 180
   ggtggccgcc gtctgcctgg gcctgaacct catcttcctt gtggcttacc tggtctgtgc 240
   atgccactgc cggcgggacg atgcggtgca gaccaagcag caccactcct gctgcatcac 300
   ctggacggcc gtggtggccg ggctcatctg ctgtgctgcg gtgggcgttg gtttctatgg 360
   aaacagcgag accaacgatg gggcgtacca gctgatgtac tccttggacg atgccaacca 420
   caccttctct gggatcgatg ctctggtttc cggaactacc cagaagatga aggtggacct 480
   agagcagcac ctggcccggc tcagtgagat ctttgctgcc cggggcgatt acctgcagac 540
   cctgaagttc atacagcaga tggcgggcag cgttgttgtt cagctctcag gactgcccgt 600
   gtggagggag gtcaccatgg agctgaccaa gctatccgac cagactggct acgtggagta 660
   ctacaggtgg ctctcctacc tcctgctctt tatcctggac ctggtcatct gcctcattgc 720
   ctgcctggga ctggccaagc gctccaagtg tctcctggcc tcgatgctgt gctgtggggc 780
   actgagcctg ctcctcagtt gggcatccct ggccgctgat ggctctgcgg cagtggccac 840
   cagtgacttc tgtgtggctc ctgacacctt catcctgaac gtcacggagg gccagatcag 900
   cacagaggtg actcgctact acctgtattg cagccagagt ggaagcagcc ccttccagca 960
   gaccctgacc accttccagc gcgcacttac caccatgcag atccaggtcg cggggctgct 1020
   gcagtttgcc gtgcccctct tctccactgc agaggaagac ctgcttgcaa tccagctcct 1080
   gctgaactcc tcagagtcca gccttcacca gctgaccgcc atggtggact gccgagggct 1140
   gcacaaggat tatctggacg ctcttgctgg catctgctac gacggcctcc agggcttgct 1200
   gtaccttggc ctcttctcct tcctggccgc cctcgccttc tccaccatga tctgtgcagg 1260
   gccaagggcc tggaagcact tcaccaccag aaacagagac tacgatgaca ttgatgatga 1320
   tgaccccttt aacccccaag cctggcgcat ggcggctcac agtcccccga ggggacagct 1380
   tcacagcttc tgcagctaca gcagtggcct gggaagtcag accagcctgc agcccccggc 1440
   ccagaccatc tccaacgccc ctgtctccga gtacatgaac caagccatgc tctttggtag 1500
   gaacccacgc tacgagaacg tgccactaat cgggagagcc tcccctccgc ctacgtactc 1560
   tcccagcatg agagccacct acctgtctgt ggcggatgag cacctgaggc actacgggaa 1620
   tcagtttcca gcctaacaga ctttcggggg ttcctgcctc ctttttccgt tctggttttt 1680
   aattagtgca aatacaagct gcgtttcttt aatagaaacc aaaggcatct ggagcccgag 1740
   aggcctcctg ctgtggcaga ggagcagctg ggattcccga ccaaagcccc agggggtgca 1800
   gaagactcac cacgcgggcc agcctctctc ttttgccctg ctctccacac cagaaatgcc 1860
   cccaggtgct tggctgcctc agaggtacca tccctgagct ggctgcctgg ccctgctcac 1920
   ccctacgcct cgcccttgcc aggaggggag tggcagtgag gagggggcca ggtcaggcac 1980
   caccatcaag agagctgtgt gttctctctg gtcccacaac gatgactctg cctcttgtca 2040
   gcccagccaa gagcccagac gacccctctg tcctcgttcc ctgtcctcgt tccctgcagg 2100
   taacatgaga agggctgatc aggagatgct ctttaagaag ttcgcacccc tgctgacacc 2160
   agaacagccc aaatcagagt tcccagggcc agacaggctc ttcctgggcc acagagggga 2220
   ggcatcagga aagctctgca gtggggggct ggtggctccg gggctggggg atcacaggct 2280
   ggtgaacccc ggtgggaaca gaggtgaaag cctgccacat tccgcctgtc tccctaaccc 2340
   tccattgcct cgcctctatt ccagaatcaa tgctgcagaa tgtgttagct gcagataggc 2400
   atggtctcag gtatgaacag acactttgaa acgactttag gtctttcttt tctccagtgt 2460
   tttaaacatg ttgattatcc aaagaattga aactcctagc acatccagtt tttacaacag 2520
   atttgcagct cattccttac cctggttagg tcactacttt tgcagatttt gctggcactg 2580
   atctggagat ctgcagatct ggaggagacg ggaaggagtc gattcttaaa taaggatcag 2640
   tgaggcatcc tgtcccaagc tactgtttgg tggggatctg ggttcatctc acccacagag 2700
   ggaggatctt taagaggaga aaaaagccaa gagggaaagc cagagttccc tgttctaggg 2760
   gactagccaa atgcctacat cagctgtccc ctccctgttg tctccaagta agtttgccag 2820
   aaaaggtttt agcaaagtgc tacaactgtg tctttatagg aggataggcc tctgccctgc 2880
   cccaccccca ccacctgtcc ccacccagtg tcccaggcca caggagctta ttggccagga 2940
   gggaataatg tcccccaata ctgcctgttg agggaccaga gttggggtct ttggtgcttc 3000
   caacctcctg ccaacctgga gttcacaaca ccagagcccc acggcctcgc acactgaagc     3060
   aggggcgtgc ggtgactcgg tgcttctgtt ttggaagaac cacctgtcat caaaacatgg     3120
   acagcagggt gttctcagct cccagcgaag cctccacaac agaatggggc cacagggcag     3180
   ccgggactcc ctgtctcacc tacattaacc catgcatact gtatgccata aactcacttt     3240
   ggtatatccg cgtcacatgc agagaggaac tctgcgacgt caaagtgttg cttcttaaag     3300
   tttcattatt ggcaactaga gggttgtttt taatgcatgg aaactaaaca gattcctcgg     3360
   ggagttcctg aaggaaccag gtgggcaaac ctttgcttat atacatgcgg cctcacctgg     3420
   aagagaaata aaccacttgt actaaaaaaa aaaaaaaaaa aaaaaaaa                  3468
<210> 43
   <211> 962
   <212> DNA
   <213> Homo sapiens
<400> 43
   gtcgtgaggc gggccttcgg gctggctcgc cgtcggctgc cggggggttg gccggggtgt       60
   cattggctct gggaagcggc agcagaggca gggaccactc ggggtctggt gtcggcacag      120
   ccatggcggg cgcgttggtg cggaaagcgg cggactatgt ccgaagcaag gatttccggg      180
   actacctcat gagtacgcac ttctggggcc cagtagccaa ctggggtctt cccattgctg      240
   ccatcaatga tatgaaaaag tctccagaga ttatcagtgg gcggatgaca tttgccctct      300
   gttgctattc tttgacattc atgagatttg cctacaaggt acagcctcgg aactggcttc      360
   tgtttgcatg ccacgcaaca aatgaagtag cccagctcat ccagggaggg cggcttatca      420
   aacacgagat gactaaaacg gcatctgcat aacaatggaa aaggaagaac aaggtcttga      480
   agggacagca ttgccagctg ctgctgagtc acagatttca ttataaatag cctccctaag      540
   gaaaatacac tgaatgctat ttttactaac cattctattt ttatagaaat agctgagagt      600
   ttctaaacca actctctgct gccttacaag tattaaatat tttacttctt tccataaaga      660
   gtagctcaaa atatgcaatt aatttaataa tttctgatga tggttttatc tgcagtaata      720
   tgtatatcat ctattagaat ttacttaatg aaaaactgaa gagaacaaaa tttgtaacca      780
   ctagcactta agtactcctg attcttaaca ttgtctttaa tgaccacaag acaaccaaca      840
   gctggccacg tacttaaaat tttgtcccca ctgtttaaaa atgttacctg tgtatttcca      900
   tgcagtgtat atattgagat gctgtaactt aatggcaata aatgatttaa atatttgtta      960
   aa                                                                     962
<210> 44
   <211> 1424
   <212> DNA
   <213> Homo sapiens
<400> 44
   gggacgtgcg gcccagcgag ttggtcggtc ccggggtcac ccgctacggg aagcaggcct      60
   cgccacagac taagaaaaat ggctttgtca gcccaacaga tacccagatg gtttaactca     120
   gttaagttga ggagcctcat taatgctgca caactcacaa aacgttttac tagaccagca     180
   agaacactgt tacatggctt ttctgctcag cctcagatat cctctgacaa ttgctttctc     240
   cagtggggat ttaagactta caggacttcc tccttatgga atagttccca gtctactagc     300
   tcaagtagtc aggagaataa ttctgcccaa agcagtctgc ttccttccat gaatgaacag     360
   tcacagaaga cacaaaatat atccagcttt gattctgagc tgtttctaga agaactggat     420
   gaattgcctc cattgtctcc aatgcagcca atttcagagg aagaggctat tcagattatt     480
   gcagaccctc cattgccacc agcttcattc acacttcgag actatgtgga tcattctgag     540
   actctgcaga agttggttct tctaggcgtg gatttgtcca agatagaaaa acatccagaa     600
   gcagcaaacc tccttctgag actggatttt gaaaaagaca ttaagcaaat gcttctgttt     660
   cttaaagatg tgggtataga ggataaccaa ctgggagcat tcctgacaaa aaatcatgca     720
   attttctctg aagaccttga aaatctgaag accagggtgg cttatctgca ttcaaaaaat     780
   ttcagtaaag cagatgttgc acagatggtc agaaaagcac catttttgct gaacttttca     840
   gtggaaagac tggataacag attgggattt tttcagaaag aacttgaact tagtgtgaag     900
   aagactagag atctggtagt tcgtctccca aggctgctaa ctggaagtct ggaacccgtg     960
   aaagaaaata tgaaggttta tcgtcttgaa cttggtttta aacataacga aattcaacat    1020
   atgatcacca gaatcccaaa gatgttaact gcaaataaaa tgaaacttac cgagacgttt    1080
   gattttgtgc acaatgtgat gagcattccc caccacatca ttgtcaagtt cccacaggta    1140
   tttaatacaa ggctgtttaa ggtcaaagaa agacacttgt ttcttaccta tttaggaaga    1200
   gcacagtatg atccagcaaa acctaactac atctctttgg acaaactagt atctattcct    1260
   gatgaaatat tttgtgaaga gattgccaaa gcatcagtac aggactttga aaaattctta    1320
   aaaacgcttt agatttttat gtatgttaaa atgcagtatt gtaaagtgaa tatatatatg    1380
   aataaatgaa tatattttta aatgccaaaa aaaaaaaaaa aaaa                      1424
<210> 45
   <211> 913
   <212> DNA
   <213> Homo sapiens
<400> 45
   agtgctgtac aaagagacag aggctgttag ctatggctga tctatccaat aaaaaagaga     60
   gaggaccgca gacgcctggc tctcatcata tgcaatacaa agtttgatca cctgcctgca    120
   aggaatgggg ctcactatga catcgtgggg atgaaaaggc tgcttcaagg cctgggctac    180
   actgtggttg acgaaaagaa tctcacagcc agggatatgg agtcagtgct gagggcattt    240
   gctgccagac cagagcacaa gtcctctgac agcacgttct tggtactcat gtctcatggc    300
   atcctagagg gaatctgcgg aactgcgcat aaaaagaaaa aaccggatgt gctgctttat    360
   gacaccatct tccagatatt caacaaccgc aactgcctca gtctaaagga caaacccaag    420
   gtcatcattg tccaggcctg cagaggtgaa aaacatgggg aactctgggt cagagactct    480
   ccagcatcct tggcactcat ctcttcacag tcatctgaga acctggaggc agattctgtt    540
   tgcaagatcc acgaggagaa ggacttcatt gctttctgtt cttcaacacc acataacgtg    600
   tcctggagag accgcacaag gggctccatc ttcattacgg aactcatcac atgcttccag    660
   aaatattctt gctgctgcca cctaatggaa atatttcgga aggtacagaa atcatttgaa    720
   gttccacagg ctaaagccca gatgcccacc atagaacgag caaccttgac aagagatttc    780
   tacctctttc ctggcaattg aaaatgaaac cacaggcagc ccagccctcc tctgtcaaca    840
   tcaaagagca catttaccag tatagcttgc atagtcaata tttggtattt caataaaagt    900
   aaagactgta tct                                                       913
<210> 46
   <211> 1796
   <212> DNA
   <213> Homo sapiens
<400> 46
   aatcaaaatt tataaaactt ctattttggt gttcaaagat cgggataaat atgtaagtgg 60
   agaattcagg taagttcaga tttttccctc cagttggttt aatttctatt tcctaaaaca 120
   ttaaaataat aatggaatga ttgaaataat aaacattttt cttattcaag atttcgtcat 180
   ggctattgta aaggaaaccc taggaaaatg gtgaaaactt gggcagaaaa agaaatgagg 240
   aacttaatca ggctaaacac agcagagata ccatgtccag aaccaataat gctaagaagt 300
   catgttcttg tcatgagttt catcggtaaa gatgacatgc ctgcaccact cttgaaaaat 360
   gtccagttat cagaatccaa ggctcgggag ttgtacctgc aggtcattca gtacatgaga 420
   agaatgtatc aggatgccag acttgtccat gcagatctca gtgaatttaa catgctgtac 480
   cacggtggag gcgtgtatat cattgacgtg tctcagtccg tggagcacga ccacccacat 540
   gccttggagt tcttgagaaa ggattgcgcc aacgtcaatg atttctttat gaggcacagt 600
   gttgctgtca tgactgtgcg ggagctcttt gaatttgtca cagatccatc cattacacat 660
   gagaacatgg atgcttatct ctcaaaggcc atggaaatag catctcaaag gaccaaggaa 720
   gaacggtcta gccaagatca tgtggatgaa gaggtgttta agcgagcata tattcctaga 780
   accttgaatg aagtgaaaaa ttatgagagg gatatggaca taattatgaa attgaaggaa 840
   gaggacatgg ccatgaatgc ccaacaagat aatattctat accagactgt tacaggattg 900
   aagaaagatt tgtcaggagt tcagaaggtc cctgcactcc tagaaaatca agtggaggaa 960
   aggacttgtt ctgattcaga agatattgga agctctgagt gctctgacac agactctgaa 1020
   gagcagggag accatgcccg ccccaagaaa cacaccacgg accctgacat tgataaaaaa 1080
   gaaagaaaaa agatggtcaa ggaagcccag agagagaaaa gaaaaaacaa aattcctaaa 1140
   catgtgaaaa aaagaaagga gaagacagcc aagacgaaaa aaggcaaata gaatgagaac 1200
   catattatgt acagtcattt tcctcagttc cttttctcgc ctgaactctt aagctgcatc 1260
   tggaagatgg cttattggtt ttaaccagat tgtcatcgtg gcactgtctg tgaagacgga 1320
   ttcaaatgtt ttcatgtaac tatgtaaaaa gctctaagct ctagagtcta gatccagtca 1380
   ctgactctgt ctggtgttga cagaggattt atttaagcta ttattttaat aaagaacttt 1440
   gtacattttt atttttatat ttttttctct tacaaatatg tttttggaag catgataaat 1500
   gtttaaatgt agtcaacatc tgtaactctt acatgagtgt ccagaggcac tcatgggaaa 1560
   attggttttg ctttctttgt acacaccaga gacccatctg aggtcatctg attataaggc 1620
   catgtttata taaagggaat ttcacccaca gttcagctgg ctgttgattt tcactgcaac 1680
   tctgcctttg tgtgtattgg cgatcatttg taatgctctt acacttcgtc tttaatgttc 1740
   tttttggagt taggacctct cagttcataa agttttttac aattcaaaaa aaaaaa 1796
<210> 47
   <211> 2495
   <212> DNA
   <213> Homo sapiens
<400> 47
   gggtggtgga tctgtcggtc ccgttttccc gtcgcacgtg gtggccactg ttggcttctg      60
   aatggtttgc aaggcggata tccacgccaa ggcctttgga tcggccgtgg gtacatccgt     120
   ctgagccgtt cctttccatc gcagagcggc ggcctccggc ggcgctctcc agtcatggac     180
   taccggcggc ttctcatgag ccgggtggtc cccgggcaat tcgacgacgc ggactcctct     240
   gacagtgaaa acagagactt gaagacagtc aaagagaagg atgacattct gtttgaagac     300
   cttcaagaca atgtgaatga gaatggtgaa ggtgaaatag aagatgagga ggaggagggt     360
   tatgacgatg atgatgatga ctgggactgg gatgaaggag ttggaaaact cgccaagggt     420
   tatgtctgga atggaggaag caacccacag gcaaatcgac agacctccga cagcagttca     480
   gccaaaatgt ctactccagc agacaaggtc ttacggaaat ttgagaataa aattaattta     540
   gataagctaa atgttactga ttccgtcata aataaagtca ccgaaaagtc tagacaaaag     600
   gaagcagata tgtatcgcat caaagataag gcagacagag caactgtaga acaggtgttg     660
   gatcccagaa caagaatgat tttattcaag atgttgacta gaggaatcat aacagagata     720
   aatggctgca ttagcacagg aaaagaagct aatgtatacc atgctagcac agcaaatgga     780
   gagagcagag caatcaaaat ttataaaact tctattttgg tgttcaaaga tcgggataaa     840
   tatgtaagtg gagaattcag atttcgtcat ggctattgta aaggaaaccc taggaaaatg     900
   gtgaaaactt gggcagaaaa agaaatgagg aacttaatca ggctaaacac agcagagata     960
   ccatgtccag aaccaataat gctaagaagt catgttcttg tcatgagttt catcggtaaa    1020
   gatgacatgc ctgcaccact cttgaaaaat gtccagttat cagaatccaa ggctcgggag    1080
   ttgtacctgc aggtcattca gtacatgaga agaatgtatc aggatgccag acttgtccat 1140
   gcagatctca gtgaatttaa catgctgtac cacggtggag gcgtgtatat cattgacgtg 1200
   tctcagtccg tggagcacga ccacccacat gccttggagt tcttgagaaa ggattgcgcc 1260
   aacgtcaatg atttctttat gaggcacagt gttgctgtca tgactgtgcg ggagctcttt 1320
   gaatttgtca cagatccatc cattacacat gagaacatgg atgcttatct ctcaaaggcc 1380
   atggaaatag catctcaaag gaccaaggaa gaacggtcta gccaagatca tgtggatgaa 1440
   gaggtgttta agcgagcata tattcctaga accttgaatg aagtgaaaaa ttatgagagg 1500
   gatatggaca taattatgaa attgaaggaa gaggacatgg ccatgaatgc ccaacaagat 1560
   aatattctat accagactgt tacaggattg aagaaagatt tgtcaggagt tcagaaggtc 1620
   cctgcactcc tagaaaatca agtggaggaa aggacttgtt ctgattcaga agatattgga 1680
   agctctgagt gctctgacac agactctgaa gagcagggag accatgcccg ccccaagaaa 1740
   cacaccacgg accctgacat tgataaaaaa gaaagaaaaa agatggtcaa ggaagcccag 1800
   agagagaaaa gaaaaaacaa aattcctaaa catgtgaaaa aaagaaagga gaagacagcc 1860
   aagacgaaaa aaggcaaata gaatgagaac catattatgt acagtcattt tcctcagttc 1920
   cttttctcgc ctgaactctt aagctgcatc tggaagatgg cttattggtt ttaaccagat 1980
   tgtcatcgtg gcactgtctg tgaagacgga ttcaaatgtt ttcatgtaac tatgtaaaaa 2040
   gctctaagct ctagagtcta gatccagtca ctgactctgt ctggtgttga cagaggattt 2100
   atttaagcta ttattttaat aaagaacttt gtacattttt atttttatat ttttttctct 2160
   tacaaatatg tttttggaag catgataaat gtttaaatgt agtcaacatc tgtaactctt 2220
   acatgagtgt ccagaggcac tcatgggaaa attggttttg ctttctttgt acacaccaga 2280
   gacccatctg aggtcatctg attataaggc catgtttata taaagggaat ttcacccaca 2340
   gttcagctgg ctgttgattt tcactgcaac tctgcctttg tgtgtattgg cgatcatttg 2400
   taatgctctt acacttcgtc tttaatgttc tttttggagt taggacctct cagttcataa 2460
   agttttttac aattcaaaaa aaaaaaaaaa aaaaa 2495
<210> 48
   <211> 2816
   <212> DNA
   <213> Homo sapiens
<400> 48
   cgccgcgggc cgggcgcggg gaggtgtcat gcgccggaac ctgcgcttgg ggccaagctc 60
   tggagctgac gcgcaggggc aaggcgcccc gcgtcccgga ctggcggctc cgcgcatgct 120
   cctcccaccg gcgtcgcagg cctcgagagg ctccggaagt actgggtgca gcctgatggc 180
   gcaggaggta gacacggcac agggcgccga gatgcggcgg ggcgcgggcg cggctcgggg 240
   acgcgcttcc tggtgctggg ccctggcgct gctttggctc gcggtggttc cgggctggtc 300
   ccgggtctcg ggcatcccct cccggcgcca ctggccggtg ccctacaagc gctttgactt 360
   ccgtccaaaa cctgatcctt attgtcaagc taagtatact ttctgtccaa ctggctcacc 420
   tatcccagtt atggagggtg atgatgacat tgaagttttt cgattacaag ccccagtatg 480
   ggaatttaaa tatggagacc tcctgggaca cttgaaaatt atgcatgatg ccattggatt 540
   cagaagtaca ttaactggca agaactacac aatggaatgg tatgaacttt tccaacttgg 600
   caactgtaca tttccccatc tccgacctga aatggatgcc cctttctggt gtaatcaagg 660
   cgctgcctgc ttttttgagg gaattgatga tgttcactgg aaggaaaatg ggacattagt 720
   tcaagtagca actatatcag gaaacatgtt caaccaaatg gcaaagtggg tgaaacagga 780
   caatgaaaca ggaatttatt atgagacatg gaatgtaaaa gccagcccag aaaagggggc 840
   agagacatgg tttgattcct acgactgttc caaatttgtg ttaaggacct ttaacaagtt 900
   ggctgaattt ggagcagagt tcaagaacat agaaaccaac tatacaagaa tatttcttta 960
   cagtggagaa cctacttatc tgggaaatga aacatctgtt tttgggccaa caggaaacaa 1020
   gactcttggt ttagccataa aaagatttta ttaccccttc aaaccacatt tgccaactaa 1080
   agaatttctg ttgagtctct tgcaaatttt tgatgcagtg attgtgcaca aacagttcta 1140
   tttgttttat aattttgaat attggttttt acctatgaaa ttccctttta ttaaaataac 1200
   atatgaagaa atccctttac ctatcagaaa caaaacactc tctggtttat aaaacacctt 1260
   aattctactg ctcttttttc tccaatcacc agcatctgtt tttcaggggg tgattttact 1320
   tttgtgaatt ccttagcctt tcttccttgg tgcataaagt taaaatgcac atcagcagaa 1380
   ttgctgcata ttaacatctc aggactcttc tcttgtaaag aagctgaaat tcgtactata 1440
   ttggccaaag tgagcgagtt aggtgatctt ggtttcaatt tccgagcctt tgttaatatg 1500
   gagaattatg gttcatatca gttatgtagg acctttggac ccagggtcct acagatagat 1560
   atggtgtgcc cagattttaa aaataccttc aaaaataaaa aatacattca gtgacatttt 1620
   catggtggga gctcttcttt ctgatatggc agttacactt tttcacttaa gtgctttagt 1680
   ttagactaac tttacaactt ctataacttt tggaaccaag tttagtatag tctgattaca 1740
   ttccattcac ctaactttag acattcgttt agacaccata actggagtga ttgtgcttct 1800
   agatgtggca aatccagtgt taacacatat ttctggctga gattttggaa ctagctagta 1860
   actggcttgt gttctttaag catactaaca tcactaaatc ttaggattta ggattgctgt 1920
   aaagatgtaa gttgtgtatg tttggcaggt cacattgaat ggcagtgata atgattaatc 1980
   aaagaacaaa tgtcatcctt gatcttgcct aatgtagttt atgtgccaaa ctttccaggg 2040
   ttttgtagtc acctagattt taagctgata gcatagtgct tcagcggttc ttctaaccgg 2100
   ggtatgcagg aacatggctg cagacacgtt tgggtaaaca ggcaccttct gacttcttca 2160
   ttgtttcctg tagttctccc tcttcccaca aagctgtcag cgcagtggaa gaggttgcac 2220
   ttctccgaga gaggacaggt ttctgttaag atcaccaagt agctgtgctt taattagagc 2280
   cagacaagct ttcaaggtcc tttaagtatt tgatgatcaa ctgaacacgt ttctattcaa 2340
   ggagaaaaca ccattcagta agaagatgga gtagatatca gataaaacaa ttcacgttta 2400
   atatgtaaat gtaccaatta tgtgattcag tttcaacttt caagtacttc ctgagaggtt 2460
   agtacattat tattgagctc tcacagaaag cttaatcata gatataatta acctgatcca 2520
   aatgagtaaa gtggacctta gaaaggctaa gtgatctttc tctggctatc cagctagtag 2580
   taatgaagtc aggtcttgaa ccccggttct gctgactgaa ttggatgcac tatagtacag 2640
   gcttttagca ccgaagtgtg gtcctcagac cagtgcctgc caaccagatg ttactggtct 2700
   gtgaggaaat aagtacagat actgacagga agcttttata aacaatttat tggagtgttt 2760
   ttgtttctgt tgggtctaat taaaaaaatt ggagcttgta aaaaaaaaaa aaaaaa 2816
<210> 49
   <211> 3859
   <212> DNA
   <213> Homo sapiens
<400> 49
   aaagatgact cctgtacggt ccctgccgtc caggaccctg gagtcaagtc agaaatggat     60
   gtgtccacag ctgattgtgt gacaaggtct ggatttgtgg gtgttttcat tggagtccag    120
   ggaccctggt accttatgtg cttttttgcc ttgtttcttt ccagattgct catttagaaa    180
   gtggatgtgt gggataacat gtccaatgtg tccgaggaga gaagaaaaag gcagcagaac    240
   attaaggaag gactgcagtt tatacagtca ccgctgtcat atccaggaac acaggagcaa    300
   tatgcggtat atttgcgtgc tctcgtgaga aatcttttta atgaaggaaa tgacgtttat    360
   cgggaacatg attggaacaa ctcgataagc cagtacacgg aagccttgaa tatagctgat    420
   tatgcaaaat ctgaagaaat tttaatcccc aaagaaataa ttgaaaaact atatataaat    480
   cgtattgcct gctattctaa tatgggtttc catgataaag ttttggagga ctgcaatata    540
   gtcctcagtt taaatgccag taactgcaaa gctctgtatc ggaaatctaa ggctttaagt    600
   gatttaggaa gatacaaaaa ggcttacgat gctgtagcaa agtgctcctt agcagtgcct    660
   caggatgagc atgtaataaa actaactcaa gaactagctc agaaattggg atttaaaata    720
   agaaaagcgt atgtcagagc tgagctctca ttaaaatcag ttcctgggga tggggctacc    780
   aaggctttga accattctgt ggaagatatt gagccagatt tattaactcc aaggcaagaa    840
   gcagttcctg ttgtctcttt accggcaccc agtttttctc atgaagttgg aagtgagctg    900
   gcctcagttc ctgttatgcc cttaacttct attttgccac tacaagtgga agagagcgct    960
   ctgccatctg cagtgctggc aaatggagga aagatgccct tcactatgcc agaagctttt   1020
   ctagatgatg gagatatggt ccttggagat gaactagatg acctgcttga ttctgcacct   1080
   gaaactaatg aaactgttat gccgtcagct ttagtcagag gaccccttca gacagccagt   1140
   gtctctccta gcatgccctt ttcggcatcg ctgttaggaa ccttacccat tggtgcgagg 1200
   tatgctcctc caccctcctt ctcagaattt tatccacctt tgacttcatc cttagaagat 1260
   ttttgttctt ctttaaattc attttcaatg agtgaatcca aacgagatct gtccacctca 1320
   acttctagag agggaacacc gcttaacaac agtaattctt cccttttact tatgaatgga 1380
   ccaggtagtt tgtttgcttc agagaatttc ctgggaattt caagtcagcc tagaaatgac 1440
   tttggaaact tttttggaag tgcagttacc aaaccatctt catcagtgac tccaagacat 1500
   cccctcgaag gaacccatga attgagacaa gcttgccaga tctgttttgt aaaatcaggc 1560
   cctaagttaa tggatttcac ttaccatgct aacatagatc ataagtgtaa gaaagatatt 1620
   ttaatcggta ggataaagaa tgttgaagat aaatcatgga aaaaaatacg tccaagacca 1680
   acaaaaacaa attatgaagg accatattat atatgtaaag atgttgctgc tgaggaggaa 1740
   tgtagatatt caggccactg cacgtttgct tattgccaag aggagataga tgtgtggaca 1800
   ctggagcgga aaggagcatt cagccgggag gctttctttg gcggcaatgg aaagattaac 1860
   cttactgtgt tcaaacttct ccaggagcat cttggggaat ttatattcct ttgtgagaaa 1920
   tgttttgatc ataagcctag aatgataagt aaaagaaata aagataattc tactgcttgt 1980
   tctcacccgg ttacaaagca tgagtttgaa gacaataagt gccttgtcca cattttgcga 2040
   gagacaacag taaaatactc caaaatacgt tcttttcatg gtcagtgtca gcttgattta 2100
   tgtcgacatg aagttcggta tggctgttta agggaagatg agtgctttta tgcccatagt 2160
   cttgtggaac tgaaagtctg gataatgcaa aatgaaacag gtatctcaca tgatgctatt 2220
   gctcaagagt ctaaacgata ttggcagaat ttggaagcaa atgtacctgg agcgcaggta 2280
   cttggtaatc aaataatgcc tggatttctt aatatgaaga taaagtttgt gtgcgcccag 2340
   tgtctgagaa acggtcaagt cattgaacca gacaaaaaca gaaaatattg tagtgcaaaa 2400
   gcaaggcatt cgtggaccaa agaccggcgt gcgatgagag tgatgtctat tgaacgtaag 2460
   aagtggatga acatccgtcc tctccccaca aagaaacaaa tgcctttaca gtttgatctg 2520
   tgcaaccata ttgcttctgg gaaaaaatgt caatatgttg gaaactgttc ctttgctcat 2580
   agtcctgagg aaagagaagt ttggacttac atgaaggaga atgggataca agatatggag 2640
   caattttacg aactatggct caagagtcaa aaaaatgaaa aaagtgaaga catagccagt 2700
   cagtcaaaca aggaaaatgg aaaacaaatt cacatgccaa cagattatgc tgaagttaca 2760
   gtggactttc actgctggat gtgtgggaaa aactgcaaca gtgagaagca gtggcagggc 2820
   cacatctcct ccgagaagca caaagagaag gttttccaca ccgaggacga ccagtactgc 2880
   tggcagcacc gcttcccaac aggctatttc agtatttgtg ataggtatat gaatggcacc 2940
   tgcccagaag gaaacagctg taaatttgca catggaaatg ccgaacttca tgaatgggaa 3000
   gaaagaagag atgccctaaa gatgaagctc aacaaagcac gaaaagatca cttaattggc 3060
   ccaaatgata atgactttgg aaaatatagt tttttgttta aagatttaaa ctaatatgct 3120
   ggcttttatg tatgatacct aatcagagca ttgaccagaa aaattgaaag tgttctgagg     3180
   cacatagcag aggagctgca gatttcctgc ttgtattggc gtatatcgtt cctcctgagc     3240
   agcaacccac agtaggtagg aaaatgggct gtttcacagg cctggccacg ctctcacgga     3300
   accactggca tcagatggtg aagtgactgc tacccggttg ccatctgttg aacagacttt     3360
   tggatgaagt gtgttgggga agaggataag gttatatcta ggacaactct ttgagttggt     3420
   ccttcatata agaatcgtga cggtaagaga ataaacactt gtactgggat cagaatacat     3480
   gatggatgaa attctttaca tgttttagca gaatgaattt gtttaatata ataaagtttg     3540
   ctacttatct gtatgtaggt tgctaaaaag gattttctta actcagattt taagccaaat     3600
   aaccatttaa cactagtatt tgttaaatgg ggtatttttc tgtatttgta tgtttcacta     3660
   taataaggga attaaggata atgtgcattg agaatatttt gaaaaataat tgactcaaat     3720
   tttatttctt ggtcttttgc tgtttaaatg atgattttga aagattaaac ctgtactgtt     3780
   ggtattgtgt tagtgtatgg accaatactg cctgtaataa agattttata tatagatgcc     3840
   tttcaaaaaa aaaaaaaaa                                                  3859
<210> 50
   <211> 7209
   <212> DNA
   <213> Homo sapiens
<400> 50
   gacgtcatca cgcagggccg agtcggcgcg gccacatcct ttaaatatgg tcttttttgg      60
   gcgcgcgcga caatgtgagg agtggggtgg agcgtgtgtg gtgtgtggct gcggcctggg     120
   caagagccgc cgcggaccat gagctgagta agttctggag ggatcctgcc tcttggagcc     180
   ttcgcagcca ggcagctgtg aactgtgagc tagagtgaag cagaaatcta ggaagatgag     240
   ctccaagatg gtcataagtg aaccaggact gaattgggat atttccccca aaaatggcct     300
   taagacattt ttctctcgag aaaattataa agatcattcc atggctccaa gtttaaaaga     360
   actacgtgtt ttatccaaca gacgtatagg agaaaatttg aatgcctcag caagttctgt     420
   agaaaatgag ccggcagtta gttcagcaac tcaagcaaag gaaaaagtta aaaccacaat     480
   tggaatggtt cttcttccaa aaccaagagt tccttatcct cgtttctctc gtttctcaca     540
   gagagagcag aggagttatg tggacttgtt ggttaaatac gcaaagattc ctgcaaattc     600
   caaagctgtt ggaataaata aaaatgacta cttgcagtac ttggatatga aaaaacatgt     660
   gaacgaagaa gttactgagt tcctaaagtt tttgcagaat tctgcaaaga aatgtgcgca     720
   ggattataat atgctttctg atgatgcccg tctcttcaca gagaaaattt taagagcttg     780
   cattgaacaa gtgaaaaagt attcagaatt ctatactctc cacgaggtca ccagcttaat     840
   gggattcttc ccattcagag tagagatggg attaaagtta gaaaaaactc ttctcgcatt     900
   gggcagtgta aaatatgtga aaacagtatt tccctcaatg cctataaagt tgcagctgtc     960
   aaaggacgat atagctacca ttgaaacgtc agaacaaaca gctgaagcta tgcattatga 1020
   tattagtaaa gatccaaatg cagagaagct tgtttccaga tatcaccctc agatagctct 1080
   aactagtcag tcattattta ccttattaaa taatcatgga ccaacgtaca aggaacagtg 1140
   ggaaattcca gtgtgtattc aagtaatacc tgttgcaggt tcaaaaccag ttaaagtaat 1200
   atatattaat tcaccacttc cccaaaagaa aatgactatg agagagagaa atcaaatctt 1260
   tcatgaagtt ccattaaaat ttatgatgtc caaaaacaca tctgttccag tctctgcagt 1320
   ctttatggac aaacctgaag agtttatatc tgaaatggac atgtcctgtg aagtcaacga 1380
   gtgccgaaaa attgagagtc ttgaaaactt gtatttggat tttgatgatg atgtcacaga 1440
   acttgaaact tttggagtaa ccaccaccaa agtatcaaaa tcaccaagtc cagcaagtac 1500
   ttccacagta cctaacatga cagatgctcc tacagccccc aaagcaggaa ctacaactgt 1560
   ggcaccaagt gcaccagaca tttctgctaa ttctagaagt ttatctcaga ttctgatgga 1620
   acaattgcaa aaggagaaac agctggtcac tggtatggat ggtggccctg aggaatgcaa 1680
   aaataaagat gatcagggat ttgaatcatg tgaaaaggta tcaaattctg acaagccttt 1740
   gatacaagat agtgacttga aaacatctga tgccttacag ttagaaaatt ctcaggaaat 1800
   tgaaacttct aataaaaatg atatgactat agatatatta catgctgatg gtgaaagacc 1860
   taatgttcta gaaaacctag acaactcaaa ggaaaagact gttggatcag aagcagcaaa 1920
   aactgaagat acagttctct gcagcagtga tacagatgag gagtgtttaa tcattgatac 1980
   agaatgtaaa aataatagtg atggaaagac agctgttgtg ggttctaact taagttccag 2040
   accagctagt ccaaattctt cctcaggaca ggcttctgta ggaaaccaga ctaatactgc 2100
   ttgtagtcct gaagagtcat gtgttttaaa aaaacctatc aaacgagtat ataaaaaatt 2160
   tgatccagtt ggagagattt taaaaatgca ggatgagctc ttaaagccaa tttccagaaa 2220
   agtaccagaa ttgcccttaa tgaatttaga aaattctaaa cagccttctg tttctgagca 2280
   attgtctggt ccttcagact cctctagttg gccgaaatct ggatggcctt ctgcatttca 2340
   gaagccaaaa ggacgattgc catatgaact tcaggactat gttgaagata catcggaata 2400
   cctagctcct caggaaggaa attttgttta taagttattt agcctgcaag acctgttgtt 2460
   actcgtacgc tgcagtgtcc agaggataga gacaagacca cgttctaaaa aacggaagaa 2520
   aatcagaaga caatttccag tttatgtact accaaaagta gagtatcaag cttgttatgg 2580
   agttgaagct ctgactgaaa gtgaactttg tcgcttatgg actgaaagtt tattgcattc 2640
   caacagctca ttttatgttg ggcatatcga tgcatttact tcaaaacttt ttctactgga 2700
   agaaattacc tcagaagaat taaaagaaaa gctttcagca ctcaagattt ccaatttatt 2760
   taacatcctc caacacattc taaagaaact aagtagcttg caggagggtt cctacttgtt 2820
   atctcatgca gcagaagatt cttcactcct gatttataag gcctctgatg gaaaagttac 2880
   taggacagca tacaatttgt ataaaacaca ttgcggcctt cctggtgtac cttccagtct 2940
   ctcagttccc tgggtcccat tagatcccag cctgttatta ccatatcata tccatcatgg 3000
   aagaatacct tgtacttttc caccgaaatc actggatacc acaacacaac aaaagattgg 3060
   tggaacgaga atgcctacac gcagccacag gaatccagtt tccatggaaa ccaaaagcag 3120
   ttgcttgcct gctcagcaag ttgaaactga aggagtggct ccacataaaa gaaaaataac 3180
   ttgaggactg taccatggaa aactaaattt aaaaaaacag ttataacagt gtttaattta 3240
   gataagtttg agggaaaata atcagtaggc aagaggaaca tttttcctgt agtagctaga 3300
   gtgccttgaa aaaatgtgtt ggctatgtga aggaatattt caactaaaat ggaatggtat 3360
   gcttttcacc cttgaagttt gaggaggatc ttgatatgtt ttaacattat catggcaggg 3420
   aaatatataa agaagaaaaa tatttttaca ttaaaccttt tctaaaaatt gtaaatagaa 3480
   aaataatttg gttttttatc aagaacaaca cttatcgtta tgtattgtgt tagttatatt 3540
   gccagtctgt tgcgactgac tcaaaaagtt aaatgttgcc actgctgaag atgattatga 3600
   gcatcgcaaa ctttgtttct gacccatttt gacagttttt atatactcct ttaaaatgat 3660
   gaatgttaca ggttaataaa gttaatacct ttaaaaactt ggtgaaattc cattacagaa 3720
   gccaaaaata aaaactccct gcctctgaaa agtcagatta ctgacttctt gtttggcaac 3780
   catcagtttg tttaataaaa gaaaaaattt ggtggtataa catgtttgat gacagatgcc 3840
   tctatctcta gattcaagct gagtgttgaa atacactgct gaaagcaaag agataggtat 3900
   gttttccaga aaaaaagtca gtgtcattgc tccagatgac aaggttaatg tggtaaagca 3960
   taagcttttt tttttttttg agatggagtc tcgctctgtt gcccaggctg gagtgcagtg 4020
   gcatgatctc agctcaccac aacatccacc tcctggatta aagcgattct cctgcctcat 4080
   cctcccgagt agctgggact acaggcacct accaccaggc ttggctaatt tttttttgta 4140
   tttttagtag agacagggtg ttaccatgtt ggccgggctg gtctcaaact cctgaccttg 4200
   tgatccgcct gccttggcct cccaaagtgc tgggattaca ggggtaagcc accgtgcctg 4260
   gccagcataa gctattttta tcctcatcta tactgattga tttaaatttt ccgggcctgc 4320
   agatgaattt ggaaaagggc ataatcaact gatgttgctg tagcaaattt ggacttaaga 4380
   aactacagac agggccgggc gtggtggctc acacctgtaa ttccagcact tcgcaaggcc 4440
   gaggcgggag aatcacttga gtccaggggt tggagaccag tctgggcaac acagacctca 4500
   tctctacaaa taaaaaagtt agccgggtgt ggtggggtgt gcctgtagtc ccagctactc 4560
   aaaaggctga ggcagaagaa ttgcttgagc ccaagaagtc aaggctacag tgagttgtga 4620
   tcgcaccaca gaactcagct tgagtatcag agtgtgaccc tgtctcaaag aaaaaaaaaa 4680
   acaggacagg cttgtttaat ctttagaacc cttacactaa aattttcaga aattatgtat 4740
   gtcatttgta aaatataagt tttgtaatcc tagataattg cttcacattg aaatttttta 4800
   gctattctgt gtaaagaata gctattcttt tggcctaatt tctgagaaat tagaccgaaa 4860
   aatctcctca agtattgtaa cagttgaaaa tacttatatt caactaccaa gttttgtttc 4920
   attttgattt tttgttataa aacattttta agttatttga gaagtaatta ttagtaaaat 4980
   tgtatcttga taaatcattt cctcatcctg ccattccctt ttataaaaca ctgagttttt 5040
   ttattcctta agtcacatat cccagaaatg ttttggttgt gaatcatttg ccagcgagcc 5100
   aagggagagg cagggattcc cttgaaatgt actcttacac ttttttattt tatttttaat 5160
   gtacatttgt gtgcaccaga atggaatgtg ctctttttaa agagctggac agtggttgct 5220
   tcagacttct gaataccaga aagacttgcc ttttaatttt taaatattac tttttacatc 5280
   ataaaatgtg ttatcttatc catgtatttg aataacatca tatataaagg tagttttttg 5340
   cttgtgctta gtgaagcctc cttttttaac atacatttga tatgctttat gttctttttc 5400
   ttgattttta gtactgagaa tttataagcc aagaagtaac tgacaatatg tttagcagga 5460
   tgtaatccca gctacttggg aagctgaggt gggaggatcg cttgagtcta ggagtttgag 5520
   gctgcagtga gctatgatgg tgccactgca ctccatcctg ggtgagagat caagacccta 5580
   tctcaaaaaa tgagcaagtt tttttttttt ttttaaatca gacttagggc tagcaaggat 5640
   gtgatcaccc tggcccattt atacctcagc agtgaaggtg taatttatac acacctcttt 5700
   gaaaacagtt taattcagta agtgagtgca actatgtgcc atgcactcca aagtaggtcc 5760
   ccacagttat tctctcatag ccttgttttt tcagatctca ccatatttat ttgtttcttt 5820
   gattttcttt tcttatagat tacaagctct gcaaggacag gggcatattt gttttgttca 5880
   ccattaagta ataaacacct aatacagtgc ctgtcatgta gttgcttagc agatattagt 5940
   tagatgcatg aatgtgtagg tatcaaagat tttttttttc aggagatcac attctagtgg 6000
   aagacactat actgctttat gtgataagtg ctttgataaa gtaagcaagt catctaacca 6060
   gatcttggta gttaaggttt tctgagatag aacttgagct gagtctttta aaaatagtag 6120
   gatttataag ggaaaattaa aggtcttcca caaagaaact aagtgaaact aagcagccta 6180
   tgaaaaggtg aaagttggag agattatagg ctatgaacat gggagatgag agagaaaact 6240
   gcaggatagg gacagaaaca ctgtgctaag acatttgaac tttgtcctgt gcaatgtggg 6300
   aaccagtagt gaaggttaat taactgacat gatcagatat gtatttgaaa agggctcttg 6360
   ttggcattag tctagagaat gcgatggagc tgggaagcga aagaagaaat gttgggagcc 6420
   tgtgttgtag taactgaaaa atggcaatgg cccagaatta aagcagcaag aataggagtg 6480
   ggctcaagtg atgtataagg aaaaaaaaaa aaatgacaac tcagttttgt tgttggtaag 6540
   gagagtaaag tatggcttct gggtttctca tttgagatac tgagtaaatg atggtgttaa 6600
   cccattgaga aaatagtgtg gttagagtgg gaagaaagtt aatttaaaat gttcattttt 6660
   ttttttagtc atttctactt ttggattcta ggtttgtgtg ctatattggt tataaccaat 6720
   ttgctgaata ataaattatc ccaaaactta gctacttaaa acaacaaaca ttatctcaca 6780
   gtttctgtaa gtcaaaagtc caggtctctc atgaagttgc agtcaggctc ttggctaggg 6840
   ctgcagtcat ctgaaggatt agtaagtcgc taagcccaca cttaagctcc acccttgaag 6900
   ggagaagttt ggaaagaatt tgtgaacata tttttaaatc acatatgcta tgaacaggtt 6960
   taggggaggg agaggaaaga aaactttaac ttttcattag catactgcat ctgtagttcc 7020
   taatgggcat gttgtaaaga tgttaagacg ttttctatgc attataggta tatagtagta 7080
   ttttgttttg ttctgttttt ttgtttggta tgtagtagtt tttaatcaaa ccttaacgta 7140
   tttgatatta agatttattg agtttcttgt tgagatgtag aaataaaggg gatccatttc 7200
   atttatcca 7209
<210> 51
   <211> 7049
   <212> DNA
   <213> Homo sapiens
<400> 51
   gacgtcatca cgcagggccg agtcggcgcg gccacatcct ttaaatatgg tcttttttgg        60
   gcgcgcgcga caatgtgagg agtggggtgg agcgtgtgtg gtgtgtggct gcggcctggg      120
   caagagccgc cgcggaccat gagctgagta agttctggag ggatcctgcc tcttggagcc      180
   ttcgcagcca ggcagctgtg aactgtgagc tagagtgaag cagaaatcta ggaagatgag      240
   ctccaagatg gtcataagtg aaccaggact gaattgggat atttccccca aaaatggcct      300
   taagacattt ttctctcgag aaaattataa agatcattcc atggctccaa gtttaaaaga      360
   actacgtgtt ttatccaaca gagagagcag aggagttatg tggacttgtt ggttaaatac      420
   gcaaagattc ctgcaaattc caaagctgtt ggaataaata aaaatgacta cttgcagtac      480
   ttggatatga aaaaacatgt gaacgaagaa gttactgagt tcctaaagtt tttgcagaat      540
   tctgcaaaga aatgtgcgca ggattataat atgctttctg atgatgcccg tctcttcaca      600
   gagaaaattt taagagcttg cattgaacaa gtgaaaaagt attcagaatt ctatactctc      660
   cacgaggtca ccagcttaat gggattcttc ccattcagag tagagatggg attaaagtta      720
   gaaaaaactc ttctcgcatt gggcagtgta aaatatgtga aaacagtatt tccctcaatg      780
   cctataaagt tgcagctgtc aaaggacgat atagctacca ttgaaacgtc agaacaaaca      840
   gctgaagcta tgcattatga tattagtaaa gatccaaatg cagagaagct tgtttccaga      900
   tatcaccctc agatagctct aactagtcag tcattattta ccttattaaa taatcatgga      960
   ccaacgtaca aggaacagtg ggaaattcca gtgtgtattc aagtaatacc tgttgcaggt     1020
   tcaaaaccag ttaaagtaat atatattaat tcaccacttc cccaaaagaa aatgactatg     1080
   agagagagaa atcaaatctt tcatgaagtt ccattaaaat ttatgatgtc caaaaacaca     1140
   tctgttccag tctctgcagt ctttatggac aaacctgaag agtttatatc tgaaatggac     1200
   atgtcctgtg aagtcaacga gtgccgaaaa attgagagtc ttgaaaactt gtatttggat     1260
   tttgatgatg atgtcacaga acttgaaact tttggagtaa ccaccaccaa agtatcaaaa 1320
   tcaccaagtc cagcaagtac ttccacagta cctaacatga cagatgctcc tacagccccc 1380
   aaagcaggaa ctacaactgt ggcaccaagt gcaccagaca tttctgctaa ttctagaagt 1440
   ttatctcaga ttctgatgga acaattgcaa aaggagaaac agctggtcac tggtatggat 1500
   ggtggccctg aggaatgcaa aaataaagat gatcagggat ttgaatcatg tgaaaaggta 1560
   tcaaattctg acaagccttt gatacaagat agtgacttga aaacatctga tgccttacag 1620
   ttagaaaatt ctcaggaaat tgaaacttct aataaaaatg atatgactat agatatatta 1680
   catgctgatg gtgaaagacc taatgttcta gaaaacctag acaactcaaa ggaaaagact 1740
   gttggatcag aagcagcaaa aactgaagat acagttctct gcagcagtga tacagatgag 1800
   gagtgtttaa tcattgatac agaatgtaaa aataatagtg atggaaagac agctgttgtg 1860
   ggttctaact taagttccag accagctagt ccaaattctt cctcaggaca ggcttctgta 1920
   ggaaaccaga ctaatactgc ttgtagtcct gaagagtcat gtgttttaaa aaaacctatc 1980
   aaacgagtat ataaaaaatt tgatccagtt ggagagattt taaaaatgca ggatgagctc 2040
   ttaaagccaa tttccagaaa agtaccagaa ttgcccttaa tgaatttaga aaattctaaa 2100
   cagccttctg tttctgagca attgtctggt ccttcagact cctctagttg gccgaaatct 2160
   ggatggcctt ctgcatttca gaagccaaaa ggacgattgc catatgaact tcaggactat 2220
   gttgaagata catcggaata cctagctcct caggaaggaa attttgttta taagttattt 2280
   agcctgcaag acctgttgtt actcgtacgc tgcagtgtcc agaggataga gacaagacca 2340
   cgttctaaaa aacggaagaa aatcagaaga caatttccag tttatgtact accaaaagta 2400
   gagtatcaag cttgttatgg agttgaagct ctgactgaaa gtgaactttg tcgcttatgg 2460
   actgaaagtt tattgcattc caacagctca ttttatgttg ggcatatcga tgcatttact 2520
   tcaaaacttt ttctactgga agaaattacc tcagaagaat taaaagaaaa gctttcagca 2580
   ctcaagattt ccaatttatt taacatcctc caacacattc taaagaaact aagtagcttg 2640
   caggagggtt cctacttgtt atctcatgca gcagaagatt cttcactcct gatttataag 2700
   gcctctgatg gaaaagttac taggacagca tacaatttgt ataaaacaca ttgcggcctt 2760
   cctggtgtac cttccagtct ctcagttccc tgggtcccat tagatcccag cctgttatta 2820
   ccatatcata tccatcatgg aagaatacct tgtacttttc caccgaaatc actggatacc 2880
   acaacacaac aaaagattgg tggaacgaga atgcctacac gcagccacag gaatccagtt 2940
   tccatggaaa ccaaaagcag ttgcttgcct gctcagcaag ttgaaactga aggagtggct 3000
   ccacataaaa gaaaaataac ttgaggactg taccatggaa aactaaattt aaaaaaacag 3060
   ttataacagt gtttaattta gataagtttg agggaaaata atcagtaggc aagaggaaca 3120
   tttttcctgt agtagctaga gtgccttgaa aaaatgtgtt ggctatgtga aggaatattt 3180
   caactaaaat ggaatggtat gcttttcacc cttgaagttt gaggaggatc ttgatatgtt 3240
   ttaacattat catggcaggg aaatatataa agaagaaaaa tatttttaca ttaaaccttt 3300
   tctaaaaatt gtaaatagaa aaataatttg gttttttatc aagaacaaca cttatcgtta 3360
   tgtattgtgt tagttatatt gccagtctgt tgcgactgac tcaaaaagtt aaatgttgcc 3420
   actgctgaag atgattatga gcatcgcaaa ctttgtttct gacccatttt gacagttttt 3480
   atatactcct ttaaaatgat gaatgttaca ggttaataaa gttaatacct ttaaaaactt 3540
   ggtgaaattc cattacagaa gccaaaaata aaaactccct gcctctgaaa agtcagatta 3600
   ctgacttctt gtttggcaac catcagtttg tttaataaaa gaaaaaattt ggtggtataa 3660
   catgtttgat gacagatgcc tctatctcta gattcaagct gagtgttgaa atacactgct 3720
   gaaagcaaag agataggtat gttttccaga aaaaaagtca gtgtcattgc tccagatgac 3780
   aaggttaatg tggtaaagca taagcttttt tttttttttg agatggagtc tcgctctgtt 3840
   gcccaggctg gagtgcagtg gcatgatctc agctcaccac aacatccacc tcctggatta 3900
   aagcgattct cctgcctcat cctcccgagt agctgggact acaggcacct accaccaggc 3960
   ttggctaatt tttttttgta tttttagtag agacagggtg ttaccatgtt ggccgggctg 4020
   gtctcaaact cctgaccttg tgatccgcct gccttggcct cccaaagtgc tgggattaca 4080
   ggggtaagcc accgtgcctg gccagcataa gctattttta tcctcatcta tactgattga 4140
   tttaaatttt ccgggcctgc agatgaattt ggaaaagggc ataatcaact gatgttgctg 4200
   tagcaaattt ggacttaaga aactacagac agggccgggc gtggtggctc acacctgtaa 4260
   ttccagcact tcgcaaggcc gaggcgggag aatcacttga gtccaggggt tggagaccag 4320
   tctgggcaac acagacctca tctctacaaa taaaaaagtt agccgggtgt ggtggggtgt 4380
   gcctgtagtc ccagctactc aaaaggctga ggcagaagaa ttgcttgagc ccaagaagtc 4440
   aaggctacag tgagttgtga tcgcaccaca gaactcagct tgagtatcag agtgtgaccc 4500
   tgtctcaaag aaaaaaaaaa acaggacagg cttgtttaat ctttagaacc cttacactaa 4560
   aattttcaga aattatgtat gtcatttgta aaatataagt tttgtaatcc tagataattg 4620
   cttcacattg aaatttttta gctattctgt gtaaagaata gctattcttt tggcctaatt 4680
   tctgagaaat tagaccgaaa aatctcctca agtattgtaa cagttgaaaa tacttatatt 4740
   caactaccaa gttttgtttc attttgattt tttgttataa aacattttta agttatttga 4800
   gaagtaatta ttagtaaaat tgtatcttga taaatcattt cctcatcctg ccattccctt 4860
   ttataaaaca ctgagttttt ttattcctta agtcacatat cccagaaatg ttttggttgt 4920
   gaatcatttg ccagcgagcc aagggagagg cagggattcc cttgaaatgt actcttacac 4980
   ttttttattt tatttttaat gtacatttgt gtgcaccaga atggaatgtg ctctttttaa 5040
   agagctggac agtggttgct tcagacttct gaataccaga aagacttgcc ttttaatttt 5100
   taaatattac tttttacatc ataaaatgtg ttatcttatc catgtatttg aataacatca 5160
   tatataaagg tagttttttg cttgtgctta gtgaagcctc cttttttaac atacatttga     5220
   tatgctttat gttctttttc ttgattttta gtactgagaa tttataagcc aagaagtaac     5280
   tgacaatatg tttagcagga tgtaatccca gctacttggg aagctgaggt gggaggatcg     5340
   cttgagtcta ggagtttgag gctgcagtga gctatgatgg tgccactgca ctccatcctg     5400
   ggtgagagat caagacccta tctcaaaaaa tgagcaagtt tttttttttt ttttaaatca     5460
   gacttagggc tagcaaggat gtgatcaccc tggcccattt atacctcagc agtgaaggtg     5520
   taatttatac acacctcttt gaaaacagtt taattcagta agtgagtgca actatgtgcc     5580
   atgcactcca aagtaggtcc ccacagttat tctctcatag ccttgttttt tcagatctca     5640
   ccatatttat ttgtttcttt gattttcttt tcttatagat tacaagctct gcaaggacag     5700
   gggcatattt gttttgttca ccattaagta ataaacacct aatacagtgc ctgtcatgta     5760
   gttgcttagc agatattagt tagatgcatg aatgtgtagg tatcaaagat tttttttttc     5820
   aggagatcac attctagtgg aagacactat actgctttat gtgataagtg ctttgataaa     5880
   gtaagcaagt catctaacca gatcttggta gttaaggttt tctgagatag aacttgagct     5940
   gagtctttta aaaatagtag gatttataag ggaaaattaa aggtcttcca caaagaaact     6000
   aagtgaaact aagcagccta tgaaaaggtg aaagttggag agattatagg ctatgaacat     6060
   gggagatgag agagaaaact gcaggatagg gacagaaaca ctgtgctaag acatttgaac     6120
   tttgtcctgt gcaatgtggg aaccagtagt gaaggttaat taactgacat gatcagatat     6180
   gtatttgaaa agggctcttg ttggcattag tctagagaat gcgatggagc tgggaagcga     6240
   aagaagaaat gttgggagcc tgtgttgtag taactgaaaa atggcaatgg cccagaatta     6300
   aagcagcaag aataggagtg ggctcaagtg atgtataagg aaaaaaaaaa aaatgacaac     6360
   tcagttttgt tgttggtaag gagagtaaag tatggcttct gggtttctca tttgagatac     6420
   tgagtaaatg atggtgttaa cccattgaga aaatagtgtg gttagagtgg gaagaaagtt     6480
   aatttaaaat gttcattttt ttttttagtc atttctactt ttggattcta ggtttgtgtg     6540
   ctatattggt tataaccaat ttgctgaata ataaattatc ccaaaactta gctacttaaa     6600
   acaacaaaca ttatctcaca gtttctgtaa gtcaaaagtc caggtctctc atgaagttgc     6660
   agtcaggctc ttggctaggg ctgcagtcat ctgaaggatt agtaagtcgc taagcccaca     6720
   cttaagctcc acccttgaag ggagaagttt ggaaagaatt tgtgaacata tttttaaatc     6780
   acatatgcta tgaacaggtt taggggaggg agaggaaaga aaactttaac ttttcattag     6840
   catactgcat ctgtagttcc taatgggcat gttgtaaaga tgttaagacg ttttctatgc     6900
   attataggta tatagtagta ttttgttttg ttctgttttt ttgtttggta tgtagtagtt     6960
   tttaatcaaa ccttaacgta tttgatatta agatttattg agtttcttgt tgagatgtag     7020
   aaataaaggg gatccatttc atttatcca                                      7049
<210> 52
   <211> 1013
   <212> DNA
   <213> Homo sapiens
<400> 52
   acgctggtgg gcctgttgtg gagtacgctt tggactgaga agcatcgagg ctataggacg      60
   cagctgttgc catgacggcc caggggggcc tggtggctaa ccgaggccgg cgcttcaagt     120
   gggccattga gctaagcggg cctggaggag gcagcagggg tcgaagtgac cggggcagtg     180
   gccagggaga ctcgctctac ccagtcggtt acttggacaa gcaagtgcct gataccagcg     240
   tgcaagagac agaccggatc ctggtggaga agcgctgctg ggacatcgcc ttgggtcccc     300
   tcaaacagat tcccatgaat ctcttcatca tgtacatggc aggcaatact atctccatct     360
   tccctactat gatggtgtgt atgatggcct ggcgacccat tcaggcactt atggccattt     420
   cagccacttt caagatgtta gaaagttcaa gccagaagtt tcttcagggt ttggtctatc     480
   tcattgggaa cctgatgggt ttggcattgg ctgtttacaa gtgccagtcc atgggactgt     540
   tacctacaca tgcatcggat tggttagcct tcattgagcc ccctgagaga atggagttca     600
   gtggtggagg actgcttttg tgaacatgag aaagcagcgc ctggtcccta tgtatttggg     660
   tcttatttac atccttcttt aagcccagtg gctcctcagc atactcttaa actaatcact     720
   tatgttaaaa agaaccaaaa gactcttttc tccatggtgg ggtgacaggt cctagaagga     780
   caatgtgcat attacgacaa acacaaagaa actataccat aacccaaggc tgaaaataat     840
   gtagaaaact ttatttttgt ttccagtaca gagcaaaaca acaacaaaaa aacataacta     900
   tgtaaacaag agaataactg ctgctaaatc aagaactgtt gcagcatctc ctttcaataa     960
   attaaatggt tgagaacaat gcataaaaaa aaaaaaaaaa aaaaaaaaaa aaa           1013
<210> 53
   <211> 2622
   <212> DNA
   <213> Homo sapiens
<400> 53
   ggtgcggggc ggaagtgggc ggctgcggga cgcgcgcgga gtcgcgcggc gggcgggacc      60
   tggccgagct ggagggcgcc ggggagcggg gctcgggcgg tccccgaggc ccggcggagc     120
   gggcttctgg ggtgtctgcg gcggcgccgg gggaacgggc tggggatggg gcgcctagcc     180
   gggcggtggc cggggcctcg gccatgttcg cggggctgca ggacctgggc gtggccaacg     240
   gcgaggacct gaaggagacc ctgaccaact gcacggagcc gctcaaggcc atcgagcagt     300
   tccagacaga gaatggtgtg ctgctgccat ctcttcagtc agccctcccc ttcttggacc     360
   tgcacgggac gccgcggctg gagttccacc agtcggtatt cgatgagctg cgggacaagc     420
   tgctggagcg agtgtcagcc atcgcttcgg aggggaaggc tgaggaaagg tacaagaagc     480
   tggaagacct tctggagaag agcttttctc tggtgaagat gccgtccctg cagcccgtgg     540
   tgatgtgcgt catgaagcac ctgcccaagg ttccggagaa aaaactgaag ctggttatgg      600
   ctgacaagga gctgtatcga gcctgcgccg tggaggtgaa gcggcagatc tggcaagaca      660
   accaggccct cttcggggac gaggtttccc cactcctgaa gcagtacatc ctggagaagg      720
   agagcgctct cttcagtaca gagctctctg tcctgcacaa ctttttcagt ccttccccca      780
   agaccaggcg ccagggcgag gtggtgcagc ggctgacgcg gatggtgggg aagaacgtga      840
   agctgtacga catggtgctg cagtttctgc gcacgctctt cctgcgcacg cggaatgtgc      900
   actactgcac gctgcgggct gagctgctca tgtccctgca cgacctggac gtgggtgaaa      960
   tctgcaccgt ggacccgtgc cacaagttca cctggtgcct ggacgcctgc atccgagagc     1020
   ggttcgtgga cagcaagagg gcgcgggagc tgcaggggtt tctcgatggc gtcaagaagg     1080
   gccaggagca ggtgctgggg gacctgtcca tgatcctgtg tgaccccttc gccatcaaca     1140
   cgctggcact gagcacagtc aggcacctgc aggagctggt cggccaggag acactgccca     1200
   gggacagccc cgacctcctg ctgctgctcc ggctgctggc gctgggccag ggagcctggg     1260
   acatgatcga cagccaggtc ttcaaggagc ccaagatgga ggtagagctc atcaccaggt     1320
   tcctcccgat gctcatgtcc ttcctggtgg atgactacac tttcaatgtg gatcagaaac     1380
   ttccggctga ggagaaagcc ccagtctcat atccaaacac acttcccgaa agcttcacta     1440
   agtttctgca ggagcagcgc atggcctgcg aggtggggct gtactacgtc ctgcacatca     1500
   ccaagcagag gaacaagaac gcgctcctcc gcctgctgcc cgggctggtg gagacctttg     1560
   gcgacttggc ctttggcgac atcttcctcc acctgctcac gggcaacctt gcgctgctgg     1620
   ccgacgaatt tgcccttgag gacttctgca gcagcctctt cgatggcttc ttcctcaccg     1680
   cctctccaag gaaggagaac gtgcaccggc acgcgctgcg gctcctcatt cacctgcacc     1740
   ccagggtggc cccgtctaag ctggaggcgt tgcagaaggc cctggagcct acaggccaga     1800
   gcggagaggc agtgaaggag ctttactccc agctcggcga gaagctggaa cagctggatc     1860
   accggaagcc cagcccggca caggctgcgg agacgccggc cctggagctg cccctcccca     1920
   gcgtgcccgc ccctgccccg ctctgagggc cctccagacc tgctcgggtg ctggggccat     1980
   gccgagtcgc ggccctgctc agccggaaga ggctcccgga cctggatgta cagggcagtc     2040
   tctcttcccg gggctatggc tgggcctgtc ctgccgtcat ggccccctgc ttcctgctcc     2100
   ttggagctgg ctcccggacc ttgcccacca tccatgcagt ggctcccagg gcagagcctc     2160
   tccttgtact ttggcagcca tagaaagcgt gctcattttc tgttttcctg tgttaggaaa     2220
   aaaccacctg ttttccaagg ggagagggcg gggcctgagg gtgggggcgg ggcctcttca     2280
   ttggcccagc ttggcgaaag cgaggcacac tgcttactgc cttggggttg tggagatgga     2340
   cccgtgacct cgtggaggcc gtgtgggggc agcagcctgg cctgtgccat ggtgggtgtc     2400
   ctggggcctg tgcggaggga gccacctcac cctgcagccc agtttgcagg tgtggccttg     2460
   tttctccttg cccagcagtg ctgccttcag cggccgtgac ggggccagct ggacacacgg     2520
   tgagattttc tcgtatgtaa ataaaaggca ttttggtaaa cgtggaaaaa aaaaaaaaaa 2580
   aaaaaaaaaa aaaaaaaaaa aaaaaaaaaa aaaaaaaaaa aa 2622
<210> 54
   <211> 2577
   <212> DNA
   <213> Homo sapiens
<400> 54
<210> 55
   <211> 2606
   <212> DNA
   <213> Homo sapiens
<400> 55
   ctttccgccc tctccccgcc tccttttcgg gcgtcccgag gccgctcccc aaccgacaac      60
   caagaccccg caggccacgc agccctggag ccgaggcccc ccgacggcgg aggcgcccgc     120
   gggtccccta cagccaaggt ccctgagtgc cagaggtggt ggtgttgctt atcttctgga     180
   accccatgca gccagatccc aggcctagcg gggctggggc ctgctgccga ttcctgcccc     240
   tgcagtcaca gtgccctgag ggggcagggg acgcggtgat gtacgcctcc actgagtgca     300
   aggcggaggt gacgccctcc cagcatggca accgcacctt cagctacacc ctggaggatc     360
   ataccaagca ggcctttggc atcatgaacg agctgcggct cagccagcag ctgtgtgacg     420
   tcacactgca ggtcaagtac caggatgcac cggccgccca gttcatggcc cacaaggtgg     480
   tgctggcctc atccagccct gtcttcaagg ccatgttcac caacgggctg cgggagcagg     540
   gcatggaggt ggtgtccatt gagggtatcc accccaaggt catggagcgc ctcattgaat     600
   tcgcctacac ggcctccatc tccatgggcg agaagtgtgt cctccacgtc atgaacggtg     660
   ctgtcatgta ccagatcgac agcgttgtcc gtgcctgcag tgacttcctg gtgcagcagc     720
   tggaccccag caatgccatc ggcatcgcca acttcgctga gcagattggc tgtgtggagt     780
   tgcaccagcg tgcccgggag tacatctaca tgcattttgg ggaggtggcc aagcaagagg 840
   agttcttcaa cctgtcccac tgccaactgg tgaccctcat cagccgggac gacctgaacg 900
   tgcgctgcga gtccgaggtc ttccacgcct gcatcaactg ggtcaagtac gactgcgaac 960
   agcgacggtt ctacgtccag gcgctgctgc gggccgtgcg ctgccactcg ttgacgccga 1020
   acttcctgca gatgcagctg cagaagtgcg agatcctgca gtccgactcc cgctgcaagg 1080
   actacctggt caagatcttc gaggagctca ccctgcacaa gcccacgcag gtgatgccct 1140
   gccgggcgcc caaggtgggc cgcctgatct acaccgcggg cggctacttc cgacagtcgc 1200
   tcagctacct ggaggcttac aaccccagtg acggcacctg gctccggttg gcggacctgc 1260
   aggtgccgcg gagcggcctg gccggctgcg tggtgggcgg gctgttgtac gccgtgggcg 1320
   gcaggaacaa ctcgcccgac ggcaacaccg actccagcgc cctggactgt tacaacccca 1380
   tgaccaatca gtggtcgccc tgcgccccca tgagcgtgcc ccgtaaccgc atcggggtgg 1440
   gggtcatcga tggccacatc tatgccgtcg gcggctccca cggctgcatc caccacaaca 1500
   gtgtggagag gtatgagcca gagcgggatg agtggcactt ggtggcccca atgctgacac 1560
   gaaggatcgg ggtgggcgtg gctgtcctca atcgtctcct ttatgccgtg gggggctttg 1620
   acgggacaaa ccgccttaat tcagctgagt gttactaccc agagaggaac gagtggcgaa 1680
   tgatcacagc aatgaacacc atccgaagcg gggcaggcgt ctgcgtcctg cacaactgta 1740
   tctatgctgc tgggggctat gatggtcagg accagctgaa cagcgtggag cgctacgatg 1800
   tggaaacaga gacgtggact ttcgtagccc ccatgaagca ccggcgaagt gccctgggga 1860
   tcactgtcca ccaggggaga atctacgtcc ttggaggcta tgatggtcac acgttcctgg 1920
   acagtgtgga gtgttacgac ccagatacag acacctggag cgaggtgacc cgaatgacat 1980
   cgggccggag tggggtgggc gtggctgtca ccatggagcc ctgccggaag cagattgacc 2040
   agcagaactg tacctgttga ggcacttttg tttcttgggc aaaaatacag tccaatgggg 2100
   agtatcattg tttttgtaca aaaaccggga ctaaaagaaa agacagcact gcaaataacc 2160
   catcttccgg gaagggaggc caggatgcct cagtgttaaa atgacatctc aaaagaagtc 2220
   caaagcggga atcatgtgcc cctcagcgga gccccgggag tgtccaagac agcctggctg 2280
   ggaaaggggg tgtggaaaga gcaggcttcc aggagagagg cccccaaacc ctctggccgg 2340
   gtaataggcc tgggtcccac tcacccatgc cggcagctgt caccatgtga tttattcttg 2400
   gatacctggg agggggccaa tgggggcctc agggggaggc cccctctgga aatgtggttc 2460
   ccagggatgg gcctgtacat agaagccacc ggatggcact tccccaccgg atggacagtt 2520
   attttgttga taagtaaccc tgtaattttc caaggaaaat aaagaacaga ctaactagtg 2580
   tctttcaccc tgaaaaaaaa aaaaaa 2606
<210> 56
   <211> 2277
   <212> DNA
   <213> Homo sapiens
<400> 56
   gggaaatggc ggccgcgggc ttggtcgctg tggcagcggc tgccgagtac tctggcacgg 60
   tagcgtcggg aggtaacctc cctggtgttc actgcggccc aagctccggg gcaggccctg 120
   gttttggccc gggctcgtgg agccgctctc tcgatcgagc cctggaggag gcggcggtca 180
   ctggggtgct gagcctgagc ggccggaaac tgagggagtt tccccgggga gcggccaacc 240
   acgacctgac ggacaccacc cgggcggacc tgtcgcgaaa tcgcctttca gaaattccta 300
   tagaagcatg tcactttgtt tctctggaaa atctcaactt gtaccaaaat tgtattcgtt 360
   atattccaga ggcaatttta aacctacaag ctctaacatt cttaaatatt agtcggaacc 420
   aactgtcaac attgccggta cacttgtgta atttgccatt gaaagtctta attgctagta 480
   ataacaaatt ggtgtcactt ccagaagaaa ttggacacct tagacatttg atggaacttg 540
   atgtgagctg caatgaaatt caaactatac cttcccaaat tggtaacctg gaggccttga 600
   gagaccttaa tgtaagaaga aatcacctag tacatttgcc tgaagagctg gcggagttgc 660
   ctttgatacg gttagacttc tcatgcaata aaattaccac aatccctgtt tgttatcgga 720
   acctcaggca cctacagacg atcaccctag ataacaatcc actacaatca cctcctgcac 780
   agatatgtat aaaaggcaaa gtccacatat ttaaatacct gaacatacaa gcttgtaaga 840
   ttgctccaga tctgccggat tatgatagga gaccgttggg ttttggctcc tgccatgaag 900
   aactgtactc aagtcgccct tatggagccc ttgattcagg cttcaatagt gtggacagtg 960
   gtgataagag atggtcaggg aatgaaccta cagatgaatt ttcagatctg cctcttcgag 1020
   tagcagagat tactaaagaa caaagactac gaagagaaag ccagtaccaa gagaaccgcg 1080
   gcagtttggt agtaacaaac ggcggagtgg aacatgatct ggatcagatt gactacatag 1140
   acagctgcac cgcagaggaa gaggaggccg aggtgagaca gcccaaggga ccagacccag 1200
   acagccttag ttcacagttt atggcgtata ttgaacagcg gcgaatctct catgagggtt 1260
   caccagtaaa gccagtagcc attagggagt ttcaaaaaac agaagatatg agaagatact 1320
   tacatcaaaa cagggttcca gctgagccat cttccctcct gtcactatca gcaagtcaca 1380
   atcagctgtc acacacagac ctggaacttc atcagagaag ggagcagtta gtagagcgca 1440
   ctcggagaga ggctcagctt gctgccctgc agtatgagga ggagaaaata aggaccaagc 1500
   agatccagag agatgctgtc ctggactttg tcaaacaaaa agcatcacaa agtccacaaa 1560
   aacagcaccc gctcctagat ggcgtagatg gtgagtgccc cttcccatcc agaaggtctc 1620
   agcacactga tgatagtgcc ttgtgcatgt cgctgtcagg gttgaatcaa gtgggctgtg 1680
   ctgctaccct gcctcattct tctgccttca cgcctcttaa gagtgatgac agacctaatg 1740
   ctctattaag ttcacctgca acagaaacag ttcatcattc ccctgcatat tcttttcctg 1800
   ctgctatcca gagaaatcag cctcagcgcc ctgaaagctt ccttttccga gcaggtgtca     1860
   gggcagaaac caacaaaggt catgcttcac cccttcctcc atctgctgca cctaccactg     1920
   attctacaga ttccataaca ggacagaatt caagacagag agaagaagag ctggaattaa     1980
   tagaccaact gcgtaaacat attgagtacc ggttgaaagt gtctctacct tgtgatctcg     2040
   gagcagctct aactgacggt gttgttcttt gccatttggc caatcatgtg cgacctcgat     2100
   ctgtcccaag cattcatgtt ccctcaccag ctgtagtaag ttgataatcc taaaaagcct     2160
   tggctattca tcagacattt gttaagcaca gtggacagac atttcttaag cattgtgcta     2220
   gatattaagg atacagatat aaagagtcca gtctctacaa aaaaaaaaaa aaaaaaa        2277
<210> 57
   <211> 4431
   <212> DNA
   <213> Homo sapiens
<400> 57
   caagagcagg cctggagacc cgggagtgcc ttggggagag ggtccccgcc ctgccgggag      60
   cccggccctc ccggtgccgg ggtggacact gttagtgtag tcattgtccc tatggagaaa     120
   ctgaggcatg gagaggctga agtcacacaa gcccgccacg atgactatca tggtggagga     180
   catcatgaag ctgctgtgct ccctttctgg ggagaggaag atgaaggcgg ctgtcaagca     240
   ctctgggaag ggtgccctgg tcacaggggc catggccttc gtcgggggtt tggtgggcgg     300
   cccaccggga ctcgccgttg ggggggctgt cggggggctg ttaggtgcct ggatgacaag     360
   tggacagttt aagccggttc ctcagatcct aatggagctg ccccctgccg agcaacagag     420
   gctctttaac gaagccgcag ccatcatcag gcacctggag tggacggacg ccgtgcagct     480
   gaccgcgctg gtcatgggca gcgaggccct gcagcagcag ctgctggcca tgctggtgaa     540
   ctacgtcacc aaggagctgc gggccgagat ccagtatgat gactaggccg cacctccggg     600
   gaggtggggg gcccctttaa atgactctgt gattctgaag aggtggcttg ggagttggga      660
   gaagcccagc ggatgccccc tggggaatct ccacatcatc agtgtattac tagtaatgtc     720
   ccgctggaga ggccaccgct gtgcagtgtc atgttccaga aattactgat gaagcagcat     780
   gtgttggtgg catgtgcact ggcctgccat gacagccctc tgactggccc cccagtgaag     840
   agtaaaggcc tgcctgccgc aggcttcgga ggcgtctgct gagtcctctc acccgcatgg     900
   gtctggggaa gtgatcacgc tcagccgacg gtctgaccac acttcatcct ccccccgggg 960
   ccttctcatc ttgggagatg actcctcttc agagcacctg ctgcaggact ggatcccacc    1020
   cccctgcagg tcctggggtc tcagggcctt ggagcagccc atgctggaat catgtttacc    1080
   tcctagtgca accgtcccct acccagggac tgtcgaatgg ccccacggag gggacgggcg    1140
   gcctgctgag tgaagccaca aataccgagt ggacttgacc ccggccccca ctaggctgca    1200
   cacctagact cgccctgcca gggcctcgct cttcccatct gaaaagtcct ggtagttctt    1260
   gaggtttact tctcaaatga aatattttta gtaaaaagta caggtatatc tcggagatat     1320
   tgtgggttca gttccagacc acctcggtaa agccaacatc acaataaagc aaggaagcgc     1380
   attgttttag tttcccagtg catctaagtc atgtttactg catattgcag tccactaaat     1440
   gtgcaatagc attatgtcta acaaatgtac aaaccttaat ttaaaaatat ttactgttca     1500
   aaatgctgac acagaaacgc aaagtgagca catgctgttg gaaaatggtg ccaaatagac     1560
   ttgcctgatg ccaggctgct acaaaccttc aatttaaaaa aaaaacacag tattcacaaa     1620
   gcatagtaga atgaggtatg cctgtattgc tctttctgaa gtggtgtgat ataaaccatc     1680
   tctaagaaat gtttctaccc taaagatttc cccagtacag tcagctctcc gtaactgtgg     1740
   tctccacatt tagatccaac cagccttgga taggaaatat ttgaaaaaag aaattgcatt     1800
   ggtactgaac acgtacagac cttttttctt tgccattatt ccctaaacaa tatggtgtag     1860
   catatttaca tagcatttat attgtatttg gtattataag aaatctagag atgatttaaa     1920
   ttatacagga aggtgtgcgt aggttacgtg caaacgctat gccattgccc atcagggact     1980
   tgagcatcct cagatgtcgg tgtctgaggg ttgaggttgc agtcctggaa cccatccccc     2040
   atggatactg aggcatagct gtactgtgtg ttttcacttt gctttcagaa ctacgacttg     2100
   aatgtgatcg attacaataa atgtttttct aaaaagccat tttcccacat attatgtgta     2160
   aaatatctct aatattcaaa gtcttacaaa tggaacaaga gggaaggtac cagatttgtg     2220
   gctttaaaga atgcaagagt aggtcaggtg aggtggctca tgcttgtaat gtcggcactt     2280
   ttggaggcca aagtgggagg atcgcttgag gccaagagtt tgaaactagc ctgggcaata     2340
   tagtgagacc tccatctcta caaaaaatgt tattccttgg tccccaggtc ctggggcagg     2400
   gcagggactt ggggctctcc tctcaggccc tggaggctcg gagggaggca cttctgctga     2460
   ggccctgctt ataggaaagc tcagtatcct ggcacctcca tgtgtgtaag gacgtcaggg     2520
   tgaattataa cccatggtga ttaaggaagg gccgagcctt tctcttgcag agcgattgct     2580
   cttctgctgg cctcgtctag cacaaatcag gtgccctagc acaggccaca ctcctggagt     2640
   taatgaagcc actttaatca tttatatgcc acttaagttg atgggggaca gggcaggggt     2700
   gggaaggagg ggttgtctgt ctgtgaaaac tgagtgtggc ttttcttgtt gaactgatca     2760
   ttcctgctct tcctgcaaat aagtcctgca tacggaccct ggaactaaaa atggaaaatc     2820
   agagcatgcc ccctcccaat tttgtatagc tttagtgggc tctaaagttg cccgttttta     2880
   gtgtgaagga aaaaacgttg atttgcagat atcgtgagaa tgaaacctca acaaagatgt     2940
   ttggttcagt gcttcaaagt tgggggacac tttttccatg ttgaacaaat gccaacttct     3000
   ccggttgctt acagcaaatc cttctggaac aatcggggct gaaattgagt tgcctttgtt     3060
   aggcgattgg gccccattca ttcttactcg tgcatcaggt cctggtctgt gtcaggccca     3120
   ggggacacag gtggtcccag ctcagaggcc cagtgtccac tgcagcccct cccacagcct     3180
   gcccacccct actgcaggga aaaatgccca gggaggagat ggtccaactc ctgatcagtt     3240
   ttgtgtccga tggagcaggc cttgctgagt gaagacactg gaactagctg ggtcctgggg 3300
   tgacttggag gctttgggcc taaaagggca gcctgaacct ggagtcttat ctcccccagg 3360
   agccgaaagc acttttcttg atttccccca ggaaatcaag cgctgcttct cagctcctgt 3420
   ggttttagta tttatatatc tgtatcttct ttgtagaaat ttatttattt ttgaataagt 3480
   aatacctgcc tggtacaaaa tttaaaaggt acgggagggc gcaagctgca agggaaggcc 3540
   tgctcctatg ccgaccccag aggcagccac tgttaccaat ttcatgtgta ttcctttaac 3600
   tctgttttaa agtaagtctc tgaaaactgt tcatttcctt ttgtcagtat ttgttgctga 3660
   aaacctagaa aaacccagaa aatataatga aataaaaact acaaatttca caacccagaa 3720
   aactgctggt aacagatttg gtgtctttcc tgcaattgtt ttaatgctat gtaggtacac 3780
   atatattttt ttcttttttt cttttttttt tttttgagat ggagtctcgc actgtcgccc 3840
   gggctggagt gcagtagcgc gatcttggtt cactgcaagc tctgcctcct gggttcaagc 3900
   cattctcctg cctcagcctc ccgagtagct ttgactacag tcgcccgcca ccatgcccgg 3960
   ctaattttta tttttttatt ttttttttat ttttagtaga gacggggttt caccatgtta 4020
   gccaggatgg tctcaatctc cttacctcgt gatctgccca cctcagcctc ccaaagtgct 4080
   gggattacag gcgtgagcca ctgcgcccgg cctatgtatg tatatttcta acgtacttgc 4140
   cttcttatcc tatatgcccc tttgaacacc tgtatcaatg aatgttaaat aatatttttg 4200
   atgtaattta aaattccttg taaatatttg taatggctgc ataatattcc actgtgccta 4260
   accatgtgcc tggtgtgaat tccatttgtg gatttttact actgtaatgc tgaagtatta 4320
   cagtaatact gtgttaatat tctctttatt gaattctggt atatagtctt tgggattcat 4380
   tcctcaaagt gaaattgctg gatttaaaag taaaaaaaaa aaaaaaaaaa a 4431
<210> 58
   <211> 4702
   <212> DNA
   <213> Homo sapiens
<400> 58
   gtgatcatgc gtgcgcgtgg gagaaaggca gggctgggcc tgcgggagcg cggccttgcg 60
   gttcccagga ctcttctccg ggcgcctcgt ctccttacgc cacccgcaac ccaagccagg 120
   gccccggtga cagcggcggg gtgggccagg accggggagg gggtgcccag cagcgacccc 180
   cggctccccc taaggccggg cgcagctcgg agccaggagc tggcccggcg cgtggcttcc 240
   cggaaggccc ggcgcagccg gaaggtggga cggagggcgg ggccagcgcc ggggccgcca 300
   ccaaggcctg cgcgaccctc cgcggggctg gggagctggg cggggagccc ggggcctgcc 360
   aggcccgggc tgcagccgcg tctgatcgcc gagcgcgccg cgtagacctc cgctccccca 420
   ggcccgccac gatgactatc atggtggagg acatcatgaa gctgctgtgc tccctttctg 480
   gggagaggaa gatgaaggcg gctgtcaagc actctgggaa gggtgccctg gtcacagggg 540
   ccatggcctt cgtcgggggt ttggtgggcg gcccaccggg actcgccgtt gggggggctg 600
   tcggggggct gttaggtgcc tggatgacaa gtggacagtt taagccggtt cctcagatcc 660
   taatggagct gccccctgcc gagcaacaga ggctctttaa cgaagccgca gccatcatca 720
   ggcacctgga gtggacggac gccgtgcagc tgaccgcgct ggtcatgggc agcgaggccc 780
   tgcagcagca gctgctggcc atgctggtga actacgtcac caaggagctg cgggccgaga 840
   tccagtatga tgactaggcc gcacctccgg ggaggtgggg ggccccttta aatgactctg 900
   tgattctgaa gaggtggctt gggagttggg agaagcccag cggatgcccc ctggggaatc 960
   tccacatcat cagtgtatta ctagtaatgt cccgctggag aggccaccgc tgtgcagtgt 1020
   catgttccag aaattactga tgaagcagca tgtgttggtg gcatgtgcac tggcctgcca 1080
   tgacagccct ctgactggcc ccccagtgaa gagtaaaggc ctgcctgccg caggcttcgg 1140
   aggcgtctgc tgagtcctct cacccgcatg ggtctgggga agtgatcacg ctcagccgac 1200
   ggtctgacca cacttcatcc tccccccggg gccttctcat cttgggagat gactcctctt 1260
   cagagcacct gctgcaggac tggatcccac ccccctgcag gtcctggggt ctcagggcct 1320
   tggagcagcc catgctggaa tcatgtttac ctcctagtgc aaccgtcccc tacccaggga 1380
   ctgtcgaatg gccccacgga ggggacgggc ggcctgctga gtgaagccac aaataccgag 1440
   tggacttgac ccggcccccc actaggctgc acacctagac tcgccctgcc agggcctcgc 1500
   tcttcccatc tgaaaagtcc tggtagttct tgaggtttac ttctcaaatg aaatattttt 1560
   agtaaaaagt acaggtatat ctcggagata ttgtgggttc agttccagac cacctcggta 1620
   aagccaacat cacaataaag caaggaagcg cattgtttta gtttcccagt gcatctaagt 1680
   catgtttact gcatattgca gtccactaaa tgtgcaatag cattatgtct aacaaatgta 1740
   caaaccttaa tttaaaaata tttactgttc aaaatgctga cacagaaacg caaagtgagc 1800
   acatgctgtt ggaaaatggt gccaaataga cttgcctgat gccaggctgc tacaaacctt 1860
   caatttaaaa aaaaaacaca gtattcacaa agcatagtag aatgaggtat gcctgtattg 1920
   ctctttctga agtggtgtga tataaaccat ctctaagaaa tgtttctacc ctaaagattt 1980
   ccccagtaca gtcagctctc cgtaactgtg gtctccacat ttagatccaa ccagccttgg 2040
   ataggaaata tttgaaaaaa gaaattgcat tggtactgaa cacgtacaga cctttttttc 2100
   ttgccattat tccctaaaca atatggtgta gcatatttac atagcattta tattgtattt 2160
   ggtattataa gaaatctaga gatgatttaa attatacagg aaggtgtgcg taggttacgt 2220
   gcaaacgcta tgccattgcc catcagggac ttgagcatcc tcagatgtcg gtgtctgagg 2280
   gttgaggttg cagtcctgga acccatcccc catggatact gaggcatagc tgtactgtgt 2340
   gttttcactt tgctttcaga actacgactt gaatgtgatc gattacaata aatgtttttc 2400
   taaaaagcca ttttcccaca tattatgtgt aaaatatctc taatattcaa agtcttacaa 2460
   atggaacaag agggaaggta ccagatttgt ggctttaaag aatgcaagag taggtcaggt    2520
   gaggtggctc atgcttgtaa tgtcggcact tttggaggcc aaagtgggag gatcgcttga    2580
   ggccaagagt ttgaaactag cctgggcaat atagtgagac ctccatctct acaaaaaatg    2640
   ttattccttg gtccccaggt cctggggcag ggcagggact tggggctctc ctctcaggcc    2700
   ctggaggctc ggagggaggc acttctgctg aggccctgct tataggaaag ctcagtatcc    2760
   tggcacctcc atgtgtgtaa ggacgtcagg gtgaattata acccatggtg attaaggaag    2820
   ggccgagcct ttctcttgca gagcgattgc tcttctgctg gcctcgtcta gcacaaatca    2880
   ggtgccctag cacaggccac actcctggag ttaatgaagc cactttaatc atttatatgc    2940
   cacttaagtt gatgggggac agggcagggg tgggaaggag gggttgtctg tctgtgaaaa    3000
   ctgagtgtgg cttttcttgt tgaactgatc attcctgctc ttcctgcaaa taagtcctgc    3060
   atacggaccc tggaactaaa aatggaaaat cagagcatgc cccctcccaa ttttgtatag    3120
   ctttagtggg ctctaaagtt gcccgttttt agtgtgaagg aaaaaacgtt gatttgcaga    3180
   tatcgtgaga atgaaacctc aacaaagatg tttggttcag tgcttcaaag ttgggggaca    3240
   ctttttccat gttgaacaaa tgccaacttc tccggttgct tacagcaaat ccttctggaa    3300
   caatcggggc tgaaattgag ttgcctttgt taggcgattg ggccccattc attcttactc    3360
   gtgcatcagg tcctggtctg tgtcaggccc aggggacaca ggtggtccca gctcagaggc    3420
   ccagtgtcca ctgcagcccc tcccacagcc tgcccacccc tactgcaggg aaaaatgccc    3480
   agggaggaga tggtccaact cctgatcagt tttgtgtccg atggagcagg ccttgctgag    3540
   tgaagacact ggaactagct gggtcctggg gtgacttgga ggctttgggc ctaaaagggc    3600
   agcctgaacc tggagtctta tctcccccag gagccgaaag cacttttctt gatttccccc    3660
   aggaaatcaa gcgctgcttc tcagctcctg tggttttagt atttatatat ctgtatcttc    3720
   tttgtagaaa tttatttatt tttgaataag taatacctgc ctggtacaaa atttaaaagg    3780
   tacgggaggg cgcaagctgc aagggaaggc ctgctcctat gccgacccca gaggcagcca    3840
   ctgttaccaa tttcatgtgt attcctttaa ctctgtttta aagtaagtct ctgaaaactg    3900
   ttcatttcct tttgtcagta tttgttgctg aaaacctaga aaaacccaga aaatataatg    3960
   aaataaaaac tacaaatttc acaacccaga aaactgctgg taacagattt ggtgtctttc    4020
   ctgcaattgt tttaatgcta tgtaggtaca catatatttt tttctttttt tctttttttt    4080
   ttttttgaga tggagtctcg cactgtcgcc cgggctggag tgcagtagcg cgatcttggt    4140
   tcactgcaag ctctgcctcc tgggttcaag ccattctcct gcctcagcct cccgagtagc    4200
   tttgactaca gtcgcccgcc accatgcccg gctaattttt atttttttat ttttttttta    4260
   tttttagtag agacggggtt tcaccatgtt agccaggatg gtctcaatct ccttacctcg    4320
   tgatctgccc acctcagcct cccaaagtgc tgggattaca ggcgtgagcc actgcgcccg    4380
   gcctatgtat gtatatttct aacgtacttg ccttcttatc ctatatgccc ctttgaacac    4440
   ctgtatcaat gaatgttaaa taatattttt gatgtaattt aaaattcctt gtaaatattt 4500
   gtaatggctg cataatattc cactgtgcct aaccatgtgc ctggtgtgaa ttccatttgt 4560
   ggatttttac tactgtaatg ctgaagtatt acagtaatac tgtgttaata ttctctttat 4620
   tgaattctgg tatatagtct ttgggattca ttcctcaaag tgaaattgct ggatttaaaa 4680
   gtaaaaaaaa aaaaaaaaaa aa 4702
<210> 59
   <211> 1537
   <212> DNA
   <213> Homo sapiens
<400> 59
   cggacccgga agtgcttggc cacagtcgca gccccggcgc cccgaagcgg gaaaaaggct 60
   gggtgccgcc gtcccccagc tgcgcaaccc taggaactct cgggttcaca gtctctcttt 120
   ttctaggaac ttggctgtgt tgtcctgcct cagagacaaa ttcatctatt gtaggcctag 180
   cccctgcctt tgaaaacaag gaaaggttgg tagaacatca acacagcatg gaatttccag 240
   ggaggtctca tttcaaaact tcataaagaa caagaaccac ctggacttct gtgagggcga 300
   tgattaaact ggcctgagtt tgaatgaaag gataatgtat gctcaacctg tgactaacac 360
   caaggaggtc aagtggcaga aggtcttgta tgagcgacag ccctttcctg ataactatgt 420
   ggaccggcga ttcctggaag agctccggaa aaacatccat gctcggaaat accaatattg 480
   ggctgtggta tttgagtcca gtgtggtgat ccagcagctg tgcagtgttt gtgtttttgt 540
   ggttatctgg tggtatatgg atgagggtct tctggccccc cattggcttt tagggactgg 600
   tctggcttct tcactgattg ggtatgtttt gtttgatctc attgatggag gtgaagggcg 660
   gaagaagagt gggcagaccc ggtgggctga cctgaagagt gccctagtct tcattacttt 720
   cacttatggg ttttcaccag tgctgaagac ccttacagag tctgtcagca ctgacaccat 780
   ctatgccatg tcagtcttca tgctgttagg ccatctcatc ttttttgact atggtgccaa 840
   tgctgccatt gtatccagca cactatcctt gaacatggcc atctttgctt ctgtatgctt 900
   ggcatcacgt cttccccggt ccctgcatgc cttcatcatg gtgacatttg ccattcagat 960
   ttttgccctg tggcccatgt tgcagaagaa actaaaggca tgtactcccc ggagctatgt 1020
   gggggtcaca ctgctttttg cattttcagc cgtgggaggc ctactgtcca ttagtgctgt 1080
   gggagccgta ctctttgccc ttctgctgat gtctatctca tgtctgtgtc cattctacct 1140
   cattcgcttg cagcttttta aagaaaacat tcatgggcct tgggatgaag ctgaaatcaa 1200
   ggaagacttg tccaggttcc tcagttaaat taggacatcc attacattat taaagcaagc 1260
   tgatagatta gcctcctaac tagtatagaa cttaaagaca gagttccatt ctggaagcag 1320
   catgtcattg tggtaagaga atagagatca aaaccaaaaa aaatgaacca aaggcttggg 1380
   tggtgagggt gcttatcctt tctgttattt tgtagatgaa aaaactttct ggggacctct 1440
   tgaattacat gctgtaacat atgaagtgat gtggtttcta ttaaaaaaat aacacatcca 1500
   aaaaaaaaaa aaaaaaaaaa aaaaaaaaaa aaaaaaa 1537
<210> 60
   <211> 1514
   <212> DNA
   <213> Homo sapiens
<400> 60
   cggacccgga agtgcttggc cacagtcgca gccccggcgc cccgaagcgg gaaaaaggct      60
   gggtgccgcc gtcccccagc tgcgcaaccc taggaactct cgggaacttg gctgtgttgt     120
   cctgcctcag agacaaattc atctattgta ggcctagccc ctgcctttga aaacaaggaa     180
   aggttggtag aacatcaaca cagcatggaa tttccaggga ggtctcattt caaaacttca     240
   taaagaacaa gaaccacctg gacttctgtg agggcgatga ttaaactggc ctgagtttga     300
   atgaaaggat aatgtatgct caacctgtga ctaacaccaa ggaggtcaag tggcagaagg     360
   tcttgtatga gcgacagccc tttcctgata actatgtgga ccggcgattc ctggaagagc     420
   tccggaaaaa catccatgct cggaaatacc aatattgggc tgtggtattt gagtccagtg     480
   tggtgatcca gcagctgtgc agtgtttgtg tttttgtggt tatctggtgg tatatggatg     540
   agggtcttct ggccccccat tggcttttag ggactggtct ggcttcttca ctgattgggt     600
   atgttttgtt tgatctcatt gatggaggtg aagggcggaa gaagagtggg cagacccggt     660
   gggctgacct gaagagtgcc ctagtcttca ttactttcac ttatgggttt tcaccagtgc     720
   tgaagaccct tacagagtct gtcagcactg acaccatcta tgccatgtca gtcttcatgc     780
   tgttaggcca tctcatcttt tttgactatg gtgccaatgc tgccattgta tccagcacac     840
   tatccttgaa catggccatc tttgcttctg tatgcttggc atcacgtctt ccccggtccc     900
   tgcatgcctt catcatggtg acatttgcca ttcagatttt tgccctgtgg cccatgttgc     960
   agaagaaact aaaggcatgt actccccgga gctatgtggg ggtcacactg ctttttgcat    1020
   tttcagccgt gggaggccta ctgtccatta gtgctgtggg agccgtactc tttgcccttc    1080
   tgctgatgtc tatctcatgt ctgtgtccat tctacctcat tcgcttgcag ctttttaaag    1140
   aaaacattca tgggccttgg gatgaagctg aaatcaagga agacttgtcc aggttcctca    1200
   gttaaattag gacatccatt acattattaa agcaagctga tagattagcc tcctaactag    1260
   tatagaactt aaagacagag ttccattctg gaagcagcat gtcattgtgg taagagaata    1320
   gagatcaaaa ccaaaaaaaa tgaaccaaag gcttgggtgg tgagggtgct tatcctttct    1380
   gttattttgt agatgaaaaa actttctggg gacctcttga attacatgct gtaacatatg    1440
   aagtgatgtg gtttctatta aaaaaataac acatccaaaa aaaaaaaaaa aaaaaaaaaa    1500
   aaaaaaaaaa aaaa                                                      1514
<210> 61
   <211> 2485
   <212> DNA
   <213> Homo sapiens
<400> 61
   tgccccgttg tgaggtgata aagtgttgcg ctccgggacg ccagcgccgc ggctgccgcc 60
   tctgctgggg tctaggctgt ttctctcgcg ccaccactgg ccgccggccg cagctccagg 120
   tgtcctagcc gcccagcctc gacgccgtcc cgggacccct gtgctctgcg cgaagccctg 180
   gccccggggg ccggggcatg ggccaggggc gcggggtgaa gcggcttccc gcggggccgt 240
   gactgggcgg gcttcagcca tgaagaccct catagccgcc tactccgggg tcctgcgcgg 300
   cgagcgtcag gccgaggctg accggagcca gcgctctcac ggaggacctg cgctgtcgcg 360
   cgaggggtct gggagatggg gcactggatc cagcatcctc tccgccctcc aggacctctt 420
   ctctgtcacc tggctcaata ggtccaaggt ggaaaagcag ctacaggtca tctcagtgct 480
   ccagtgggtc ctgtccttcc ttgtactggg agtggcctgc agtgccatcc tcatgtacat 540
   attctgcact gattgctggc tcatcgctgt gctctacttc acttggctgg tgtttgactg 600
   gaacacaccc aagaaaggtg gcaggaggtc acagtgggtc cgaaactggg ctgtgtggcg 660
   ctactttcga gactactttc ccatccagct ggtgaagaca cacaacctgc tgaccaccag 720
   gaactatatc tttggatacc acccccatgg tatcatgggc ctgggtgcct tctgcaactt 780
   cagcacagag gccacagaag tgagcaagaa gttcccaggc atacggcctt acctggctac 840
   actggcaggc aacttccgaa tgcctgtgtt gagggagtac ctgatgtctg gaggtatctg 900
   ccctgtcagc cgggacacca tagactattt gctttcaaag aatgggagtg gcaatgctat 960
   catcatcgtg gtcgggggtg cggctgagtc tctgagctcc atgcctggca agaatgcagt 1020
   caccctgcgg aaccgcaagg gctttgtgaa actggccctg cgtcatggag ctgacctggt 1080
   tcccatctac tcctttggag agaatgaagt gtacaagcag gtgatcttcg aggagggctc 1140
   ctggggccga tgggtccaga agaagttcca gaaatacatt ggtttcgccc catgcatctt 1200
   ccatggtcga ggcctcttct cctccgacac ctgggggctg gtgccctact ccaagcccat 1260
   caccactgtt gtgggagagc ccatcaccat ccccaagctg gagcacccaa cccagcaaga 1320
   catcgacctg taccacacca tgtacatgga ggccctggtg aagctcttcg acaagcacaa 1380
   gaccaagttc ggcctcccgg agactgaggt cctggaggtg aactgagcca gccttcgggg 1440
   ccaattccct ggaggaacca gctgcaaatc acttttttgc tctgtaaatt tggaagtgtc 1500
   atgggtgtct gtgggttatt taaaagaaat tataacaatt ttgctaaacc attacaatgt 1560
   taggtctttt ttaagaagga aaaagtcagt atttcaagtt ctttcacttc cagcttgccc 1620
   tgttctaggt ggtggctaaa tctgggccta atctgggtgg ctcagctaac ctctcttctt 1680
   cccttcctga agtgacaaag gaaactcagt cttcttgggg aagaaggatt gccattagtg 1740
   acttggacca gttagatgat tcactttttg cccctaggga tgagaggcga aagccacttc 1800
   tcatacaagc ccctttattg ccactacccc acgctcgtct agtcctgaaa ctgcaggacc      1860
   agtttctctg ccaaggggag gagttggaga gcacagttgc cccgttgtgt gagggcagta      1920
   gtaggcatct ggaatgctcc agtttgatct cccttctgcc acccctacct cacccctagt      1980
   cactcatatc ggagcctgga ctggcctcca ggatgaggat gggggtggca atgacaccct      2040
   gcaggggaaa ggactgcccc ccatgcacca ttgcagggag gatgccgcca ccatgagcta      2100
   ggtggagtaa ctggtttttc ttgggtggct gatgacatgg atgcagcaca gactcagcct      2160
   tggcctggag cacatgctta ctggtggcct cagtttacct tccccagatc ctagattctg      2220
   gatgtgagga agagatccct cttcagaagg ggcctggcct tctgagcagc agattagttc      2280
   caaagcaggt ggcccccgaa cccaagcctc acttttctgt gccttcctga gggggttggg      2340
   ccggggagga aacccaaccc tctcctgtgt gttctgttat ctcttgatga gatcattgca      2400
   ccatgtcaga cttttgtata tgccttgaaa ataaatgaaa gtgagaatcc tcaaaaaaaa      2460
   aaaaaaaaaa aaaaaaaaaa aaaaa                                            2485
<210> 62
   <211> 2499
   <212> DNA
   <213> Homo sapiens
<400> 62
   taccacaaat ttactggctt aaaacgacgc aagtctgtag gtcagaagtc tgacacgggt      60
   cttaactggt gacccgagtc agatttggga cacaaagaac agaaaccaag ctgtgcaggt     120
   ttctgacagg cagtccggtt agggagccct acagcaaccc gccggtcctc tctctcaggc     180
   agttgctgcc atggctcatt attccaaccg gttctcctca gcccagtcta tctcagtggc     240
   tccattcata gggtgatgtg cccggcggga cactaaccct aaccaagcag agagacggtc     300
   atgcccgtca cgacctcggc cctcgccccg gccgaggctt ctcctgcagg tcgcgagaat     360
   caggtgcgtc agcggcgtcc gggaacgccg gaagagccag tggagcggct ctgtagtcca     420
   aagtaccccg tcgaccccag cacggccgct ccaccgcctc ctactagacc cagtcctagg     480
   gactgcgcag tcgcagagct ccgtccgagt accggaagcc taggccgcca gcacttccgg     540
   gaagtgactt cgtctccgaa gccgattggt tgttgctttg ctcccgctcg cgtcggtggc     600
   gtttttcctg cagcgcgtgc gtgctgcgct actgagcagc gccatggagg actctgaagc     660
   actgggcttc gaacacatgg gcctcgatcc ccggctcctt caggctgtca ccgatctggg     720
   ctggtcgcga cctacgctga tccaggagaa ggccatccca ctggccctag aagggaagga     780
   cctcctggct cgggcccgca cgggctccgg gaagacggcc gcttatgcta ttccgatgct     840
   gcagctgttg ctccatagga aggcgacagg tccggtggta gaacaggcag tgagaggcct     900
   tgttcttgtt cctaccaagg agctggcacg gcaagcacag tccatgattc agcagctggc     960
   tacctactgt gctcgggatg tccgagtggc caatgtctca gctgctgaag actcagtctc    1020
   tcagagagct gtgctgatgg agaagccaga tgtggtagta gggaccccat ctcgcatatt 1080
   aagccacttg cagcaagaca gcctgaaact tcgtgactcc ctggagcttt tggtggtgga 1140
   cgaagctgac cttctttttt cctttggctt tgaagaagag ctcaagagtc tcctctgtca 1200
   cttgccccgg atttaccagg cttttctcat gtcagctact tttaacgagg acgtacaagc      1260
   actcaaggag ctgatattac ataacccggt tacccttaag ttacaggagt cccagctgcc      1320
   tgggccagac cagttacagc agtttcaggt ggtctgtgag actgaggaag acaaattcct 1380
   cctgctgtat gccctgctca agctgtcatt gattcggggc aagtctctgc tctttgtcaa 1440
   cactctagaa cggagttacc ggctacgcct gttcttggaa cagttcagca tccccacctg 1500
   tgtgctcaat ggagagcttc cactgcgctc caggtgccac atcatctcac agttcaacca 1560
   aggcttctac gactgtgtca tagcaactga tgctgaagtc ctgggggccc cagtcaaggg 1620
   caagcgtcgg ggccgagggc ccaaagggga caaggcctct gatccggaag caggtgtggc 1680
   ccggggcata gacttccacc atgtgtctgc tgtgctcaac tttgatcttc ccccaacccc 1740
   tgaggcctac atccatcgag ctggcaggac agcacgcgct aacaacccag gcatagtctt 1800
   aacctttgtg cttcccacgg agcagttcca cttaggcaag attgaggagc ttctcagtgg 1860
   agagaacagg ggccccattc tgctccccta ccagttccgg atggaggaga tcgagggctt 1920
   ccgctatcgc tgcagggatg ccatgcgctc agtgactaag caggccattc gggaggcaag 1980
   attgaaggag atcaaggaag agcttctgca ttctgagaag cttaagacat actttgaaga 2040
   caaccctagg gacctccagc tgctgcggca tgacctacct ttgcaccccg cagtggtgaa 2100
   gccccacctg ggccatgttc ctgactacct ggttcctcct gctctccgtg gcctggtgcg 2160
   ccctcacaag aagcggaaga agctgtcttc ctcttgtagg aaggccaaga gagcaaagtc 2220
   ccagaaccca ctgcgcagct tcaagcacaa aggaaagaaa ttcagaccca cagccaagcc 2280
   ctcctgaggt tgttgggcct ctctggagct gagcacattg tggagcacag gcttacaccc 2340
   ttcgtggaca ggcgaggctc tggtgcttac tgcacagcct gaacagacag ttctggggcc 2400
   ggcagtgctg ggccctttag ctccttggca cttccaagct ggcatcttgc cccttgacaa 2460
   cagaataaaa attttagctg ccccaaaaaa aaaaaaaaa 2499
<210> 63
   <211> 2865
   <212> DNA
   <213> Homo sapiens
<400> 63
   gtcgccggaa gtggggcggg actctattgt ggcggtgagg aacaggaagc cctgaagggt 60
   caaaagaaat acaaaagcaa aggctatttt cttttttttt ttctttcttt cattcattcc 120
   ttcctctgtt tctttctttc ttcctttcat ttttttttct tttttaagag cgagcggctc 180
   tgcggtggcg gtttggggtg ggcgccgccg aggtgaggtc gtctcgcctc ccgcgcgccg 240
   gtagattggt tgtttcatta tggatggagg ggatgatggt aaccttatta tcaaaaagag      300
   gtttgtgtct gaggcagaac tagatgaacg gcgcaaaagg aggcaagaag aatgggagaa      360
   agttcgaaaa cctgaagatc cagaagaatg tccagaggag gtttatgacc ctcgatctct      420
   atatgaaagg ctacaggaac agaaggacag gaagcagcag gagtacgagg aacagttcaa      480
   attcaaaaac atggtaagag gcttagatga agatgagacc aacttccttg atgaggtttc      540
   tcgacagcag gaactaatag aaaagcaacg aagagaagaa gaactgaaag aactgaagga      600
   atacagaaat aacctcaaga aggttggaat ttctcaagag aacaagaagg aagtggaaaa      660
   gaaactgact gtgaagccta tagaaaccaa gaacaagttc tcccaggcga agctgttggc      720
   aggagctgtg aagcataaga gctcagagag tggcaacagt gtgaaaagac tgaaaccgga      780
   ccctgagcca gatgacaaga atcaagagcc ctcatcctgc aagtctctcg gaaacacctc      840
   cctgagtggc ccctccatcc actgcccctc tgctgcagta tgtatcggca tcctcccagg        900
   cctgggtgcc tactctggga gcagcgactc cgagtccagc tcagacagcg aaggcaccat      960
   caatgccacc ggaaagattg tctcctccat cttccgaacc aacaccttcc tcgaggcccc     1020
   ctagtttctc cgtccctaca cagggagctc ctccccaagg gtagatcgga ccgttcatgc     1080
   tgcctatagg cattatgtcc ctcaaaaaaa aactcctttg cctgcatcct gtgtacaaca     1140
   tgacattttt aaccaatcca atctaaaaat gtgccagaat ccacctgtgg cccgaatcgt     1200
   gtttggttcc tctttctact ccactgcaga tgaccaaacc tgtcccgctg ccactttcct     1260
   cactgatatt gggaggaggg caaggcccag ccgaagttcc actaaaaatg ccccaggaga     1320
   ataggcaccg gctggcttgc caaagggttt gggttttatt gctttctgtt ttttcttttc     1380
   ccgacagcac aaagaagtaa gggcagttat tggacaggtg ttatttaaac attctattgt     1440
   aaatgaatgt gttgtttggt tctactgcat tgtggagcat gcgggggaag agaactgacc     1500
   caggtagtga aatggagccc ttccctggaa ctaaccagtc cttgatgttg tgtgactaag     1560
   taaagatgat aaaccccatc tgctgggggt gtcacttcac actcggcatg cattgtgaaa     1620
   gctttccata cccttggcca ttccctctct cctctctctc caaccccatt tatgcaggaa     1680
   gggactgcta acaagaacgc ttccatctca aacctttctt ctgcctggga aattatttta    1740
   tgtttgtttt tgaaataaag gatttagttt aagattctaa attttagaga aacaaacgta     1800
   ggccttgttt actaatagcc agacatcaga actgcaggta ggtatgttaa tgagatgact     1860
   tatttctggc agctcctgga atcctaatat tgtaaatgag tgggacacac ttgcatattg     1920
   tgaccattct attgaggccc ttctctgttt aatgcatatt atacttgtgc ttttaactgt     1980
   ggaatctatt tctaacctaa aggtgctgcc ctagtacttt tctttgctgc ctctgctgct     2040
   ctttttcctt tccaaacagc aactctgagg ccatgagcag ccaaaaacta gaggtactgc     2100
   tccacctcgt ctcataaagg gaaacgggct catcccttgg attctggagg agggagaggg     2160
   agatggtgtg gaggcctcga ggacagagat agacatgagc tttgacaaca atctgtaggc 2220
   tctcctgctt tagaataagc atgtaccatt ctttatccat tccccttatt cctacatcaa 2280
   ttgtttttac tttcttgggt gtgagactga gtgagacaca cacaaaatgt gttgacactg 2340
   tgatgccggc aggcagagca gctactgact ttgaacatgg gcagagaggc ccctggatct 2400
   catccagccc actccttttc cccttccagt acagtgacac tctggtgccc attggcagat 2460
   ggcgacttcc ctgcacccat aactgatgct ttgtgaattc ttcctccttt tcagaactac 2520
   tctgtgctaa ttgttctgcc agtatgggcg catcagctcc atcctgacaa acaagacatt 2580
   taggtaaaac tttgtaggca ccttctgctt ctctgcttca ttgttcctgt gatagtcctg 2640
   ttgttattac agcatgtacc caaaacagcc tcacattgtt acaggaggca ggccaggaca 2700
   tcaaagtcat catctttatg tggcatgact cttaagaggc cattactgta tctcatggcc 2760
   tcttgatgtg gaaagaagtt gacagagggt tgcagggttt aaaaacatcc attaacatga 2820
   aagctaataa acctgtcaga gaacaagaaa aaaaaaaaaa aaaaa 2865
<210> 64
   <211> 4353
   <212> DNA
   <213> Homo sapiens
<400> 64
   ttctctcacg aagccccgcc cgcggagagg ttccatattg ggtaaaatct cggctctcgg      60
   agagtcccgg gagctgttct cgcgagagta ctgcgggagg ctcccgtttg ctggctcttg     120
   gaaccgcgac cactggagcc ttagcgggcg cagcagctgg aacgggagta ctgcgacgca     180
   gcccggagtc ggccttgtag gggcgaaggt gcagggagat cgcggcgggc gcagtcttga      240
   gcgccggagc gcgtccctgc ccttagcggg gcttgcccca gtcgcagggg cacatccagc     300
   cgctgcggct gacagcagcc gcgcgcgcgg gagtctgcgg ggtcgcggca gccgcacctg     360
   cgcgggcgac cagcgcaagg tccccgcccg gctgggcggg cagcaagggc cggggagagg     420
   gtgcgggtgc aggcgggggc cccacagggc caccttcttg cccggcggct gccgctggaa     480
   aatgtctcag gagaggccca cgttctaccg gcaggagctg aacaagacaa tctgggaggt     540
   gcccgagcgt taccagaacc tgtctccagt gggctctggc gcctatggct ctgtgtgtgc     600
   tgcttttgac acaaaaacgg ggttacgtgt ggcagtgaag aagctctcca gaccatttca     660
   gtccatcatt catgcgaaaa gaacctacag agaactgcgg ttacttaaac atatgaaaca     720
   tgaaaatgtg attggtctgt tggacgtttt tacacctgca aggtctctgg aggaattcaa     780
   tgatgtgtat ctggtgaccc atctcatggg ggcagatctg aacaacattg tgaaatgtca     840
   gaagcttaca gatgaccatg ttcagttcct tatctaccaa attctccgag gtctaaagta     900
   tatacattca gctgacataa ttcacaggga cctaaaacct agtaatctag ctgtgaatga     960
   agactgtgag ctgaagattc tggattttgg actggctcgg cacacagatg atgaaatgac    1020
   aggctacgtg gccactaggt ggtacagggc tcctgagatc atgctgaact ggatgcatta    1080
   caaccagaca gttgatattt ggtcagtggg atgcataatg gccgagctgt tgactggaag    1140
   aacattgttt cctggtacag accatattaa ccagcttcag cagattatgc gtctgacagg    1200
   aacacccccc gcttatctca ttaacaggat gccaagccat gaggcaagaa actatattca    1260
   gtctttgact cagatgccga agatgaactt tgcgaatgta tttattggtg ccaatcccct    1320
   ggctgtcgac ttgctggaga agatgcttgt attggactca gataagagaa ttacagcggc    1380
   ccaagccctt gcacatgcct actttgctca gtaccacgat cctgatgatg aaccagtggc    1440
   cgatccttat gatcagtcct ttgaaagcag ggacctcctt atagatgagt ggaaaagcct    1500
   gacctatgat gaagtcatca gctttgtgcc accacccctt gaccaagaag agatggagtc    1560
   ctgagcacct ggtttctgtt ctgttgatcc cacttcactg tgaggggaag gccttttcac    1620
   gggaactctc caaatattat tcaagtgcct cttgttgcag agatttcctc catggtggaa    1680
   gggggtgtgc gtgcgtgtgc gtgcgtgtta gtgtgtgtgc atgtgtgtgt ctgtctttgt    1740
   gggagggtaa gacaatatga acaaactatg atcacagtga ctttacagga ggttgtggat    1800
   gctccagggc agcctccacc ttgctcttct ttctgagagt tggctcaggc agacaagagc    1860
   tgctgtcctt ttaggaatat gttcaatgca aagtaaaaaa atatgaattg tccccaatcc    1920
   cggtcatgct tttgccactt tggcttctcc tgtgacccca ccttgacggt ggggcgtaga    1980
   cttgacaaca tcccacagtg gcacggagag aaggcccata ccttctggtt gcttcagacc    2040
   tgacaccgtc cctcagtgat acgtacagcc aaaaaggacc aactggcttc tgtgcactag    2100
   cctgtgatta acttgcttag tatggttctc agatcttgac agtatatttg aaactgtaaa    2160
   tatgtttgtg ccttaaaagg agagaagaaa gtgtagatag ttaaaagact gcagctgctg    2220
   aagttctgag ccgggcaagt cgagagggct gttggacagc tgcttgtggg cccggagtaa    2280
   tcaggcagcc ttcataggcg gtcatgtgtg catgtgagca catgcgtata tgtgcgtctc    2340
   tctttctccc tcacccccag gtgttgccat ttctctgctt acccttcacc tttggtgcag    2400
   aggtttcttg aatatctgcc ccagtagtca gaagcaggtt cttgatgtca tgtacttcct    2460
   gtgtactctt tatttctagc agagtgagga tgtgttttgc acgtcttgct atttgagcat    2520
   gcacagctgc ttgtcctgct ctcttcagga ggccctggtg tcaggcaggt ttgccagtga    2580
   agacttcttg ggtagtttag atcccatgtc acctcagctg atattatggc aagtgatatc    2640
   acctctcttc agcccctagt gctattctgt gttgaacaca attgatactt caggtgcttt    2700
   tgatgtgaaa atcatgaaaa gaggaacagg tggatgtata gcatttttat tcatgccatc    2760
   tgttttcaac caactatttt tgaggaatta tcatgggaaa agaccagggc ttttcccagg    2820
   aatatcccaa acttcggaaa caagttattc tcttcactcc caataactaa tgctaagaaa    2880
   tgctgaaaat caaagtaaaa aattaaagcc cataaggcca gaaactcctt ttgctgtctt    2940
   tctctaaata tgattacttt aaaataaaaa agtaacaagg tgtcttttcc actcctatgg    3000
   aaaagggtct tcttggcagc ttaacattga cttcttggtt tggggagaaa taaattttgt 3060
   ttcagaattt tgtatattgt aggaatcctt tgagaatgtg attccttttg atggggagaa 3120
   agggcaaatt attttaatat tttgtatttt caactttata aagataaaat atcctcaggg 3180
   gtggagaagt gtcgttttca taacttgctg aatttcaggc attttgttct acatgaggac 3240
   tcatatattt aagccttttg tgtaataaga aagtataaag tcacttccag tgttggctgt 3300
   gtgacagaat cttgtatttg ggccaaggtg tttccatttc tcaatcagtg cagtgataca 3360
   tgtactccag agggacaggg tggaccccct gagtcaactg gagcaagaag gaaggaggca 3420
   gactgatggc gattccctct cacccgggac tctccccctt tcaaggaaag tgaaccttta 3480
   aagtaaaggc ctcatctcct ttattgcagt tcaaatcctc accatccaca gcaagatgaa 3540
   ttttatcagc catgtttggt tgtaaatgct cgtgtgattt cctacagaaa tactgctctg 3600
   aatattttgt aataaaggtc tttgcacatg tgaccacata cgtgttagga ggctgcatgc 3660
   tctggaagcc tggactctaa gctggagctc ttggaagagc tcttcggttt ctgagcataa 3720
   tgctcccatc tcctgatttc tctgaacaga aaacaaaaga gagaatgagg gaaattgcta 3780
   ttttatttgt attcatgaac ttggctgtaa tcagttatgc cgtataggat gtcagacaat 3840
   accactggtt aaaataaagc ctatttttca aatttagtga gtttctcaag tttattatat 3900
   ttttctcttg tttttattta atgcacaata tggcattata tcaatatcct ttaaactgtg 3960
   acctggcata cttgtctgac agatcttaat actactccta acatttagaa aatgttgata 4020
   aagcttctta gttgtacatt ttttggtgaa gagtatccag gtctttgctg tggatgggta 4080
   aagcaaagag caaatgaacg aagtattaag cattggggcc tgtcttatct acactcgagt 4140
   gtaagagtgg ccgaaatgac agggctcagc agactgtggc ctgagggcca aatctggccc 4200
   accacctgtt tggtgtagcc tgctaagaat ggcttttaca tttttaaatg gttgggaaag 4260
   aaaaaaaaag aagtagtaga ttttgtagca tgtgatgtaa gtaatgtaaa acttaaattc 4320
   cagtatccat aaataaagtt ttatgagaac aga 4353
<210> 65
   <211> 4353
   <212> DNA
   <213> Homo sapiens
<400> 65
   ttctctcacg aagccccgcc cgcggagagg ttccatattg ggtaaaatct cggctctcgg      60
   agagtcccgg gagctgttct cgcgagagta ctgcgggagg ctcccgtttg ctggctcttg     120
   gaaccgcgac cactggagcc ttagcgggcg cagcagctgg aacgggagta ctgcgacgca     180
   gcccggagtc ggccttgtag gggcgaaggt gcagggagat cgcggcgggc gcagtcttga      240
   gcgccggagc gcgtccctgc ccttagcggg gcttgcccca gtcgcagggg cacatccagc     300
   cgctgcggct gacagcagcc gcgcgcgcgg gagtctgcgg ggtcgcggca gccgcacctg     360
   cgcgggcgac cagcgcaagg tccccgcccg gctgggcggg cagcaagggc cggggagagg 420
   gtgcgggtgc aggcgggggc cccacagggc caccttcttg cccggcggct gccgctggaa 480
   aatgtctcag gagaggccca cgttctaccg gcaggagctg aacaagacaa tctgggaggt 540
   gcccgagcgt taccagaacc tgtctccagt gggctctggc gcctatggct ctgtgtgtgc 600
   tgcttttgac acaaaaacgg ggttacgtgt ggcagtgaag aagctctcca gaccatttca 660
   gtccatcatt catgcgaaaa gaacctacag agaactgcgg ttacttaaac atatgaaaca 720
   tgaaaatgtg attggtctgt tggacgtttt tacacctgca aggtctctgg aggaattcaa 780
   tgatgtgtat ctggtgaccc atctcatggg ggcagatctg aacaacattg tgaaatgtca 840
   gaagcttaca gatgaccatg ttcagttcct tatctaccaa attctccgag gtctaaagta 900
   tatacattca gctgacataa ttcacaggga cctaaaacct agtaatctag ctgtgaatga 960
   agactgtgag ctgaagattc tggattttgg actggctcgg cacacagatg atgaaatgac 1020
   aggctacgtg gccactaggt ggtacagggc tcctgagatc atgctgaact ggatgcatta 1080
   caaccagaca gttgatattt ggtcagtggg atgcataatg gccgagctgt tgactggaag 1140
   aacattgttt cctggtacag accatattga tcagttgaag ctcattttaa gactcgttgg 1200
   aaccccaggg gctgagcttt tgaagaaaat ctcctcagag tctgcaagaa actatattca 1260
   gtctttgact cagatgccga agatgaactt tgcgaatgta tttattggtg ccaatcccct 1320
   ggctgtcgac ttgctggaga agatgcttgt attggactca gataagagaa ttacagcggc 1380
   ccaagccctt gcacatgcct actttgctca gtaccacgat cctgatgatg aaccagtggc 1440
   cgatccttat gatcagtcct ttgaaagcag ggacctcctt atagatgagt ggaaaagcct 1500
   gacctatgat gaagtcatca gctttgtgcc accacccctt gaccaagaag agatggagtc 1560
   ctgagcacct ggtttctgtt ctgttgatcc cacttcactg tgaggggaag gccttttcac 1620
   gggaactctc caaatattat tcaagtgcct cttgttgcag agatttcctc catggtggaa 1680
   gggggtgtgc gtgcgtgtgc gtgcgtgtta gtgtgtgtgc atgtgtgtgt ctgtctttgt 1740
   gggagggtaa gacaatatga acaaactatg atcacagtga ctttacagga ggttgtggat 1800
   gctccagggc agcctccacc ttgctcttct ttctgagagt tggctcaggc agacaagagc 1860
   tgctgtcctt ttaggaatat gttcaatgca aagtaaaaaa atatgaattg tccccaatcc 1920
   cggtcatgct tttgccactt tggcttctcc tgtgacccca ccttgacggt ggggcgtaga 1980
   cttgacaaca tcccacagtg gcacggagag aaggcccata ccttctggtt gcttcagacc 2040
   tgacaccgtc cctcagtgat acgtacagcc aaaaaggacc aactggcttc tgtgcactag 2100
   cctgtgatta acttgcttag tatggttctc agatcttgac agtatatttg aaactgtaaa 2160
   tatgtttgtg ccttaaaagg agagaagaaa gtgtagatag ttaaaagact gcagctgctg 2220
   aagttctgag ccgggcaagt cgagagggct gttggacagc tgcttgtggg cccggagtaa 2280
   tcaggcagcc ttcataggcg gtcatgtgtg catgtgagca catgcgtata tgtgcgtctc 2340
   tctttctccc tcacccccag gtgttgccat ttctctgctt acccttcacc tttggtgcag 2400
   aggtttcttg aatatctgcc ccagtagtca gaagcaggtt cttgatgtca tgtacttcct 2460
   gtgtactctt tatttctagc agagtgagga tgtgttttgc acgtcttgct atttgagcat 2520
   gcacagctgc ttgtcctgct ctcttcagga ggccctggtg tcaggcaggt ttgccagtga 2580
   agacttcttg ggtagtttag atcccatgtc acctcagctg atattatggc aagtgatatc 2640
   acctctcttc agcccctagt gctattctgt gttgaacaca attgatactt caggtgcttt 2700
   tgatgtgaaa atcatgaaaa gaggaacagg tggatgtata gcatttttat tcatgccatc 2760
   tgttttcaac caactatttt tgaggaatta tcatgggaaa agaccagggc ttttcccagg 2820
   aatatcccaa acttcggaaa caagttattc tcttcactcc caataactaa tgctaagaaa 2880
   tgctgaaaat caaagtaaaa aattaaagcc cataaggcca gaaactcctt ttgctgtctt 2940
   tctctaaata tgattacttt aaaataaaaa agtaacaagg tgtcttttcc actcctatgg 3000
   aaaagggtct tcttggcagc ttaacattga cttcttggtt tggggagaaa taaattttgt 3060
   ttcagaattt tgtatattgt aggaatcctt tgagaatgtg attccttttg atggggagaa 3120
   agggcaaatt attttaatat tttgtatttt caactttata aagataaaat atcctcaggg 3180
   gtggagaagt gtcgttttca taacttgctg aatttcaggc attttgttct acatgaggac 3240
   tcatatattt aagccttttg tgtaataaga aagtataaag tcacttccag tgttggctgt 3300
   gtgacagaat cttgtatttg ggccaaggtg tttccatttc tcaatcagtg cagtgataca 3360
   tgtactccag agggacaggg tggaccccct gagtcaactg gagcaagaag gaaggaggca 3420
   gactgatggc gattccctct cacccgggac tctccccctt tcaaggaaag tgaaccttta 3480
   aagtaaaggc ctcatctcct ttattgcagt tcaaatcctc accatccaca gcaagatgaa 3540
   ttttatcagc catgtttggt tgtaaatgct cgtgtgattt cctacagaaa tactgctctg 3600
   aatattttgt aataaaggtc tttgcacatg tgaccacata cgtgttagga ggctgcatgc 3660
   tctggaagcc tggactctaa gctggagctc ttggaagagc tcttcggttt ctgagcataa 3720
   tgctcccatc tcctgatttc tctgaacaga aaacaaaaga gagaatgagg gaaattgcta 3780
   ttttatttgt attcatgaac ttggctgtaa tcagttatgc cgtataggat gtcagacaat 3840
   accactggtt aaaataaagc ctatttttca aatttagtga gtttctcaag tttattatat 3900
   ttttctcttg tttttattta atgcacaata tggcattata tcaatatcct ttaaactgtg 3960
   acctggcata cttgtctgac agatcttaat actactccta acatttagaa aatgttgata 4020
   aagcttctta gttgtacatt ttttggtgaa gagtatccag gtctttgctg tggatgggta 4080
   aagcaaagag caaatgaacg aagtattaag cattggggcc tgtcttatct acactcgagt 4140
   gtaagagtgg ccgaaatgac agggctcagc agactgtggc ctgagggcca aatctggccc 4200
   accacctgtt tggtgtagcc tgctaagaat ggcttttaca tttttaaatg gttgggaaag 4260
   aaaaaaaaag aagtagtaga ttttgtagca tgtgatgtaa gtaatgtaaa acttaaattc 4320
   cagtatccat aaataaagtt ttatgagaac aga 4353
<210> 66
   <211> 1431
   <212> DNA
   <213> Homo sapiens
<400> 66
   ttctctcacg aagccccgcc cgcggagagg ttccatattg ggtaaaatct cggctctcgg 60
   agagtcccgg gagctgttct cgcgagagta ctgcgggagg ctcccgtttg ctggctcttg 120
   gaaccgcgac cactggagcc ttagcgggcg cagcagctgg aacgggagta ctgcgacgca 180
   gcccggagtc ggccttgtag gggcgaaggt gcagggagat cgcggcgggc gcagtcttga 240
   gcgccggagc gcgtccctgc ccttagcggg gcttgcccca gtcgcagggg cacatccagc 300
   cgctgcggct gacagcagcc gcgcgcgcgg gagtctgcgg ggtcgcggca gccgcacctg 360
   cgcgggcgac cagcgcaagg tccccgcccg gctgggcggg cagcaagggc cggggagagg 420
   gtgcgggtgc aggcgggggc cccacagggc caccttcttg cccggcggct gccgctggaa 480
   aatgtctcag gagaggccca cgttctaccg gcaggagctg aacaagacaa tctgggaggt 540
   gcccgagcgt taccagaacc tgtctccagt gggctctggc gcctatggct ctgtgtgtgc 600
   tgcttttgac acaaaaacgg ggttacgtgt ggcagtgaag aagctctcca gaccatttca 660
   gtccatcatt catgcgaaaa gaacctacag agaactgcgg ttacttaaac atatgaaaca 720
   tgaaaatgtg attggtctgt tggacgtttt tacacctgca aggtctctgg aggaattcaa 780
   tgatgtgtat ctggtgaccc atctcatggg ggcagatctg aacaacattg tgaaatgtca 840
   gaagcttaca gatgaccatg ttcagttcct tatctaccaa attctccgag gtctaaagta 900
   tatacattca gctgacataa ttcacaggga cctaaaacct agtaatctag ctgtgaatga 960
   agactgtgag ctgaagattc tggattttgg actggctcgg cacacagatg atgaaatgac 1020
   aggctacgtg gccactaggt ggtacagggc tcctgagatc atgctgaact ggatgcatta 1080
   caaccagaca gttgatattt ggtcagtggg atgcataatg gccgagctgt tgactggaag 1140
   aacattgttt cctggtacag accatattga tcagttgaag ctcattttaa gactcgttgg 1200
   aaccccaggg gctgagcttt tgaagaaaat ctcctcagag tctgcaagaa actatattca 1260
   gtctttgact cagatgccga agatgaactt tgcgaatgta tttattggtg ccaatcccct 1320
   gggtaagttg accatatatc ctcacctcat ggatattgaa ttggttatga tataaattgg 1380
   ggatttgaag aagagtttct ccttttgacc aaataaagta ccattagttg a 1431
<210> 67
   <211> 4274
   <212> DNA
   <213> Homo sapiens
<400> 67
   ttctctcacg aagccccgcc cgcggagagg ttccatattg ggtaaaatct cggctctcgg      60
   agagtcccgg gagctgttct cgcgagagta ctgcgggagg ctcccgtttg ctggctcttg     120
   gaaccgcgac cactggagcc ttagcgggcg cagcagctgg aacgggagta ctgcgacgca     180
   gcccggagtc ggccttgtag gggcgaaggt gcagggagat cgcggcgggc gcagtcttga     240
   gcgccggagc gcgtccctgc ccttagcggg gcttgcccca gtcgcagggg cacatccagc     300
   cgctgcggct gacagcagcc gcgcgcgcgg gagtctgcgg ggtcgcggca gccgcacctg     360
   cgcgggcgac cagcgcaagg tccccgcccg gctgggcggg cagcaagggc cggggagagg     420
   gtgcgggtgc aggcgggggc cccacagggc caccttcttg cccggcggct gccgctggaa     480
   aatgtctcag gagaggccca cgttctaccg gcaggagctg aacaagacaa tctgggaggt     540
   gcccgagcgt taccagaacc tgtctccagt gggctctggc gcctatggct ctgtgtgtgc     600
   tgcttttgac acaaaaacgg ggttacgtgt ggcagtgaag aagctctcca gaccatttca     660
   gtccatcatt catgcgaaaa gaacctacag agaactgcgg ttacttaaac atatgaaaca     720
   tgaaaatgtg attggtctgt tggacgtttt tacacctgca aggtctctgg aggaattcaa     780
   tgatgtgtat ctggtgaccc atctcatggg ggcagatctg aacaacattg tgaaatgtca     840
   gaagcttaca gatgaccatg ttcagttcct tatctaccaa attctccgag gtctaaagta     900
   tatacattca gctgacataa ttcacaggga cctaaaacct agtaatctag ctgtgaatga     960
   agactgtgag ctgaagattc tggattttgg actggctcgg cacacagatg atgaaatgac    1020
   aggctacgtg gccactaggt ggtacagggc tcctgagatc atgctgaact ggatgcatta    1080
   caaccagaca gttgatattt ggtcagtggg atgcataatg gccgagctgt tgactggaag    1140
   aacattgttt cctggtacag accatattga tcagttgaag ctcattttaa gactcgttgg    1200
   aaccccaggg gctgagcttt tgaagaaaat ctcctcagag tctctgtcga cttgctggag    1260
   aagatgcttg tattggactc agataagaga attacagcgg cccaagccct tgcacatgcc    1320
   tactttgctc agtaccacga tcctgatgat gaaccagtgg ccgatcctta tgatcagtcc    1380
   tttgaaagca gggacctcct tatagatgag tggaaaagcc tgacctatga tgaagtcatc    1440
   agctttgtgc caccacccct tgaccaagaa gagatggagt cctgagcacc tggtttctgt    1500
   tctgttgatc ccacttcact gtgaggggaa ggccttttca cgggaactct ccaaatatta    1560
   ttcaagtgcc tcttgttgca gagatttcct ccatggtgga agggggtgtg cgtgcgtgtg    1620
   cgtgcgtgtt agtgtgtgtg catgtgtgtg tctgtctttg tgggagggta agacaatatg    1680
   aacaaactat gatcacagtg actttacagg aggttgtgga tgctccaggg cagcctccac    1740
   cttgctcttc tttctgagag ttggctcagg cagacaagag ctgctgtcct tttaggaata    1800
   tgttcaatgc aaagtaaaaa aatatgaatt gtccccaatc ccggtcatgc ttttgccact    1860
   ttggcttctc ctgtgacccc accttgacgg tggggcgtag acttgacaac atcccacagt    1920
   ggcacggaga gaaggcccat accttctggt tgcttcagac ctgacaccgt ccctcagtga 1980
   tacgtacagc caaaaaggac caactggctt ctgtgcacta gcctgtgatt aacttgctta 2040
   gtatggttct cagatcttga cagtatattt gaaactgtaa atatgtttgt gccttaaaag 2100
   gagagaagaa agtgtagata gttaaaagac tgcagctgct gaagttctga gccgggcaag 2160
   tcgagagggc tgttggacag ctgcttgtgg gcccggagta atcaggcagc cttcataggc 2220
   ggtcatgtgt gcatgtgagc acatgcgtat atgtgcgtct ctctttctcc ctcaccccca 2280
   ggtgttgcca tttctctgct tacccttcac ctttggtgca gaggtttctt gaatatctgc 2340
   cccagtagtc agaagcaggt tcttgatgtc atgtacttcc tgtgtactct ttatttctag 2400
   cagagtgagg atgtgttttg cacgtcttgc tatttgagca tgcacagctg cttgtcctgc 2460
   tctcttcagg aggccctggt gtcaggcagg tttgccagtg aagacttctt gggtagttta 2520
   gatcccatgt cacctcagct gatattatgg caagtgatat cacctctctt cagcccctag 2580
   tgctattctg tgttgaacac aattgatact tcaggtgctt ttgatgtgaa aatcatgaaa 2640
   agaggaacag gtggatgtat agcattttta ttcatgccat ctgttttcaa ccaactattt 2700
   ttgaggaatt atcatgggaa aagaccaggg cttttcccag gaatatccca aacttcggaa 2760
   acaagttatt ctcttcactc ccaataacta atgctaagaa atgctgaaaa tcaaagtaaa 2820
   aaattaaagc ccataaggcc agaaactcct tttgctgtct ttctctaaat atgattactt 2880
   taaaataaaa aagtaacaag gtgtcttttc cactcctatg gaaaagggtc ttcttggcag 2940
   cttaacattg acttcttggt ttggggagaa ataaattttg tttcagaatt ttgtatattg 3000
   taggaatcct ttgagaatgt gattcctttt gatggggaga aagggcaaat tattttaata 3060
   ttttgtattt tcaactttat aaagataaaa tatcctcagg ggtggagaag tgtcgttttc 3120
   ataacttgct gaatttcagg cattttgttc tacatgagga ctcatatatt taagcctttt 3180
   gtgtaataag aaagtataaa gtcacttcca gtgttggctg tgtgacagaa tcttgtattt 3240
   gggccaaggt gtttccattt ctcaatcagt gcagtgatac atgtactcca gagggacagg 3300
   gtggaccccc tgagtcaact ggagcaagaa ggaaggaggc agactgatgg cgattccctc 3360
   tcacccggga ctctccccct ttcaaggaaa gtgaaccttt aaagtaaagg cctcatctcc 3420
   tttattgcag ttcaaatcct caccatccac agcaagatga attttatcag ccatgtttgg 3480
   ttgtaaatgc tcgtgtgatt tcctacagaa atactgctct gaatattttg taataaaggt 3540
   ctttgcacat gtgaccacat acgtgttagg aggctgcatg ctctggaagc ctggactcta 3600
   agctggagct cttggaagag ctcttcggtt tctgagcata atgctcccat ctcctgattt 3660
   ctctgaacag aaaacaaaag agagaatgag ggaaattgct attttatttg tattcatgaa 3720
   cttggctgta atcagttatg ccgtatagga tgtcagacaa taccactggt taaaataaag 3780
   cctatttttc aaatttagtg agtttctcaa gtttattata tttttctctt gtttttattt 3840
   aatgcacaat atggcattat atcaatatcc tttaaactgt gacctggcat acttgtctga     3900
   cagatcttaa tactactcct aacatttaga aaatgttgat aaagcttctt agttgtacat     3960
   tttttggtga agagtatcca ggtctttgct gtggatgggt aaagcaaaga gcaaatgaac     4020
   gaagtattaa gcattggggc ctgtcttatc tacactcgag tgtaagagtg gccgaaatga     4080
   cagggctcag cagactgtgg cctgagggcc aaatctggcc caccacctgt ttggtgtagc     4140
   ctgctaagaa tggcttttac atttttaaat ggttgggaaa gaaaaaaaaa gaagtagtag     4200
   attttgtagc atgtgatgta agtaatgtaa aacttaaatt ccagtatcca taaataaagt     4260
   tttatgagaa caga                                                       4274
<210> 68
   <211> 5386
   <212> DNA
   <213> Homo sapiens
<400> 68
   cgccttgccc aaggcctcag caaccgacgt tcgaaagcca ggagaaaagg cgaatgataa 60
   agggcgctcc acgcatgcgt taagaagccg ccccaactcc cccgcggcgt tctttcttgg 120
   aacaaaacta gcgcggagcc acggaactcc gcagtttgcg tagacttgaa tttcctattc 180
   ctcggacgat ccatgtggaa tccgaaaaat agaaatgaag gtacatatgc acacaaaatt 240
   ttgcctcatt tgtttgctga catttatttt tcatcattgc aaccattgcc atgaagaaca 300
   tgaccatggc cctgaagcgc ttcacagaca gcatcgtgga atgacagaat tggagccaag 360
   caaattttca aagcaagctg ctgaaaatga aaaaaaatac tatattgaaa aactttttga 420
   gcgttatggt gaaaatggaa gattatcctt ttttggtttg gagaaacttt taacaaactt 480
   gggccttgga gagagaaaag tagttgagat taatcatgag gatcttggcc acgatcatgt 540
   ttctcattta gatattttgg cagttcaaga gggaaagcat tttcactcac ataaccacca 600
   gcattcccat aatcatttaa attcagaaaa tcaaactgtg accagtgtat ccacaaaaag 660
   aaaccataaa tgtgatccag agaaagagac agttgaagtg tctgtaaaat ctgatgataa 720
   acatatgcat gaccataatc accgcctacg tcatcaccat cgtttgcatc atcatcttga 780
   tcataacaac actcaccatt ttcataatga ttccattact cccagtgagc gtggggagcc 840
   tagcaatgaa ccttcaacag agaccaataa aacccaggaa caatctgatg ttaaactacc 900
   gaaaggaaag aggaagaaaa aagggaggaa aagtaatgaa aattctgagg ttattacacc 960
   aggttttccc cctaaccatg atcagggtga acagtatgag cataatcggg tccacaaacc 1020
   tgatcgtgta cataacccag gtcattctca tgtacatctt ccagaacgta atggtcatga 1080
   tcctggtcgt ggacaccaag atcttgatcc tgataatgaa ggtgaacttc gacatactag 1140
   aaagagagaa gcaccacatg ttaaaaataa tgcaataatt tctttgagaa aagatctaaa 1200
   tgaagatgac catcatcatg aatgtttgaa cgtcactcag ttattaaaat actatggtca 1260
   tggtgccaac tctcccatct caactgattt atttacatac ctttgccctg cattgttata 1320
   tcaaatcgac agcagacttt gtattgagca ttttgacaaa cttttagttg aagatataaa 1380
   taaggataaa aacctggttc ctgaagatga ggcaaatata ggggcatcag cctggatttg 1440
   tggtatcatt tctatcactg tcattagcct gctttccttg ctaggcgtga tcttggttcc 1500
   tatcattaac caaggatgct tcaaattcct tcttacattc cttgttgcat tagctgtagg 1560
   aacaatgagt ggagacgccc ttcttcatct actgccccat tctcagggtg gacatgatca 1620
   cagtcaccaa catgcacatg ggcatggaca ttctcatgga catgaatcta acaagttttt 1680
   ggaagaatat gatgctgtat tgaaaggact tgttgctcta ggaggcattt acttgctatt 1740
   tatcattgaa cactgcatta gaatgtttaa gcactacaaa caacaaagag gaaaacagaa 1800
   atggtttatg aaacagaaca cagaagaatc aactattgga agaaagcttt cagatcacaa 1860
   gttaaacaat acaccagatt ctgactggct tcaactcaag cctcttgccg gaactgatga 1920
   ctcggttgtt tctgaagatc gacttaatga aactgaactg acagatttag aaggccaaca 1980
   agaatcccct cctaaaaatt acctttgtat agaagaggag aaaatcatag accattctca 2040
   cagtgatgga ttacatacca ttcatgagca tgatctccat gctgctgcac ataaccacca 2100
   cggcgagaac aaaactgtgc tgaggaagca taatcaccag tggcaccaca agcattctca 2160
   tcattcccat ggcccctgtc attctggatc cgatctgaaa gaaacaggaa tagctaatat 2220
   agcctggatg gtgatcatgg gggatggcat ccacaacttc agtgatgggc tcgcaattgg 2280
   tgcagctttc agtgctggat tgacaggagg aatcagtact tctatagccg tcttctgtca 2340
   tgaactgcca catgaattag gagattttgc agttcttctt aaagcaggca tgactgtaaa 2400
   gcaagcaatt gtatacaacc tcctctctgc catgatggct tacataggca tgctcatagg 2460
   cacagctgtt ggtcagtatg ccaataacat cacactttgg atctttgcag tcactgcagg 2520
   catgttcctc tatgtagcct tggtggatat gcttccagaa atgttgcatg gtgatggtga 2580
   caatgaagaa catggctttt gtcctgtggg gcaattcatc cttcagaatt taggattgct 2640
   ctttggattt gccattatgc tggtgattgc cctctatgaa gataaaattg tgtttgacat 2700
   ccagttttga cctttcccag taatcactgt tgattacgag aatgttacca tgcagctttg 2760
   catctgttcc ttgtactgta tgcacattgc tcaaaggaaa gtcagtggct tgcactactt 2820
   acaagtttca tagatttgag cctaaccaca agaggctggt gcttagtact gttttccctg 2880
   cacgtagggg tcttttaaaa atataaagct tgtgataaag agaggagaat atgggactcc 2940
   atgaaccagt gttgatatgt ttgattaaga cttttcacaa aataatcata taaaacacta 3000
   gtctctttat tagtagaaac ttctgtggct atgcagaaat agagatcgaa ccaaaaaaaa 3060
   tcatttaaac tttaaaaata ttttaaatgg actttgggga gacatttttt gtgtgtttta 3120
   agaatgaatt gtagtgctct ttaattcagc tacatatatt catgtggtga tagggatcaa 3180
   cttgacacaa ctttgaaact gcataaagta gacataggaa ctagaggaaa gctcaggctg 3240
   cattagagta tgaatttagc attgggaaaa gcccttattc ttgaatctag agttactatt 3300
   tttgtatata tttgcatagt gtttaaacct gcagcctaaa ctactgaaat ttgtgattgt 3360
   atgtttgtgt gagcttcagt ttaatgaaag attcataatg gttctttgta ttattataat 3420
   acttggtgtt ggggtgttct ttctgttttg ttttttactt taattttgtt ttgatttttt 3480
   tttttttttt ttggcggggg taggtgaggg tttggagcat gtggtctttt taaaaaattg 3540
   taaccctcta gaaaatatca aagaaatgaa ccagacgtgg tttaaatagt tgattttcct 3600
   attttaacag taccaactag ttaattggga aatgtaagtt ctgaatgttc acattgcttt 3660
   accagtttgg cactggaacc aagagcacat gtcgtggctg gctacaaggt tgtaaagcag 3720
   aaaatcgaag tttaccatgt ctgtaatgtg tacatgaagt gtcaatttag aacagttact 3780
   aggataaact ccattattgc catggctgtc atggtaccca agtgacttgg aagatgcatt 3840
   taaattactc agctgaaatc acttgatcat cttgtgccaa gatatgctgt tggtgcctga 3900
   tagggattag tcttttaggt gccctgttct cctaccataa ttgtgaatga tttgtgagaa 3960
   gtgcaagcca tgtttatcct gaatttttac ttaataattt gtattactag tcatatgcat 4020
   gtagctttct gtttacatcc tatgccacat ggtcttcatt tatgccaggt aaactgtatt 4080
   tgaactatgt gcagctagct ttgttttaat ctgcttggca accagtgtag ctgctgtaac 4140
   aatctatctt attgttcaaa tatataagag ccaaactctt ttccattcca tctaaaatgt 4200
   tttcatttag tactcttctt tcctcctact ctatgaactt caaaacaaaa acaaaacttt 4260
   gagagcagca catgcatcca ggtatttata gattattgcc agtgtctttt ctgtatgcta 4320
   taagcaaggg agcttaggtg ttatttcttt aatttatgct tgaatctgaa aaattatttc 4380
   tgacttactc catggcctcc ttataataag tagaagtttt atatataatt aattttcagc 4440
   attgggcact gaattaggac agtcctcatc tcattgcttg gcccttcaag caacctagct 4500
   aaaaggtgct gatattttat ttagtactgc caacttcaag tgatttagat atctatctat 4560
   ctagatttct gaaccaagat atatttatag ttcacttttg ggtttttata cccacggtag 4620
   gattctgcat tccagcatta aatctgcttc attttagaac ctttataaaa gcaatagctg 4680
   gaatatactc ccagttttaa aataaatgcc tgattgattt aaagcaagta ggttatgctg 4740
   aagtatataa agaagtttta tattctctca aaaatggtat tatctttctt tatttgctag 4800
   attcttacaa atcttttaag agggctgtaa cagttgctgc tagtattagg gttccacatc 4860
   attctaatgt atagtttcaa gtcttaatag acaatctgaa ttccactaca tttcttttgg 4920
   ctccaacatt ccttttagct tgaccagtct aatttaaaat gtgtttgttg gaggtcatta 4980
   acgttacttg tacaatgctg tcactgtgtg acatccatat gaattttggt atatatcaat 5040
   caatcaatca atcacattgc attcaatcaa tcagctgtga ttgattgatt atgcttagaa 5100
   atactatagt aactagatgc agtgtgaatt ttttccatta acaaacaaac aagtcagtgg 5160
   cttaaatgtg attatggtcc tgcaaggtga ttcttgctaa aatatctaaa cttttgtttt 5220
   gttttaactg aatcattttt taacttaaaa agctggaaaa tatcaaatgc tgtttttttt 5280
   ttttcattgt caacagtggt gtgtcatttt atgtatgttc ctaatgctta tggaactcct 5340
   ccaaaataaa gttactcaaa gagagcaaat aaaaaaaaaa aaaaaa 5386
<210> 69
   <211> 5337
   <212> DNA
   <213> Homo sapiens
<400> 69
   gagtggacct tgtacgccgc aagcgtagca gggtgtcaga cgcgccggtt tctgcgacgc 60
   agttagcgca gtctgctttg gtgaatacac gatttggtgc agccggggtt tggtaccgag 120
   cggagaggag atgcacacgg cactcgagtg tgaggaaaaa tagaaatgaa ggtacatatg 180
   cacacaaaat tttgcctcat ttgtttgctg acatttattt ttcatcattg caaccattgc 240
   catgaagaac atgaccatgg ccctgaagcg cttcacagac agcatcgtgg aatgacagaa 300
   ttggagccaa gcaaattttc aaagcaagct gctgaaaatg aaaaaaaata ctatattgaa 360
   aaactttttg agcgttatgg tgaaaatgga agattatcct tttttggttt ggagaaactt 420
   ttaacaaact tgggccttgg agagagaaaa gtagttgaga ttaatcatga ggatcttggc 480
   cacgatcatg tttctcattt agatattttg gcagttcaag agggaaagca ttttcactca 540
   cataaccacc agcattccca taatcattta aattcagaaa atcaaactgt gaccagtgta 600
   tccacaaaaa gaaaccataa atgtgatcca gagaaagaga cagttgaagt gtctgtaaaa 660
   tctgatgata aacatatgca tgaccataat caccgcctac gtcatcacca tcgtttgcat 720
   catcatcttg atcataacaa cactcaccat tttcataatg attccattac tcccagtgag 780
   cgtggggagc ctagcaatga accttcaaca gagaccaata aaacccagga acaatctgat 840
   gttaaactac cgaaaggaaa gaggaagaaa aaagggagga aaagtaatga aaattctgag 900
   gttattacac caggttttcc ccctaaccat gatcagggtg aacagtatga gcataatcgg 960
   gtccacaaac ctgatcgtgt acataaccca ggtcattctc atgtacatct tccagaacgt 1020
   aatggtcatg atcctggtcg tggacaccaa gatcttgatc ctgataatga aggtgaactt 1080
   cgacatacta gaaagagaga agcaccacat gttaaaaata atgcaataat ttctttgaga 1140
   aaagatctaa atgaagatga ccatcatcat gaatgtttga acgtcactca gttattaaaa 1200
   tactatggtc atggtgccaa ctctcccatc tcaactgatt tatttacata cctttgccct 1260
   gcattgttat atcaaatcga cagcagactt tgtattgagc attttgacaa acttttagtt 1320
   gaagatataa ataaggataa aaacctggtt cctgaagatg aggcaaatat aggggcatca 1380
   gcctggattt gtggtatcat ttctatcact gtcattagcc tgctttcctt gctaggcgtg 1440
   atcttggttc ctatcattaa ccaaggatgc ttcaaattcc ttcttacatt ccttgttgca 1500
   ttagctgtag gaacaatgag tggagacgcc cttcttcatc tactgcccca ttctcagggt     1560
   ggacatgatc acagtcacca acatgcacat gggcatggac attctcatgg acatgaatct     1620
   aacaagtttt tggaagaata tgatgctgta ttgaaaggac ttgttgctct aggaggcatt     1680
   tacttgctat ttatcattga acactgcatt agaatgttta agcactacaa acaacaaaga     1740
   ggaaaacaga aatggtttat gaaacagaac acagaagaat caactattgg aagaaagctt     1800
   tcagatcaca agttaaacaa tacaccagat tctgactggc ttcaactcaa gcctcttgcc     1860
   ggaactgatg actcggttgt ttctgaagat cgacttaatg aaactgaact gacagattta     1920
   gaaggccaac aagaatcccc tcctaaaaat tacctttgta tagaagagga gaaaatcata     1980
   gaccattctc acagtgatgg attacatacc attcatgagc atgatctcca tgctgctgca     2040
   cataaccacc acggcgagaa caaaactgtg ctgaggaagc ataatcacca gtggcaccac     2100
   aagcattctc atcattccca tggcccctgt cattctggat ccgatctgaa agaaacagga     2160
   atagctaata tagcctggat ggtgatcatg ggggatggca tccacaactt cagtgatggg     2220
   ctcgcaattg gtgcagcttt cagtgctgga ttgacaggag gaatcagtac ttctatagcc     2280
   gtcttctgtc atgaactgcc acatgaatta ggagattttg cagttcttct taaagcaggc     2340
   atgactgtaa agcaagcaat tgtatacaac ctcctctctg ccatgatggc ttacataggc     2400
   atgctcatag gcacagctgt tggtcagtat gccaataaca tcacactttg gatctttgca     2460
   gtcactgcag gcatgttcct ctatgtagcc ttggtggata tgcttccaga aatgttgcat     2520
   ggtgatggtg acaatgaaga acatggcttt tgtcctgtgg ggcaattcat ccttcagaat     2580
   ttaggattgc tctttggatt tgccattatg ctggtgattg ccctctatga agataaaatt     2640
   gtgtttgaca tccagttttg acctttccca gtaatcactg ttgattacga gaatgttacc     2700
   atgcagcttt gcatctgttc cttgtactgt atgcacattg ctcaaaggaa agtcagtggc     2760
   ttgcactact tacaagtttc atagatttga gcctaaccac aagaggctgg tgcttagtac     2820
   tgttttccct gcacgtaggg gtcttttaaa aatataaagc ttgtgataaa gagaggagaa     2880
   tatgggactc catgaaccag tgttgatatg tttgattaag acttttcaca aaataatcat     2940
   ataaaacact agtctcttta ttagtagaaa cttctgtggc tatgcagaaa tagagatcga     3000
   accaaaaaaa atcatttaaa ctttaaaaat attttaaatg gactttgggg agacattttt     3060
   tgtgtgtttt aagaatgaat tgtagtgctc tttaattcag ctacatatat tcatgtggtg     3120
   atagggatca acttgacaca actttgaaac tgcataaagt agacatagga actagaggaa     3180
   agctcaggct gcattagagt atgaatttag cattgggaaa agcccttatt cttgaatcta     3240
   gagttactat ttttgtatat atttgcatag tgtttaaacc tgcagcctaa actactgaaa     3300
   tttgtgattg tatgtttgtg tgagcttcag tttaatgaaa gattcataat ggttctttgt     3360
   attattataa tacttggtgt tggggtgttc tttctgtttt gttttttact ttaattttgt     3420
   tttgattttt tttttttttt tttggcgggg gtaggtgagg gtttggagca tgtggtcttt     3480
   ttaaaaaatt gtaaccctct agaaaatatc aaagaaatga accagacgtg gtttaaatag 3540
   ttgattttcc tattttaaca gtaccaacta gttaattggg aaatgtaagt tctgaatgtt 3600
   cacattgctt taccagtttg gcactggaac caagagcaca tgtcgtggct ggctacaagg 3660
   ttgtaaagca gaaaatcgaa gtttaccatg tctgtaatgt gtacatgaag tgtcaattta 3720
   gaacagttac taggataaac tccattattg ccatggctgt catggtaccc aagtgacttg 3780
   gaagatgcat ttaaattact cagctgaaat cacttgatca tcttgtgcca agatatgctg 3840
   ttggtgcctg atagggatta gtcttttagg tgccctgttc tcctaccata attgtgaatg 3900
   atttgtgaga agtgcaagcc atgtttatcc tgaattttta cttaataatt tgtattacta 3960
   gtcatatgca tgtagctttc tgtttacatc ctatgccaca tggtcttcat ttatgccagg 4020
   taaactgtat ttgaactatg tgcagctagc tttgttttaa tctgcttggc aaccagtgta 4080
   gctgctgtaa caatctatct tattgttcaa atatataaga gccaaactct tttccattcc 4140
   atctaaaatg ttttcattta gtactcttct ttcctcctac tctatgaact tcaaaacaaa 4200
   aacaaaactt tgagagcagc acatgcatcc aggtatttat agattattgc cagtgtcttt 4260
   tctgtatgct ataagcaagg gagcttaggt gttatttctt taatttatgc ttgaatctga 4320
   aaaattattt ctgacttact ccatggcctc cttataataa gtagaagttt tatatataat 4380
   taattttcag cattgggcac tgaattagga cagtcctcat ctcattgctt ggcccttcaa 4440
   gcaacctagc taaaaggtgc tgatatttta tttagtactg ccaacttcaa gtgatttaga 4500
   tatctatcta tctagatttc tgaaccaaga tatatttata gttcactttt gggtttttat 4560
   acccacggta ggattctgca ttccagcatt aaatctgctt cattttagaa cctttataaa 4620
   agcaatagct ggaatatact cccagtttta aaataaatgc ctgattgatt taaagcaagt 4680
   aggttatgct gaagtatata aagaagtttt atattctctc aaaaatggta ttatctttct 4740
   ttatttgcta gattcttaca aatcttttaa gagggctgta acagttgctg ctagtattag 4800
   ggttccacat cattctaatg tatagtttca agtcttaata gacaatctga attccactac 4860
   atttcttttg gctccaacat tccttttagc ttgaccagtc taatttaaaa tgtgtttgtt 4920
   ggaggtcatt aacgttactt gtacaatgct gtcactgtgt gacatccata tgaattttgg 4980
   tatatatcaa tcaatcaatc aatcacattg cattcaatca atcagctgtg attgattgat 5040
   tatgcttaga aatactatag taactagatg cagtgtgaat tttttccatt aacaaacaaa 5100
   caagtcagtg gcttaaatgt gattatggtc ctgcaaggtg attcttgcta aaatatctaa 5160
   acttttgttt tgttttaact gaatcatttt ttaacttaaa aagctggaaa atatcaaatg 5220
   ctgttttttt tttttcattg tcaacagtgg tgtgtcattt tatgtatgtt cctaatgctt 5280
   atggaactcc tccaaaataa agttactcaa agagagcaaa taaaaaaaaa aaaaaaa 5337
<210> 70
   <211> 2644
   <212> DNA
   <213> Homo sapiens
<400> 70
   gcgctcgcca cgcccatgcc tccgtcgctg cgcggcccac cccggatgtc agccccccgc      60
   gccgaccaga atccgtgaac atggcgaacg aggttatcaa gtgcaaggct gcagttgctt     120
   gggaggctgg aaagcctctc tccatagagg agatagaggt ggcaccccca aaggctcatg     180
   aagttcgaat caagatcatt gccactgcgg tttgccacac cgatgcctat accctgagtg     240
   gagctgatcc tgagggttgt tttccagtga tcttgggaca tgaaggtgct ggaattgtgg     300
   aaagtgttgg tgagggagtt actaagctga aggcgggtga cactgtcatc ccactttaca     360
   tcccacagtg tggagaatgc aaattttgtc taaatcctaa aactaacctt tgccagaaga     420
   taagagtcac tcaagggaaa ggattaatgc cagatggtac cagcagattt acttgcaaag     480
   gaaagacaat tttgcattac atgggaacca gcacattttc tgaatacaca gttgtggctg     540
   atatctctgt tgctaaaata gatcctttag cacctttgga taaagtctgc cttctaggtt     600
   gtggcatttc aaccggttat ggtgctgctg tgaacactgc caagttggag cctggctctg     660
   tttgtgccgt ctttggtctg ggaggagtcg gattggcagt tatcatgggc tgtaaagtgg     720
   ctggtgcttc ccggatcatt ggtgtggaca tcaataaaga taaatttgca agggccaaag     780
   agtttggagc cactgaatgt attaaccctc aggattttag taaacccatc caggaagtgc     840
   tcattgagat gaccgatgga ggagtggact attcctttga atgtattggt aatgtgaagg     900
   tcatgagagc agcacttgag gcatgtcaca agggctgggg cgtcagcgtc gtggttggag     960
   tagctgcttc aggtgaagaa attgccactc gtccattcca gctggtaaca ggtcgcacat    1020
   ggaaaggcac tgcctttgga ggatggaaga gtgtagaaag tgtcccaaag ttggtgtctg    1080
   aatatatgtc caaaaagata aaagttgatg aatttgtgac tcacaatctg tcttttgatg    1140
   aaatcaacaa agcctttgaa ctgatgcatt ctggaaagag cattcgaact gttgtaaaga    1200
   tttaattcaa aagagaaaaa taatgtccat cctgtcgtga tgtgatagga gcagcttaac    1260
   aggcagggag aagcgcctcc aacctcacag cctcgtagag cttcacagct actccagaaa    1320
   atagggttat gtgtgtcatt catgaatctc tataatcaag gacaaggata attcagtcat    1380
   gaacctgttt tctggatgct cctccacata aataattgct agtttattaa ggaatatttt    1440
   aacataataa aagtaatttc tacatttgtg tggaaattgt cttgttttat gctgtcatca    1500
   ttgtcacggt ttgtctgccc attatcttca ttctgcaagg gaaagggaaa ggaagcaggg    1560
   cagtggtggg tgtctgaaac ctcagaaaca taacgttgaa cttttaaggg tctcagtccc    1620
   cgttgattaa agaacagatc ctagccatca gtgacaaagt taatcaggac ccaagtctgc    1680
   ttctgtgata ttatcttgaa gggaggtact gtgccttgtt catacctgta ccccaaattc    1740
   ctaggatggc atctgccctt cagggggcac taaaatgtat tattgaaaca gcattctggg     1800
   cttaaatagg tgtatgtatg tgttggttgt gactgtacta tttctagtat agtgaactac     1860
   atactgaata tccaagttct cagcacctac ttttgtcaaa tcttaacatt ttgccacttc     1920
   gagatcacat tgccattcct cccctccaga ggtaacaatt atccacaatt tgatgtttat     1980
   cattcctgtg ttgttgtact ttcactgtgt ataacctaaa ccatctactc tttagtactg     2040
   ttttatatat ttttaagcct catacttgct cattctacag cttttttcac tcattattgt     2100
   ataattatat ctgaagctct cgttcattaa ttttagtcct gtgtagcaga attcaattac     2160
   gggaactacc ataatttatc tgttctccag ttgaaggcat gaagttgttg ccagtttctg     2220
   tattataaca ctgtagtgga acattcttct gcattgggct cactgcgtgt tacctaagac     2280
   gtatcacaga ataaacacat ttagccttat agacattgcc aaattgctct tcaaagtaaa     2340
   tgtgagtttt tgtgaattac atgagtatgg aatggtgttt tattatgact ttagtttgca     2400
   ttttcctcaa ttctcgttaa atccttcatt ctaatggaca ttttattgtg aagaacctgt     2460
   tcatatcctg tgctcaactt tgtattgaat tatttttctc tgaataattt ttaggagttc     2520
   ttttattcta gacatcaatc atttgtcagt tttatatgtt gcaaatatct tctagtctat     2580
   cttgtgactt ttctttttac tttatggtat tttgttgaat aaagttttaa tgtagtcaca     2640
   taaa                                                                  2644
<210> 71
   <211> 7966
   <212> DNA
   <213> Homo sapiens
<400> 71
   ggggccgcgc cgggtagcgt ttctttttag tgcctgaggc agctctggct cggagagcct       60
   tttgctagcc ccacggggac ctctgtgcac ggatggaccc gcccggacct ggcgggaagc      120
   ggcctggcag gcggcggccc cggcggcatc agcagagaca ggacggggcc gacgccgcgg      180
   gcccctgagg cgtgcgtgcc caccgggccc ggcggcggca ccatgatgcc gggcgagacc      240
   cattcggcgg cgcccgggac ggcggcggac ctgtcgcgat gtcagggctg cgcctctctg      300
   cagcagaatt tgaatgaata tgttgaagca ttaattacct tgaaacaaaa aattatcaat      360
   acagataatt tgttaacaga atatcagaag aaatgtgatg agctgcagtt tgcaagaaga      420
   gagaatagta atctgcatca ccaagtggaa gagatgcttc aaaaaatttc tcctctacag      480
   aaatgtcagg aagaactggg atctttaaaa gcagagctag aagagaaaaa gagttcttta      540
   aagttgtatc aggatactca tcaggaatat gctcgtgtaa aggaagaatg cttgaagagt      600
   gatgctcaga agaagaaact agaagctaag gtgaagaagc tgcaagaggc tgctgtcaag      660
   caaactcagg acttcaagca actgagaaat gaaaagaaaa tacttgaaaa ggaatttaag      720
   aagacacagg aaaggcttga cgaattttct aaacagaaaa atgaaaagga gttgagacat      780
   attggaacac aaatttcaag tgattcatat ggaagcatag ataaaagaaa agtgaaactg      840
   cttctgaagg aactctggct ctgtgtaaac acaacacaca gactacctgg tgaaggcagc 900
   aggtgtgtcc cagaaaaacc tgccaaagca atcaccagct ccagagtgcc tggggaagat 960
   ggtacgctac ctccaacaca gggcagccct ctcaggacct caaatgtgca gacatgcctc 1020
   acaaaactgt ccatggagat aaaggaggac tttttatgtc aaaatgtgga aaaacagagc 1080
   tccagtggaa caaattgtag ttctgaccat gtttttaatg agaatggaaa tcttgaggtt 1140
   ttagtacaaa gtcatcgtga cggtggtagt actgaatttg ttgatcatga tcattttttt 1200
   gatgaagatc ttcaagctgc aattgacttc ttcaaacttc cccctcctct tctgtcacca 1260
   gtgccctcgc cccctccgat gtcatcacct cacccgggtt ccttaccgtc ttcatttgca 1320
   cctgaaacct actttggaga atacacagat tccagcgata atgactcagt ccagcttaga 1380
   aattctgctg agtgtgtttc agaagatgat acaactgaat cacagaatta ttttggctca 1440
   ttgagaaaaa ataaaggaag tggcacatgg gaggaaaagc ccaaatcaca tgaagctatc 1500
   caagctctga atacatggga agtaaataaa gtgacaactt ctggactcga gactttcaca 1560
   gcaacactga gagaatcttc tgccacacac tccttagttg gtgaaaaaca ctggaccaca 1620
   gcatctcgat ccatgagtga tagaaaaaga gacattttac atgagacaaa aacacaaatg 1680
   gaggttaggg agatggataa gtcagtacaa actgagaaga ccattcataa actcactcga 1740
   ggtctatgca ttgagagatt gtctgccagc cctgcacaag agaaggaagc tgcccctggg 1800
   aagtctgagt tgtgttcttc tccccttggc aaaaggccat taaatgaact catggaatct 1860
   gaaggaaaaa ccgtattgtc taaaatgatg ggatcgccca aatcagagtt tactaagtgg 1920
   acacgaatta atgaaatcac ttctgaacca gaccgtatca cagtttctgg ccattttcac 1980
   agactatcta gagaattgga aaaggaaaaa gaagatactc aagggttcac tttaggagaa 2040
   tcacctgaat cagaagatga tgactcaggt gatggaatgg atgtagcagg gcttgacatt 2100
   gaaaccagtt tttcttcctc ttctaccttg gtagcattgt ctgttggcag taatccccag 2160
   tcttcttctg ggttagactg tggtaatgat acagatatta ctactaaagt attctctact 2220
   gaaccgcatc attcagaaca taaattgcaa actaaaactt taaacacatt acatctgcag 2280
   tctgagccac cggagtgttc tataggagga aacaacttgg agaatagctt gtgtgccttg 2340
   agccctgaat tgggagcatc taattttaat gatcagaaga gcagtgggat agaatataca 2400
   aaagtagtaa aaggcttgac caaaatacat tcacttcctc ggtcagtatt tatgaaagct 2460
   acaaaagatg ggcaatgtga aagtcaagat ccaagaattg agctcacact aaataagcca 2520
   gatttcacat cattaatagg ttctcaggct gccttgatca agagtggttt gggttttgtt 2580
   aaaagtactt catggcacca tagtgattta ttaaggaaag gtggcgaaga aagtctgaga 2640
   gccaaatcag aacatgaaca gaagactagc catcagttac aaaaggcaat gccattccta 2700
   caaaatagag gaccaacacc caagcctgat cttcttagag aaaataacaa tcctgtagaa 2760
   ttcaagacca ctgcatcggt gttgcctaat caagtatcag ttatcacaaa acagacaaga 2820
   cctgaaaagg ttcagagtgc caaattggaa cacttgaggc cacatagggt tgagcctacc 2880
   ttagtaacag aaaatagtgg caacaaaacc ggtatgtcaa ctgtagcaaa atgtgatggg 2940
   gaaagagatg atacaacaca aaacatcacg gaggtggctg ctgtgaaaag catttcacca 3000
   gaagtttctg cctctaggag aaaattagat tttaattctc caggtggttc ttcaccagta 3060
   gaaaattctg attgttccac aaatagcaga ttatctttct ctcctgaaaa tatcctcatc 3120
   caaaaccaag acattgtgag agaagctgca gtgcagggag atgggcagaa gcaaaggcag 3180
   cctcaggcca cagatctgga ctccagtggg acacatggca gtgagatgct tccagccaca 3240
   gaagtgactg tgtcaggagg gttttctgtt gaagaaacca gctgtggaga cacagggaga 3300
   tctggtggtg aggccctggc tgttgcaaat gattctacca gcacaccaca aaatgctaat 3360
   ggactttgga aattgaaatc tacaactccc ggtggtgctt tgcctgagtg ttttggcacc 3420
   acagacacta ctttttcttc agcattttgc agaaaacatg gagagacaca ggatacctcc 3480
   caaagtagcc tgcctggtac cttacattgt tacacaggca ttcgagaggg gggagacgac 3540
   actgaggtag agagtgaggc atttagctgc agtgagggga gcgaacagca agatgctcct 3600
   gatgactcac agaaaaattt aggagacaca gatgctgctg tagccgaggt gagaccttcc 3660
   ttagaggtag gttatttgac gtcagctctg caagatttta acataagtac tttttctgag 3720
   ctggatagac tttccacatc agaggttgtg atgtttcttg agagctgtca gttaggggat 3780
   tatagttcag gggactctgt ttctgaatgt tctagtaaag gaaccctaag taaagaaatg 3840
   aacaaagaat taaaggcaag tgaaatagga gaaaaataca gaaagcaacc ctgtgaggaa 3900
   gaaacacttg gaacctgtga agagtggatt gaatcagagg aagatgatta ttcgttaaaa 3960
   aatacaagtc agctcactca gtgttctttg gaaactctgt ctgaggttct gaccaagatt 4020
   aggcaagaac ttcaaacaaa ttctgaagat tgcaatggta aagatactgg cagtttattg 4080
   ctcttaaatg taaataacaa catgaccact gagaatttaa aagagaaaag tccatttcgg 4140
   gaaacgactg gctcctcatc acatgcttca gaaccaaccc cacaagcagc tgccttggac 4200
   actgagggca gctctcccat cagcggtatg cctcagaatg aaaaccctca gagcagacca 4260
   gaggcccgtt cagatgcagg caggcaaacc gatggtgggg aagaagacct gccagaacct 4320
   gtggagccat cagccttgtg ctctgactct gtgatggagc catccataga gcaaagttct 4380
   aactgcgagg ccgaaacaac atttcagtgt cagatagcaa cagtgacctc agaagttata 4440
   aacgtactta taaataagga tcagaatcta gtcattgaaa agggggacaa ctggacaatc 4500
   atcagtggtg tagctgtctt gccacatgtg gaccaggtca cactgtgtga cattcctgga 4560
   gacatcccta tttctcagga tcaaggagag ctggaagctg gttgcatccc agtgacttct 4620
   gctgagaagt ccccagaggc cagtcacact ggccctgcat ttcaggaggc tccatgtggc 4680
   aataatcttt catgtcccca agaggatgtt tcaagcagtg gtcagagcac caactttgat 4740
   aagagtcgtt tgcgaaatag acccgttaag cctagtatat ggattagttc tcaaatctat 4800
   gatcaaaact tcgagactca gattgttgcg tctgatcaca catattataa ctcaaaacta 4860
   gagccatctg gcaaaaataa gaatcgatca aagatttcaa acaaagatca gtcaaacaaa 4920
   ccagtaaaaa cttcagcgtc gagcagagtt gaaactcatc agagtgaagt tgctcagtca 4980
   ttttcagggg aaaaagctaa tacaaaaact caaagaagcc aaactcagac cattttagca 5040
   aatgctgata catccactcc tacagattgt tctcctgaca cactgagtaa aatacggcaa 5100
   gaggtggggc ctcctttgcc gcctctgctt gctcctctga tagctacacc tccaaggact 5160
   tcacagccac tgtctccact gatatcgagt tctagtcctt cctcaccagc ctctcctgtt 5220
   ggccaggttt ctcccttccg tgaaacccca gtgcctcctg ccatgtctcc atggccagag 5280
   gaccccagac gtgcctctcc tccagatcct tctccatctc catctgcagc ttcagccagt 5340
   gagagggtag tgccgtctcc tctgcagttc tgtgcggcca cgccgaagca cgcacttcct 5400
   gtgcctggcc gactcccacc ctgtgcatct ggccacgctg ctgtgggagg gcctcaggag 5460
   aattctgtga aaatccttga caccatgtat ccagagttat ctgccagggc ccggaccctc 5520
   aacatcctca aagggaatat tcaactcaca cgaggtccgc ctgctgactg taagaattta 5580
   ccgggacctg ccagtgctat gataggattc aaaacgatca cttcagcagc aactgctttt 5640
   gtcaaaactg ggagcagctc tggtggtgac tgtaaccaag acaagtcaag agatttgggg 5700
   actcagcagg attcaagcgg gaaaagaaca ctgtcaacgt ctacactgag aagtgctaaa 5760
   agactgcgcc tggacactgg gtccccagaa ccagaaacca ggggagtcac tgcagaagga 5820
   atccacaaaa acctcccagg gaacctccct ccagctgaag ttgcaacaac aaatgaggaa 5880
   agaagttgtt ctagtccagc cgtcagtgca gtttcacagt tgcctttaag cccaaaagaa 5940
   actgtggagt cccatgataa agccatagct aatgccctga agaaaattgc agagttttct 6000
   tttgatctgt tacctgtcat tcgtagtcat gtgtatgtgg gaaatatctc caaaaagccc 6060
   gtaatgagag atcaagagaa ggaagttgtt tatgaattta gcacaacaaa aaagcattta 6120
   gcagagtgct tgcttcactc tattctctca gaactaaaaa ttcagaagat atctatggac 6180
   cacaattaca ttcacgccct ctgcagggtg tatgtgggta tttgtcggca actcggagac 6240
   ttggaaagag ctcgtttgtt ttgctacagc ctacttaaag aagattttcc ggagtctgaa 6300
   aaattaactt tgtttattgc aaacatgtgg catgatatat ttctctctca atcggtgatt 6360
   aataaagcaa tgcagttagt tgccaggcaa cgtgctaaag gagaggttct gaactgcttg 6420
   agagcttttc ttaattggga aaagaatgcc ccggtagatg ttggcttcat ggtttctaag 6480
   ctgcttttga ccatacagtt atgtccaaaa acagaatttc aacctagtga aaaatttggt 6540
   gaagacctaa gtgataacac ttgggaatac atatttgcca ttgatctgct ctgctgccat 6600
   cagaaatgga tctggacgca tgataacatc ataagtaagg agctgtggcc tgtgatggat 6660
   aaatggataa aatacagaaa aggacatgca aacattgcgt atactcctga tattattata 6720
   gcctcaatac tgaggctgat tggtcgttta ggccaattgg gtttgaaaga aggatttcca 6780
   tctgctgtga aaaatattag ttcggttatt ggtatgttta tacagcatgc tcacgatgaa 6840
   gatataccat ggggtataca gttagcagcc gtgtatgctc tttgtgactt gagtcccagc 6900
   aatccagcag aaatttccaa gatcctggaa gcttggcgga gagaggcctc caaaagcgtt 6960
   ccgtctgcga ttgtcagctg cctagaggaa gtcagtgccc tgagcacaga ggagcttggc 7020
   tgacctggga tgccactgag gcttgagaag tgccttgaca cattttgaac acaaatagtt 7080
   tgatcagctt tcagaataca aagggaggtt tcaaaacaaa aagacataaa atagataaaa 7140
   cagaccagag gctctcctta ttgtttggca aggagacagg agaaacaagc agtcgcatag 7200
   tcgtttttcc ctaaatctaa tacgtttcac ttaaggctgt tgtgatctgt gacatatggg 7260
   ttatattatt gtgtctttgt caaacaacaa aaacttgaac ttagcccttt ttttgctgca 7320
   gaaagtgtcc ttttagtggc ttcttaaaat tgagtggcat tttataatga acttaccaat 7380
   ataaaaacat gatttggttc ctgagctgtt gttgttggac ttgtgttcca atgagtgact 7440
   aggaaaaaat aaattggcaa aaacctagag ttttctgcta tctttgctgg aaatgagttg 7500
   caaaagtttt tctcaagatg tagtgcgtaa ttgatcagag caaaacatgc agagccctta 7560
   gcagaaaccc actttaatgc attttcttca tatccctaaa gttccttaaa aatatgtgac 7620
   aatgcatcag gaagaggaga actgaagagt agaagttccc ttgcagattt ttttatcagt 7680
   gacatgtaat gagcaattca cagatgagcg caggcagagc tctgtgtgcc gtgtacatat 7740
   ggaccgtgct atgatgtgtc tcacattgga tgatattcca ctttgggaat tttagtattt 7800
   gtatatagaa aatgggttta ataactcacc atggttttga tttgtcttat attcgttatt 7860
   tcttaaaact cttgtatgtg tttttataat aaaaaataaa agtaagccat ggatattaaa 7920
   aaaaaaaaaa aaaaaaaaaa aaaaagaaag aaaaaaaaaa aaaaaa 7966
<210> 72
   <211> 1523
   <212> DNA
   <213> Homo sapiens
<400> 72
   tggctgagtt cttcctttga caagatggcg gcaggaggca gtggcgttgg tgggaagcgc      60
   agctcgaaaa gcgatgccga ttctggtttc ctggggctgc ggcccacttc ggtggaccca     120
   gcgctgaggc ggcggcggcg aggcccaaga aataagaagc ggggctggcg gcggcttgct     180
   caggagccgc tggggctgga ggttgaccag ttcctggaag acgtgcggct acaggagcgc     240
   acgagcggtg gcttgttgtc agaggcccca aatgaaaaac tcttcttcgt ggacactggc     300
   tccaaggaaa aagggctgac aaagaagaga accaaagtcc agaagaagtc actgcttctc     360
   aagaaacccc ttcgggttga cctcatcctc gagaacacat ccaaagtccc tgcccccaaa     420
   gacgtcctcg cccaccaggt ccccaacgcc aagaagctca ggcggaagga gcagctatgg     480
   gagaagctgg ccaagcaggg cgagctgccc cgggaggtgc gcagggccca ggcccggctc     540
   ctcaaccctt ctgcaacaag ggccaagccc gggccccagg acaccgtaga gcggcccttc     600
   tacgacctct gggcctcaga caaccccctg gacaggccgt tggttggcca ggatgagttt     660
   ttcctggagc agaccaagaa gaaaggagtg aagcggccag cacgcctgca caccaagccg     720
   tcccaggcac ccgccgtgga ggtggcgcct gccggagctt cctacaatcc atcctttgaa     780
   gaccaccaga ccctgctctc agcggcccac gaggtggagt tgcagcggca gaaggaggcg     840
   gagaagctgg agcggcagct ggccctgccc gccacggagc aggccgccac ccaggagtcc     900
   acattccagg agctgtgcga ggggctgctg gaggagtcgg atggtgaggg ggagccaggc     960
   cagggcgagg ggccggaggc tggggatgcc gaggtctgtc ccacgcccgc ccgcctggcc    1020
   accacagaga agaagacgga gcagcagcgg cggcgggaga aggctgtgca caggctgcgg    1080
   gtacagcagg ccgcgttgcg ggccgcccgg ctccggcacc aggagctgtt ccggctgcgc    1140
   gggatcaagg cccaggtggc cctgaggctg gcggagctgg cgcggcggca gaggcggcgg    1200
   caggcgcggc gggaggctga ggctgacaag ccccgaaggc tggggcggct caagtaccag    1260
   gcacctgaca tcgacgtgca gctgagctcg gagctgacag actcgctcag gaccctgaag    1320
   cccgagggca acatccttcg agaccggttc aagagcttcc agaggaggaa tatgatcgag    1380
   cctcgagaga gagccaagtt caaacgcaag tacaaggtga agctggtgga gaagcgggcg    1440
   ttccgtgaga tccagttgta gctgccatca gatgccggag actcgccctt caataaaaaa    1500
   tctcttctag ctgatcagtg gga                                            1523
<210> 73
   <211> 2611
   <212> DNA
   <213> Homo sapiens
<400> 73
   gcaggcgcag agtccccggg ccaagatggc tgcgcggtgc tccacacgct ggttgctggt 60
   ggttgtgggg accccgcggc tgccggctat atcgggtaga ggggcccggc cgcccaggga 120
   gggcgtggtg ggggcatggc tgagccgcaa gctgagcgtc cccgcctttg cgtcttccct 180
   gacctcttgc ggcccccgag cgctgctgac attgagacct ggtgtcagcc ttacaggaac 240
   aaaacataac cctttcattt gtactgcctc cttccacacg agtgcccctt tggccaaaga 300
   agattattat cagatattag gagtgcctcg aaatgccagc cagaaagaga tcaagaaagc 360
   ctattatcag cttgccaaga agtatcaccc tgacacaaat aaggatgatc ccaaagccaa 420
   ggagaagttc tcccagctgg cagaagccta tgaggttttg agtgatgagg tgaagaggaa 480
   gcagtacgat gcctacggct ctgcaggctt cgatcctggg gccagcggct cccagcatag 540
   ctactggaag ggaggcccca ctgtggaccc cgaggagctg ttcaggaaga tctttggcga 600
   gttctcatcc tcttcatttg gagatttcca gaccgtgttt gatcagcctc aggaatactt 660
   catggagttg acattcaatc aagctgcaaa gggggtcaac aaggagttca ccgtgaacat 720
   catggacacg tgtgagcgct gcaacggcaa ggggaacgag cccggcacca aggtgcagca 780
   ttgccactac tgtggcggct ccggcatgga aaccatcaac acaggccctt ttgtgatgcg 840
   ttccacgtgt aggagatgtg gtggccgcgg ctccatcatc atatcgccct gtgtggtctg 900
   caggggagca ggacaagcca agcagaaaaa gcgagtgatg atccctgtgc ctgcaggagt 960
   cgaggatggc cagaccgtga ggatgcctgt gggaaaaagg gaaattttca ttacgttcag 1020
   ggtgcagaaa agccctgtgt tccggaggga cggcgcagac atccactccg acctctttat 1080
   ttctatagct caggctcttc ttgggggtac agccagagcc cagggcctgt acgagacgat 1140
   caacgtgacg atcccccctg ggactcagac agaccagaag attcggatgg gtgggaaagg 1200
   catcccccgg attaacagct acggctacgg agaccactac atccacatca agatacgagt 1260
   tccaaagagg ctaacgagcc ggcagcagag cctgatcctg agctacgccg aggacgagac 1320
   agatgtggag gggacggtga acggcgtcac cctcaccagc tctggaaaaa gatccactgg 1380
   aaactaggcc gggaagcagc agcccctcca agggccaggg cacctgggag acgggaggat 1440
   tccagaacag cagcactgag ctcccacccg cagagcctct ggacggcctt ggcaacagca 1500
   aaatcatggg acaacacctc tctccacgga aaggtcacag tggacagccc gggcagtagg 1560
   atgcagcccc agaggctggt ggcagtttcc tgtccattgg taggtgacgg cccctggctc 1620
   aggcagaggg agatggttag actcttgcag ggctaaaact ctaatttgga attgaatatt 1680
   gtggatatct tagttaaagg ccatgcttac agcttagaaa tgaagcctta agctgcatca 1740
   agttacgaag tgattaattt ccttctcagc aaacctccgg gaggttccag aatgagttct 1800
   tcctgacagg ttgtcttcac tgggagcgtg gggcccccag gccccaccag caccgtcctc 1860
   ccctaatgag gggccctgcc gaggcatcag ctgctctgct cagttagttt ttattcccgg 1920
   ggtaccaagc agctgcacag tcggtgcctg ggaggcacgt agaggcccag agagtccctg 1980
   ggggttctgc tctgaccgtg tgggtggtga tccttgtcag gatgtacagt ccttgctccc 2040
   accccatccg ggatggccgc ctgtccctga ctattgagtc ctgttgttgt aagccaggca 2100
   tggagggctc ctgcccttct gctgagccac agcccattgc agcactgtgc tggccagact 2160
   tcagctgcct tgggaactga agccctgcca ctgttgctag tcaggggctt ggttctccca 2220
   cttacactgt tgacatctat tttctgaagt gtgtttaaat tattcagtgc taatcattgt 2280
   tttttccttt gtaaatgttg attcagaaaa ggaaagcaca ggctaagcag ttgaaggttc 2340
   cccaccattc agtgagagca gaacccccat tccccagcct ctgctggtag catgtcgcag 2400
   tttccatgtg tttcaggatc ttcgggctgt cgttagacag gttaatgaag aacacttctc 2460
   aacagtttcc tttttgtttt cctttataat tcactaaaat aaagcatcta ttagtgtctg 2520
   atttaggaat gtaaaatgat tctgtattaa tgtaaataag attatctatt gcaaaaagat 2580
   atttcaaacc taaaaaaaaa aaaaaaaaaa a 2611
<210> 74
   <211> 2728
   <212> DNA
   <213> Homo sapiens
<400> 74
   gcaggcgcag agtccccggg ccaagatggc tgcgcggtgc tccacacgct ggttgctggt 60
   ggttgtgggg accccgcggc tgccggctat atcgggtaga ggggcccggc cgcccaggga 120
   gggcgtggtg ggggcatggc tgagccgcaa gctgagcgtc cccgcctttg cgtcttccct 180
   gacctcttgc ggcccccgag cgctgctgac attgagacct ggtgtcagcc ttacaggaac 240
   aaaacataac cctttcattt gtactgcctc cttccacacg agtgcccctt tggccaaaga 300
   agattattat cagatattag gagtgcctcg aaatgccagc cagaaagaga tcaagaaagc 360
   ctattatcag cttgccaaga agtatcaccc tgacacaaat aaggatgatc ccaaagccaa 420
   ggagaagttc tcccagctgg cagaagccta tgaggttttg agtgatgagg tgaagaggaa 480
   gcagtacgat gcctacggct ctgcaggctt cgatcctggg gccagcggct cccagcatag 540
   ctactggaag ggaggcccca ctgtggaccc cgaggagctg ttcaggaaga tctttggcga 600
   gttctcatcc tcttcatttg gagatttcca gaccgtgttt gatcagcctc aggaatactt 660
   catggagttg acattcaatc aagctgcaaa gggggtcaac aaggagttca ccgtgaacat 720
   catggacacg tgtgagcgct gcaacggcaa ggggaacgag cccggcacca aggtgcagca 780
   ttgccactac tgtggcggct ccggcatgga aaccatcaac acaggccctt ttgtgatgcg 840
   ttccacgtgt aggagatgtg gtggccgcgg ctccatcatc atatcgccct gtgtggtctg 900
   caggggagca ggacaagcca agcagaaaaa gcgagtgatg atccctgtgc ctgcaggagt 960
   cgaggatggc cagaccgtga ggatgcctgt gggaaaaagg gaaattttca ttacgttcag 1020
   ggtgcagaaa agccctgtgt tccggaggga cggcgcagac atccactccg acctctttat 1080
   ttctatagct caggctcttc ttgggggtac agccagagcc cagggcctgt acgagacgat 1140
   caacgtgacg atcccccctg ggactcagac agaccagaag attcggatgg gtgggaaagg 1200
   catcccccgg attaacagct acggctacgg agaccactac atccacatca agatacgagt 1260
   tccaaagagg ctaacgagcc ggcagcagag cctgatcctg agctacgccg aggacgagac 1320
   agatgtggag gggacggtga acggcgtcac cctcaccagc tctggtggca gcaccatgga 1380
   tagctccgca ggaagcaagg ctaggcgtga ggctggggag gacgaggagg gattcctttc 1440
   caaacttaag aaaatgttta cctcatgata tcccagccga ggaaaaagat ccactggaaa 1500
   ctaggccggg aagcagcagc ccctccaagg gccagggcac ctgggagacg ggaggattcc 1560
   agaacagcag cactgagctc ccacccgcag agcctctgga cggccttggc aacagcaaaa 1620
   tcatgggaca acacctctct ccacggaaag gtcacagtgg acagcccggg cagtaggatg 1680
   cagccccaga ggctggtggc agtttcctgt ccattggtag gtgacggccc ctggctcagg      1740
   cagagggaga tggttagact cttgcagggc taaaactcta atttggaatt gaatattgtg      1800
   gatatcttag ttaaaggcca tgcttacagc ttagaaatga agccttaagc tgcatcaagt      1860
   tacgaagtga ttaatttcct tctcagcaaa cctccgggag gttccagaat gagttcttcc      1920
   tgacaggttg tcttcactgg gagcgtgggg cccccaggcc ccaccagcac cgtcctcccc      1980
   taatgagggg ccctgccgag gcatcagctg ctctgctcag ttagttttta ttcccggggt      2040
   accaagcagc tgcacagtcg gtgcctggga ggcacgtaga ggcccagaga gtccctgggg      2100
   gttctgctct gaccgtgtgg gtggtgatcc ttgtcaggat gtacagtcct tgctcccacc      2160
   ccatccggga tggccgcctg tccctgacta ttgagtcctg ttgttgtaag ccaggcatgg      2220
   agggctcctg cccttctgct gagccacagc ccattgcagc actgtgctgg ccagacttca      2280
   gctgccttgg gaactgaagc cctgccactg ttgctagtca ggggcttggt tctcccactt      2340
   acactgttga catctatttt ctgaagtgtg tttaaattat tcagtgctaa tcattgtttt      2400
   ttcctttgta aatgttgatt cagaaaagga aagcacaggc taagcagttg aaggttcccc      2460
   accattcagt gagagcagaa cccccattcc ccagcctctg ctggtagcat gtcgcagttt      2520
   ccatgtgttt caggatcttc gggctgtcgt tagacaggtt aatgaagaac acttctcaac      2580
   agtttccttt ttgttttcct ttataattca ctaaaataaa gcatctatta gtgtctgatt      2640
   taggaatgta aaatgattct gtattaatgt aaataagatt atctattgca aaaagatatt      2700
   tcaaacctaa aaaaaaaaaa aaaaaaaa                                         2728
<210> 75
   <211> 3818
   <212> DNA
   <213> Homo sapiens
<400> 75
<210> 76
   <211> 20
   <212> DNA
   <213> Artificial Sequence
<220>
   <223> Oligonucleotide
<400> 76
   ctcctgcctg catgttctct 20
<210> 77
   <211> 16
   <212> DNA
   <213> Artificial sequence
<220>
   <223> Oligonucleotide
<400> 77
   tgttgttgga gcctgg 16
<210> 78
   <211> 19
   <212> DNA
   <213> Artificial sequence
<220>
   <223> Oligonucleotide
<400> 78
   acagaggggt ccactctcc 19
<210> 79
   <211> 73
   <212> DNA
   <213> Artificial sequence
<220>
   <223> Oligonucleotide
<400> 79
   ctcctgcctg catgttctct ctgttgttgg agcctggagc cttgctctct gggcacagag 60
   gggtccactc tcc 73
<210> 80
   <211> 22
   <212> DNA
   <213> Artificial sequence
<220>
   <223> Oligonucleotide
<400> 80
   gaaacgatgg gacctcagtc tt 22
<210> 81
   <211> 17
   <212> DNA
   <213> Artificial sequence
<220>
   <223> Oligonucleotide
<400> 81
   cttcagcaga ggacttg 17
<210> 82
   <211> 25
   <212> DNA
   <213> Artificial sequence
<220>
   <223> Oligonucleotide
<400> 82
   tattttgtat ttggcaactg tgggc 25
<210> 83
   <211> 65
   <212> DNA
   <213> Artificial sequence
<220>
   <223> Oligonucleotide
<400> 83
   gaaacgatgg gacctcagtc ttcttcagca gaggacttga tattttgtat ttggcaactg 60
   tgggc 65
<210> 84
   <211> 20
   <212> DNA
   <213> Artificial sequence
<220>
   <223> Oligonucleotide
<400> 84
   ccccttccta ctcccagaca 20
<210> 85
   <211> 16
   <212> DNA
   <213> Artificial sequence
<220>
   <223> Oligonucleotide
<400> 85
   caccctcgct tcagcc 16
<210> 86
   <211> 23
   <212> DNA
   <213> Artificial sequence
<220>
   <223> Oligonucleotide
<400> 86
   cagtttcctc atctgtccag tgg 23
<210> 87
   <211> 62
   <212> DNA
   <213> Artificial sequence
<220>
   <223> Oligonucleotide
<400> 87
   ccccttccta ctcccagaca cccaccctcg cttcagccac agtttcctca tctgtccagt 60
   gg 62
<210> 88
   <211> 17
   <212> DNA
   <213> Artificial sequence
<220>
   <223> Oligonucleotide
<400> 88
   gccttccctg cctgtgg 17
<210> 89
   <211> 20
   <212> DNA
   <213> Artificial sequence
<220>
   <223> Oligonucleotide
<400> 89
   ccacttttaa tgggtcctcc 20
<210> 90
   <211> 17
   <212> DNA
   <213> Artificial sequence
<220>
   <223> Oligonucleotide
<400> 90
   caacggggtc agggagg 17
<210> 91
   <211> 68
   <212> DNA
   <213> Artificial sequence
<220>
   <223> Oligonucleotide
<400> 91
   gccttccctg cctgtggggg tcatgctgcc acttttaatg ggtcctccac ccaacggggt 60
   cagggagg 68
<210> 92
   <211> 25
   <212> DNA
   <213> Artificial sequence
<220>
   <223> Oligonucleotide
<400> 92
   acgtctggtt caaagagttg gatat 25
<210> 93
   <211> 21
   <212> DNA
   <213> Artificial sequence
<220>
   <223> Oligonucleotide
<400> 93
   taacttccag gagttcctca t 21
<210> 94
   <211> 20
   <212> DNA
   <213> Artificial sequence
<220>
   <223> Oligonucleotide
<400> 94
   gtgataaaga tgggcgtggc 20
<210> 95
   <211> 88
   <212> DNA
   <213> Artificial sequence
<220>
   <223> Oligonucleotide
<400> 95
   acgtctggtt caaagagttg gatatcaaca ctgatggtgc agttaacttc caggagttcc 60
   tcattctggt gataaagatg ggcgtggc 88
<210> 96
   <211> 17
   <212> DNA
   <213> Artificial sequence
<220>
   <223> Oligonucleotide
<400> 96
   ggccaccctg cctctac 17
<210> 97
   <211> 19
   <212> DNA
   <213> Artificial sequence
<220>
   <223> Oligonucleotide
<400> 97
   tgtcaaactg tcttggctg 19
<210> 98
   <211> 15
   <212> DNA
   <213> Artificial sequence
<220>
   <223> Oligonucleotide
<400> 98
   ggctaggggc tgggg 15
<210> 99
   <211> 79
   <212> DNA
   <213> Artificial sequence
<220>
   <223> Oligonucleotide
<400> 99
   ggccaccctg cctctaccca accagggccc cggggcctgt tatgtcaaac tgtcttggct 60
   gtggggctag gggctgggg 79
<210> 100
   <211> 29
   <212> DNA
   <213> Artificial sequence
<220>
   <223> Oligonucleotide
<400> 100
   tgcagaactt tcaggatgac tattaattc 29
<210> 101
   <211> 18
   <212> DNA
   <213> Artificial sequence
<220>
   <223> Oligonucleotide
<400> 101
   ttgagtggtc caagcctg 18
<210> 102
   <211> 21
   <212> DNA
   <213> Artificial sequence
<220>
   <223> Oligonucleotide
<400> 102
   tgttttgaac ccacagcagt g 21
<210> 103
   <211> 85
   <212> DNA
   <213> Artificial sequence
<220>
   <223> Oligonucleotide
<400> 103
   tgcagaactt tcaggatgac tattaattcc tctcagatgt catttttgag tggtccaagc 60
   ctgctgtttt gaacccacag cagtg 85
<210> 104
   <211> 25
   <212> DNA
   <213> Artificial sequence
<220>
   <223> Oligonucleotide
<400> 104
   taccttctac aggtcccctc tgagc 25
<210> 105
   <211> 25
   <212> DNA
   <213> Artificial sequence
<220>
   <223> Oligonucleotide
<400> 105
   tgtttcctgg tacagaccat attaa 25
<210> 106
   <211> 25
   <212> DNA
   <213> Artificial sequence
<220>
   <223> Oligonucleotide
<400> 106
   gaacaccggg ccatggctga agaag 25

## Description

### INTRODUCTION

Cytokine targeting drugs such as TNFα-blocking agents (herein after referred to as "TBA") are increasingly used in the treatment of various inflammatory diseases. The first indications in which such TBA were approved are rheumatoid arthritis and Crohn's disease. Rheumatoid arthritis (RA) is a chronic, progressive, debilitating auto-immune disease of largely unknown etiology that affects approximately 1% of the population (1). RA is characterized by chronic inflammation of the synovium, which ultimately leads to joint damage, pain and disability (2). The clinical spectrum of RA is heterogeneous, ranging from mild to severe, with variability in secondary organ system involvement. Disease heterogeneity is further illustrated by the current variation in treatment response rates. First line treatment is usually initiated with so called disease-modifying anti-rheumatic drugs (DMARDs), such as methotrexate (MTX). Approximately 30% of patients display a suboptimal response or intolerance to traditional DMARDs (3). In these patients, second line treatment is initiated with "biologics", agents that block molecules or cells thought to be instrumental to disease progression, such as tumor necrosis factor-α (TNFα) and interleukin-1 (IL-1) or B and T-cells. There are indeed nine biologic agents currently available, each with overlapping or unique mechanisms of action (4). The response rates to such treatments vary widely, with a great number of patients remaining refractory to treatment or demonstrating only partial improvement (5). The incomplete understanding of drug mechanisms of action together with disease heterogeneity means that there are no methods of identifying patient suitability for the various biologies prior to the initiation of the treatment. Establishing a rational basis on which to select patients for specific biologies would help patients to be treated more efficiently; those that would be likely to respond would initiate the biologic in question whereas those unlikely to respond could be provided with another treatment.

In absence of reliable literature on efficacy and safety of biologies and given the percentage of patients that do not respond or experience severe adverse effects, the destructive nature of RA, and the societal costs of inefficacious biological treatments, there is a strong need to make predictions on success before starting the therapy. A clinically or radiographic-based test will most probably assess conditions too late for protecting joints from irreversible destruction. Ideally, a molecular biomarker signature as a predictor for therapy responsiveness should be obtained prior to the start of therapy in a readily available bio-sample, such as peripheral blood. Given the systemic nature of RA and the communication between the systemic and organ-specific compartments, the peripheral blood may not directly have implications for the understanding of disease pathogenesis, but it is especially suitable to analyze gene expression profiles that provide a framework to select clinically relevant biomarkers. Furthermore, blood-based tests remain less invasive for the patients than synovial tissue-based tests.

Ultimately, this may lead to a personalized form of medicine, whereby the best suited therapy will be applied to an individual patient.

The same is true for other inflammatory diseases for which TBA have been approved or for which preliminary results indicate that TBA might be a useful treatment. For the moment, TBA have been approved, in addition to the treatment of RA or Crohn's disease, for the treatment of ankylosing spondylitis, psoriatic arthritis, plaque psoriasis, and ulcerative colitis (see notably FDA labels of infliximab and etanercept). In addition, preliminary results suggest that TBA may be useful in the treatment of several other inflammatory diseases, such as vasculitis (notably Behcet's disease, Churg-Strauss vasculitis, polyarteritis nodosa, and giant cell arthritis); Wegener's granulomatosis; sarcoidosis; adult-onset Still's disease, polymyositis/dermatomyositis, and systemic lupus erythematosus (SLE) (31 and 32).

In all cases, only a proportion (although sometimes a high proportion) of patients treated with TBA display a clinical response to the treatment (see notably FDA labels of infliximab (Remicade®) and etanercept (Enbrel®)). For all diseases in which TBA may be useful, it would thus be very helpful to be able to predict the capacity of a subject to respond or not to TBA treatment.

A very powerful way to gain insight into the molecular signatures underlying pathophysiological processes has arisen from DNA microarray technology, which allows the identification of the fraction of genes that are differentially expressed in blood and tissue samples among patients with clinically defined disease. These differentially expressed genes may provide insight into biological pathways contributing to disease and represent classifiers for early diagnosis, prognosis, and response prediction.

Several pitfalls were experienced using this multistage and relatively expensive technology, which highly depends on perfectly standardized conditions. Factors that might influence the sensitivity and reproducibility range from sample differences, variation in amount and quality of starting RNA material, amplification and labeling strategies and dyes, to probe sequence and hybridization conditions. In addition, the lack of standardized approaches for normalization and usage of data analysis algorithms could influence the outcome. Furthermore, most microarray studies are not prospectively planned and often do not have detailed protocols, but rather tend to make use of existing samples. Therefore, verification of results is an essential step in microarrays studies and quality criteria have to be set.

Several groups have explored the possibility of identifying molecular traits (single nucleotide polymorphisms, gene expression etc.) capable of classifying patients according to their response to treatment based on retrospective analyses of biological samples (synovium or peripheral blood) collected at treatment baseline. In particular, much interest has been paid to the TNFα-blocking agent Infliximab, with the first report several years ago by T Lequerré and colleagues of gene expression-based prediction of response to therapy (6). Since then, several other groups have similarly reported on large-scale gene expression analyses of peripheral blood as a means to predict response to Infliximab (7) (8) (9) (34). All of these studies reported on differentially expressed genes and combinations thereof for the prediction of response to therapy.

These studies provided important proof of concept for the prediction of response to Infliximab at baseline of therapy. Nevertheless, as with all studies of this kind, the use of microarray technology, measuring thousands of genes simultaneously in relatively small cohorts of patients, runs the risk of over-fitting data, leading to false positive results. Moreover, the mono-centric nature of these studies may limit the relevance of the genes identified to a wider and more demographically varied population.

The present invention overcomes these drawbacks by combining information from multiple existing studies. This approach can increase the reliability and generalizability of results. Quantitative approaches in which individual studies addressing a set of related research hypothesis are statistically integrated and analyzed to determine the effectiveness of interventions (meta-analysis) showed the broad utility of applying meta-analytic approaches to genome-wide data for the purpose of biological discovery. Meta-analysis were already used to identify genes differentially expressed between two groups, to compare results obtained on different microarray platforms (cross-platform classification), to identify overlaps between samples from heterologous datasets, to identify co-expressed genes or to reconstruct gene networks.

Meta-analyses of multiple gene expression microarray datasets provide discriminative gene expression signatures that are identified and validated on a large number of microarray samples, generated by different laboratories and microarray technologies. Predictive models generated by this approach are better validated than those generated on a single data set, while showing high predictive power and improved generalization performance.

In the present invention, the meta-analysis was performed according to the stepwise approach in conducting meta-analysis on microarray datasets (1-identify suitable microarray studies; 2-extract data from studies (this step also involved getting additional information from the authors of selected studies); 3-prepare the individual datasets; 4-annotate the individual datasets; 5-resolve the many-to-many relationship between probes and genes; 6-combine the study-specific estimates; 7-analyze, present, and interpret results) described in Ramasamy *et al.* (10).

Sixty one genes differentially expressed between future responders and non responders to Infliximab therapy have thus been identified. Furthermore, two individual genes have been found to be highly correlated to the Infliximab responsive or non-responsive phenotype of tested subjects, and several combinations of a minimum number of genes are proposed as being predictive of the primary (week 14 and week 22) response to anti-TNF treatment in RA patients. These combinations comprise genes that are known to be involved in inflammatory or immune processes rather than in the metabolism pathway of Infliximab, which clearly gives a rational for their general usefulness for predicting TNFα-blocking agents (TBA) responsive or non-responsive phenotype of subjects suffering from other inflammatory diseases, notably those for which TBA have been approved or have been shown to be useful in preliminary studies.

### DETAILED DESCRIPTION OF THE INVENTION

The invention thus relates to a method for the in vitro diagnosis or prognosis of a TNFα-blocking agent (hereafter referred to as TBA) responding or non-responding phenotype, comprising:
(a) determining from a blood sample of a subject suffering from an inflammatory disease an expression profile comprising or consisting of:
   (i) the gene MAPK14; or
   (ii) the genes MAPK14 and S100A9; or
   (iii) the genes MAPK14 and GNLY;
   and optionally one or more housekeeping gene(s),
(b) comparing the obtained expression profile with at least one reference responding expression profile and at least one reference non-responding expression profile using statistical models or machine learning technologies, and
(c) determining the responding or non-responding phenotype from said comparison.

The invention also relates to a method for designing a TBA treatment for a subject suffering from an inflammatory disease, said method comprising:
(a) determining from a blood sample of said subject an expression profile comprising or consisting of:
   (i) the gene MAPK14;
   (ii) the genes MAPK14 and S100A9; or
   (iii) the genes MAPK14 and GNLY;
   and optionally one or more housekeeping gene(s),
(b) comparing the obtained expression profile with at least one reference responding expression profile and at least one reference non-responding expression profile using statistical models or machine learning technologies,
(c) determining the responding or non-responding phenotype from said comparison, and
(d) designing the dose of TBA treatment according to said identified TBA responding or non-responding phenotype.

More generally, the present description also discloses a method for the in vitro diagnosis or prognosis of a cytokine targeting drug (hereafter referred to as CyTD, such as aTNFα-blocking agent, hereafter referred to as TBA) responding or non-responding phenotype, comprising:
(a) determining from a biological sample of a subject suffering from an inflammatory disease an expression profile comprising or consisting of:
   (iv) the gene MAPK14; or the genes MAPK14 and S100A9; or the genes MAPK14 and GNLY; or the 61 genes of Table 2, or
   (v) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
   (vi) Equivalent Expression Profile of anyone of the expression profiles of (i) and (ii),
   and optionally one or more housekeeping gene(s),
(b) comparing the obtained expression profile with at least one reference expression profile, and
(c) determining the CyTD responding or non-responding phenotype from said comparison.

The present description also discloses a method for designing a CyTD treatment for a subject suffering from an inflammatory disease, said method comprising:
(a) determining from a biological sample of said subject an expression profile comprising or consisting of:
   (iv) the gene MAPK14; or the genes MAPK14 and S100A9; or the genes MAPK14 and GNLY; or the 61 genes of Table 2, or
   (v) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
   (vi) Equivalent Expression Profile of anyone of the expression profiles of (i) and (ii),
   and optionally one or more housekeeping gene(s),
(b) comparing the obtained expression profile with at least one reference expression profile,
(c) determining the CyTD responding or non-responding phenotype from said comparison, and
(d) designing the dose of CyTD treatment according to said identified CyTD responding or non-responding phenotype.

The present description is also drawn to a method of treatment of a subject suffering from an inflammatory disease with a CyTD, comprising:
(a) determining from a biological sample of the said subject the presence of a CyTD responding or non-responding phenotype using a method disclosed in the present description, and
(b) adapting the CyTD treatment in function of the result of step (a).

Said adaptation of the CyTD treatment may consist in:
- a reduction or suppression of the said CyTD treatment if the subject has been diagnosed as CyTD non-responding, or
- the continuation of the said CyTD treatment if the subject has been diagnosed as CyTD responding.

The present description also refers to a new use of a CyTD in the treatment of an inflammatory disease, comprising the steps of:
(a) determining from a biological sample of a subject suffering from an inflammatory disease the presence of a CyTD responding or non-responding phenotype using a method disclosed in the present description, and
(b) determining the dose of CyTD to administer with respect to the result of step (a).

Optionally; the dose of CyTD determined in step (b) is administered to the subject.

The present description thus also relates to a CyTD, for use in treating an inflammatory disease, wherein the CyTD is administered to a subject suffering from said inflammatory disease who has been diagnosed and/or prognosed as responsive using a method disclosed in the present description.

The present description also relates to the use of a CyTD for preparing a drug for the treatment of an inflammatory disease in subjects suffering from said inflammatory disease who have been diagnosed and/or prognosed as responsive using a method disclosed in the present description.

In all the present description, an "inflammatory disease" refers to a disease involving uncontrolled inflammation processes leading to body damages, and includes any disease generally considered as inflammatory diseases by those skilled in the art. Advantageously, said inflammatory disease is known to involve, at least in some cases, a pathogenic inflammatory cytokine (IL-1, IL-6, IL15, IL-17, IL-18, IL-23 or TNF-α) secretion. The methods disclosed in the present description then permit to diagnose the presence of such a pathogenic inflammatory cytokine secretion in a tested subject, to predict his/her capacity to respond to a CyTD treatment, and thus to adapt his/her treatment in view of his CyTD responding/non-responding phenotype. In the invention, said inflammatory disease is known to involve, at least in some cases, a pathogenic TNF-α secretion. The methods according to the invention then permit to diagnose the presence of such a pathogenic TNF-α secretion in a tested subject, to predict his/her capacity to respond to a TBA treatment, and thus to adapt his/her treatment in view of his TBA responding/non-responding phenotype. Such inflammatory diseases may be of autoimmune or non-autoimmune origin. Non limiting examples of inflammatory diseases for which the methods and kits according to the invention are useful for determining a TBA responding or non-responding phenotype include rheumatoid arthritis (RA), Crohn's disease, ankylosing spondylitis, psoriatic arthritis, plaque psoriasis, ulcerative colitis, vasculitis (notably Behcet's disease, Churg-Strauss vasculitis, polyarteritis nodosa, and giant cell arthritis); Wegener's granulomatosis; sarcoidosis; adult-onset Still's disease, polymyositis/dermatomyositis, and systemic lupus erythematosus (SLE). An advantageous group of diseases for which the methods of the invention are useful are those in the treatment of which TBA have been approved: rheumatoid arthritis (RA), Crohn's disease, ankylosing spondylitis, psoriatic arthritis, plaque psoriasis, ulcerative colitis. The methods according to the invention are particularly useful for RA-suffering patients for determining a TBA responding or non-responding phenotype.

For rheumatoid arthritis (RA), since first line treatment is usually initiated with so called disease-modifying anti-rheumatic drugs (DMARDs), the present description also refers to a method of treatment of an RA-suffering subject, comprising the steps of:
(a) administering a therapeutic dose of a DMARD to the said subject suffering from RA,
(b) determining from a biological sample of the said RA-suffering subject the presence of a CyTD responding or non-responding phenotype using a method disclosed in the present description, and
(c) determining the dose of CyTD to administer with respect to the result of step (b).

Thus the present description also refers to a combination of a DMARD and a CyTD for the treatment of RA, comprising the steps of:
(a) administering a therapeutic dose of a DMARD to a subject suffering from RA,
(b) determining from a biological sample of the said RA-suffering subject the presence of a CyTD responding or non-responding phenotype using a method disclosed in the present description, and
(c) determining the dose of CyTD to administer with respect to the result of step (b).

Optionnally; the dose of CyTD determined in step (c) is administered to the subject.

In a preferred embodiment, the DMARD is methotrexate (MTX).

By "cytokine targeting drug" or "CyTD", it is meant any molecule neutralizing a cytokine signalling, notably by binding to and neutralizing the cytokine or its receptor. Such a binding and neutralizing molecule may notably be an antibody or a fragment thereof specific for said cytokine or cytokine receptor, cytokine receptor antagonists, or any other molecule, such as a recombinant protein, binding to and neutralizing said cytokine or cytokine receptor. Said CyTD preferably targets an inflammatory cytokine such as IL-1, IL-6, IL-15, IL-17, IL-18, IL-23 or TNF-α or a receptor of such inflammatory cytokines. Molecules targeting IL-1 signalling include monoclonal antibodies to IL-1, such as Canakinumab (commercial name Ilaris®), a human anti-IL-1β monoclonal antibody; antagonists of IL-1 receptor such as anakinra (commercial name Kineret®), and a fusion protein between IgG1 Fc portion and ligand-binding domains of human IL-1RI and IL-1AcP such as Rilonacept (nom commercial Arcalyst^{™}) Molecules targeting II-6 signalling notably include Tocilizumab, an anti-IL-6R monoclonal antibody. Molecules targeting II-15 signalling notably include HuMax-IL-15 (AMG 714), an anti-IL-15 monoclonal antibody. Molecules targeting II-17 signalling notably include AIN457, an anti-IL-17A monoclonal antibody. In all the present description, a preferred CyTD is a "TNFα-blocking agent" or "TBA".

By "TNFα-blocking agent" or "TBA", it is herein meant a biological agent which is capable of neutralizing the effects of TNFα. Said agent is a preferentially a protein such as a soluble TNFα receptor, e.g. Pegsunercept, or an antibody. In a further preferred embodiment, the said agent is a monoclonal antibody. In an even further preferred embodiment, the said agent is selected in the group consisting of Etanercept (Enbrel®), Infliximab (Remicade®), Adalimumab (Humira®), and Certolizumab pegol (Cimzia®). In an even more preferred embodiment, the said agent is Infliximab.

In a particularly preferred embodiment of any method according to the present invention, the inflammatory disease is rheumatoid arthritis and the TBA is Infliximab, a particular TBA.

According to the present description, a "CyTD responding phenotype" is defined as a response state of a subject to the administration of a CyTD. A "response state" means that the said subject (referred to as a CyTD responding subject or a responding subject or a responsive subject: for the purpose of this application, these terms are similar) responds to the treatment, i.e. that the treatment is efficacious in the said subject. The definition of response is an improvement in clinical symptoms. The quantification of such response is made according to ACR20, ACR50, ACR70 criteria (11) and/or EULAR criteria at weeks 14 or 22 or change in DAS28 >1.2. Even more preferred is EULAR response criteria at 14 weeks. These criteria (31) have been established by organizations regrouping the professionals in the field (ACR: American College of Rheumatology; EULAR: European League Against Rheumatism). These criteria are thus well known to the skilled person in the art and need not be detailed here.

In contrast, a "CyTD non-responding phenotype" refers to the absence in said subject (referred to herein as a CyTD non-responding subject or a non responding subject or a non-responsive subject: these terms should be construed in the context of this application as having the same meaning) of a state of response, meaning that said subject remains refractory to the treatment.

In a preferred embodiment of any of the above-described *in vitro* methods of diagnosis/prognosis, and in particular in methods of diagnosis/prognosis according to the invention, the said subject is an RA-suffering subject. An "RA-suffering subject" is a subject fulfilling the American College of Rheumatology (ACR) criteria for RA (11). The said subject may not be treated with a CyTD; alternatively, the said subject may be treated with a CyTD.

It will easily be conceived that when the said subject is not treated with a CyTD, the methods disclosed in the present description permit a prognosis of the responsiveness/non responsiveness of the said subject. Thus, in this embodiment, the method disclosed in the present description allows the person skilled in the art to prognose (i.e. to identify) the subjects susceptible of responding to the CyTD treatment. This is important because of the destructive nature of RA and the societal costs of inefficacious biological treatments. Moreover, since this allows for identification of non responsive subjects before any treatment is initiated, the risks for one treated subject to encounter severe adverse effects are greatly diminished.

When the subject is treated with a CyTD, the methods disclosed in the present description are useful for diagnosing if a subject responds to the said CyTD, and whether the said subject would thus benefit from a continuation of the said treatment. Moreover, they are useful for diagnosing subjects who are not responding to the treatment, i.e. who are refractory to the CyTD, and should thus swiftly shifted to another therapy. In regard of the debilitating nature of RA, this achievement is crucial. In particular, the methods disclosed in the present description allow for a diagnosis at week 14 or 22 after the beginning of the CyTD treatment.

In the present description, what is described for CyTD also particularly applies to TBA, which is the particular CyTD in any method or kit according to the invention.

A "biological sample" may be any sample that may be taken from a subject, such as a serum sample, a plasma sample, an urine sample, a blood sample, a lymph sample, or a biopsy. In the invention, the sample is a blood sample. In the invention, such a sample must allow for the determination of an expression profile comprising or consisting of:
(i) the gene MAPK14;
(ii) the genes MAPK14 and S100A9; or
(iii) the genes MAPK14 and GNLY;
and optionally one or more housekeeping gene(s).

In other methods disclosed in the present description, such a sample must allow for the determination of an expression profile comprising or consisting of:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s).

Preferred biological samples for the determination of an expression profile include samples such as a blood sample, a plasma sample, a lymph sample, or a biopsy. Preferably, the biological sample is a blood sample. Indeed, such a blood sample may be obtained by a completely harmless blood collection from the patient and thus allows for a non-invasive diagnosis of a CyTD responding or non-responding phenotype. In the invention, the sample is a blood sample.

In the invention, by "expression profile" is meant the expression levels of a group of genes comprising or consisting of:
(i) the gene MAPK14;
(ii) the genes MAPK14 and S100A9; or
the genes MAPK14 and GNLY; and optionally one or more housekeeping gene(s), since these expression profiles have been demonstrated to be particularly relevant for assessing the responding/non responding phenotype of a subject. In a most preferred embodiment, the expression profile for diagnosing responsiveness at week 14 or week 22 after the beginning of the TBA treatment comprises or preferably consists of the genes MAPK14 and S100A9, and optionally one or more housekeeping gene(s). In another most preferred embodiment, the expression profile for diagnosing responsiveness at week 14 or week 22 after the beginning of the TBA treatment comprises or preferably consists of the genes MAPK14 and GNLY, and optionally one or more housekeeping gene(s).

In other methods disclosed in the present description, by "expression profile" is meant the expression levels of a group of genes comprising or consisting of:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s), since these expression profiles have been demonstrated to be particularly relevant for assessing the responding/non responding phenotype of a subject. The expression profile for diagnosing if the subject is responding at week 14 or week 22 after the beginning of the CyTD treatment may comprise or preferably consist of the genes IL2RB, S100A9, and CASP5, or of the gene S100A9 or Equivalent Expression Profile thereof, and optionally one or more housekeeping gene(s). Alternatively, the expression profile for diagnosing responsiveness at week 14 or week 22 after the beginning of the CyTD treatment (in particular a TBA treatment) may comprise or preferably consist of the gene S100A9, or Equivalent Expression Profile thereof, and optionally one or more housekeeping gene(s).

The determination of the presence of a CyTD responding or non-responding phenotype is carried out thanks to the comparison of the obtained expression profile with at least one reference expression profile in step (b).

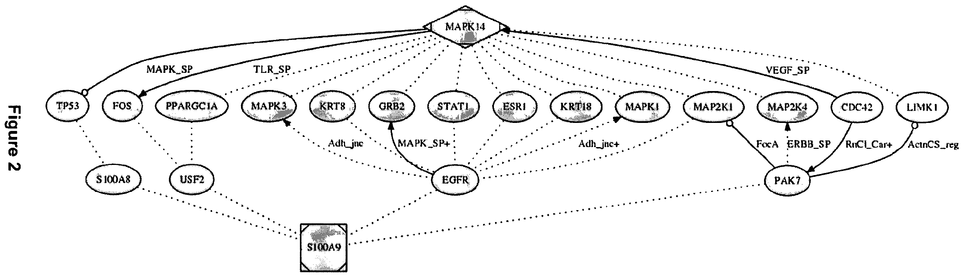

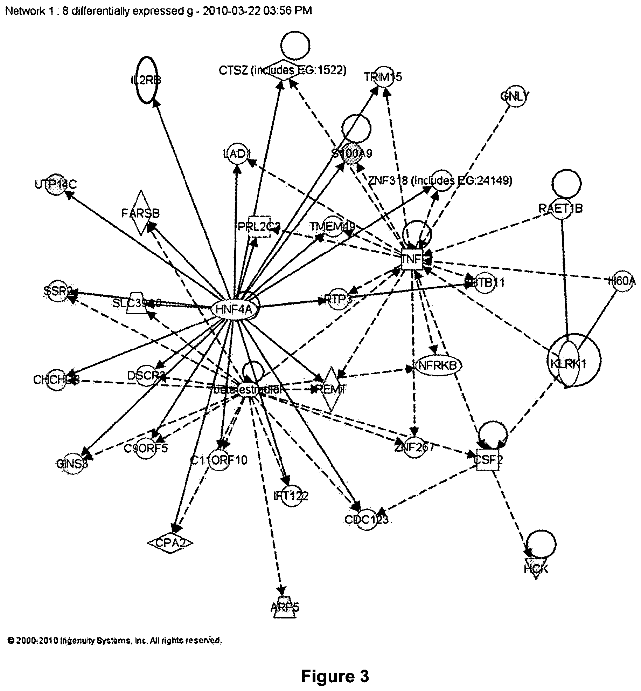

The term "Equivalent Expression Profile" herein refers to expression profiles of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C,
wherein the addition, deletion or substitution of some of the genes does not change significantly the reliability of the test and is considered as an "acceptable expression profile". As an example, the addition or substitution of some of the genes of the sets described in the present description by other genes belonging to the same metabolic pathway should also be considered as an equivalent expression profile. For example S100A8 is equivalent to S100A9, and any above mentioned (i) or (ii) expression profile in which S100A9 is replaced by S100A8 (i.e. expression profiles comprising or consisting of the genes MAPK14 and S100A8; 8 or the 61 genes of Table 2 in which S100A9 is replaced by S100A8; or the gene S100A8; or the genes S100A8, IL2RB, and CASP5; or the genes S100A8, IL2RB, KLRK1, HCK, and GNLY; or the genes S100A8, IL2RB, KLRK1, HCK, GNLY, CTSZ, ARF5, and UTP14C) should be considered as Equivalent Expression Profiles of above mentioned (i) and (ii) expression profiles. More generally are considered as Equivalent Expression Profiles, any expression profile wherein some of the genes are replaced by genes belonging to the same biological network, such as described in figures 2 and 3 below.

The term "Acceptable Expression Profile" herein refers to an expression profile which is capable of correctly classifying at least 60% of the analyzed samples, preferably 65%, and more preferably 70%, has a sensitivity and specificity of at least 60% preferably 65%, and more preferably 70.The sensitivity value is defined as the ratio of the number of patients actually clinically responding to the CyTD treatment and classified as responding using the test disclosed in the present description amongst all patients treated with the CyTD. Specificity measures the proportion of patients actually clinically not responding to the CyTD treatment which are correctly identified using the test disclosed in the present description amongst all patients treated with the CyTD.

By "Best Expression Profile" is meant an expression profile which is able to correctly classify at least 80% of the analyzed samples, has either a sensitivity or a sensitivity of at least 80%.

Although the lists of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C,
have been determined as the Best Expression Profiles to assess responsiveness/non responsiveness, an Equivalent Expression Profile such as defined above, still permits to assess responsiveness, with an acceptable reliability. In particular embodiments, sublists of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C,
still permit to assess responsiveness with a good reliability and should be considered as Acceptable Expression Profiles.

In the invention, while the expression profile used for determining the CyTD (notably TBA) responsive or non-responsive phenotype may comprise and not only consist of:
(i) the gene MAPK14;
(ii) the genes MAPK14 and S100A9; or
(iii) the genes MAPK14 and GNLY;
and optionally one or more housekeeping gene(s), it is preferred that the expression profiles consists or essentially consists of one of the above described (i), (ii) or (iii) expression profiles, optionally with one or more housekeeping gene(s), meaning that no more than 50, 40, 30, 25, 20, preferably no more than 15, preferably no more than 10, preferably no more than 9, 8, 7, 6, 5, 4, 3, 2, or 1 genes that are not a gene belonging one the above described (i), (ii) or (iii) expression profiles or a housekeeping gene are included in the expression profile.

In other methods disclosed in the present description, while the expression profile used for determining the CyTD (notably TBA) responsive or non-responsive phenotype may comprise and not only consist of:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s), it is preferred that the expression profiles consists or essentially consists of one of the above described (i), (ii) or (iii) expression profiles, optionally with one or more housekeeping gene(s), meaning that no more than 50, 40, 30, 25, 20, preferably no more than 15, preferably no more than 10, preferably no more than 9, 8, 7, 6, 5, 4, 3, 2, or 1 genes that are not a gene belonging one the above described (i) or (ii) expression profiles or a housekeeping gene are included in the expression profile.

By "housekeeping genes", it is meant genes that are constitutively expressed at a relatively constant level across many or all known conditions, because they code for proteins that are constantly required by the cell, hence, they are essential to a cell and always present under any conditions. It is assumed that their expression is unaffected by experimental conditions. The proteins they code are generally involved in the basic functions necessary for the sustenance or maintenance of the cell. Non-limitating examples of housekeeping genes that may be used in methods of the invention include:
- HPRT1 (hypoxanthine phosphoribosyltransferase 1),
- UBC (ubiquitin C),
- YWHAZ (tyrosine 3-monooxygenase/tryptophan 5-monooxygenase activation protein, zeta polypeptide),
- B2M (beta-2-microglobulin),
- GAPDH (glyceraldehyde-3-phosphate dehydrogenase),
- FPGS (folylpolyglutamate synthase),
- DECR1 (2,4-dienoyl CoA reductase 1, mitochondrial),
- PPIB (peptidylprolyl isomerase B (cyclophilin B)),
- ACTB (actin β),
- PSMB2 (proteasome (prosome, macropain) subunit, beta type, 2),
- GPS1 (G protein pathway suppressor 1),
- CANX (calnexin),
- NACA (nascent polypeptide-associated complex alpha subunit),
- TAX1 BP1 (Tax1 (human T-cell leukemia virus type I) binding protein 1), and
- PSMD2 (proteasome (prosome, macropain) 26S subunit, non-ATPase, 2).

Preferably, the number of housekeeping genes used for normalization in methods according to the invention is comprised between one and five with a preference for three.

The determination of the presence of a responsive or non responsive phenotype is carried out thanks to comparing the obtained expression profile with at least one reference profile in step (b).

A "reference expression profile" is a predetermined expression profile, obtained from a biological sample from a subject with a known particular response state. In order to have this comparison meaningful and robust, the "reference expression profile" is determined by the training of an algorithm resulting from the following steps:
- the collection of the expression profiles of biological samples obtained from responsive and non-responsive patients,
- the training of an algorithmon the expression profiles of the aforementioned patients for classifying said expression profiles as responsive and non-responsive phenotypes.

The "comparison of an obtained expression profile with a reference expression profile" can be understood as the application of the adjusted algorithm on the obtained expression profile.

The reference expression profile used for comparison with the test sample in step (b) may have been obtained from biological samples from CyTD responsive subjects ("CyTD responsive reference expression profile" or "responsive reference expression profile"; as used herein these expressions are synonymous), and/or from biological samples from CyTD non-responsive subjecst ("CyTD non-responsive reference expression profile" or "non-responsive reference expression profile" ; as used herein these expressions have the same meaning).

Preferably, at least one reference expression profile is a CyTD responsive reference expression profile. Alternatively, at least one reference expression profile may be a CyTD non-responsive reference expression profile. More preferably, the determination of the presence or absence of a CyTD responsive phenotype is carried out by comparison with at least one responder and at least one non-responder reference expression profiles. The diagnosis or prognostic may thus be performed using one responsive reference expression profile and one non-responsive reference expression profile. Advantageously, to get a stronger diagnosis or prognostic, said diagnosis or prognostic is carried out using several responsive reference expression profiles and several non-responsive reference expression profiles.

The comparison of a tested subject expression profile with said reference expression profiles, which permits prediction of the tested subject's clinical response based on his/her expression profile, can be done by those skilled in the art using statistical models or machine learning technologies. The PLS (Partial Least Square) regression is particularly relevant to give prediction in the case of small reference samples. The comparison may also be performed using Support Vector Machines (SVM), logistic regression, Linear Discriminant Analysis, Random Forests, k-NN (Nearest Neighbour) or PAM (Predictive Analysis of Microarrays) statistical methods.

The expression profile may be determined by any technology known by a man skilled in the art. In particular, each gene expression level may be measured at the genomic and/or nucleic and/or proteic level. In a preferred embodiment, the expression profile is determined by measuring the amount of nucleic acid transcripts of each gene. In another embodiment, the expression profile is determined by measuring the amount of protein produced by each of the genes.

The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-know in the art. From the mRNA or cDNA sample, the amount of nucleic acid transcripts may be measured using any technology known by a man skilled in the art, including nucleic microarrays, quantitative PCR, and hybridization with a labelled probe.

In a preferred embodiment, the expression profile is determined using quantitative PCR. Quantitative, or real-time, PCR is a well known and easily available technology for those skilled in the art and does not need a precise description.

In a particular embodiment, the determination of the expression profile using quantitative PCR may be performed as follows. Briefly, the real-time PCR reactions are carried out using the TaqMan Universal PCR Master Mix (Applied Biosystems). 6 µL cDNA is added to a 9 µL PCR mixture containing 7.5 µL TaqMan Universal PCR Master Mix, 0.75 µL of a 20X mixture of probe and primers and 0.75µl water. The reaction consisted of one initiating step of 2 min at 50 deg. C, followed by 10 min at 95 deg. C, and 40 cycles of amplification including 15 sec at 95 deg. C and 1 min at 60 deg. C. The reaction and data acquisition can be performed using the ABI 7900HT Fast Real-Time PCR System (Applied Biosystems). The number of template transcript molecules in a sample is determined by recording the amplification cycle in the exponential phase (cycle threshold or C_{T}), at which time the fluorescence signal can be detected above background fluorescence. Thus, the starting number of template transcript molecules is inversely related to C_{T}. The level of expression of a gene is measured using the "ΔΔCT method", briefly a gene is normalized by the value of one or a group of reference/housekeeping genes and/or by a reference sample such as a pooled sample or a commercially available reference such as the qPCR Human Universal Reference cDNA, random primed ; Ozyme ; ref 639654.

In another preferred embodiment, the expression profile is determined by the use of a nucleic microarray.

According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 25 to about 60 base pairs or less in length.

To determine the expression profile of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art.

In a preferred embodiment of the invention, the nucleic microarray is an oligonucleotide microarray comprising or consisting of oligonucleotides specific for:
(i) the gene MAPK14;
(ii) the genes MAPK14 and S100A9; or
(iii) the genes MAPK14 and GNLY;
and optionally one or more housekeeping gene(s).

In other methods disclosed in the present description, the nucleic microarray may be an oligonucleotide microarray comprising or consisting of oligonucleotides specific for:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s).

Preferably, the oligonucleotides are about 50 bases in length.

Suitable microarray oligonucleotides specific for any gene of Table 2 may be designed, based on the genomic sequence of each gene (see Table 2 Genbank accession numbers), using any method of microarray oligonucleotide design known in the art. In particular, any available software developed for the design of microarray oligonucleotides may be used, such as, for instance, the OligoArray software (available at http://berry.engin.umich.edu/oligoarray/), the GoArrays software (available at http://www.isima.fr/bioinfo/goarrays/), the Array Designer software (available at http://www.premierbiosoft.com/dnamicroarray/index.html), the Primer3 software (available at http://frodo.wi.mit.edu/primer3/primer3-code.html), or the Promide software (available at http://oligos.molgen.mpg.de/).

In another embodiment, the expression profile is determined by the use of a protein microarray.

In a particular embodiment of a method according to the invention, said method may further comprise determining at least one additional parameter useful for the diagnosis. Such "parameters useful for the diagnosis" are parameters that cannot be used alone for a diagnosis but that have been described as displaying significantly different values between responsive subjects and subjects who are clearly refractory and may thus also be used to refine and/or confirm the diagnosis according to the above described method according to the invention. They may notably include relevant clinical parameters depending on the inflammatory disease. For rheumatoid arthritis (RA), such clinical parameters include an assessment of the subject's pain, duration of morning stiffness, the number of swollen joints, the number of painful joints etc. Preferably, the parameters useful or diagnosis are determined from a non invasive biological sample of the subject. In particular, for RA, they may be selected from standard biological parameters specific for RA. According to the invention, "standard biological parameters specific for RA" are biological parameters usually used by clinicians to monitor the efficacy of a treatment of RA. These standard biological parameters specific for RA or autoimmune diseases usually comprise serum or plasma concentrations of particular proteins which are well known of those skilled in the art. The said standard biological parameters specific for RA can be determined by tests which include the Antinuclear Antibody test (ANA test), C-Reactive Protein test (CRP test), Erythrocyte sedimentation rate (ESR test), Cyclic Citrullinated Peptide Antibody test (CCP test), and the Rheumatoid Factor test. These tests are well known to the person skilled in the art and not be detailed here. They may be used on their own or in combination.

In the invention, such additional parameters may be used to confirm the diagnosis obtained using the expression profile comprising or consisting of:
(i) the gene MAPK14;
(ii) the genes MAPK14 and S100A9; or
(iii) the genes MAPK14 and GNLY;
and optionally one or more housekeeping gene(s).

In other methods disclosed in the present description, such additional parameters may be used to confirm the diagnosis obtained using the expression profile comprising or consisting of:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s).

The invention further concerns the use of a kit comprising at least one reagent for the determination of an expression profile comprising, or consisting of:
(i) the gene MAPK14;
(ii) the genes MAPK14 and S100A9; or
(iii) the genes MAPK14 and GNLY
and optionally one or more housekeeping gene(s), for the *in vitro* diagnosis of a CyTD responsive or non responsive phenotype.

The present description also discloses a kit for the *in vitro* diagnosis of a CyTD responsive or non responsive phenotype, comprising at least one reagent for the determination of an expression profile comprising, or consisting of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s).

By "a reagent for the determination of an expression profile" is meant a reagent which specifically allows for the determination of said expression profile, i.e. a reagent specifically intended for the specific determination of the expression level of the genes comprised in the expression profile. This definition excludes generic reagents useful for the determination of the expression level of any gene, such as Taq polymerase or an amplification buffer, although such reagents may also be included in a kit used according to the invention.

In a preferred embodiment, said kit is dedicated to the *in vitro* diagnosis of a TBA responsive or non responsive phenotype based on expression profiles comprising or consisting of:
(i) the gene MAPK14;
(ii) the genes MAPK14 and S100A9; or
(iii) the genes MAPK14 and GNLY;
and optionally one or more housekeeping gene(s).

In other kits disclosed in the present description, said kit may be dedicated to the *in vitro* diagnosis of a CyTD responsive or non responsive phenotype based on expression profiles comprising or consisting of:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s).

By "dedicated", it is meant that reagents for the determination of an expression profile in the kit essentially consist of reagents for determining the expression level of the above (i), (ii) or (iii) expression profiles, optionally with one or more housekeeping gene(s), and thus comprise a minimum of reagents for determining the expression of other genes than those mentioned in above described (i), (ii) or (iii) expression profiles and housekeeping genes. For instance, a dedicated kit preferably comprises no more than 50, 40, 30, 25, 20, preferably no more than 15, preferably no more than 10, preferably no more than 9, 8, 7, 6, 5, 4, 3, 2, or 1 reagent(s) for determining the expression level of a gene that does not belong to one of the above described (i), (ii) or (iii) expression profiles and that is not a housekeeping gene.

Such a kit used for the in vitro diagnosis of a CyTD responsive or non responsive phenotype may further comprise instructions for determination of the presence or absence of a responsive phenotype.

Such a kit used for the *in vitro* diagnosis of a responsive phenotype may also further comprise at least one reagent for the determining of at least one additional parameter useful for the diagnosis such as standard biological parameters. In particular, the said reagent is useful for performing of any of the following tests: the Antinuclear Antibody test (ANA test), C-Reactive Protein test (CRP test), Erythrocyte sedimentation rate (ESR test), Cyclic Citrullinated Peptide Antibody test (CCP test), and the Rheumatoid Factor test.

In any kit for the *in vitro* diagnosis of a responsive phenotype used according to the invention, the reagent(s) for the determination of an expression profile comprising, or consisting of:
(i) the gene MAPK14;
(ii) the genes MAPK14 and S100A9; or
(iii) the genes MAPK14 and GNLY;
and optionally one or more housekeeping gene(s), preferably include specific amplification primers and/or probes for the specific quantitative amplification of transcripts of:
(i) the gene MAPK14;
(ii) the genes MAPK14 and S100A9; or
(iii) the genes MAPK14 and GNLY;
and optionally one or more housekeeping gene(s), and/or
a nucleic microarray for the detection of:
(i) the gene MAPK14;
(ii) the genes MAPK14 and S100A9; or
(iii) the genes MAPK14 and GNLY;
and optionally one or more housekeeping gene(s).

In any other kit for the *in vitro* diagnosis of a responsive phenotype disclosed in the present description, the reagent(s) for the determination of an expression profile comprising, or consisting of:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s), preferably include specific amplification primers and/or probes for the specific quantitative amplification of transcripts of:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s), and/or
a nucleic microarray for the detection of:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s).

The determination of the expression profile may thus be performed using quantitative PCR and/or a nucleic microarray, preferably an oligonucleotide microarray, and/or protein microarrays.

In addition, the instructions for the determination of the presence or absence of a CyTD (notably TBA) phenotype preferably include at least one reference expression profile, or at least one reference sample for obtaining a reference expression profile. In a preferred embodiment, at least one reference expression profile is a responsive expression profile. Alternatively, at least one reference expression profile may be a non responsive expression profile. More preferably, the determination of the level of responsiveness is carried out by comparison with both responsive and non-responsive expression profiles as described above.

The invention is also directed to the use of a nucleic acid microarray comprising or consisting of nucleic acids specific for:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s), for the *in vitro* diagnosis of a CyTD responsive or non responsive phenotype.

The present description is also directed to a nucleic acid microarray comprising or consisting of nucleic acids specific for:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s).

Said nucleic acid microarray may comprise additional nucleic acids specific for additional genes and optionally one or more housekeeping gene(s), but preferably consists of a maximum of 500, 400, 300, 200 preferably 100, 90, 80, 70 more preferably 60, 50, 45, 40, 35, 30, 25, 20, 15, 10, or even less (for instance 9, 8, 7, 6, 5, 4, 3, 2 or 1) distinct nucleic acids.

In a preferred embodiment, said nucleic acid microarray comprises no more than 50, 40, 30, 25, 20, preferably no more than 15, preferably no more than 10, preferably no more than 9, 8, 7, 6, 5, 4, 3, 2, or 1 distinct nucleic acids specific for a gene that does not belong to one of the above described (i), (ii) or (iii) expression profiles and that is not a housekeeping gene.

Advantageously, said microarray consists of nucleic acids specific for:
(i) the gene MAPK14;
(ii) the genes MAPK14 and S100A9; or
(iii) the genes MAPK14 and GNLY;
and optionally one or more housekeeping gene(s).

Alternatively, a microarray disclosed in the present description may consist of nucleic acids specific for:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s).

In a preferred embodiment, said nucleic acid microarray is an oligonucleotide microarray comprising or consisting of oligonucleotides specific for:
(i) the gene MAPK14;
(ii) the genes MAPK14 and S100A9; or
(iii) the genes MAPK14 and GNLY;
and optionally one or more housekeeping gene(s).

Another nucleic acid microarray disclosed in the present description is an oligonucleotide microarray comprising or consisting of oligonucleotides specific for:
(i) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(ii) Equivalent Expression Profile of anyone of the expression profiles of (i),
and optionally one or more housekeeping gene(s).

Having generally described this invention, a further understanding of characteristics and advantages of the invention can be obtained by reference to certain specific examples and figures which are provided herein for purposes of illustration only.

### DESCRIPTION OF THE FIGURES

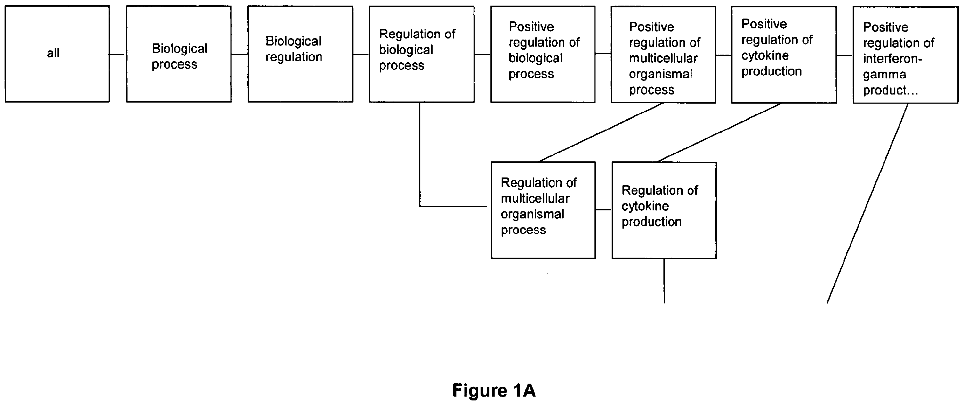

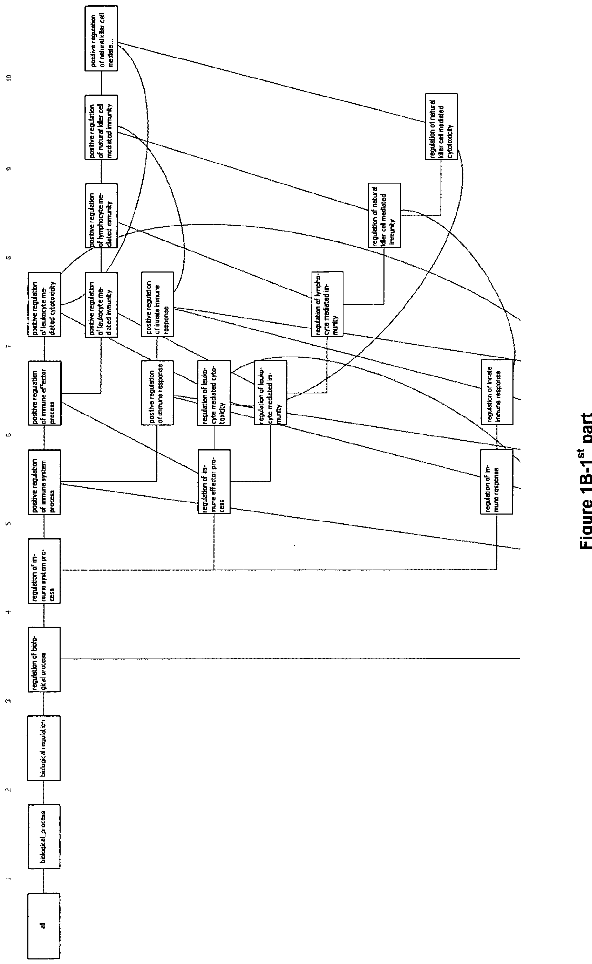

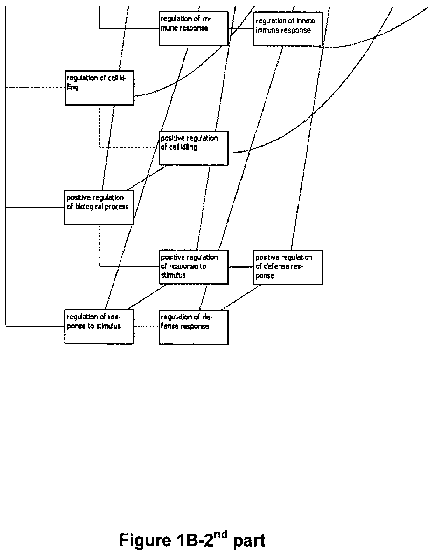

**Figure 1****:** The two most significantly enriched GO terms for the top differentially expressed 8 genes are: "Positive Regulation of interferon gamma production" (A) and "Positive regulation of natural killer cell mediated cytotoxicity" (B) (p-value <5x10⁻³).
**Figure 2****:** Pathway analysis for MAPK14 and S100A9 using PathGen
**Figure 3** **: The top differentially expressed 8 genes issued from Ingenuity® System analysis belong to a single biological network** (Cell-to-cell signalling and interaction, haematological system development and function, immune cell trafficking)

In Figure 3, symbols represent different types of molecules;
- upward triangles: phosphatase,
- downward triangles: kinase,
- horizontal ellipses: transcription regulator,
- vertical ellipses: transmembrane receptor,
- rhombuses: enzyme,
- horizontal rectangles: ligand-dependent nuclear receptor,
- vertical rectangles: g-protein coupled receptor,
- simple circles: other types of molecules,
- double circles: complex group,
Shaded shapes represent the molecules that are part of the 61 list.

The types of line represent the type of interaction:
- plain arrows: means the first molecule directly acts on the second one (such as but not limited to activation)
- dotted arrows: means the first molecule indirectly acts on the second one
- A line ending with a short segment indicates inhibition
- A simple line represents interaction (such a but not limited to protein-protein interaction)

### EXAMPLES

### Example 1: meta-analysis of two datasets (Bienkowska et al.(9) and Julia et al.(8))

### Materials and Methods

In this example, the materials and methodologies used in subsequent examples 1 to 3 are described.

*Data Identification and Data Extraction:* Studies were selected on the basis that they had been performed on RA patients naive to biologies who had started therapy with Infliximab and measurement of their response to treatment was available at 14 or 22 weeks. Large scale gene expression information had to be available at baseline (prior to treatment). Following the steps described in Ramasamy *et al.* (10), we identified four studies that matched our research criteria: Lequerré *et al.* (6), Sekiguchi *et al.* (7), Bienkowska *et al.* (9) and Julià *et al.* (8). The expression data, the phenotypes and the annotation data were all downloaded from GEO (GSE3592, GSE8350, GSE12051 and GSE 15258 respectively).

All four studies identified "Gene expression signatures of response to anti-TNF therapy". Interestingly, however, no two publications used the same approach and this can partly explain the lack of overlap between the reported signatures. To make the four studies more comparable, we contacted the authors to obtain additional individual information such as the DAS28 at baseline and week 14 or week 22 to use a single definition of response (EULAR criteria - with "moderate" and "good" responders considered as responders) or detail of treatment to ensure that only Infliximab-treated patients were analyzed. We therefore reclassified patients as responders based on the EULAR definition at week 14 and week 22 and performed a binary analysis of good and moderate responders versus non-responders. This binary grouping is particularly suited for the identification of non responders. The final dataset is summarized in **Table 1** and was the most homogeneous data we could obtain.

**Table 1: Summary of datasets after alignment of clinical criteria**

| **Study Reference** | **Bienkowska *et al.*** | **Julia *et al.*** | **Lequerre *et al.*** | **Sekiguchi *et al.*** |
|---|---|---|---|---|
| Sample type | whole blood | whole blood | PBMCs | whole blood |
| Microarrays | Affymetrix | Illumina | Custom | Custom |
| Number of probes | 54675 | 17454 | 13824 | 776 |
| Studied response *criteria* | EULAR @ 14wks | | | EULAR @22wks |

| Treatment | Infliximab (mono-therapy) | Infliximab (bi-therapy) | | |
|---|---|---|---|---|
| Number of R/NR | 20/8 | 37/7 | 9/4 | 15/3 |
| Mean age (R/NR) | NA | 52/57 | 56/57 | 55/53 |
| Mean RA duration (R/NR) | NA | 13/19 | 13/10 | 9.3/4.8 |
| Mean DAS28 at baseline (R/NR) | 5.4/5 | 6/5.8 | 6.1/6 | 6.2/6.2 |

Data Quality and Processing: Data from Bienkowska et al. was the only data for which we downloaded the raw .CEL files and processed them using our internal protocols (normalization was performed using GC-RMA in refiner array by GENEDATA Expressionist® (Genedata AG, Basel, Switzerland)). Six chips were flagged with quality issues due to increased distortion. However, due to the limited number of arrays available for Infliximab samples, we included them in our analysis.

Data from Lequerré et al., Julia et al. and Sekiguchi et al. were all downloaded as expression matrices, which correspond to expression values after normalization. The data by Lequerré et al. included technical replicates; we averaged the technical replicates and excluded the control samples from the analysis.

To translate probe information into gene information we used the NCBI's Geneld as provided in the respective annotation files found on GEO. When multiple probes were available, we selected probes with the same NCBI GI number across platforms. When probes differed in terms of GI, they were selected randomly. Therefore for each gene, only one probe contributed to the analysis.

Statistical Analysis: Because of the limited number of probes on the arrays used by Sekiguchi et al. and the major difference introduced by the sample source in the study by Lequerré et al. (PBMCs as opposed to whole blood), we initially performed a meta-analysis only on the Bienkowska et al. and Julia et al. data sets. We then performed the meta-analysis adding the data from Sekiguchi et al. and lastly, the data from Lequerré et al. Thus three meta-analyses were performed (examples 1, 2, and 3 respectively).

The statistical analysis was performed in R using the MetaArray package (D Ghosh and H Choi, 2009). This package implements the latent variable model described in (H Choi, 2005) as well as the integrative correlation (12). For the probability of expression (POE) estimation we used the EM estimation method. Probes were filtered on 100% presence and based on an average correlation >-0.2. Individual probes contribution was estimated using the t-test as implemented in the multtest library in R. We set the threshold for significance at 2.5 based on visual inspection of the distribution plots. The list of probes identified using this approach was then further evaluated in the individual data sets to assess their predictive performance. This was done in Genedata Expressionist® using the Fisher LDA predictor and leave-one-out cross validation. The assessment of biological processes enrichment was done in Genedata Expressionist® (Genedata AG, Basel, Switzerland) using the Gene Ontology Fisher's Exact Test function. The list of significant probes was compared to the list of probes that were tested (the common probes). Pathway analysis was performed using PathGen (available at http://dna.cs.byu.edu/pathgen).

### Results

The initial meta-analysis of the two datasets (i.e. Bienkowska *et al.* and Julia *et al*.) was performed on 4022 probes. Some heterogeneity between the two studies could be due to remaining clinical differences such as severity of disease, use of mono- versus bi-therapy or to probe differences due to the different platforms used (Affymetrix versus Illumina microarrays). Based on the t-test of the POE from the merged datasets, we ranked the genes based on their significance. **Table 2** provides the gene symbol and direction of the 61 most differentially expressed genes between responders and non responders. Interestingly this gene list overlaps only by two genes with the genes identified by Julia *et al.* and by one gene identified by Bienkowska *et al.* To assess the discriminatory performance of a group of genes we selected the top 8 genes and applied them to the individual data sets. The performance can be found in **Table 3.** This eight gene set performs extremely well in the dataset of Julia *et al.:* by LOO, 7 out of 44 samples were misclassified giving an overall error rate of 84%, the PPV was as high as 97%.

**Table 2: Ranked list of first 61 differentially expressed probes. The top genes are the most significant ones.**

| **Gene Symbol** | **Name** | **Accession Nb** | **Direction** |
|---|---|---|---|
| **IL2RB** | interleukin 2 receptor, beta | NM_000878.2 (SEQ ID NO :1) | Increased |
| **S100A9** | S100 calcium binding protein A9 | NM_002965.3 (SEQ ID NO :2) | Decreased |
| **KLRK1** | killer cell lectin-like receptor subfamily K, member 1 | NM_007360.2 (SEQ ID NO :3) | Increased |
| **HCK** | hemopoietic cell kinase | NM_001172129.1 (SEQ ID NO :4) | Decreased |
| **GNLY** | granulysin | NM_006433.3 (SEQ ID NO :5) | Increased |
| **CTSZ** | cathepsin Z | NM_001336.3 (SEQ ID NO :6) | Decreased |
| **ARF5** | ADP-ribosylation factor 5 | NM_001142272.1 (SEQ ID NO :7) | Decreased |
| **UTP14C** | U3 small nucleolar ribonucleoprotein, homolog C | NM_021645.5 (SEQ ID NO :8) | Increased |
| DRAP1 | DR1-associated protein 1 (negative cofactor 2 alpha) | NM_006442.3 (SEQ ID NO :9) | Decreased |
| SHKBP1 | SH3KBP1 binding protein 1 | NM_138392.3 (SEQ ID NO :10) | Decreased |
| IARS | isoleucyl-tRNA synthetase | NM_002161.4 (SEQ ID NO :11) | Increased |
| ATP6V1E1 | ATPase, H⁺ transporting, lysosomal | NM_001039367.1 | Decreased |
| | 31kDa, V1 subunit E1 | (SEQ ID NO :12) | |
| RPP21 | Ribonuclease P/MRP 21kDa subunit | NM_024839.1 (SEQ ID NO :13) | Increased |
| SFRS5 | splicing factor, arginine/serine-rich 5 | NM_001039465.1 (SEQ ID NO :14) | Increased |
| LOC285636 | chromosome 5 open reading frame 51 | NM_175921.4 (SEQ ID NO :15) | Increased |
| NTNG2 | netrin G2 | NM_032536.2 (SEQ ID NO :16) | Decreased |
| ASF1A | ASF1 anti-silencing function 1 homolog A | NM_014034.2 (SEQ ID NO :17) | Increased |
| DYSF | dysferlin | NM_001130455.1 (SEQ ID NO :18) | Decreased |
| EIF4H | eukaryotic translation initiation factor 4H | NM_031992.1 (SEQ ID NO :19) | Increased |
| USP39 | ubiquitin specific peptidase 39 | NM_006590.2 (SEQ ID NO :20) | Increased |
| ANAPC4 | Anaphase promoting complex subunit 4 | NM_013367.2 (SEQ ID NO :21) | Increased |
| NUDT15 | nudix | NM_018283.1 (SEQ ID NO :22) | Decreased |
| TRAPPC3 | trafficking protein particle complex 3 | NM_014408.3 (SEQ ID NO :23) | Increased |
| SRPRB | signal recognition particle receptor, B subunit | NM_021203.3 (SEQ ID NO :24) | Increased |
| UBE2Z | ubiquitin-conjugating enzyme E2Z | NM_023079.3 (SEQ ID NO :25) | Increased |
| RCN2 | reticulocalbin 2 | NM_002902.2 (SEQ ID NO :26) | Increased |
| RAB9A | member RAS oncogene family | NM_004251.3 (SEQ ID NO :27) | Increased |
| SBDS | Shwachman-Bodian-Diamond syndrome | NM_023248.1 (SEQ ID NO :28) | Increased |
| SYNE1 | spectrin repeat containing, nuclear envelope 1 | NM_015293.2 (SEQ ID NO :29) | Increased |
| | | NM_033071.2 | |
| | | (SEQ ID NO :30) | |
| | | NM_133650.2 (SEQ ID NO :31) | |
| | | NM_182961.2 (SEQ ID NO :32) | |
| PGLYRP1 | peptidoglycan recognition protein 1 | NM_005091.1 (SEQ ID NO :33) | Decreased |
| FLJ10769 | carbohydrate kinase domain containing | NM_018210.2 (SEQ ID NO :34) | Increased |
| MFAP1 | microfibrillar-associated protein 1 | NM_005926.2 (SEQ ID NO :35) | Increased |
| HK3 | hexokinase 3 | NM_002115.2 (SEQ ID NO :36) | Decreased |
| MLLT11 | myeloid/lymphoid or mixed-lineage leukemia (trithorax homolog, Drosophila); translocated to, 11 | NM_006818.3 (SEQ ID NO :37) | Increased |
| GPR137B | G protein-coupled receptor 137B | NM_003272.3 (SEQ ID NO :38) | Increased |
| CD63 | CD63 | NM_001040034.1 (SEQ ID NO :39) | Decreased |
| | | NM_001780.4 (SEQ ID NO :40) | |
| TARSL2 | threonyl-tRNA synthetase-like 2 | NM_152334.2 (SEQ ID NO :41) | Increased |
| TTYH2 | tweety homolog 2 | NM_032646.5 (SEQ ID NO :42) | Increased |
| BRP44L | brain protein 44-like | NM_016098.2 (SEQ ID NO :43) | Increased |
| MTERFD1 | MTERF domain containing 1 | NM_015942.3 (SEQ ID NO :44) | Increased |
| CASP5 | caspase 5 | NM_001136111.1 (SEQ ID NO :45) | Decreased |
| RIOK1 | RIO kinase 1 | NM_153005.1 (SEQ ID NO :46) | Increased |
| | | NM_031480.2 (SEQ ID NO :47) | |
| CLN5 | ceroid-lipofuscinosis, neuronal 5 | NM_006493.2 (SEQ ID NO :48) | Increased |
| ZC3H7A | zinc finger CCCH-type containing 7A | NM_014153.2 (SEQ ID NO :49) | Increased |
| NARG2 | NMDA receptor regulated 2 | NM_024611.4 (SEQ ID NO :50) | Increased |
| | | NM_001018089.1 (SEQ ID NO :51) | |
| TMEM85 | transmembrane protein 85 | NM_016454.2 (SEQ ID NO :52) | Increased |
| COBRA1 | cofactor of BRCA1 | NM_015456.3 (SEQ ID NO :53) | Increased |
| KEAP1 | kelch-like ECH-associated protein 1 | NM_012289.3 (SEQ ID NO :54) | Decreased |
| | | NM_203500.1 (SEQ ID NO :55) | |
| LRCH3 | leucine-rich repeats and calponin homology (CH) domain containing 3 | NM_032773.2 (SEQ ID NO :56) | Increased |
| C19orf12 | chromosome 19 open reading frame 12 | NM_001031726.2 (SEQ ID NO :57) | Increased |
| | | NM_031448.3 (SEQ ID NO :58) | |
| PIGC | phosphatidylinositol glycan anchor biosynthesis, class C | NM_002642.3 (SEQ ID NO :59) | Increased |
| | | NM_153747.1 (SEQ ID NO :60) | |
| DGAT2 | diacylglycerol O-acyltransferase 2 | NM_032564.3 (SEQ ID NO :61) | Decreased |
| DDX56 | DEAD box polypeptide 56 | NM_019082.2 (SEQ ID NO :62) | Increased |
| NIP30 | NEFA-interacting nuclear protein | NM_024946.2 (SEQ ID NO :63) | Increased |
| MAPK14 | mitogen-activated protein kinase 14 | NM_001315.2 (SEQ ID NO :64) | Decreased |
| | | NM_139012.2 (SEQ ID NO :65) | |
| | | NM_139013.2 (SEQ ID NO :66) | |
| | | NM_139014.2 (SEQ ID NO :67) | |
| SLC39A10 | solute carrier family 39 (zinc transporter), member 10 | NM_001127257.1 (SEQ ID NO :68) | Increased |
| | | NM_020342.2 (SEQ ID NO :69) | |
| ADH5 | alcohol dehydrogenase 5 | NM_000671.3 (SEQ ID NO :70) | Increased |
| KIAA0947 | KIAA0947 | NM_015325.1 (SEQ ID NO :71) | Increased |
| GLTSCR2 | glioma tumor suppressor candidate region gene 2 | NM_015710.4 (SEQ ID NO :72) | Increased |
| DNAJA3 | DnaJ (Hsp40) homolog, subfamily A, member 3 | NM_001135110.1 (SEQ ID NO :73) | Increased |
| | | NM_005147.4 (SEQ ID NO :74) | |
| FAM134C | family with sequence similarity 134, member C | NM_178126.3 (SEQ ID NO :75) | Increased |

**Table 3: Discriminatory performance of the gene set based on the top 8 genes in the data of Julià et al.**

| | True R (Julià *et al*.) | True NR (*Julià et al*.) | Sum | Correct [%] |
|---|---|---|---|---|
| Predicted R (Julià *et al.)* | 31 | 1 | 32 | 96.88 |
| Predicted NR (Julià *et al.)* | 6 | 6 | 12 | 50 |
| Sum | 37 | 7 | - | - |
| Correct [%] | 83.78 | 85.71 | - | - |

The top 8 most significantly differentially expressed genes (IL2RB, S100A9, KLRK1, HCK, GNLY, CTSZ, ARF5, and UTP14C) have all been associated to the same biological network ("Cell-to-cell signalling and interaction, haematological system development and function, immune cell trafficking") by an Ingenuity® Systems analysis (Figure 3).

Furthermore, the top 5 most significantly differentially expressed genes (IL2RB, S100A9, KLRK1, HCK, GNLY) are particularly relevant to RA physiopathology.

### IL2RB (Interleukin-2 receptor subunit beta)

IL2RB gene encodes the beta subunit of the IL2R, which is present in the moderate and high affinity forms of the receptor required for signal transduction from IL2. Several studies showed that SNPs in IL2RB gene (especially rs743777) are significantly associated with RA (13).

It has been recently proposed that thymic selection of an auto-reactive T-cell repertoire is an important risk factor for rheumatoid arthritis (RA) (14).

The IL2RB gene is thus an attractive candidate for RA because of the key role played by IL2 in T cell activation and regulation.

### S100A9 (S100 calcium-binding protein A9)

S100A9 gene codes for the S100A9 protein also called myeloid-related protein 14 (MRP14) or calgranulin B that belongs to the S100 family of proteins. S100 proteins are known to be associated with several pathological conditions such as cystic fibrosis (15), cancer (16) and inflammatory diseases (17).

S100A9 has also been shown to induce neutrophil degranulation (which may account for the tissue damage in RA) via a MAPK-dependent mechanism (18).

A recently published study showed that S100 proteins are associated with RA inflammation and auto-antibody production (19).

### KLRK1

KLRK1 gene encodes the natural killer group 2D (NKG2D) protein which belongs to the family of activating NK cell receptors (NKRs).

In RA patients, a significant proportion of CD4⁺CD28⁻ T cells were shown to express NKG2D which stimulated auto-reactive responses against RA synoviocytes (20).

### HCK

HCK gene encodes the tyrosine-protein kinase HCK (hematopoietic cell kinase) predominantly expressed in hematopoietic cell types.

HCK has been shown to be involved in functional pro-inflammatory responses such as the FcR-mediated respiratory burst (21), neutrophil migration (22) and neutrophil degranulation (23) that play important roles in the early phases of RA physiopathology Furthermore, it has also been shown that HCK mediates IL-2 signaling in human monocytes (24).

### GNLY

GNLY gene codes for Granulysin, a saponin-like protein present in cytotoxic granules of cytolytic T cells and NK cells and released upon antigen stimulation.

Granulysin is a chemoattractant for T lymphocytes, monocytes and other inflammatory cells and induces the expression of a number of cytokines, including RANTES, MCP-1, MCP-3, MIP-1α, IL-10, IL-1, IL-6 and IFN-α (25).

Granulysin is also involved in several diseases including infection, cancer, transplantation, autoimmunity, skin and reproductive maladies (26).

### Example 2: meta-analysis of three datasets (Bienkowska et al. Julia et al, and Sekiguchi et al.)

A second meta-analysis was performed after merging the dataset of Sekiguchi *et al.* This dramatically reduced the number of genes being tested, bringing it down to 290. Keeping the same threshold for the test statistic as previously (2.5) only three of the 61 previously described (see Example 1) genes were significant: IL2RB, S100A9 and CASP5. However, these genes widen the prediction of the Infliximab treatment to a 22 weeks follow-up. Besides the relationship between IL2RB and S100A9 and physiopathology of RA (see above), CASP5 gene also has a relevant significance in this auto-immune disorder.

### CASP5

CASP5 gene encodes the Caspase 5 enzyme that proteolytically cleaves other proteins at an aspartic acid residue. It is an inflammatory caspase that plays a role in the immune system (27) and in the complex process of cellular apoptosis (28).

The aberrant decrease in apoptosis or increased cell cycle activity of fibroblast-like or macrophage-like synoviocytes is responsible for the synovial hyperplasia and contributes to the destruction of cartilage and bone in RA patients thus suggesting a potential role of apoptosis-related caspases in the physiopathology of RA (29).

### Example 3: meta-analysis of four datasets (Bienkowska et al. Julia et al, Sekiguchi et al., and Leguerré et al.)

Further adding the data by Lequerré *et al.,* and thus leading to a total number of 103 samples analysed in 179 genes identified only two of the 61 previously described (see Example 1) genes that are significantly increased in non responders: MAPK14 et S100A9 (Figure 2). The combination of these two genes is thus able to predict response to Infliximab treatment on a biological sample (whole blood or PBMCs) at week 14 or week 22.

### MAPK14

MAPK14 gene encodes the mitogen-activated protein kinase 14, a member of the MAP kinase family. MAP kinases act as an integration point for multiple biochemical signals, and are involved in a wide variety of cellular processes such as proliferation, differentiation, transcription regulation and development. The MAP kinase pathway is particularly influencing the biosynthesis of pro-inflammatory cytokines TNFα and IL-1β both at a translational and a transcriptional level (30). This pathway might thus be a key anti-inflammatory target for RA treatment.

### Example 4: Quantitative PCR confirmation of the efficiency of signatures obtained from meta-analysis of microarray data

### Patients and methods

### Patients

Peripheral blood samples from 40 patients, with rheumatoid arthritis and classified as responders and non responders to Infliximab treatment at week 14 according to the EULAR definition of response (Good and moderate responders were classified as responders and poor responders were classified as non responders), were identified and analyzed by RT-PCR. The clinical and demographic variables of the patients are summarized in Table 4.

**Table 4: Patient clinical characteristics**

| | |
|---|---|
| Number of Responders | 34 |
| Number of Non Responders | 6 |
| Average DAS28 value at baseline | 6 |
| Average DAS28 value at week 14 | 4 |
| Number of Males | 5 |
| Number of Females | 35 |
| Average ESR at baseline | 40 |
| Average CRP at baseline | 2 |

### Methods

Peripheral blood was collected into RNA PAXgene® tubes (PreAnalytix, Switzerland) and RNA from whole blood was extracted according to the methods described in Julia *et al.* (8). Samples underwent quality control and were dried using speed vacuum prior to the RT and PCR steps. The real-time PCR reactions was carried out on a total of 40 samples using the TaqMan Universal PCR Master Mix (Applied Biosystems). 6 µL cDNA was added to a 9 µL PCR mixture containing 7.5 µL TaqMan Universal PCR Master Mix, 0.75 µL of a 20X mixture of probe and primers and 0.75µl water. The reaction consisted of one initiating step of 2 min at 50 deg. C, followed by 10 min at 95 deg. C, and 40 cycles of amplification including 15 sec at 95 deg. C and 1 min at 60 deg. C. The reaction and data acquisition was performed using the ABI 7900HT Fast Real-Time PCR System (Applied Biosystems). The "ΔΔCT method" was used as a measure of gene expression. Post extraction steps and analyses were carried out at TcLand Expression ISO13485 laboratory.

For 7 out of the 11 genes, probes were custom designed and the sequence information can be found in Table 5a. For 3 out of the 11 genes, probes were ordered from Applied Biosystems directly and are referenced in Table 5b.

**Table 5a : Sequence information for the 7probes that were custom designed.**

| Gene Symbol | RefSeq | Forward Primer Sequence | Probe Sequence | Reverse Primer Sequence | Amplicon Sequence |
|---|---|---|---|---|---|
| **ARF5** | NM_001 662.3 | | | | |
| **CTSZ** | NM_001 336.3 | | | | |
| **HCK** | NM_002 110.3 | | | | |
| **KLRK1** | NM_007 360.2 | | | | |
| **S100A8** | NM_002 964.3 | | | | |
| **S100A9** | NM_002 965.3 | | | | |
| **UTP14C** | NM_021 645.5 | | | | |

**Table 5b: Probe information for the three genes for which probes were ordered from Applied Biosystems repositoryResults**

| Gene Symbol | Probe ID | Context Sequence |
|---|---|---|
| GNLY | Hs01120727_m1 | TACCTTCTACAGGTCCCCTCTGAGC (SEQ ID NO :104) |
| MAPK14 | Hs01051153_m1 | TGTTTCCTGGTACAGACCATATTAA (SEQ ID NO :105) |
| IL2RB | HS00386697 | GAACACCGGGCCATGGCTGAAGAAG (SEQ ID NO :106) |

Following the results from the meta-analysis, Taqman probes were ordered through applied or designed in house. After our internal quality control steps 8 out of the 11 genes were tested on the RA samples. The 40 samples, that represent a subset of the original samples used for microarray analysis in the study of Julia *et al*., were analyzed by RT-PCR following our internal protocols. The following statistical analysis has been performed: Identification of individually differentially expressed genes between the two groups of responders versus non responders (Table 6). The selection criteria used was a significant t-test of at least 0.05. Additionally the discriminatory power of the combination of 1, 2, 5 and 8 genes was assessed using a logistic regression and classification rates were provided (Table 7).

**Table 6: P-values and ΔΔCT for Responder and Non Responder groups. Genes underlined and in italic were confirmed as being discriminant (P-value<0.05).**

| **Gene Symbol** | **P-Value** | **Average ΔΔCT value in non responder group (ΔΔCT_{NonRESP})** | **Average ΔΔCT value in responder group (ΔΔCT_{RESP})** |
|---|---|---|---|
| ARF5 | 0.9284 | 0.3438 | 0.3557 |
| *CTSZ* | 0.4426 | -0.6069 | -0.6848 |
| *GNLY* | *0.0017* | -4.253 | -3.2036 |
| *HCK* | 0.1963 | -3.3665 | -3.5577 |
| *IL2RB* | *0.0282* | -3.527 | -3.1646 |
| *KLRK1* | 0.0829 | -1.5087 | -0.9914 |
| *MAPK14* | *0.0003* | 1.364 | 0.831 |
| *S100A8* | 0.1768 | -4.8138 | -5.2706 |
| S100A9 | 0.0642 | -5.1389 | -5.6202 |

**Table 7: Classification performance using logistic regression of claimed list gene combinations.**

| **Model** | **Sensitivity** | **Specificity** | **Error Rate** |
|---|---|---|---|
| S100A9 | 0% | 100% | 16% |
| MAPK14, S100A9 | 83% | 96% | 5% |
| IL2RB, S100A9, KLRK1,HCK and GNLY | 83% | 96% | 6% |
| IL2RB, S100A9, KLRK1, HCK, GNLY, CTSZ, ARF5 and UTP14C | 100% | 100% | 0% |

### Conclusion

The above results confirm that the particular combinations (signatures) of genes identified as predicting the response to infliximab of RA-suffering patients based on meta-analysis of microarray data obtained in several independent studies are actually efficient (high sensititity and specificity, low error rate) for predicting the response to influximab treatment of a validation group of RA-suffering patients.

### Example 5: Prediction of the response to Infliximab at week 14 of included patients using different sub-combinations of genes

### Patients

The same patients as in Example 4 have been studied.

### Results

The identification of an optimal subgroup of genes that together best discriminate between the responders and non responders was then performed. A logistic regression was applied and the classification error was used to identify the optimal subset of genes.

The most discriminating gene on its own is MAPK14 (Table 7), the optimal two-gene combination is MAPK14 with GNLY.

**Table 7: Classification performance using logistic regression of claimed list gene combinations.**

| **Model** | **Sensitivity** | **Specificity** | **Error Rate** |
|---|---|---|---|
| MAPK14 | 50% | 97% | 11% |
| MAPK14, GNLY | 100% | 100% | 0% |

### Conclusion

These results show that MAPK14, already identified as significant in Example 3 by meta-analysis of microarray data obtained from 4 independent studies, is actually highly predictive, even alone, of response to Infliximab treatment in RA-suffering patients. Moreover, in addition to the combination of genes S100A9 and MAPK14, already identified as useful in Example 3 and confirmed by qPCR experiments in Example 4, the combination of genes MAPK14 and GNLY (GNLY being already identified as significant in Example 1) is also highly predictive of response to Infliximab in RA-suffering patients, all patients tested being correctly classified using this combination.

Globally, the results presented in Examples 1 to 5 support the tight correlation of two individual genes, MAPK14 and S100A9, or equivalent correlated genes, with the Infliximab responsive or non-responsive phenotype of RA-suffering subjects. In addition, combination of one or both of these genes with a small number of other genes found associated to Infliximab responsive or non-responsive phenotype of RA-suffering subjects (notably the 61 genes of Table 2, and more particularly genes GNLY, IL2RB, S100A9, KLRK1, HCK, CTSZ, ARF5, and UTP14C), permit a highly sensitive and specific (very low error rate) prediction of the Infliximab responsive or non-responsive phenotype of RA-suffering subjects.

Finally, we note that genes found to be highly correlated to the Infliximab responsive or non-responsive phenotype of RA-suffering subjects are not genes that might be involved in the metabolism of Infliximab, but rather genes that may be associated to the disease or its underlying dysfunctions themselves. In particular, many genes found to be highly correlated to the Infliximab responsive or non-responsive phenotype of RA-suffering subjects are known to be involved in inflammatory or immune processes. This clearly gives a rational for the extension of the methods of the invention for the prediction of the TBA responsive or non-responsive phenotype of subjects suffering from other inflammatory diseases, in particular those involving pathogenic TNFα secretion and even more particularly those for which TBA have been approved or have been shown in preliminary studies to be useful.

### Example 6: Correlation between gene expression and inflammatory response measured by DAS28, CRP and ESR in rheumatoid arthritis patients

Clinical parameters measuring inflammation at baseline such as DAS28, C-reactive protein (CRP) and erythrocyte sedimentation rate (ESR) do not discriminate between responders and non responders (respective p-values for t-test are 0.7792, 0.4839 and 0.1755). To assess whether the gene expression levels are correlated to the clinical parameters of inflammation, a correlation coefficient was computed (Table 8). Table 8 indicates that the genes in the signature are not correlated to the clinical parameters and thus add independent information. Only the gene expression levels of IL2RB correlate negatively to CRP (correlation coefficient = -0.35, p-value 0.03), please note this p-value is uncorrected for multiple testing.

**Table 8: Correlation coefficient of gene expression versus clinical parameters indicate lack of correlation between gene expression levels and clinical parameters of inflammation.**

| | **DAS28_w0** | **CRP** | **ESR** |
|---|---|---|---|
| **MAPK14** | 0.06 | 0.17 | 0.17 |
| **S100A9** | -0.07 | 0.09 | 0 |
| **S100A8** | 0.1 | 0.09 | 0.13 |
| **IL2RB** | -0.19 | -0.35 | -0.25 |
| **KLRK1** | -0.17 | -0.19 | -0.15 |
| **HCK** | -0.23 | -0.09 | -0.21 |
| **GNLY** | -0.17 | -0.25 | -0.23 |
| **CTSZ** | 0.13 | 0.17 | -0.05 |
| **ARF5** | 0 | 0.1 | -0.08 |
| **UTP14C** | 0.11 | -0.08 | 0.16 |

These results support the claim that our gene expression signature provides independant discriminatory power over clinical variables.

### REFERENCES

1. Lee DM, Weinblatt ME. Rheumatoid arthritis. Lancet. 2001 Sep 15;358(9285):903-11.
2. Choy EH, Panayi GS. Cytokine pathways and joint inflammation in rheumatoid arthritis. N Engl J Med. 2001 Mar 22;344(12):907-16.
3. Kooloos WM, de Jong DJ, Huizinga TW, Guchelaar HJ. Potential role of pharmacogenetics in anti-TNF treatment of rheumatoid arthritis and Crohn's disease. Drug Discovery Today. 2007;12(3-4):125-31.
4. Isaacs JD. Antibody engineering to develop new antirheumatic therapies. Arthritis Res Ther. 2009;11 (3):225.
5. Hetland ML, Christensen IJ, Tarp U, Dreyer L, Hansen A, Hansen IT, et al. Direct comparison of treatment responses, remission rates, and drug adherence in patients with rheumatoid arthritis treated with adalimumab, etanercept, or infliximab: results from eight years of surveillance of clinical practice in the nationwide Danish DANBIO registry. Arthritis Rheum. 2010 Jan;62(1):22-32.
6. Lequerre T, Gauthier-Jauneau AC, Bansard C, Derambure C, Hiron M, Vittecoq O, et al. Gene profiling in white blood cells predicts infliximab responsiveness in rheumatoid arthritis. Arthritis Res Ther. 2006;8(4):R105.
7. Sekiguchi N, Kawauchi S, Furuya T, Inaba N, Matsuda K, Ando S, et al. Messenger ribonucleic acid expression profile in peripheral blood cells from RA patients following treatment with an anti-TNF-alpha monoclonal antibody, infliximab. Rheumatology (Oxford). 2008 Jun;47(6):780-8.
8. Julia A, Erra A, Palacio C, Tomas C, Sans X, Barcelo P, et al. An eight-gene blood expression profile predicts the response to infliximab in rheumatoid arthritis. PLoS One. 2009;4(10):e7556.
9. Bienkowska JR, Dalgin GS, Batliwalla F, Allaire N, Roubenoff R, Gregersen PK, et al. Convergent Random Forest predictor: methodology for predicting drug response from genome-scale data applied to anti-TNF response. Genomics. 2009 Dec;94(6):423-32.
10. Ramasamy A, Mondry A, Holmes CC, Altman DG. Key issues in conducting a meta-analysis of gene expression microarray datasets. PLoS Med. 2008 Sep 30;5(9):e184.
11. Arnett FC, Edworthy SM, Bloch DA, McShane DJ, Fries JF, Cooper NS, et al. The American Rheumatism Association 1987 revised criteria for the classification of rheumatoid arthritis. Arthritis Rheum. 1988 Mar;31(3):315-24.
12. Parmigiani G, Garrett-Mayer ES, Anbazhagan R, Gabrielson E. A cross-study comparison of gene expression studies for the molecular classification of lung cancer. Clin Cancer Res. 2004 May 1;10(9):2922-7.
13. Barton A, Thomson W, Ke X, Eyre S, Hinks A, Bowes J, et al. Rheumatoid arthritis susceptibility loci at chromosomes 10p15, 12q13 and 22q13. Nat Genet. 2008 Oct;40(10):1156-9.
14. Goronzy JJ, Weyand CM. Developments in the scientific understanding of rheumatoid arthritis. Arthritis Res Ther. 2009;11(5):249.
15. Lorenz E, Muhlebach MS, Tessier PA, Alexis NE, Duncan Hite R, Seeds MC, et al. Different expression ratio of S100A8/A9 and S100A12 in acute and chronic lung diseases. Respir Med. 2008 Apr;102(4):567-73.
16. Cheng P, Corzo CA, Luetteke N, Yu B, Nagaraj S, Bui MM, et al. Inhibition of dendritic cell differentiation and accumulation of myeloid-derived suppressor cells in cancer is regulated by S100A9 protein. J Exp Med. 2008 Sep 29;205(10):2235-49.
17. Lim SY, Raftery M, Goyette J, Hsu K, Geczy CL. Oxidative modifications of S100 proteins: functional regulation by redox. J Leukoc Biol. 2009 86(3): 577-87.
18. Simard JC, Girard D, Tessier PA. Induction of neutrophil degranulation by S100A9 via a MAPK-dependent mechanism. J Leukoc Biol. 2010 Jan 26.
19. Chen YS, Yan W, Geczy CL, Brown MA, Thomas R. Serum levels of soluble receptor for advanced glycation end products and of S100 proteins are associated with inflammatory, autoantibody, and classical risk markers of joint and vascular damage in rheumatoid arthritis. Arthritis Res Ther. 2009;11(2):R39.
20. Groh V, Bruhl A, El-Gabalawy H, Nelson JL, Spies T. Stimulation of T cell autoreactivity by anomalous expression of NKG2D and its MIC ligands in rheumatoid arthritis. Proc Natl Acad Sci USA. 2003 Aug 5;100(16):9452-7.
21. Paul R, Obermaier B, Van Ziffle J, Angele B, Pfister HW, Lowell CA, et al. Myeloid Src kinases regulate phagocytosis and oxidative burst in pneumococcal meningitis by activating NADPH oxidase. J Leukoc Biol. 2008 Oct;84(4):1141-50.
22. Fumagalli L, Zhang H, Baruzzi A, Lowell CA, Berton G. The Src family kinases Hck and Fgr regulate neutrophil responses to N-formyl-methionyl-leucyl-phenylalanine. J Immunol. 2007 Mar 15;178(6):3874-85.
23. Mocsai A, Ligeti E, Lowell CA, Berton G. Adhesion-dependent degranulation of neutrophils requires the Src family kinases Fgr and Hck. J Immunol. 1999 Jan 15;162(2):1120-6.
24. Bosco MC, Curiel RE, Zea AH, Malabarba MG, Ortaldo JR, Espinoza-Delgado I. IL-2 signaling in human monocytes involves the phosphorylation and activation of p59hck. J Immunol. 2000 May 1;164(9):4575-85.
25. Deng A, Chen S, Li Q, Lyu SC, Clayberger C, Krensky AM. Granulysin, a cytolytic molecule, is also a chemoattractant and proinflammatory activator. J Immunol. 2005 May 1;174(9):5243-8.
26. Krensky AM, Clayberger C. Biology and clinical relevance of granulysin. Tissue Antigens. 2009 Mar;73(3):193-8.
27. Martinon F, Tschopp J. Inflammatory caspases and inflammasomes: master switches of inflammation. Cell Death Differ. 2007 Jan;14(1):10-22.
28. Kurokawa M, Kornbluth S. Caspases and kinases in a death grip. Cell. 2009 Sep 4;138(5):838-54.
29. Morel J, Audo R, Hahne M, Combe B. Tumor necrosis factor-related apoptosis-inducing ligand (TRAIL) induces rheumatoid arthritis synovial fibroblast proliferation through mitogen-activated protein kinases and phosphatidylinositol 3-kinase/Akt. J Biol Chem. 2005 Apr 22;280(16):15709-18.
30. Korb A, Tohidast-Akrad M, Cetin E, Axmann R, Smolen J, Schett G. Differential tissue expression and activation of p38 MAPK alpha, beta, gamma, and delta isoforms in rheumatoid arthritis. Arthritis Rheum. 2006 Sep;54(9):2745-
31. Fransen J, van Riel PLCM. The Disease Activity Score and the EULAR response criteria. Clin Exp Rheumatol. 2005 23(5 Suppl 39): S93-9.
32. Lorenz HM et al. Arthritis Res. 2002;4 Suppl 3:S17-24
33. Atzeni F et al. Autoimmun Rev. 2007 Sep;6(8):529-36.
34. WO2010/034864.

## Claims

1. A method for the *in vitro* diagnosis or prognosis of a TNF-α blocking agent (TBA) responding or non-responding phenotype, comprising:
(a) determining from a blood sample of a subject suffering from an inflammatory disease an expression profile comprising or consisting of:
(i) the gene MAPK14;
(ii) the genes MAPK14 and S100A9; or
(iii) the genes MAPK14 and GNLY;
and optionally one or more housekeeping gene(s)
(b) comparing the obtained expression profile with at least one reference responding expression profile and at least one reference non-responding expression profile using statistical models or machine learning technologies, and
(c) determining the responding or non-responding phenotype from said comparison.

2. A method for designing a TBA treatment for a subject suffering from an inflammatory disease, said method comprising:
(a) determining from a blood sample of said subject an expression profile comprising or consisting of:
(i) the gene MAPK14;
(ii) the genes MAPK14 and S100A9; or
(iii) the genes MAPK14 and GNLY;
and optionally one or more housekeeping gene(s)
(b) comparing the obtained expression profile with at least one reference responding expression profile and at least one reference non-responding expression profile using statistical models or machine learning technologies,
(c) determining the responding or non-responding phenotype of said subject from said comparison, and
(d) designing a dose of TBA treatment according to the said identified responding or non-responding phenotype.

3. The method of claim 1 or claim 2, wherein the expression profile of step (a) comprises or consists of the genes MAPK14 and GNLY.

4. The method of anyone of claims 1 to 3, wherein the expression profile is determined by measuring the amount of nucleic acid transcripts of said gene(s).

5. The method of claim 4, wherein the expression profile is determined using quantitative PCR or an oligonucleotide microarray.

6. The method of anyone of claims claims 1 to 3, wherein the expression profile is determined using a genomic microarray or a proteic microarray.

7. The method of anyone of claims 1 to 6, wherein the expression profile of step (a) is determined at 14 or 22 weeks after the beginning of the CyTD treatment.

8. The method of anyone of claims 1 to 7, wherein said TBA is selected in the group consisting of Etanercept (Enbrel®), Infliximab (Remicade®), Adalimumab (Humira®), and Certolizumab pegol (Cimzia®).

9. The method according to anyone of claims 1 to 8, wherein said inflammatory disease is selected from the group consisting of rheumatoid arthritis (RA), Crohn's disease, ankylosing spondylitis, psoriatic arthritis, plaque psoriasis, ulcerative colitis, vasculitis; Wegener's granulomatosis; sarcoidosis; adult-onset Still's disease, polymyositis/dermatomyositis, and systemic lupus erythematosus (SLE).

10. The method of claim 9, wherein said inflammatory disease is rheumatoid arthritis.

11. The method according to claim 10, further comprising determining at least one additional parameter, said additional parameter being determined by a test selected from the Antinuclear Antibody test (ANA test), C-Reactive Protein test (CRP test), Cyclic Citrullinated Peptide Antibody test (CCP test), and the Rheumatoid Factor test.

12. Use of a kit comprising at least one reagent for the determination of an expression profile comprising or consisting of:
(i) the gene MAPK14;
(ii) the genes MAPK14 and S100A9; or
(iii) the genes MAPK14 and GNLY;
and optionally one or more housekeeping gene(s),
for the *in vitro* diagnosis of a TBA responding or non-responding phenotype.

13. The use of claim 12, wherein said kit further comprises at least one reagent for determining at least one additional parameter, said reagent selected from the Antinuclear Antibody test (ANA test), C-Reactive Protein test (CRP test), Cyclic Citrullinated Peptide Antibody test (CCP test), and the Rheumatoid Factor test.

14. Use of a nucleic acid microarray, comprising nucleic acids specific for:
(i) the gene MAPK14;
(ii) the genes MAPK14 and S100A9; or
(iii) the genes MAPK14 and GNLY;
and optionally one or more housekeeping gene(s),
for the *in vitro* diagnosis of a TBA responding or non-responding phenotype.

15. The use of claim 14, wherein said nucleic acid microarray is an oligonucleotide microarray.

## Patentansprüche

1. Ein Verfahren zur *in vitro* Diagnose oder Prognose eines TNF-α Faktor blockierendes Mittel (TBA) respondierenden oder nicht-respondierenden Phänotyps, wobei das Verfahren umfasst:
a) die Bestimmung eines Expressionsprofils, in der Blutprobe eines unter einer entzündlichen Krankheit leidenden Subjekts, wobei dieses Expressionsprofil das folgendes umfasst oder ausfolgendem besteht:
(i) das Gen MAPK 14;
(ii) die Gene MAPK14 und S100A9; oder
(iii) die Gene MAPK14 und GNLY;
und gegebenenfalls ein oder mehrere Haushaltsgen(e)
b) den Vergleich des erhaltenen Expressionsprofils mit mindestens einem Referenzrespondierenden Expressionsprofil und mindestens einem Referenz nicht-respondierenden Expressionsprofil, unter Verwendung von statistischen Modellen oder Technologien für maschinelles Lernen
und
c) die Bestimmung eines respondierenden oder nicht-respondierenden Phänotyps vom dem genannten Vergleich.

2. Ein Verfahren zum Design einer TBA-Behandlung für einen unter einer entzündlichen Krankheit leidenden Subjekt, wobei dieses Verfahren umfasst:
a) die Bestimmung eines Expressionsprofils, in der Blutprobe des genannten Subjekts, das folgendes umfasst oder ausfolgendem besteht:
(i) das Gen MAPK 14;
(ii) die Gene MAPK14 und S100A9; oder
(iii) die Gene MAPK14 und GNLY;
und gegebenenfalls ein oder mehrere Haushaltsgen(e)
b) den Vergleich des erhaltenen Expressionsprofils mit mindestens einem Referenz respondierenden Expressionsprofil und mindestens einem Referenz nicht-respondierenden Expressionsprofil, unter Verwendung von statistischen Modellen oder Technologien für maschinelles Lernen
c) die Bestimmung eines respondierenden oder nicht-respondierenden Phänotyps des genannten Subjekts von dem genannten Vergleich, und
d) das Design einer Dosis für TBA-Behandlung gemäss des genannten identifizierten respondierenden oder nicht-respondierenden Phänotyps.

3. Ein Verfahren nach Anspruch 1 oder Anspruch 2, in welchem das Expressionsprofil der Phase (a) MAPK14 und GNLY umfasst oder aus MAPK14 und GNLY besteht.

4. Ein Verfahren nach einem der Ansprüche 1 bis 3, in welchem das Expressionsprofil durch die Quantifizierung von Nucleinsäuretranskripten der genannten Gene bestimmt wird.

5. Ein Verfahren nach Anspruch 4, in welchem das Expressionsprofil durch die quantitative Polymerase-Kettenreaktion (quantitative PCR) oder dank eines Oligonukleotiden- Mikroarrays bestimmt wird.

6. Ein Verfahren nach einem der Ansprüche 1 bis 3, in welchem das Expressionsprofil unter Verwendung von einem genomischen Mikroarray oder einem Protein-Chip bestimmt wird.

7. Ein Verfahren nach einem der Ansprüche 1 bis 6, in welchem das Expressionsprofil der Phase (a), 14 oder 22 Wochen nach Anfang der CyTD-Behandlung bestimmt wird.

8. Ein Verfahren nach einem der Ansprüche 1 bis 7, in welchem das genannte TBA aus einer Gruppe bestehend aus Etanercept (EnbreI^{R}), Infliximab (Remicade^{R}), Adalimumab (Humira^{R}), und Certolizumab Pegol (Cimzia^{R}), ausgewählt wird.

9. Ein Verfahren nach einem der Ansprüche 1 bis 8, in welchem die entzündliche Krankheit aus einer Gruppe ausgewählt wird, die aus folgende Pathologien besteht: rheumatoide Arthritis (RA), Morbus Crohn, Spondylitis ankylosans, psoriatrische Arthritis, Schuppenflechte Psoriasis (Psoriasis vulgaris), Colitis ulcerosa, Vaskulitis, Wegener-Granulomatose, Sarkoidose, Still-Syndrom des Erwachsenen, Polymyositis/Dermatomyositis und systemische Lupus erythematodes (SLE).

10. Ein Verfahren nach Anspruch 9, in welchem die entzündliche Krankheit rheumatoide Arthritis ist.

11. Ein Verfahren nach Anspruch 10 das weiter die Bestimmung von mindestens einem zusätzlichen Parameter umfasst, wobei dieser genannte Parameter durch einen Test bestimmt wird, der zwischen dem ANA-Test (Antinukleare Antikörper), dem CRP-Test (C-reaktive Protein), dem Anti-CCP-Test (zyklisches zitrulliniertes Peptid Antikörper), und dem Rheumatoid Faktor-Test ausgewählt wird.

12. Verwendung von einem Kit, das mindestens ein Reagenzien für die Bestimmung von einem Expressionsprofil umfasst, wobei dieses Expressionsprofil folgende Gene umfasst oder aus folgenden Genen besteht:
(i) das Gen MAPK 14;
(ii) die Gene MAPK14 und S100A9; oder
(iii) die Gene MAPK14 und GNLY;
und gegebenenfalls ein oder mehrere Haushaltsgen(e)
zur *in vitro* Diagnose eines TBA respondierenden oder nicht-respondierenden Phänotyps.

13. Verwendung nach Anspruch 12, in welchem das genannte Kit nebenbei mindestens ein Reagenzien zur Bestimmung von mindestens einem zusätzlichen Parameter enthält, wobei das genannte Reagenzien aus dem ANA-Test (Antinukleare Antikörper), dem CRP-Test (C-reaktive Protein), dem Anti-CCP-Test (zyklisches zitrulliniertes Peptid Antikörper), und dem rheumatoiden Faktor-Test ausgewählt wird.

14. Verwendung vom einem Nukleinsäuren-Mikroarray, das spezifische Nukleinsäuren für folgendes umfasst:
(i) das Gen MAPK 14;
(ii) die Gene MAPK14 und S100A9; oder
(iii) die Gene MAPK14 und GNLY;
und gegebenenfalls ein oder mehrere Haushaltsgen(e)
zur *in vitro* Diagnose eines TBA respondierenden oder nicht-respondierenden Phänotyps.

15. Verwendung nach Anspruch 14, in welchem das genannte Nukleinsäuren-Mikroarray ein Oligonukleotiden-Mikroarray ist.

## Revendications

1. Un procédé pour diagnostiquer ou pronostiquer *in vitro* le phénotype répondeur ou non-répondeur à un traitement avec un agent bloquant le facteur TNFα (TBA), comprenant :
a. la détermination à partir d'un échantillon de sang d'un sujet souffrant d'une maladie inflammatoire, du profil d'expression de gènes consistant en ou comprenant :
i. le gène MAPK14 ;
ii. les gènes MAPK14 et S100A9 ; ou
iii. les gènes MAPK14 et GNLY ;
et optionnellement un ou plusieurs gène(s) domestique(s)
b. la comparaison du profil d'expression obtenu avec au moins un profil d'expression répondeur de référence et au moins un profil d'expression non-répondeur de référence au moyen de modèles statistiques ou de technologies d'apprentissage machine, et
c. la détermination du phénotype répondeur ou non-répondeur de ladite comparaison.

2. Un procédé pour le design d'un traitement avec un agent TBA pour un sujet souffrant d'une maladie inflammatoire, ledit procédé comprenant:
a. la détermination à partir d'un échantillon de sang dudit sujet, du profil d'expression de gènes consistant en ou comprenant :
i. le gène MAPK14 ;
ii. les gènes MAPK14 et S100A9 ; ou
iii. les gènes MAPK14 et GNLY ;
et optionnellement un ou plusieurs gène(s) domestique(s)
b. la comparaison du profil d'expression obtenu avec au moins un profil d'expression répondeur de référence et au moins un profil d'expression non-répondeur de référence au moyen de modèles statistiques ou de technologies d'apprentissage machine,
c. la détermination du phénotype répondeur ou non-répondeur dudit sujet à partir de ladite comparaison, et
d. l'élaboration d'une dose d'un traitement avec un agent TBA en fonction du phénotype répondeur ou non-répondeur identifié.

3. Le procédé selon la revendication 1 ou 2, dans lequel le profil d'expression de l'étape (a) consiste en ou comprend les gènes MAPK14 et GNLY.

4. Le procédé selon l'une quelconque des revendications 1 à 3, dans lequel le profil d'expression est déterminé par la mesure de la quantité des transcripts d'acides nucléiques desdits gènes.

5. Le procédé selon la revendication 4, dans lequel le profil d'expression est déterminé par PCR quantitative ou une micropuce à oligonucléotides.

6. Le procédé selon l'une quelconque des revendications 1 à 3, dans lequel le profil d'expression est déterminé par micropuce génomique ou micropuce protéique.

7. Le procédé selon l'une quelconque des revendications 1 à 6, dans lequel le profil d'expression de l'étape (a) est déterminé 14 ou 22 semaines après le début du traitement CyTD.

8. Le procédé selon l'une quelconque des revendications 1 à 7, dans lequel l'agent TBA est sélectionné dans le groupe consistant en Etanercept (Enbrel^{R}), Infliximab (Remicade^{R}), Adalimumab (Humira^{R}), et Certolizumab pegol (Cimzia^{R}).

9. Le procédé selon l'une quelconque des revendications 1 à 8, dans lequel la maladie inflammatoire est sélectionnée dans un groupe consistant en la polyarthrite rhumatoïde (RA), la maladie de Crohn, la spondylarthrite ankylosante, le rhumatisme psoriasique, le psoriasis en plaques, la colite ulcéreuse, les vascularites, la granulomatose de Wegener, la sarcoïdose, la maladie de Still de l'adulte, la polymyosite/dermatopolymyosite et le Lupus érythémateux disséminé (SLE).

10. Le procédé de la revendication 9, dans lequel la maladie inflammatoire est la polyarthrite rhumatoïde.

11. Le procédé selon la revendication 10, comprenant en plus la détermination d'au moins un paramètre supplémentaire, ledit paramètre supplémentaire étant déterminé par un test sélectionné parmi le test d'anticorps anti-nucléaire (ANA test), le test de la protéine C-réactive (CRP test), le test d'anticorps anti-peptides citrullinés cycliques (CCP test) et le test du facteur rhumatoïde.

12. L'utilisation d'un kit comprenant au moins un réactif pour la détermination d'un profil d'expression consistant en ou comprenant:
i. le gène MAPK14 ;
ii. les gènes MAPK14 et S100A9 ; ou
iii. les gènes MAPK14 et GNLY ;
et optionnellement un ou plusieurs gène(s) domestique(s)
pour le diagnostic *in vitro* d'un phénotype répondeur ou non-répondeur à un agent TBA.

13. L'utilisation selon la revendication 12, dans laquelle le kit comprend en outre au moins un réactif pour la détermination d'au moins un paramètre supplémentaire, ledit réactif étant sélectionné parmi le test d'anticorps anti-nucléaire (ANA test), le test de la protéine C-réactive (CRP test), le test d'anticorps anti-peptides citrullinés cycliques (CCP test) et le test du facteur rhumatoïde.

14. L'utilisation d'une micropuce à acide nucléique, comprenant des acides nucléiques spécifiques pour :
i. le gène MAPK14 ;
ii. les gènes MAPK14 et S100A9 ; ou
iii. les gènes MAPK14 et GNLY
et optionnellement un ou plusieurs gène(s) domestique(s),
pour le diagnostic in vitro du phénotype répondeur ou non-répondeur à un agent TBA.

15. L'utilisation selon la revendication 14, dans laquelle la micropuce à acide nucléique est une micropuce à oligonucléotides.
